# EUROPEAN PATENT SPECIFICATION

(11) **EP 2 069 354 B1**
(45) Date of publication and mention of the grant of the patent: **02.11.2011**
(21) Application number: 07814272.6
(22) Date of filing: 20.08.2007
(51) Int. Cl.: C07D 491/048, A61K 31/4355, A61K 31/5025, A61K 31/519, A61P 35/00, A61P 29/00

(54) **Aza-benzofuranyl compounds and methods of use**
Aza-Benzofuranylverbindungen und Anwendungsverfahren dafür
Composés aza-benzofuranyle et leurs procédés d'utilisation

(30) Priority: 21.08.2006 US 839161 P; 22.12.2006 US 871591 P; 11.05.2007 US 917623 P; 18.06.2007 US 944741 P
(43) Date of publication of application: 17.06.2009
(73) Proprietor: Genentech, Inc., South San Francisco CA 94080-4990 (US)
(72) Inventor: PRICE, Stephen, Harlow Essex CM19 5TR (GB); WILLIAMS, Karen, Harlow Essex CM19 5TR (GB); SAVY, Pierre Pascal, Harlow Essex CM19 5TR (GB); DYKE, Hazel Joan, Harlow Essex CM19 5TR (GB); MONTANA, John Gary, Harlow Essex CM19 5TR (GB); STANLEY, Mark S., Pacifica, California 94044 (US); BAO, Liang, Foster City, California 94404 (US)
(74) Representative: Kiddle, Simon John
(86) International application number: PCT/US2007/076344
(87) International publication number: WO 2008/024725

(56) References cited:
- EP-A- 1 582 521
- WO-A-2005/047292
- WO-A-2005/061476
- WO-A-2007/027855
- US-B1- 6 653 346

## Description

FIELD OF THE INVENTION

The invention relates to azabenzofuranyl compounds with anti-cancer and/or anti-inflammatory activity and more specifically to azabenzofuranyl compounds which inhibit MEK kinase activity, The invention also relates to the compounds for use in *in vitro, in situ,* and *in vivo* methods of diagnosis or treatment of mammalian cells, or associated pathological conditions.

BACKGROUND OF THE INVENTION

In the quest to understand how Ras transmits extracellular growth signals, the MAP (mitogen-activated protein) kinase (MAPK) pathway has emerged as the crucial route between membrane-bound Ras and the nucleus. The MAPK pathway encompasses a cascade of phosphorylation events involving three key kinases, namely Raf, MEK (MAP kinase kinase) and ERK (MAP kinase). Active GTP-bound Ras results in the activation and indirect phosphorylation of Raf kinase. Raf then phosphorylates MEKI and 2 on two serine residues (S218 and S222 for MEK 1 and S222 and S226 for MEK2) (Ahn et al., Methods in Enzymology 2001, 332, 417-431). Activated MEK then phosphorylates its only known substrates, the MAP kinases, ERK1 and 2. ERK phosphorylation by MEK occurs on Y204 and T202 for ERK1 and Y 185 and T 183 for ERK2 (Ahn ct al., Methods in Enzymology 2001, 332, 417-431). Phosphorylated ERK dimerizes and then translocates to the nucleus where it accumulates (Khokhlatchev et al., Gell 1998, 93, 605-615). In the nucleus, ERK is involved in several important cellular functions, including but not limited to nuclear transport, signal transduction, DNA repair, nucleosome assembly and translocation, and mRNA processing and translation (Ahn et al., Molecular Cell 2000, 6,1343-1354). Overall, treatment of cells with growth factors leads to the activation of ERK1 and 2 which results in proliferation and, in some cases, differentiation (Lewis et al., Adv. Cancer Res. 1998, 74, 49-139).

There has been strong evidence that genetic mutations and/or overexpression of protein kinases involved in the MAP kinase pathway lead to uncontrolled cell proliferation and, eventually, tumor formation, in proliferative diseases. For example, some cancers contain mutations which result in the continuous activation of this pathway due to continuous production of growth factors. Other mutations can lead to defects in the deactivation of the activated GTP-bound Ras complex, again resulting in activation of the MAP kinase pathway. Mutated, oncogenic forms ofRas are found in 50% of colon and >90% pancreatic cancers as well as many others types of cancers (Kohl et al., Science 1993, 260, 1834-1837). Recently, bRaf mutations have been identified in more than 60% of malignant melanoma (Davies, H. et al., Nature 2002, 417, 949-954). These mutations in bRaf result in a constitutively active MAP kinase cascade. Studies of primary tumor samples and cell lines have also shown constitutive or overactivation of the MAP kinase pathway in cancers of pancreas, colon, lung, ovary and kidney (Hoshino, R. et al., Oncogene 1999, 18, 813-822).

MEK has emerged as an attractive therapeutic target in the MAP kinase cascade pathway. MEK, downstream of Ras and Raf, is highly specific for the phosphorylation of MAP kinase; in fact, the only known substrates for MEK phosphorylation are the MAP kinases, ERK 1 and 2. Inhibition of MEK has been shown to have potential therapeutic benefit in several studies. For example, small molecule MEK inhibitors have been shown to inhibit human tumor growth in nude mouse xenografts, (Sebolt-Leopold et al., Nature-Medicine 1999, 5 (7), 810-816); Trachet et al., AACR Apr. 6-10, 2002, Poster #5426; Tecle, H. IBC 2.sup.nd International Conference of Protein Kinases, Sep. 9-10, 2002), block static allodynia in animals (WO 01/05390 published Jan. 25, 2001) and inhibit growth of acute myeloid leukemia cells (Milella et al., J Clin Invest 2001, 108 (6), 851-859).

Several small molecule MEK inhibitors have also been discussed in, for example, WO02/06213, WO 03/077855 and WO03/077914. There still exists a need for new MEK inhibitors as effective and safe therapeutics for treating a variety of proliferative disease states, such as conditions related to the hyperactivity of MEK, as well as diseases modulated by the MEK cascade.

WO 2005/061476 A2 describes aroylfuranes and aroylthipohenes and uses of these compounds in treatment of neoplastic and autoimmune diseases.

US 6 653 346 describes benzofuranyl derivatives which are useful in the treatment of ischemic or inflammatory conditions and in the manufacture of pharmaceutical and cosmetic formulations for the treatment of such conditions.

EP 1 582 521 A describes aza-benzofuranyl derivatives which are useful in treatment of diseases caused by thrombus or embolus,

WO 2005/047292 A describes thienopyrimidine derivatives as inhibitors of tyrosinkinases especially TIE-2, and Raf-kinases useful in the treatment of tumors.

WO 2007/027855 A describes aza-benzofuranyl compounds that are useful for inhibiting Raf-kinases and for treating disorders mediated thereby.

SUMMARY OF THE INVENTION

The invention relates generally to aza-benzofuran compounds of Formula I (and/or solvates and salts thereof) with anti-cancer and/or anti-inflammatory activity, and more specifically with MEK kinase inhibitory activity. Certain hyperproliferative and inflammatory disorders are characterized by the modulation of MEK kinase function, for example by mutations or overexpression of the proteins. Accordingly, the compounds of the invention and compositions thereof are useful in the treatment of hyperproliferative disorders such as cancer and/or inflammatory diseases such as rheumatoid arthritis. 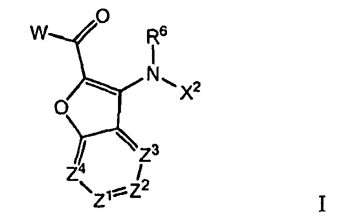 wherein:
Z¹ is CR¹;
Z² is CR² or N;
Z³ is CR³ or N;
Z⁴ is CR⁴ or N;
where one or two of Z², Z³, and Z⁴ are N;
R¹, R², R³ and R⁴ are independently selected from H, halo, CN, CF₃, -OCF₃, -NO₂, -(CR¹⁴R¹⁵)ₙC(=Y)R¹¹, -(CR¹⁴R¹⁵)ₙC(=Y)OR¹¹, -(CR¹⁴R¹⁵)ₙC(=Y)NR¹¹R¹², -(CR¹⁴R¹⁵)ₙNR¹¹R¹², -(CR¹⁴R¹⁵)ₙOR¹¹, -(CR¹⁴R¹⁵)ₙNR¹²C(=Y)R¹¹, carbocyclyl, heterocyclyl, aryl, and heteroaryl; 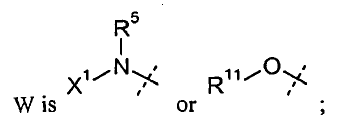
R⁵ and R⁶ are independently selected from H or C₁-C₁₂ alkyl;
X¹ is selected from R¹¹, -OR¹¹, -S(O)R¹¹, and -S(O)₂R¹¹; when X¹ is R¹¹ or -OR¹¹, R¹¹ or -OR¹¹ of X¹ and -R⁵ are optionally taken together with the nitrogen atom to which they are attached to form a 4-7 membered saturated or unsaturated ring having 0-2 additional heteroatoms selected from O, S and N, wherein said ring is optionally substituted with one or more groups selected from halo, CN, CF₃, -OCF₃, -NO₂, oxo, -(CR¹⁹R²⁰)ₙNR¹⁶R¹⁷, -(CR¹⁹R²⁰)ₙOR¹⁶, -(CR¹⁹R²⁰)ₙS(O)R¹⁶, -(CR¹⁹R²⁰)ₙS(O)₂R¹⁶, and R²¹;
X² is aryl
R¹¹, R¹² and R¹³ are independently H, C₁-C₁₂ alkyl, C₂-C₈ alkenyl, C₂-C₈ alkynyl, carbocyclyl, heterocyclyl, aryl, or heteroaryl,
R¹⁴ and R¹⁵ are independently selected from H, C₁-C₁₂ alkyl
m and n are independently selected from 0, 1, 2, 3, 4, 5, or 6;
Y is independently O, NR¹¹, or S;
wherein each said alkyl, alkenyl, alkynyl, carbocyclyl, heterocyclyl, aryl and heteroaryl of R¹, R², R³, R⁴, R⁵, R⁶, X¹, X² R¹¹, R¹², R¹³, R¹⁴, and R¹⁵ is independently optionally substituted with one or more groups independently selected from halo, CN, CF₃, -OCF₃, -NO₂, oxo, -Si(C₁-C₆ alkyl), -(CR¹⁹R²⁰)ₙC(=Y')R¹⁶, -(CR¹⁹R²⁰)ₙ C(=Y')OR¹⁶, -(CR¹⁹R²⁰)ₙC(=Y')NR¹⁶R¹⁷, -(CR¹⁹R²⁰)ₙNR¹⁶R¹⁷, -(CR¹⁹R²⁰)ₙOR¹⁶, -(CR¹⁹R²⁰)ₙ-SR¹⁶, -(CR¹⁹R²⁰)ₙ NR¹⁶C(=Y')R¹⁷, -(CR¹⁹R²⁰)ₙ NR¹⁶C(=Y')OR¹⁷, -(CR¹⁹R²⁰)ₙ NR¹⁸C(=Y')NR¹⁶R¹⁷, -(CR¹⁹R²⁰)ₙNR¹⁷SO₂R¹⁶, -(CR¹⁹R²⁰)ₙOC(=Y')R¹⁶ -(CR¹⁹R²⁰)ₙOC(=Y')OR¹⁶, -(CR¹⁹R²⁰)nOC(=Y')NR¹⁶R¹⁷, and R²¹;
each R¹⁶, R¹⁷ and R¹⁸ is independently H, C₁-C₁₂ alkyl, C₂-C₈ alkenyl, C₂-C₈ alkynyl, carbocyclyl, heterocyclyl, aryl, or heteroaryl, wherein said alkyl, alkenyl, alkynyl,carbocyclyl, heterocyclyl, aryl, or heteroaryl is optionally substituted with one or more groups selected from halo, CN, -OCF₃, CF₃, -NO₂, C₁-C₆ alkyl, -OH, -O(C₁-C₆ alkyl), -NH₂, -NH(C₁-C₆ alkyl), -N(C₁-C₆ alkyl)₂, -CO₂H, -CO₂(C₁-C₆ alkyl),
R¹⁹ and R²⁰ are independently selected from H, C₁-C₁₂ alkyl;
R²¹ is C₁-C₁₂ alkyl, C₂-C₈ alkenyl, C₂-C₈ alkenyl, carbocyclyl, heterocyclyl, aryl, or heteroaryl, wherein each member of R²¹ is optionally substituted with one or more groups selected from halo, oxo, CN, -OCF₃, CF₃, -NO₂, C₁-C₆ alkyl, -OH, -O(C₁-C₆ alkyl), -NH₂, -NH(C₁-C₆ alkyl), -N(C₁-C₆ alkyl)₂, -CO₂H, -CO₂(C₁-C₆ alkyl),
each Y' is independently O, NR²², or S; and
R²² is H or C₁-C₁₂ alkyl.

The present invention includes a composition (e.g., a pharmaceutical composition) comprising a compound of Formula I (and/or solvates, hydrates and/or salts thereof) and a carrier (a pharmaceutically acceptable carrier). The present invention also includes a composition (e.g., a pharmaceutical composition) comprising a compound of Formula I (and/or solvates, hydrates and/or salts thereof) and a carrier (a pharmaceutically acceptable carrier), further comprising a second chemotherapeutic and/or a second anti-inflammatory agent. The present compositions are useful for inhibiting abnormal cell growth or treating a hyperproliferative disorder in a mammal (e.g., human). The present compositions are also useful for treating inflammatory diseases in a mammal (e.g., human).

The present invention is useful in a method of inhibiting abnormal cell growth or treating a hyperproliferative disorder in a mammal (e.g., human) comprising administering to said mammal a therapeutically effective amount of a compound of Formula I (and/or solvates and salts thereof) or a composition thereof, alone or in combination with a second chemotherapeutic agent.

The present invention is also useful in a method of treating an inflammatory disease in a mammal (e.g., human) comprising administering to said mammal a therapeutically effective amount of a compound of Formula I (and/or solvates and salts thereof) or a composition thereof, alone or in combination with a second anti-inflammatory agent.

The present invention includes the present compounds for use in *in vitro, in situ,* and *in vivo* methods of diagnosis or treatment of mammalian cells, organisms, or associated pathological conditions,

DETAILED DESCRIPTION OF EXEMPLARY EMBODIMENTS

Reference will now be made in detail to certain embodiments of the invention, examples of which are illustrated in the accompanying structures and formulas. White the invention will be described in conjunction with the enumerated embodiments, it will be understood that they are not intended to limit the invention to those embodiments. On the contrary, the invention is intended to cover all alternatives, modifications, and equivalents included within the scope of the claims. In the event that one or more of the incorporated literature, patents, and similar materials differs from or contradicts this application, including but not limited to defined terms, term usage, described techniques, or the like, this application controls.

DEFINITIONS

The term "alkyl" as used herein refers to a saturated linear or branched-chain monovalent hydrocarbon radical of one to twelve carbon atoms. Examples of alkyl groups include, but are not limited to, methyl (Me, -CH₃), ethyl (Et, -CH₂CH₃), 1-propyl (n-Pr, n-propyl, -CH₂CH₂CH₃), 2-propyl (i-Pr, i-propyl, -CH(CH₃)₂), 1-butyl (n-Bu, n-butyl, - CH₂CH₂CH₂CH₃), 2-mothyl-l-propyl (i-Bu, i-butyl, -CH₂CH(CH;}y), 2-butyl (s-Bu, s-butyl, - CH(CH₃)CH₂CH₃), 2-methyl-2-propyl (t-Bu, t-butyl, -C(CH₃)₃), 1-pentyl (n-pentyl, - CH₂CH₂CH₂CH₂CH₃), 2-pentyl (-CH(CH₃)CH₂CH₂CH₃), 3-pentyl (-CH(CH₂CH₃)₂), 2-methyl-2-butyl (-C(CH₃)₂CH_{z}CH₃), 3-methyl-2-butyl (-CH(CH₃)CHCCH₃)₂), 3-methyl-1-butyl (-CH₂CH₂CH(CH₃)₂), 2-methyl-1-butyl (-CH₂CH(CH₃)CH₂CH₃), 1-hexyl (-CH₂CH₂CH₂CH₂CH₂CH₃), 2-hexyl (-CH(CH₃)CH₂CH₂CH₂CH₃), 3-hcxyl (-CH(CH₂CH₃)(CH₂CH₂CH₃)), 2-methyl-2-pentyl (-C(CH₃)CH₂CH₂CH₃), 3-methyl-2-pentyl (-CH(CH₃)CH(CH₃)CH₂CH₃), 4-methyl-2-pentyl (-CH(CH₃)CH₂CH(CH₃)₂), 3-methyl-3-pentyl (-C(CH₃)(CH₂CH₃)₂), 2-mothyl-3-pentyl (-CH(CH₂CH₃)CH(CH₃)₂), 2,3-dimethyl-2-butyl (-C(CH₃)₂CH(CH₃)₂), 3,3-dimethyl-2-butyl (-CH(CH₃)C(CH₃)₃, 1-heptyl, 1-octyl, and the like.

The term "alkenyl" refers to linear or branched-chain monovalent hydrocarbon radical of two to twelve carbon atoms with at least one site of unsaturation, i,e., a carbon-carbon, sp² double bond, wherein the alkenyl radical includes radicals having "cis" and "trans" orientations, or alternatively, "E" and "Z" orientations. Examples include, but are not limited to, ethylenyl or vinyl (-CH=CH₂), allyl (-CH₂CH=CH₂), and the like.

The term "alkynyl" refers to a linear or branched monovalent hydrocarbon radical of two to twelve carbon atoms with at least one site of unsaturation, i.e., a carbon-carbon, sp triple bond. Examples include, but are not limited to, ethynyl (-C=CH), propynyl (propargyl, -CH₂C≡CH), and the like.

The terms "carbocycle", "carbocyclyl", "carbocyclic ring" and "cycloalkyl" refer to a monovalent non-aromatic, saturated or partially unsaturated ring having 3 to 12 carbon atoms as a monocyclic ring or 7 to 12 carbon atoms as a bicyclic ring. Bicyclic carbocycles having 7 to 12 atoms can be arranged, for example, as a bicyclo [4,5], [5,5], [5,6] or [6,6] system, and bicyclic carbocycles having 9 or 10 ring atoms can be arranged as a bicyclo [5,6] or [6,6] system, or as bridged systems such as bicyclo[2.2.1]heptane, bicyclo[2.2.2]octane and bicyclo[3.2.2]nonane. Examples of monocyclic carbocycles include, but are not limited to, cyclopropyl, cyclobutyl, cyclopentyl, 1-cyclopent-1-enyl, 1-cyclopent-2-enyl, 1-cyclopent-3-enyl, cyclohexyl, 1-cyclohex-l-enyl, 1-cyclohex-2-enyl, 1-cyclohex-3-enyl, cyclohexadienyl, cycloheptyl, cyclooctyl, cyclononyl, cyclodecyl, cycloundecyl, cyclododecyl, and the like.

"Aryl" means a monovalent aromatic hydrocarbon radical of 6-18 carbon atoms derived by the removal of one hydrogen atom from a single carbon atom of a parent aromatic ring system. Some aryl groups are represented in the exemplary structures as "Ar". Aryl includes bicyclic radicals comprising an aromatic ring fused to a saturated, partially unsaturated ring, or aromatic carbocyclic or heterocyclic ring. Typical aryl groups include, but are not limited to, radicals derived from benzene (phenyl), substituted benzenes, naphthalene, anthracene, indenyl, indanyl, 1,2-dihydronapthalene, 1,2,3,4-tetrahydronapthyl, and the like.

The terms "heterocycle," "heterocyclyl" and "heterocyclic ring" are used interchangeably herein and refer to a saturated or a partially unsaturated (i.e., having one or more double and/or triple bonds within the ring) carbocyclic radical of 3 to 18 ring atoms in which at least one ring atom is a heteroatom selected from nitrogen, oxygen and sulfur, the remaining ring atoms being C, where one or more ring atoms is optionally substituted independently with one or more substituents described below. A heterocycle may be a monocycle having 3 to 7 ring members (2 to 6 carbon atoms and 1 to 4 heteroatoms selected from N, O, P, and S) or a bicycle having 7 to 10 ring members (4 to 9 carbon atoms and 1 to 6 heteroatoms selected from N, O, P, and S), for example: a bicyclo [4,5], [5,5], [5,6], or [6,6] system. Heterocycles are described in Paquette, Leo A.; "Principles of Modern Heterocyclic Chemistry" (W.A. Benjamin, New York, 1968), particularly Chapters 1, 3, 4, 6, 7, and 9; "The Chemistry of Heterocyclic Compounds, A series of Monographs" (John Wiley & Sons, New York, 1950 to present), in particular Volumes 13, 14, 16, 19, and 28; and J. Am. Chem. Soc. (1960) 82:5566. "Heterocyclyl" also includes radicals where heterocycle radicals are fused with a saturated, partially unsaturated ring, or aromatic carbocyclic or heterocyclic ring. Examples of heterocyclic rings include, but are not limited to, pyrrolidinyl, tetrahydrofuranyl, dihydrofuranyl, tetrahydrothienyl, tetrahydropyranyl, dihydropyranyl, tetrahydrothiopyranyl, piperidino, morpholino, thiomorpholino, thioxanyl, piperazinyl, homopiperazinyl, azetidinyl, oxetanyl, thietanyl, homopiperidinyl, oxepanyl, thiepanyl, oxazepinyl, diazepinyl, thiazepinyl, 2-pyrrolinyl, 3-pyrrolinyl, indolinyl, 2H-pyranyl, 4H-pyranyl, dioxanyl, 1,3-dioxolanyl, pyrazolinyl, dithianyl, dithiolanyl, dihydropyranyl, dihydrothienyl, dihydrofuranyl, pyrazolidinylimidazolinyl, imidazolidinyl, 3-azabicyco[3.1.0]hexanyl, 3-azabicyclo[4.1.0]heptanyl, and azabicyclo[2.2.2]hexanyl. Spiro moieties are also included within the scope of this definition. Examples of a heterocyclic group wherein ring atoms are substituted with oxo (=O) moieties are pyrimidinonyl and 1,1-dioxo-thiomorpholinyl.

The term "heteroaryl" refers to a monovalent aromatic radical of 5- or 6-membered rings, and includes fused ring systems (at least one of which is aromatic) of 5-18 atoms, containing one or more heteroatoms independently selected from nitrogen, oxygen, and sulfur. Examples of heteroaryl groups are pyridinyl (including, for example, 2-hydroxypyridinyl), imidazolyl, imidazopyridinyl, pyrimidinyl (including, for example, 4-hydroxypyrimidinyl), pyrazolyl, triazolyl, pyrazinyl, tetrazolyl, furyl, thienyl, isoxazolyl, thiazolyl, oxazolyl, isothiazolyl, pyrrolyl, quinolinyl, isoquinolinyl, indolyl, benzimidazolyl, benzofuranyl, cinnolinyl, indazolyl, indolizinyl, phthalazinyl, pyridazinyl, triazinyl, isoindolyl, pteridinyl, purinyl, oxadiazolyl, triazolyl, thiadiazolyl, furazanyl, benzofurazanyl, benzothiophenyl, benzothiazolyl, benzoxazolyl, quinazolinyl, quinoxalinyl, naphthyridinyl, and furopyridinyl.

The heterocycle or heteroaryl groups may be carbon (carbon-linked) or nitrogen (nitrogen-linked) attached where such is possible. By way of example and not limitation, carbon bonded heterocycles or heteroaryls are bonded at position 2, 3, 4, 5, or 6 of a pyridine, position 3, 4, 5, or 6 of a pyridazine, position 2, 4, 5, or 6 of a pyrimidine, position 2, 3, 5, or 6 of a pyrazine, position 2, 3, 4, or 5 of a furan, tetrahydrofuran, thiofuran, thiophene, pyrrole or tetrahydropyrrole, position 2, 4, or 5 of an oxazole, imidazole or thiazole, position 3, 4, or 5 of an isoxazole, pyrazole, or isothiazole, position 2 or 3 of an aziridine, position 2, 3, or 4 of an azetidine, position 2, 3, 4, 5, 6, 7, or 8 of a quinoline or position 1, 3, 4, 5, 6, 7, or 8 of an isoquinoline.

By way of example and not limitation, nitrogen bonded heterocycles or heteroaryls are bonded at position 1 of an aziridine, azetidine, pyrrole, pyrrolidine, 2-pyrroline, 3-pyrroline, imidazole, imidazolidine, 2-imidazoline, 3-imidazoline, pyrazole, pyrazoline, 2-pyrazoline, 3-pyrazoline, piperidine, piperazine, indole, indoline, 1H-indazole, position 2 of a isoindole, or isoindoline, position 4 of a morpholine, and position 9 of a carbazole, or β-carboline.

The term "halo" refers to F, C1, Br or I. The heteroatoms present in heteroaryl or heterocyclcyl include the oxidized forms such as N⁺→O⁻, S(O) and S(O)₂.

The terms "treat" and "treatment" refer to both therapeutic treatment and prophylactic or preventative measures, wherein the object is to prevent or slow down (lessen) an undesired physiological change or disorder, such as the development or spread of cancer. For purposes of this invention, beneficial or desired clinical results include, but are not limited to, alleviation of symptoms, diminishment of extent of disease, stabilized (i.e., not worsening) state of disease, delay or slowing of disease progression, amelioration or palliation of the disease state, and remission (whether partial or total), whether detectable or undetectable. "Treatment" can also mean prolonging survival as compared to expected survival if not receiving treatment. Those in need of treatment include those already with the condition or disorder as well as those prone to have the condition or disorder or those in which the condition or disorder is to be prevented.

The phrase "therapeutically effective amount" means an amount of a compound of the present invention that (i) treats or prevents the particular disease, condition, or disorder, (ii) attenuates, ameliorates, or eliminates one or more symptoms of the particular disease, condition, or disorder, or (iii) prevents or delays the onset of one or more symptoms of the particular disease, condition, or disorder described herein. In the case of cancer, the therapeutically effective amount of the drug may reduce the number of cancer cells; reduce the tumor size; inhibit (i.e., slow to some extent and preferably stop) cancer cell infiltration into peripheral organs; inhibit (i.e., slow to some extent and preferably stop) tumor metastasis; inhibit, to some extent, tumor growth; and/or relieve to some extent one or more of the symptoms associated with the cancer. To the extent the drug may prevent growth and/or kill existing cancer cells, it may be cytostatic and/or cytotoxic. For cancer therapy, efficacy can be measured, for example, by assessing the time to disease progression (TTP) and/or determining the response rate (RR).

The terms "abnormal cell growth" and "hyperproliferative disorder" are used interchangeably in this application. "Abnormal cell growth", as used herein, unless otherwise indicated, refers to cell growth that is independent of normal regulatory mechanisms (e.g., loss of contact inhibition). This includes, for example, the abnormal growth of: (1) tumor cells (tumors) that proliferate by expressing a mutated tyrosine kinase or overexpression of a receptor tyrosine kinase; (2) benign and malignant cells of other proliferative diseases in which aberrant tyrosine kinase activation occurs; (3) any tumors that proliferate by receptor tyrosine kinases; (4) any tumors that proliferate by aberrant serine/threonine kinase activation; and (5) benign and malignant cells of other proliferative diseases in which aberrant serine/threonine kinase activation occurs.

The terms "cancer" and "cancerous" refer to or describe the physiological condition in mammals that is typically characterized by unregulated cell growth. A "tumor" comprises one or more cancerous cells. Examples of cancer include, but are not limited to, carcinoma, lymphoma, blastoma, sarcoma, and leukemia or lymphoid malignancies. More particular examples of such cancers include squamous cell cancer (e.g., epithelial squamous cell cancer), lung cancer including small- cell lung cancer, non-small cell lung cancer ("NSCLC"), adenocarcinoma of the lung and squamous carcinoma of the lung, cancer of the peritoneum, hepatocellular cancer, gastric or stomach cancer including gastrointestinal cancer, pancreatic cancer, glioblastoma, cervical cancer, ovarian cancer, liver cancer, bladder cancer, hepatoma, breast cancer, colon cancer, rectal cancer, colorectal cancer, endometrial or uterine carcinoma, salivary gland carcinoma, kidney or renal cancer, prostate cancer, vulval cancer, thyroid cancer, hepatic carcinoma, anal carcinoma, penile carcinoma, acute leukemia, as well as head/brain and neck cancer.

A "chemotherapeutic agent" is a chemical compound useful in the treatment of cancer. Examples of chemotherapeutic agents include Erlotinib (TARCEVA®, Genentech/OSI Pharm.), Bortezomib (VELCADE®, Millennium Pharm.), Fulvestrant (FASLODEX®, AstraZeneca), Sutent (SU11248, Pfizer), Letrozole (FEMARA®, Novartis), Imatinib mesylate (GLEEVEC®, Novartis), PTK787/ZK 222584 (Novartis), Oxaliplatin (Eloxatin®, Sanofi), 5-FU (5-fluorouracil), Leucovorin, Rapamycin (Sirolimus, RAPAMUNE®, Wyeth), Lapatinib (TYKERB®, GSK572016, Glaxo Smith Kline), Lonafarnib (SCH 66336), Sorafenib (BAY43-9006, Bayer Labs), and Gefitinib (IRESSA®, AstraZeneca), AG1478, AG1571 (SU 5271; Sugen), alkylating agents such as thiotepa and CYTOXAN® cyclosphosphamide; alkyl sulfonates such as busulfan, improsulfan and piposulfan; aziridines such as benzodopa, carboquone, meturedopa, and uredopa; ethylenimines and methylamelamines including altretamine, triethylenemelamine, triethylenephosphoramide, triethylenethiophosphoramide and trimethylomelamine; acetogenins (especially bullatacin and bullatacinone); a camptothecin (including the synthetic analog topotecan); bryostatin; callystatin; CC-1065 (including its adozelesin, carzelesin and bizelesin synthetic analogs); cryptophycins (particularly cryptophycin 1 and cryptophycin 8); dolastatin; duocarmycin (including the synthetic analogs, KW-2189 and CB1-TM1); eleutherobin; pancratistatin; a sarcodictyin; spongistatin; nitrogen mustards such as chlorambucil, chlornaphazine, chlorophosphamide, estramustine, ifosfamide, mechlorethamine, mechlorethamine oxide hydrochloride, melphalan, novembichin, phenesterine, prednimustine, trofosfamide, uracil mustard; nitrosureas such as carmustine, chlorozotocin, fotemustine, lomustine, nimustine, and ranimnustine; antibiotics such as the enediyne antibiotics (e.g., calicheamicin, especially calicheamicin gammalII and calicheamicin omegaI1 (Angew Chem. Intl. Ed. Engl. (1994) 33:183-186); dynemicin, including dynemicin A; bisphosphonates, such as clodronate; an esperamicin; as well as neocarzinostatin chromophore and related chromoprotein enediyne antibiotic chromophores), aclacinomysins, actinomycin, authramycin, azaserine, bleomycins, cactinomycin, carabicin, carminomycin, carzinophilin, chromomycinis, dactinomycin, daunorubicin, detorubicin, 6-diazo-5-oxo-L-norleucine, ADRIAMYCIN® (doxorubicin), morpholino-doxorubicin, cyanomorpholino-doxorubicin, 2-pyrrolino-doxorubicin and deoxydoxorubicin), epirubicin, esorubicin, idarubicin, marcellomycin, mitomycins such as mitomycin C, mycophenolic acid, nogalamycin, olivomycins, peplomycin, porfiromycin, puromycin, quelamycin, rodorubicin, streptonigrin, streptozocin, tubercidin, ubenimex, zinostatin, zorubicin; anti-metabolites such as methotrexate and 5-fluorouracil (5-FU); folic acid analogs such as denopterin, methotrexate, pteropterin, trimetrexate; purine analogs such as fludarabine, 6-mercaptopurine, thiamiprine, thioguanine; pyrimidine analogs such as ancitabine, azacitidine, 6-azauridine, carmofur, cytarabine, dideoxyuridine, doxifluridine, enocitabine, floxuridine; androgens such as calusterone, dromostanolone propionate, epitiostanol, mepitiostane, testolactone; anti-adrenals such as aminoglutethimide, mitotane, trilostane; folic acid replenisher such as frolinic acid; aceglatone; aldophosphamide glycoside; aminolevulinic acid; eniluracil; amsacrine; bestrabucil; bisantrene; edatraxate; defofamine; demecolcine; diaziquone; elfornithine; elliptinium acetate; an epothilone; etoglucid; gallium nitrate; hydroxyurea; lentinan; lonidainine; maytansinoids such as maytansine and ansamitocins; mitoguazone; mitoxantrone; mopidanmol; nitraerine; pentostatin; phenamet; pirarubicin; losoxantrone; podophyllinic acid; 2-ethylhydrazide; procarbazine; PSK® polysaccharide complex (JHS Natural Products, Eugene, OR); razoxane; rhizoxin; sizofiran; spirogermanium; tenuazonic acid; triaziquone; 2,2',2"-trichlorotriethylamine; trichothecenes (especially T-2 toxin, verracurin A, roridin A and anguidine); urethan; vindesine; dacarbazine; mannomustine; mitobronitol; mitolactol; pipobroman; gacytosine; arabinoside ("Ara-C"); cyclophosphamide; thiotepa; taxoids, e.g., TAXOL® (paclitaxel; Bristol-Myers Squibb Oncology, Princeton, N.J.), ABRAXANE™ (Cremophor-free), albumin-engineered nanoparticle formulations of paclitaxel (American Pharmaceutical Partners, Schaumberg, Illinois), and TAXOTERE® (doxetaxel; Rhône-Poulenc Rorer, Antony, France); chloranmbucil; GEMZAR® (gemcitabine); 6-thioguanine; mercaptopurine; methotrexate; platinum analogs such as cisplatin and carboplatin; vinblastine; etoposide (VP-16); ifosfamide; mitoxantrone; vincristine; NAVELBINE® (vinorelbine); novantrone; teniposide; edatrexate; daunomycin; aminopterin; capecitabine (XELODA®); ibandronate; CPT-11; topoisomerase inhibitor RFS 2000; difluoromethylornithine (DMFO); retinoids such as retinoic acid; and pharmaceutically acceptable salts, acids and derivatives of any of the above.

Also included in the definition of "chemotherapeutic agent" are: (i) anti-hormonal agents that act to regulate or inhibit hormone action on tumors such as antiestrogens and selective estrogen receptor modulators (SERMs), including, for example, tamoxifen (including NOLVADEX®; tamoxifen citrate), raloxifene, droloxifene, 4-hydroxytamoxifen, trioxifene, keoxifene, LY117018, onapristone, and FARESTON® (toremifine citrate); (ii) aromatase inhibitors that inhibit the enzyme aromatase, which regulates estrogen production in the adrenal glands, such as, for example, 4(5)-imidazoles, aminoglutethimide, MEGASE® (megestrol acetate), AROMASIN® (exemestane; Pfizer), formestanie, fadrozole, DIVISOR® (vorozole), FEMARA® (letrozole; Novartis), and ARIMIDEX® (anastrozole; AstraZeneca); (iii) anti-androgens such as flutamide, nilutamide, bicalutamide, leuprolide, and goserelin; as well as troxacitabine (a 1,3-dioxolane nucleoside cytosine analog); (iv) protein kinase inhibitors; (v) lipid kinase inhibitors; (vi) antisense oligonucleotides, particularly those which inhibit expression of genes in signaling pathways implicated in aberrant cell proliferation, such as, for example, PKC-alpha, Ralf and H-Ras; (vii) ribozymes such as VEGF expression inhibitors (e.g., ANGIOZYME®) and HER-2 expression inhibitors; (viii) vaccines such as gene therapy vaccines, for example, ALLOVECTIN®, LEUVECTIN®, and VAXID®; PROLEUKIN® rIL-2; a topoisomerase 1 inhibitor such as LURTOTECAN®; ABARELIX® rmRH; (ix) anti-angiogenic agents such as bevacizumab (AVASTIN®, Genentech); and (x) pharmaceutically acceptable salts, acids and derivatives of any of the above. Other anti-angiogenic agents include MMP-2 (matrix-metalloproteinase 2) inhibitors, MMP-9 (matrix-metallopmtcinasc 9) inhibitors, COX-II (cyclooxygenase II) inhibitors, and VEGF receptor tyrosine kinase inhibitors. Examples of such useful matrix metalloproteinase inhibitors that can be used in combination with the present compounds/compositions are described in WO 96133172, WO 96/27583, EP 818442, EP 1004578, WO 98/07697, WO 98/03516, WO 98/34918, WO 98/34915, WO 98/33768, WO 98/30566, EP 606,046, EP 931,788, WO 90/05719, WO 99/52910. WO 99/52889, WO 99/29667, WO 99/07675, EP 945864, U.S. Pat. No. 5,863,949, U.S. Pat. No. 5,861,510, and EP 780,386. Examples of VEGF receptor tyrosine kinase inhibitors include 4-(4-bromo-2-fluorounilino)-6-methoxy-7-(1-methylpiperidin-4-ylmethoxy)quinazolino (ZD6474; Example 2 within WO 01/32651), 4-(4-fluoro-2-mothylindol-5-yloxy)-6-methoxy-7-(3-pyrrolidin-1-ylpropoxy)- quinazoline (AZD2171; Example 240 within WO 00/47212), vatalanib (PTK787; WO 98/35985) and SU11248 (sunitinib; WO 01/60814), and compounds such as those disclosed in PCT Publication Nos. WO 97/22596, WO 97/30035, WO 97/32856, and WO 98/13354),

Other examples of chemotherapeutic agents that can be used in combination with the present compounds include inhibitors of PI3K (phosphoinositide-3 kinase), such as those reported in Yaguchi et at (2006) Jour. of the Nat. Cancer Inst, 98(8):545-556; US 7173029; US 7037915; US 6608056; US 6608053; US 6838457; US 6770641; US 6653320; US 6403588; WO 2006/046031; WO 2006/046035; WO 2006/046040; WO 2007/042806; WO 2007/042810; WO 20041017950; US 20041092561; WO 2004/007491; WO 2004/006916; WO 2003/037886; US 2003/149074; WO 20031035618; WO 2003/034997; US 2003/158212; EP 1417976; US 2004/053946; JP 2001247477; JP 08175990; JP 08176070; US 6703414; and WO 97/15658. Specific examples of such PI3K inhibitors include SF-1126 (PI3K inhibitor, Semafore Pharmaceuticals), BEZ-235 (PI3K inhibitor, Novartis), XL-147 (PI3K inhibitor, Exelixis, Inc.).

The term "inflammatory diseases" as used in this application includes, but not limited to, rheumatoid arthritis, atherosclerosis, congestive hear failure, inflammatory bowel disease (including, but not limited to, Crohn's disease and ulcerative colitis), chronic obstructive pulmonary disease in the lung, fibrotic disease in the liver and kidney, Crohn's disease, skin diseases such as psoriasis, eczema and scleroderma, osteoarthritis, multiple sclerosis, asthma, diseases and disorders related to diabetic complications, fibrotic organ failure in organs such as lung, liver, kidney, and inflammatory complications of the cardiovascular system such as acute coronary syndrome.

An "anti-inflammatory agent" is a compound useful in the treatment of inflammation. Examples of anti-inflammatory agents include injectable protein therapeutics such as Enbrel®, Remicade®, Humira® and Kineret®. Other examples of anti-inflammatory agents include non-steroidal anti-inflammatory agents (NSAIDs), such as ibuprofen or aspirin (which reduce swelling and alleviate pain); disease-modifying anti-rheumatic drugs (DMARDs) such as methotrexate; 5-aminosalicylates (sulfasalazine and the sulfa-free agents); corticosteroids; immunomodulators such as 6-mercaptoputine ("6-MP"), azathioprine ("AZA"), cyclosporines, and biological response modifiers such as Remicade.RTM. (infliximab) and Enbrel.RTM. (etanercept); fibroblast growth factors; platelet derived growth factors; enzyme blockers such as Arava.RTM. (leflunomide); and/or a cartilage protecting agent such as hyaluronic acid, glucosamine, and chondroitin.

A "liposome" is a small vesicle composed of various types of lipids, phospholipids and/or surfactant which is useful for delivery of a drug (such as MEK kinase inhibitors disclosed herein and, optionally, a chemotherapeutic agent) to a mammal. The components of the liposome are commonly arranged in a bilayer formation, similar to the lipid arrangement of biological membranes.

The term "package insert" is used to refer to instructions customarily included in commercial packages of therapeutic products, that contain information about the indications, usage, dosage, administration, contraindications and/or warnings concerning the use of such therapeutic products.

The term "chiral" refers to molecules which have the property of non-superimposability of the mirror image partner, while the term "achiral" refers to molecules which are superimposable on their mirror image partner.

The term "stereoisomer" refers to compounds which have identical chemical constitution and connectivity, but different orientations of their atoms in space that cannot be interconverted by rotation about single bonds.

"Diastereomer" refers to a stereoisomer with two or more centers of chirality and whose molecules are not mirror images of one another. Diastereomers have different physical properties, e.g. melting points, boiling points, spectral properties, and reactivities. Mixtures of diastereomers may separate under high resolution analytical procedures such as crystallization, electrophoresis and chromatography.

"Enantiomers" refer to two stereoisomers of a compound which are non-superimposable mirror images of one another.

Stereochemical definitions and conventions used herein generally follow S. P. Parker, Ed., McGraw-Hill Dictionary of Chemical Terms (1984) McGraw-Hill Book Company, New York; and Eliel, E. and Wilen, S., "Stereochemistry of Organic Compounds", John Wiley & Sons, Inc., New York, 1994. The compounds of the invention may contain asymmetric or chiral centers, and therefore exist in different stereoisomeric forms. It is intended that all stereoisomeric forms of the compounds of the invention, including but not limited to, diastereomers, enantiomers and atropisomers, as well as mixtures thereof such as racemic mixtures, form part of the present invention. Many organic compounds exist in optically active forms, i.e., they have the ability to rotate the plane of plane-polarized light. In describing an optically active compound, the prefixes D and L, or *R* and *S,* are used to denote the absolute configuration of the molecule about its chiral center(s). The prefixes d and 1 or (+) and (-) are employed to designate the sign of rotation of plane-polarized light by the compound, with (-) or 1 meaning that the compound is levorotatory. A compound prefixed with (+) or d is dextrorotatory. For a given chemical structure, these stereoisomers are identical except that they are mirror images of one another. A specific stereoisomer may also be referred to as an enantiomer, and a mixture of such isomers is often called an enantiomeric mixture. A 50:50 mixture of enantiomers is referred to as a racemic mixture or a racemate, which may occur where there has been no stereoselection or stereospecificity in a chemical reaction or process. The terms "racemic mixture" and "racemate" refer to an equimolar mixture of two enantiomeric species, devoid of optical activity.

The term "tautomer" or "tautomeric form" refers to structural isomers of different energies which are interconvertible via a low energy barrier. For example, proton tautomers (also known as prototropic tautomers) include interconversions via migration of a proton, such as keto-enol and imine-enamine isomerizations. Valence tautomers include interconversions by reorganization of some of the bonding electrons.

The phrase "pharmaceutically acceptable salt" as used herein, refers to pharmaceutically acceptable organic or inorganic salts of a compound of the invention. Exemplary salts include, but are not limited, to sulfate, citrate, acetate, oxalate, chloride, bromide, iodide, nitrate, bisulfate, phosphate, acid phosphate, isonicotinate, lactate, salicylate, acid citrate, tartrate, oleate, tannate, pantothenate, bitartrate, ascorbate, succinate, maleate, gentisinate, fumarate, gluconate, glucuronate, saccharate, formate, benzoate, glutamate, methanesulfonate "mesylate", ethanesulfonate, benzenesulfonate, p-toluenesulfonate, pamoate (i.e., 1,1'-methylene-bis -(2-hydroxy-3-naphthoate)) salts, alkali metal (e.g., sodium and potassium) salts, alkaline earth metal (e.g., magnesium) salts, and ammonium salts. A pharmaceutically acceptable salt may involve the inclusion of another molecule such as an acetate ion, a succinate ion or other counter ion. The counter ion may be any organic or inorganic moiety that stabilizes the charge on the parent compound. Furthermore, a pharmaceutically acceptable salt may have more than one charged atom in its structure. Instances where multiple charged atoms are part of the pharmaceutically acceptable salt can have multiple counter ions. Hence, a pharmaceutically acceptable salt can have one or more charged atoms and/or one or more counter ion.

If the compound of the invention is a base, the desired pharmaceutically acceptable salt may be prepared by any suitable method available in the art, for example, treatment of the free base with an inorganic acid, such as hydrochloric acid, hydrobromic acid, sulfuric acid, nitric acid, methanesulfonic acid, phosphoric acid and the like, or with an organic acid, such as acetic acid, maleic acid, succinic acid, mandelic acid, fumaric acid, malonic acid, pyruvic acid, oxalic acid, glycolic acid, salicylic acid, a pyranosidyl acid, such as glucuronic acid or galacturonic acid, an alpha hydroxy acid, such as citric acid or tartaric acid, an amino acid, such as aspartic acid or glutamic acid, an aromatic acid, such as benzoic acid or cinnamic acid, a sulfonic acid, such as p-toluenesulfonic acid or ethanesulfonic acid, or the like.

If the compound of the invention is an acid, the desired pharmaceutically acceptable salt may be prepared by any suitable method, for example, treatment of the free acid with an inorganic or organic base, such as an amine (primary, secondary or tertiary), an alkali metal hydroxide or alkaline earth metal hydroxide, or the like. Illustrative examples of suitable salts include, but are not limited to, organic salts derived from amino acids, such as glycine and arginine, ammonia, primary, secondary, and tertiary amines, and cyclic amines, such as piperidine, morpholine and piperazine, and inorganic salts derived from sodium, calcium, potassium, magnesium, manganese, iron, copper, zinc, aluminum and lithium.

The phrase "pharmaceutically acceptable" indicates that the substance or composition must be compatible chemically and/or toxicologically, with the other ingredients comprising a formulation, and/or the mammal being treated therewith.

A "solvate" refers to an association or complex of one or more solvent molecules and a compound of the invention. Examples of solvents that form solvates include, but are not limited to, water, isopropanol, ethanol, methanol, DMSO, ethyl acetate, acetic acid, and ethanolamine. The term "hydrate" refers to the complex where the solvent molecule is water.

The term "protecting group" refers to a substituent that is commonly employed to block or protect a particular functionality while reacting other functional groups on the compound. For example, an "amino-protecting group" is a substituent attached to an amino group that blocks or protects the amino functionality in the compound. Suitable amino-protecting groups include acetyl, trifluoroacetyl, t-butoxycarbonyl (BOC), benzyloxycarbonyl (CBZ) and 9-fluorenylmethylenoxycarbonyl (Fmoc). Similarly, a "hydroxy-protecting group" refers to a substituent of a hydroxy group that blocks or protects the hydroxy functionality. Suitable protecting groups include acetyl and silyl. A "carboxy-protecting group" refers to a substituent of the carboxy group that blocks or protects the carboxy functionality. Common carboxy-protecting groups include phenylsulfonylethyl, cyanoethyl, 2-(trimethylsilyl)ethyl, 2-(trimethylsilyl)ethoxymethyl, 2-(p-toluenesulfonyl)ethyl, 2-(p-nitrophenylsulfenyl)ethyl, 2-(diphenylphosphino)-ethyl, nitroethyl and the like. For a general description of protecting groups and their use, see T. W. Greene, Protective Groups in Organic Synthesis, John Wiley & Sons, New York, 1991.

The terms "compound of this invention," and "compounds of the present invention" and "compounds of Formula I, ", unless otherwise indicated, include compounds of Formula I and stereoisomers, geometric isomers, tautomers, solvates, and salts (e.g., pharmaceutically acceptable salts).

The present invention provides azabenzofuranyl compounds of Formula I as described above useful as kinase inhibitors, particularly useful as MEK kinase inhibitors. The present invention includes compounds of Formulae I-a, I-b, I-d, I-f, I-g, II-a, II-b, II-d, II-f, II-g, III-a, III-b, III-d, III-f and III-g and all other variables are as defined in Formula I.

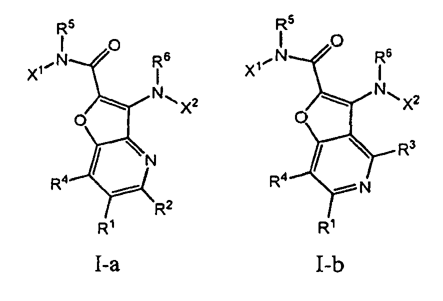

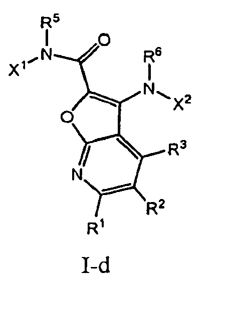

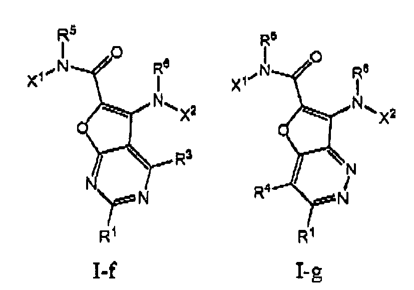

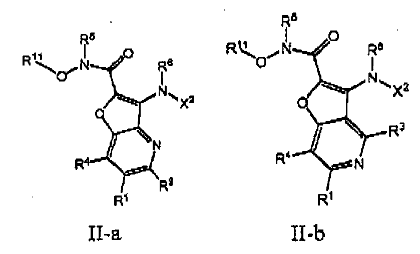

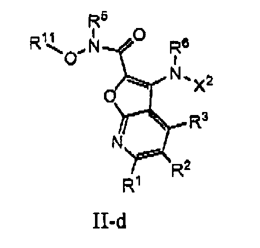

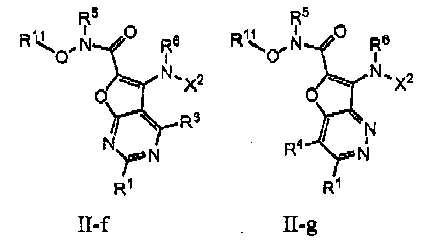

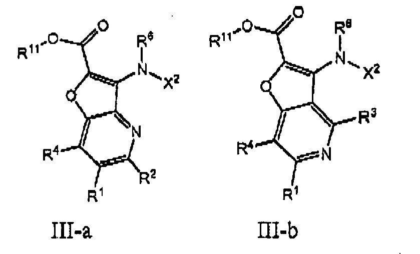

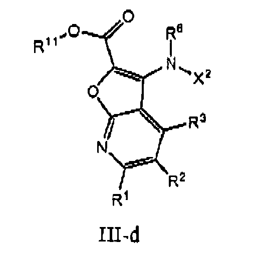

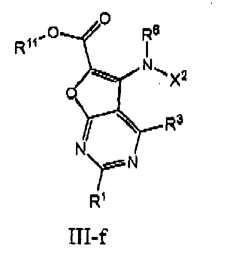

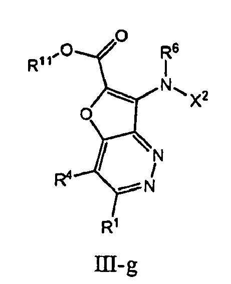

In an embodiment of the present invention, compounds are of Formulae I-b, I-f, I-g, II-b, II-f, II-g, III-b, III-f and III-g and all other variables are as defined in Formula I.

In an embodiment of the present invention, R¹ is H, halo, CN, CF₃, -NR¹¹R¹², -OR¹¹, -C(=O)NR¹¹R¹², or C₁-C₆ alkyl, and all other variables are as defined in Formula I, I-a, I-b, I-d, I-f, I-g, II-a, II-b, II-d, II-f, II-g, III-a, III-b, III-d, III-f, or III-g.

In another embodiment of the present invention, R¹ is H, halo, CN, CF₃, C₁-C₆ alkyl, -NR¹¹R¹² wherein R¹¹ and R¹² are independently H or C₁-C₆ alkyl, or -OR¹¹ wherein R¹¹ is H or C₁-C₆ alkyl; and all other variables are as defined in Formula I, I-a, I-b, I-d, I-f, I-g, II-a, II-b, II-d, II-f, II-g, III-a, III-b, III-d, III-f, or III-g.

In another embodiment of the present invention, R¹ is H, Cl, CN, CF₃, methyl, -NH₂, -NH(CH₃), -N(CH₃)₂, -OH, or -OCH₃; and all other variables are as defined in Formula I, I-a, I-b, I-d, I-f, I-g, II-a, II-b, II-d, II-f, II-g, III-a, III-b, III-d, III-f, or III-g.

In another embodiment of the present invention, R¹ is H; and all other variables are as defined in Formula I, I-a, I-b, I-d, I-f, I-g, II-a, II-b, II-d, II-f, II-g, III-a, III-b, III-d, III-f, or III-g.

In an embodiment of the present invention, R² is H, halo, CN, CF₃, -NR¹¹R¹², -OR¹¹, -C(=O)NR¹¹R¹², or C₁-C₆ alkyl, and all other variables are as defined in Formula I, I-a, I-d, II-a, II-d, III-a or III-d, or as defined above.

In another embodiment of the present invention, R² is H, halo, CN, CF₃, C₁-C₆ alkyl, -NR¹¹R¹² wherein R¹¹ and R¹² are independently H or C₁-C₆ alkyl, or -OR¹¹ wherein R¹¹ is H or C₁-C₆ alkyl; and all other variables are as defined in Formula I, I-a, I-d, II-a, II-d, III-a, or III-d, or as defined above.

In another embodiment of the present invention, R² is H, C1, CN, CF₃, methyl, -NH₂, -NH(CH₃), -N(CH₃)₂, -OH, or -OCH₃; and all other variables are as defined in Formula I, I-a, I-d, II-a, II-d, III-a or III-d, or as defined above.

In an embodiment of the present invention, R³ is H, halo, CN, CF₃, -NR¹¹R¹², -OR¹¹, -C(=O)NR¹¹R¹², or C₁-C₆ alkyl, and all other variables are as defined in Formula I, I-a, I-d, II-a, II-d, III-a, or III-d, or as defined above.

In another embodiment of the present invention, R³ is H, halo, CF₃, C₁-C₆ alkyl; and all other variables are as defined in Formula I, I-a, I-d, II-a, II-d, III-a, or III-d, or as defined above.

In another embodiment of the present invention, R³ is H, F, CF₃, or methyl; and all other variables are as defined in Formula I, I-a, I-d, II-a, II-d, III-a, or III-d, or as defined above.

In another embodiment of the present invention, R³ is H, F, C1, CF₃, methyl or CN; and all other variables are as defined in Formula I, I-a, I-d, II-a, II-d, III-a, or III-d, or as defined above.

In an embodiment of the present invention, R⁴ is H, halo, CN, CF₃, -NR¹¹R¹², -OR¹¹, -C(=O)NR¹¹R¹², or C₁-C₆ alkyl, and all other variables are as defined in Formula I, I-a, I-b, I-g, II-a, II-b, II-g, III-a, III-b, or III-g, or as defined above.

In another embodiment of the present invention, R⁴ is H, halo, CN, CF₃, -NR¹¹R¹² or -C(=O)NR¹¹R¹² wherein R¹¹ and R¹² are independently H or C₁-C₆ alkyl, or -OR¹¹ wherein R¹¹ is H or C₁-C₆ alkyl ; and all other variables are as defined in Formula I, I-a, I-b, I-g, II-a, II-b, II-g, III-a, III-b, or III-g, or as defined above.

In another embodiment of the present invention, R⁴ is H, Br, Cl, CN, CF₃, -NH₂, -NH(CH₃), -N(CH₃)₂, -C(O)NH₂, -C(O)NHCH₃, -C(O)N(CH₃)₂, -OH, or -OCH₃; and all other variables are as defined in Formula I, I-a, I-b, I-g, II-a, II-b, II-g, III-a, III-b, or III-g, or as defined above.

In another embodiment of the present invention, R⁴ is H, Br, C1, CN, CF₃, -NH₂, -NH(CH₃), -N(CH₃)₂, -C(O)NH₂, -C(O)NHCH₃, -C(O)N(CH₃)₂, -OH, or -OCH₃; and all other variables are as defined in Formula I, I-a, I-b, I-g, II-a, II-b, II-g, III-a, III-b, or III-g, or as defined above.

In another embodiment of the present invention, R⁴ is halo, -OH, or C₁-C₆ alkyl optionally substituted by halo; and all other variables are as defined in Formula I, I-a, I-b, I-g, II-a, II-b, II-g, III-a, III-b, or III-g, or as defined above.

In another embodiment of the present invention, R⁴ is independently CI, Br, Me, Et, F, CHF₂, CF₃, or -OH; and all other variables are as defined in Formula I, I-a, I-b, I-g, II-a, II-b, II-g, III-a, III-b, or III-g, or as defined above.

In an embodiment of the present invention, R⁵ is H or C₁-C₆ alkyl; and all other variables are as defined in Formula I, I-a, I-b, I-d, I-f, I-g, II-a, II-b, II-d, II-f, or II-g or as defined above.

In another embodiment of the present invention, R⁵ is H or methyl; and all other variables are as defined in Formula I, I-a, I-b, I-d, I-f, I-g, II-a, II-b, II-d, II-f, or II-g or as defined above.

In another embodiment of the present invention, R⁵ is H; and all other variables are as defined in Formula I, I-a, I-b, I-d, I-f, I-g, II-a, II-b, II-d, II-f, or II-g or as defined above.

In another embodiment of the present invention, R⁵ is methyl; and all other variables are as defined in Formula I, I-a, I-b, I-d, I-f, I-g, II-a, II-b, II-d, II-f, or II-g or as defined above.

In an embodiment of the present invention, R⁶ is H or C₁-C₆ alkyl; and all other variables are as defined in Formula I, I-a, I-b, I-d, I-f, I-g, II-a, II-b, II-d, II-f, III-a, III-b, III-d, III-f or III-g or as defined above.

In another embodiment of the present invention, R⁶ is H or methyl; and all other variables are as defined in Formula I, I-a, I-b, I-d, I-f, I-g, II-a, II-b, II-d, II-f, III-a, III-b, III-d, III-f or III-g or as defined above.

In another embodiment of the present invention, R⁶ is H; and all other variables are as defined in Formula I, I-a, I-b, I-d, I-f, I-g, II-a, II-b, II-d, II-f, III-a, III-b, III-d, III-f or III-g or as defined above.

In another embodiment of the present invention, R⁶ is methyl; and all other variables are as defined in Formula I, I-a, I-b, I-d, I-f, I-g, II-a, II-b, II-d, II-f, III-a, III-b, III-d, III-f or III-g or as defined above.

In an embodiment of the present invention, X¹ is OR¹¹ (i.e., Formula II-a to II-i); and all other variables are as defined in Formula I or I-a, I-b, I-d, I-f or I-g, or as defined above.

In an embodiment of the present invention, X¹ is OR¹¹ wherein R¹¹ is H; and all other variables are as defined in Formula I or I-a, I-b, I-d, I-f or I-g, or as defined above.

In another embodiment of the present invention, X¹ is OR¹¹ wherein R¹¹ is C₁-C₁₂ alkyl (e.g., C₁₋C₆ alkyl) substituted with one or more groups independently selected from halo, CN, CF₃, -OCF₃, -NO₂, oxo, -Si(C₁-C₆ alkyl), -(CR¹⁹R²⁰)ₙ C(=Y_{'})R¹⁶, -(CR¹⁹R²⁰)ₙC(=Y')OR¹⁶, -(CR¹⁹R²⁰)ₙC(=Y')NR¹⁶R¹⁷, -(CR¹⁹R²⁰)ₙNR¹⁶R¹⁷, -(CR¹⁹R²⁰)ₙOR¹⁶, -(CR¹⁹R²⁰)ₙ-SR¹⁶, -(CR¹⁹R²⁰)ₙ NR¹⁶C(=Y')R¹⁷, -(CR¹⁹R²⁰)ₙ NR¹⁶C(=Y')OR¹⁷, -(CR¹⁹R²⁰)ₙ NR¹⁸C(=Y')NR¹⁶R¹⁷, -(CR¹⁹R²⁰)ₙNR¹⁷SO₂R¹⁶, -(CR¹⁹R²⁰)ₙOC(=Y')R¹⁶, -(CR¹⁹R²⁰)ₙOC(=Y')OR¹⁶, -(CR¹⁹R²⁰)ₙ OC(=Y')NR¹⁶R¹⁷, and R²¹; and all other variables are as defined in Formula I or I-a to I-i, or as defined above.

In another embodiment of the present invention, X¹ is: 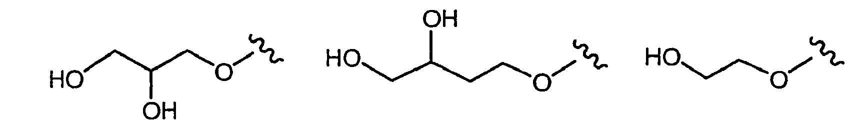 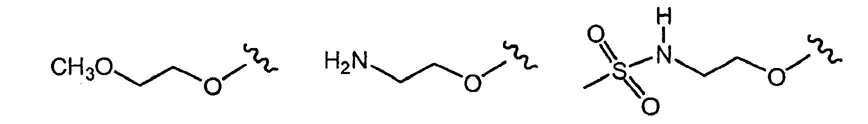 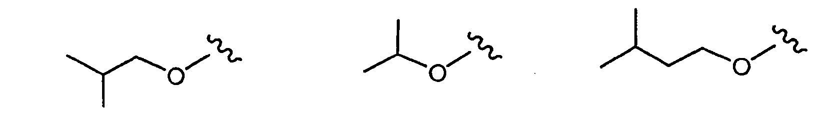 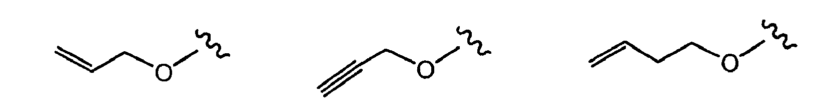 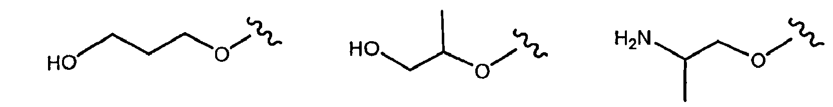 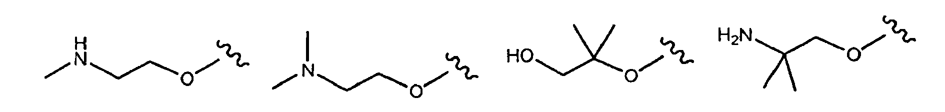 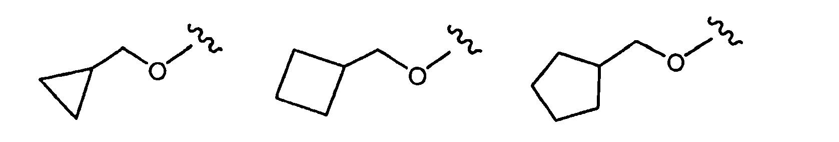 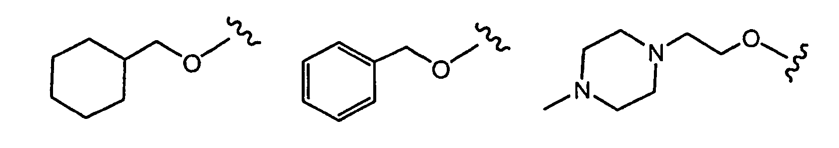 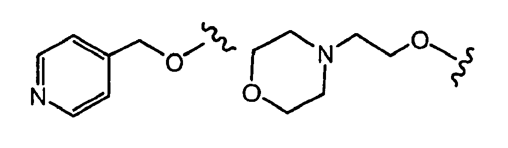 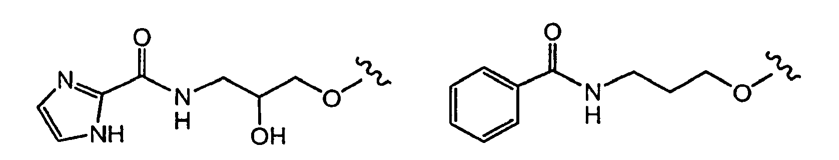 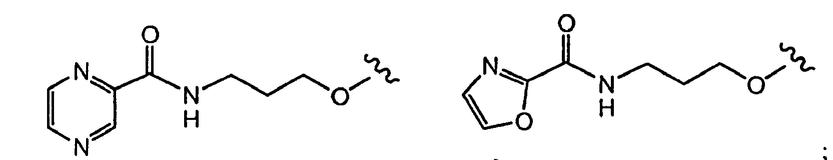 and all other variables are as defined in Formula I or I-a, I-b, I-d, I-f or I-g or as defined above.

In another embodiment of the present invention, X¹ is 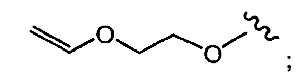 and all other variables are as defined in Formula I or I-a, I-b, I-d, I-f or I-g or as defined above.

In another embodiment of the present invention, X¹ is 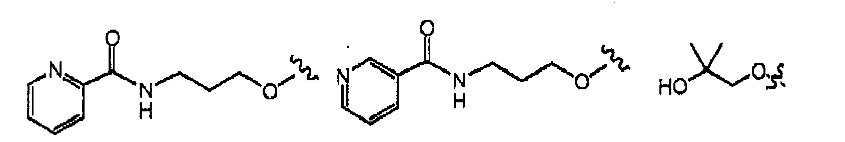 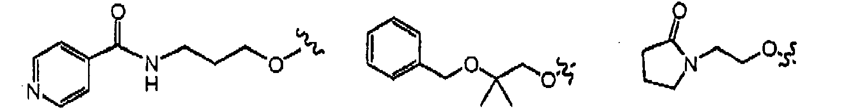 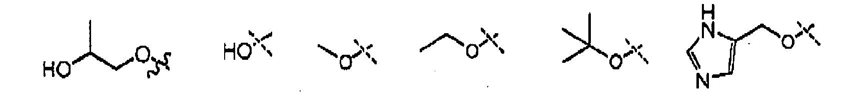 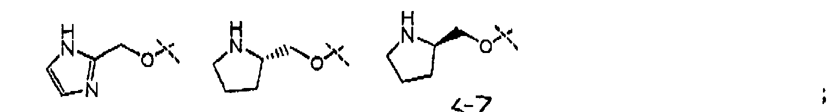 and all other variables are as defined in Formula I or I-a, I-b, I-d, I-f or I-g or as defined above.

In another embodiment of the present invention, X¹ is 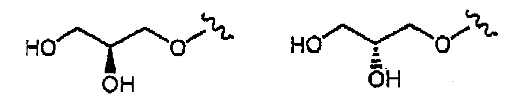 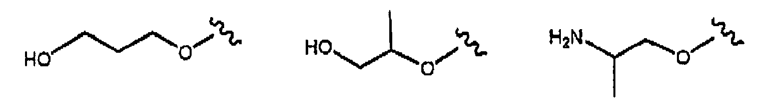 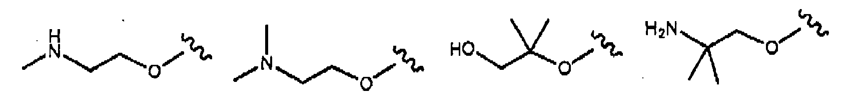 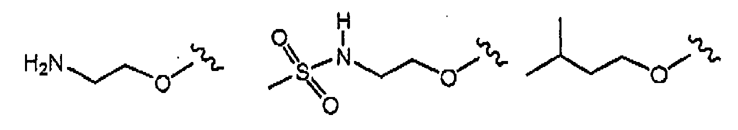 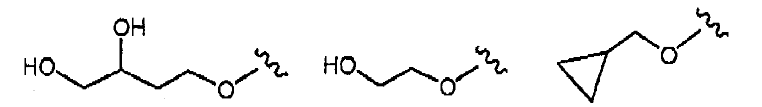 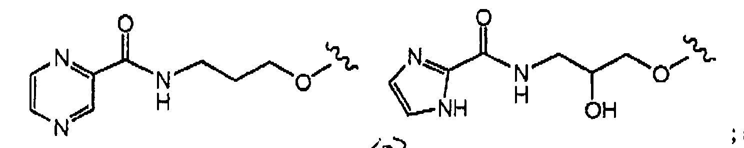 and all other variables arc as defined in Formula I or I-a, I-b, I-d, I-f or I-g or as defined above.

In another embodiment of the present invention, X¹ is 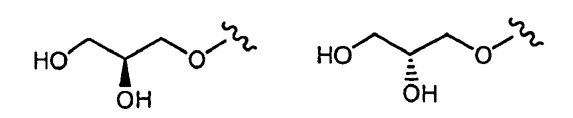 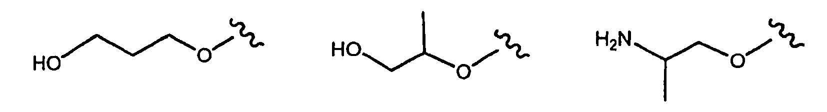 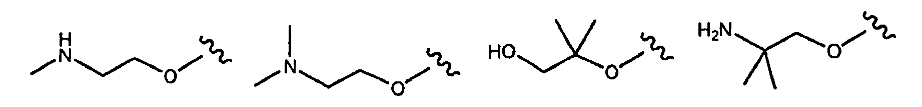 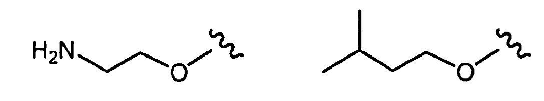 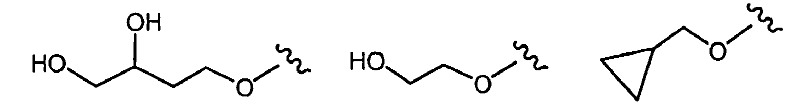 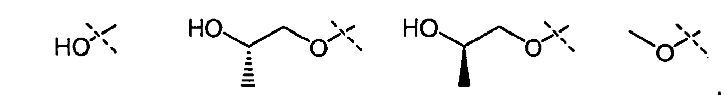 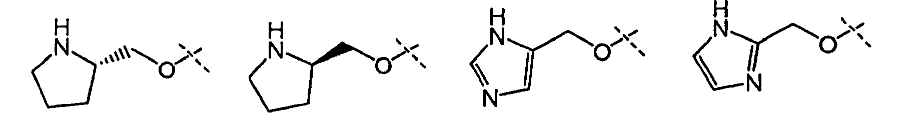 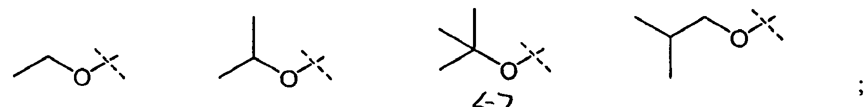 and all other variables are as defined in Formula I or I-a, I-b, I-d, I-f, or I-g or as defined above.

In another embodiment of the present invention, X¹ is OR¹¹ wherein R¹¹ is heterocyclyl (e.g., 4- to 6-membered heterocyclyl) optionally substituted with one or more groups independently selected from halo, CN, CF₃, -OCF₃, -NO₂, oxo, -Si(C₁-C₆ alkyl), -(CR¹⁹R²⁰)ₙC(=Y')R¹⁶, -(CR¹⁹R²⁰)ₙC(=Y')OR¹⁶, -(CR¹⁹R²⁰)ₙC(=Y')NR¹⁶R¹⁷, -(CR¹⁹R²⁰)ₙNR¹⁶R¹⁷, -(CR¹⁹R²⁰)ₙOR¹⁶, -(CR¹⁹R²⁰)ₙ-SR¹⁶, -(CR¹⁹R²⁰)ₙNR¹⁶C(=Y')R¹⁷, -(CR¹⁹R²⁰)ₙ NR¹⁶C(=Y')OR¹⁷, -(CR¹⁹R²⁰)ₙNR1^{8C}(=Y')NR¹⁶R¹⁷, -(CR¹⁹R²⁰)ₙNR¹⁷SO₂R¹⁶, -(CR¹⁹R²⁰)ₙOC(=Y')R¹⁶, -(CR¹⁹R²⁰)ₙOC(=Y')OR¹⁶, -(CR¹⁹R²⁰)ₙOC(=Y')NR¹⁶R¹⁷, and R²¹; and all other variables are as defined in Formula I or I-a, I-b, I-b, I-f or I-g or as defined above.

In another embodiment of the present invention, X¹ is OR¹¹ wherein R¹¹ is 4-to 6-membered heterocyclyl having 1 nitrogen ring atom wherein said heterocyclyl is optionally substituted with one or more groups independently selected from halo, CN, CF₃, -OCF₃, -NO₂, oxo, -Si(C₁-C₆ alkyl), -(CR¹⁹R²⁰)ₙC(=Y')R¹⁶, -(CR¹⁹R²⁰)ₙ C(=Y')OR¹⁶, -(CR¹⁹R²⁰)ₙC(=Y')NR¹⁶R¹⁷, -(CR¹⁹R²⁰)ₙNR¹⁶R¹⁷, -(CR¹⁹R²⁰)ₙOR¹⁶, -(CR¹⁹R²⁰)ₙ-SR¹⁶, -(CR¹⁹R²⁰)ₙNR¹⁶C(=Y')R¹⁷, -(CR¹⁹R²⁰)ₙNR¹⁶C(=Y')OR¹⁷, -(CR¹⁹R²⁰)ₙNR¹⁸C(=Y')NR¹⁶R¹⁷,-(CR¹⁹R²⁰)ₙNR¹⁷SO₂R¹⁶,-(CR¹⁹R²⁰)ₙOC(=Y')R¹⁶, -(CR¹⁹R²⁰)ₙOC(=Y')OR¹⁶, -(CR¹⁹R²⁰)ₙOC(=Y')NR¹⁶R¹⁷, and R²¹; and all other variables are as defined in Formula I or I-a, I-b, I-b, I-f or I-g or as defined above.

In another embodiment of the present invention, X¹ is 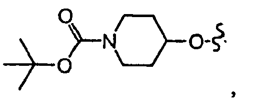 or 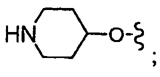 and all other variables are as defined in Formula I or I-a, I-b, I-b, I-f or I-g or as defined above.

In another embodiment of the present invention, X¹ is 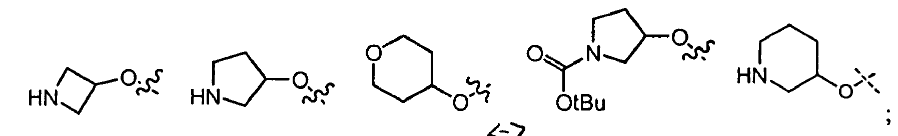 and all other variables are as defined in Formula I or I-a, I-b, I-b, I-f or I-g or as defined above.

In another embodiment of the present invention, X¹ is 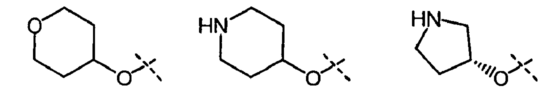 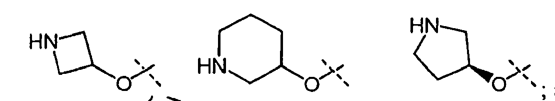 and all other variables are as defined in Formula I or I-a, I-b, I-b, I-f or I-g or as defined above.

In an embodiment of the present invention, X¹ is R¹¹, and X¹ and R⁵ are taken together with the nitrogen atom to which they are attached to form a 5-7 membered saturated or unsaturated cyclic ring having 0-2 additional heteroatoms selected from O, S and N, wherein said cyclic ring is optionally substituted with one or more groups selected from halo, CN, CF₃, -OCF₃, -NO₂, oxo, -(CR¹⁹R²⁰)ₙNR¹⁶R¹⁷, -(CR¹⁹R²⁰)ₙOR¹⁶, -(CR¹⁹R²⁰)ₙS(O)R¹⁶, -(CR¹⁹R²⁰)ₙS(O)₂R¹⁶, and R²¹; and all other variables are as defined in Formula I or I-a, I-b, I-d, I-f or I-g or as defined above.

In another embodiment of the present invention, X¹ is R¹¹, and X¹ and R⁵ are taken together with the nitrogen atom to which they are attached to form a 5-6 membered saturated cyclic ring having 0-2 additional heteroatoms selected from O, S and N, wherein said cyclic ring is optionally substituted with one or more groups selected from halo, CN, CF₃, -OCF₃, -NO₂, oxo, -(CR¹⁹R²⁰)ₙNR¹⁶R¹⁷, -(CR¹⁹R²⁰)ₙOR¹⁶, -(CR¹⁹R²⁰)ₙS(O)R¹⁶, -(CR¹⁹R²⁰)ₙS(O)₂R¹⁶, and R²¹; and all other variables are as defined in Formula I or I-a, I-b, I-d, I-f or I-g or as defined above.

In another embodiment of the present invention, W is: 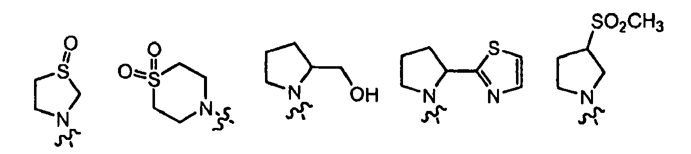 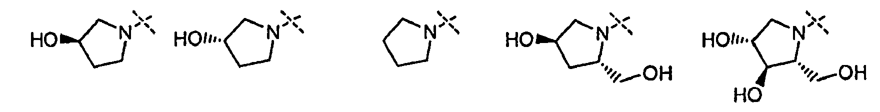 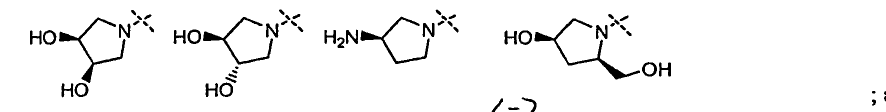 and all other variables are as defined in Formula I or I-a, I-b, I-d, I-f or I-g or as defined above.

In another embodiment of the present invention, W is: 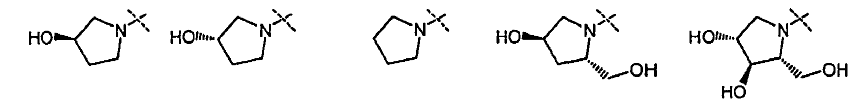 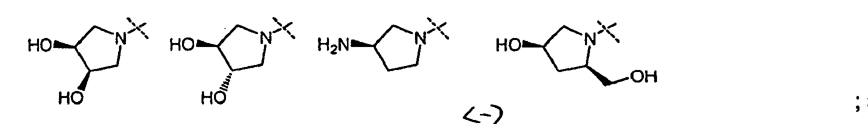 and all other variables are as defined in Formula I or I-a, I-b, I-d, I-f or I-g or as defined above.

In another embodiment of the present invention, W is: 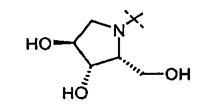 ; and all other variables are as defined in Formula I or I-a, I-b, I-d, I-f or I-g or as defined above.

In an embodiment of the present invention, X¹ is R¹¹, and X¹ and R⁵ are taken together with the nitrogen atom to which they are attached to form a 4-membered saturated or unsaturated cyclic ring having 0-1 additional heteroatoms selected from O, S and N, wherein said cyclic ring is optionally substituted with one or more groups selected from halo, CN, CF₃, -OCF₃, -NO₂, oxo, -(CR¹⁹R²⁰)ₙNR¹⁶R¹⁷, -(CR¹⁹R²⁰)ₙOR¹⁶, -(CR¹⁹R²⁰)ₙS(O)R¹⁶, -(CR¹⁹R²⁰)ₙS(O)₂R¹⁶, and R²¹; and all other variables are as defined in Formula I or I-a, I-b, I-d, I-f or I-g or as defined above.

In another embodiment of the present invention, W is: 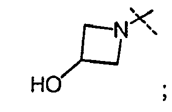 and all other variables are as defined in Formula I or I-a, I-b, I-d, I-f or I-g or as defined above.

In an embodiment of the present invention, X¹ is -OR¹¹, and -OR¹¹ of X¹ and R⁵ are taken together with the nitrogen atom to which they are attached to form a 4-7 membered saturated or unsaturated cyclic ring having 0-2 additional heteroatoms selected from O, S and N, wherein said cyclic ring is optionally substituted with one or more groups selected from halo, CN, CF₃, -OCF₃, -NO₂, oxo, -(CR¹⁹R²⁰)ₙNR¹⁶R¹⁷, -(CR¹⁹R²⁰)ₙOR¹⁶, -(CR¹⁹R²⁰)ₙS(O)R¹⁶, -(CR¹⁹R²⁰)ₙS(O)₂R¹⁶, and R²¹; and all other variables are as defined in Formula I or I-a, I-b, I-d, I-f or I-g or as defined above.

In another embodiment of the present invention, X¹ is -OR¹¹, and -OR¹¹ of X¹ and R⁵ are taken together with the nitrogen atom to which they are attached to form a 5-7 membered saturated or unsaturated cyclic ring having 0-2 additional heteroatoms selected from O, S and N, wherein said cyclic ring is optionally substituted with one or more groups selected from hallo, CN, CF₃, -OCF₃, -NO₂, oxo, -(CR¹⁹R²⁰)ₙNR¹⁶R¹⁷, -(CR¹⁹R²⁰)ₙOR¹⁶, -(CR¹⁹R²⁰)ₙS(O)R¹⁶, -(CR¹⁹R²⁰)ₙS(O)₂R¹⁶, and R²¹; and all other variables are as defined in Formula I or I-a to I-i, or as defined above

In another embodiment of the present invention, X¹ is -OR¹¹, and -OR¹¹ of X¹ and R⁵ are taken together with the nitrogen atom to which they are attached to form a 5-6 membered saturated cyclic ring having 0-2 additional heteroatoms selected from O S and N, wherein said cyclic ring is optionally substituted with one or more groups selected from halo, CN, CF₃, -OCF₃, -NO₂, oxo, -(CR¹⁹R²⁰)ₙNR¹⁶R¹⁷, -(CR¹⁹R²⁰)ₙOR¹⁶, -(CR¹⁹R²⁰)ₙS(O)₂R¹⁶, and R²¹; and all other variables are as defined in Formula I or I-a, I-b, I-d, I-f, or I-g or as defined above.

In another embodiment of the present invention, W is: 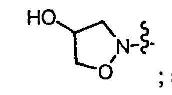 and all other variables are as defined in Formula I or I-a, I-b, I-d, I-f, or I-g or as defined above.

In an embodiment of the present invention, X¹ is R¹¹; and all other variables are as defined in Formula I, I-a, I-b, I-d, I-f or I-g, or as defined above.

In another embodiment of the present invention, X¹ is R¹¹ wherein R¹¹ is H; and all other variables are as defined in Formula I, I-a, I-b, I-d, I-f or I-g, or as defined above.

In another embodiment of the present invention, X¹ is R¹¹ wherein R¹¹ is C₁-C₁₂ alkyl (e.g., C₁-C₆ alkyl) substituted with one or more groups independently selected from halo, CN, CF₃, -OCF₃, -NO₂, oxo, -Si(C₁-C₆ alkyl), -(CR¹⁹R²⁰)ₙ C(=Y')R¹⁶, -(CR¹⁹R²⁰)ₙC(=Y')OR¹⁶, -(CR¹⁹R²⁰)ₙC(=Y')NR¹⁶R¹⁷_{,} -(CR¹⁹R²⁰)ₙNR¹⁶R¹⁷, -(CR¹⁹R²⁰)ₙOR¹⁶, -(CR¹⁹R²⁰)ₙ-SR¹⁶, -(CR¹⁹R²⁰)ₙNR¹⁶C(=Y')R¹⁷, -(CR¹⁹R²⁰)ₙ NR¹⁶C(=Y')OR¹⁷, -(CR¹⁹R²⁰)ₙ NR¹⁸C(=Y')NR¹⁶R¹⁷, -(CR¹⁹R²⁰)ₙNR¹⁷SO₂R¹⁶, -(CR¹⁹R²⁰)ₙOC(=Y')R¹⁶, -(CR¹⁹R²⁰)ₙOC(=Y')OR¹⁶, -(CR¹⁹R²⁰)ₙOC(=Y')NR¹⁶R¹⁷, and R²¹; and all other variables are as defined in Formula I, I-a, I-b, I-d, I-f, or I-g, or as defined above.

In another embodiment of the present invention, X¹ is 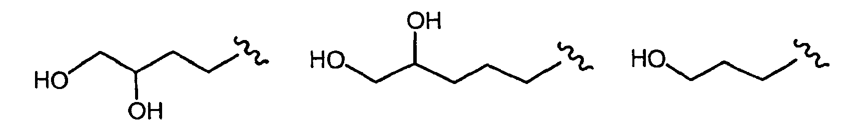 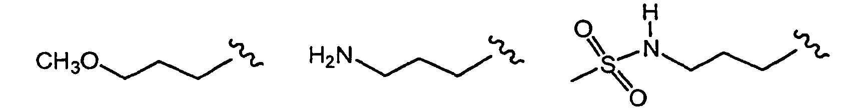 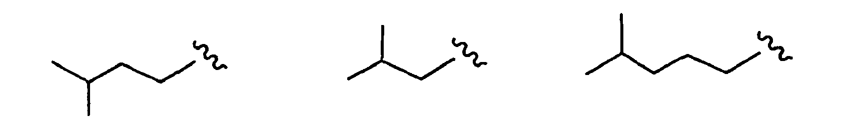 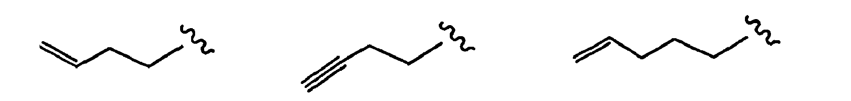 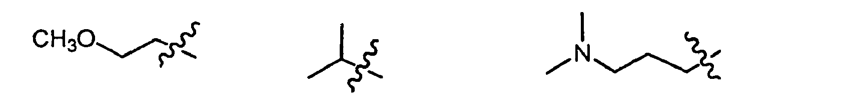 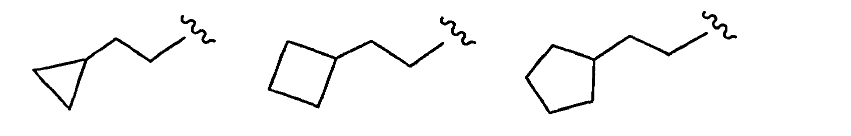 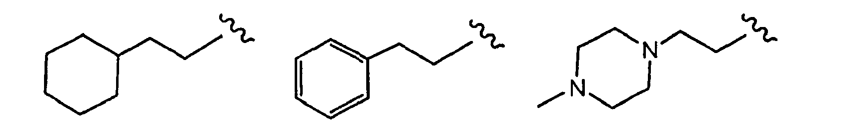 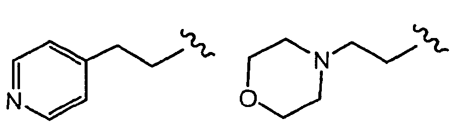 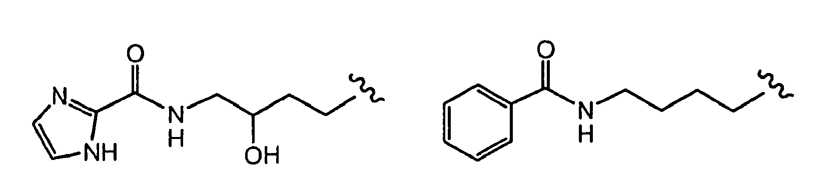 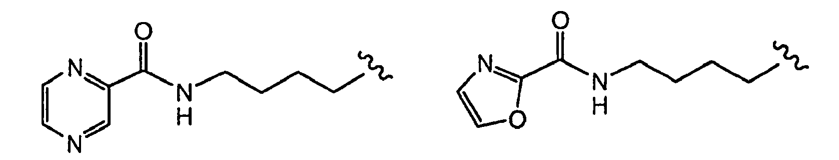 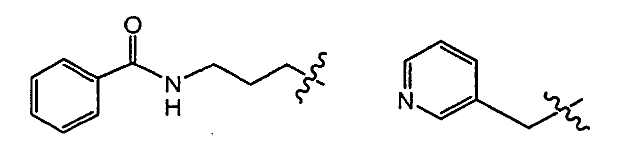 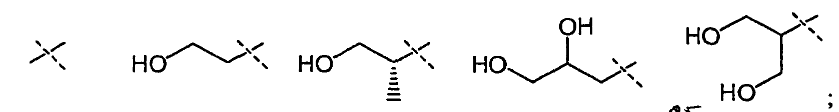 and all other variables are as defined in Formula I, I-a, I-b, I-d, I-f, or I-g, or as defined above.

In another embodiment of the present invention, X¹ is 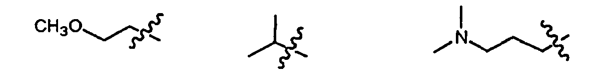 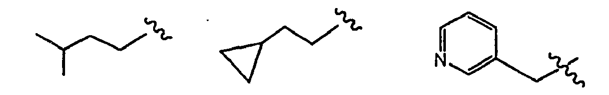 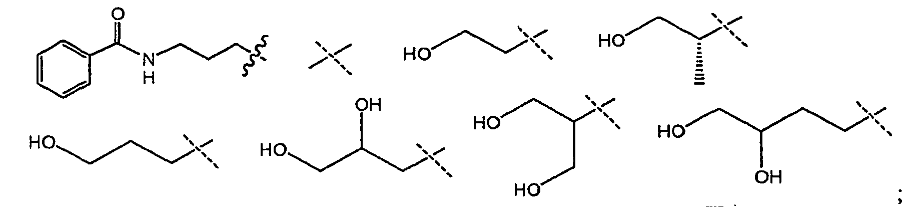 and all other variables are as defined in Formula I, I-a, I-b, I-d, I-f or I-g, or as defined above.

In another embodiment of the present invention, X¹ is -S(O)₂R¹¹, and all other variables are as defined in Formula I, I-a, I-b, I-d, I-f or I-g, or as defined above.

In another embodiment of the present invention, X¹ is -S(O)₂R¹¹ wherein R¹¹ is H or methyl; and all other variables are as defined in Formula I, I-a, I-b, I-d, I-f or I-g, or as defined above.

In an embodiment of the present invention, W is -OR¹¹ (i.e., Formula III-a, III-b, III-d, III-f or III-g) wherein R¹¹ of W is H or C₁-C₁₂ alkyl; and all other variables are as defined above.

In another embodiment of the present invention, W is -OR¹¹ (i.e., Formula III-a, III-b, III-d, III-f or III-g) wherein R¹¹ of W is H; and all other variables are as defined above.

In another embodiment of the present invention, W is -OR¹¹ (i.e., Formula III-a, III-b, III-d, III-f or III-g wherein R¹¹ of W is C₁-C₆ alkyl; and all other variables are as defined above.

In an embodiment of the present invention, X² is aryl (e.g., phenyl), wherein said aryl is optionally substituted with one or more groups independently selected from halo, CN, CF₃, -OCF₃, -NO₂, oxo, -Si(C₁-C₆ alkyl), -(CR¹⁹R²⁰)ₙ C(=Y')R¹⁶, -(CR¹⁹R²⁰)ₙC(=Y')OR¹⁶, -(CR¹⁹R²⁰)ₙC(=Y')NR¹⁶R¹⁷, -(CR¹⁹R²⁰)ₙNR¹⁶R¹⁷, -(CR¹⁹R²⁰)ₙOR¹⁶, -(CR¹⁹R²⁰)ₙ-SR¹⁶, -(CR¹⁹R²⁰)ₙNR^{l6}C(=Y')R¹⁷, -(CR¹⁹R²⁰)ₙ NR¹⁶C(=Y')OR¹⁷, -(CR¹⁹R²⁰)ₙNR¹⁸C(=Y')NR¹⁶R¹⁷, -(CR¹⁹R²⁰)ₙNR¹⁷SO₂R¹⁶, -(CR¹⁹R²⁰)ₙOC(=Y')R¹⁶, -(CR¹⁹R²⁰)ₙOC(=Y')OR¹⁶, -(CR¹⁹R²⁰)ₙOC(=Y')NR¹⁶R¹⁷, and R²¹; and all other variables are as defined in Formula I-a, I-b, I-d, I-f, I-g, II-a, II-b, II-d, II-f, II,g, III-a, III-b, III-d, III-f or III-g or as defined above.

In another embodiment of the present invention, X² is 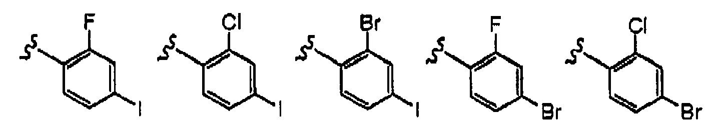 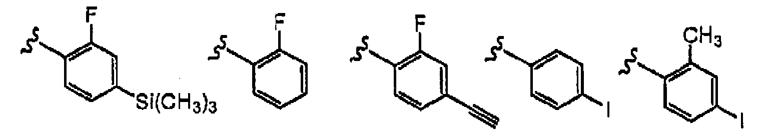 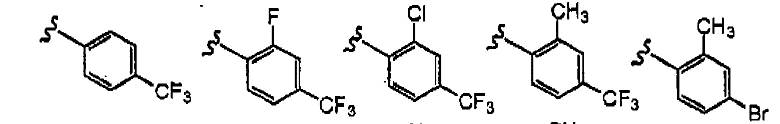 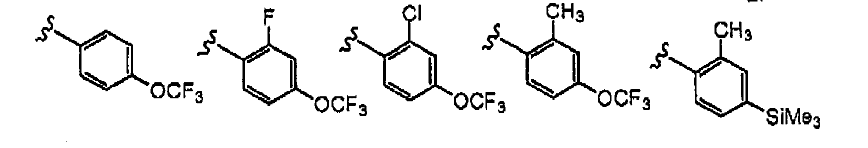 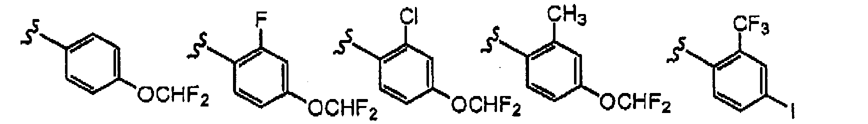 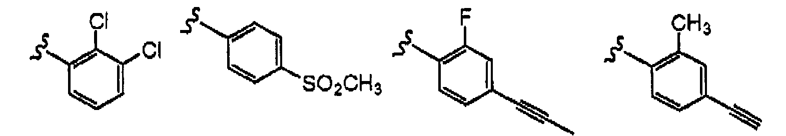 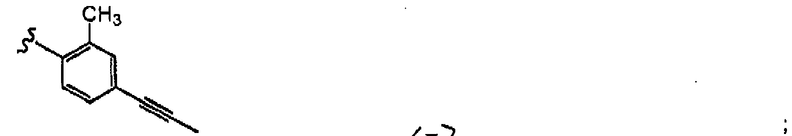 and all other variables arc as defined in Formula I, I-a, I-b, I-d, I-f, I-g, II-a, II-b, II-d, II-f, II-g, III-a, III- b, III-d, III-f or 111-g or as defined above.

In another embodiment of the present invention, X² is 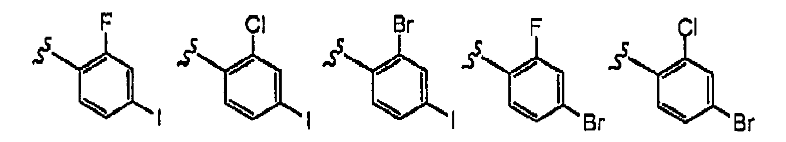 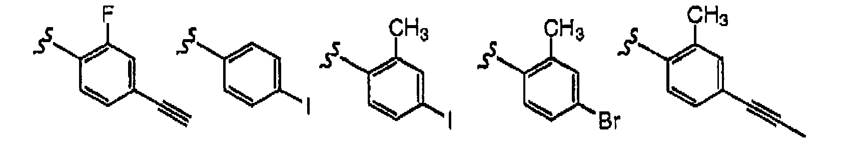 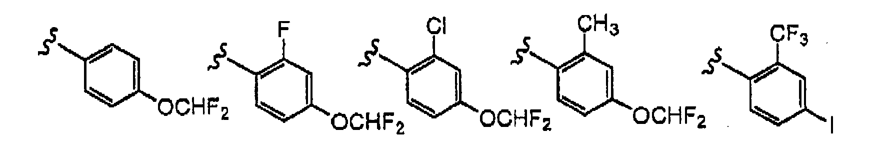 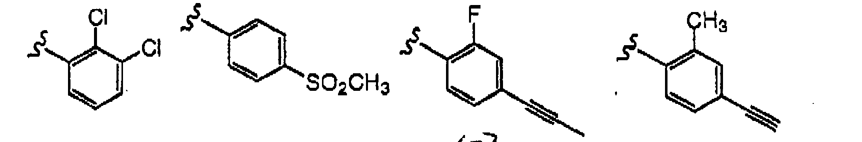 and all other variables are as defined in Formula I, I-a, I-b, I-d, I-f, I-g, II-a, II-b, II-d, II-f, II-g, III-a, III- b, III-d, III-f or III-g or as above,

In another embodiment of the present invention, X² is 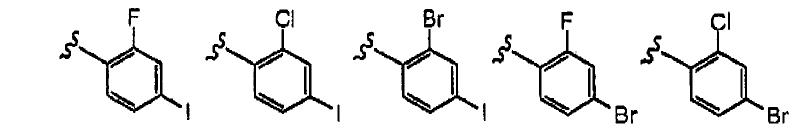 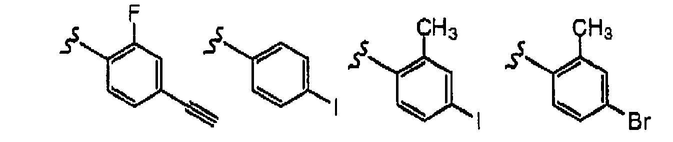 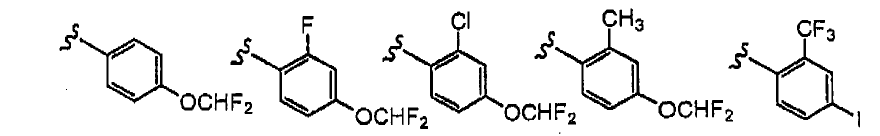 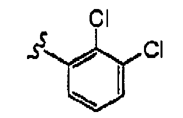 and all other variables are as defined in Formula I, I-a, I-b, I-d, I-f, I-g, II-a, II-b, II-d, II-f, II-g, III-a, III- b, III-d, III-f or III-g or as defined above.

In another embodiment of the present invention, X² is 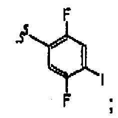 and all other variables are as defined in Formula I, I-a, I-b, I-d, I-f, I-g, II-a, II-b, II-d, II-f, II-g, 111-a, III- b, III-d, III-f or III-g or as defined above.

In another embodiment of the present invention, X² is 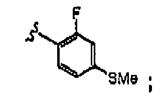 and all other variables are as defined in Formula I, I-a, I-b, I-d, I-f, I-g, II-a, II-b, II-d, II-f, II-g, 111-a, III- b, III-d, III-f or III-g or as defined above.

In another embodiment of the present invention, X² is C₆-C₁₀ aryl substituted with C₁-C₄ alkyl; and all other variables are as defined in Formula I, I-a, I-b, I-d, I-f, I-g, II-a, II-b, II-d, II-f, II-g, 111-a, III- b, III-d, III-f or III-g or as defined above.

In another embodiment of the present invention, X² is 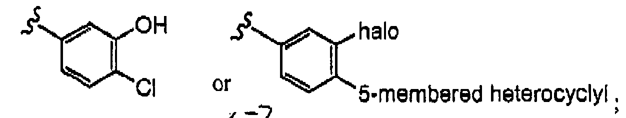 and all other variables are as defined in Formula I, I-a, I-b, I-d, I-f, I-g, II-a, II-b, II-d, II-f, II-g, III-a, III- b, III-d, III-f or III-g or as defined above.

In another embodiment of the present invention, X² is 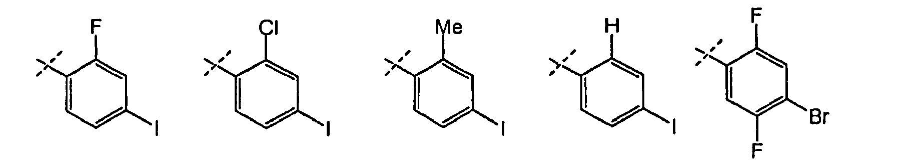 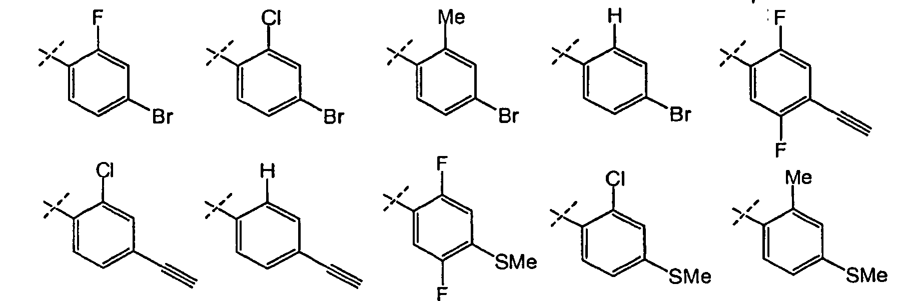 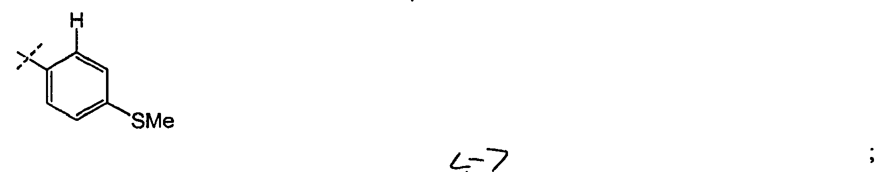 and all other variables are as defined in Formula I, I-a, I-b, I-d, I-f, I-g, II-a, II-b, II-d, II-f, II-g, III-a, III-b, III-d, III-f or III-g as defined above.

In another embodiment of the present invention, X² is 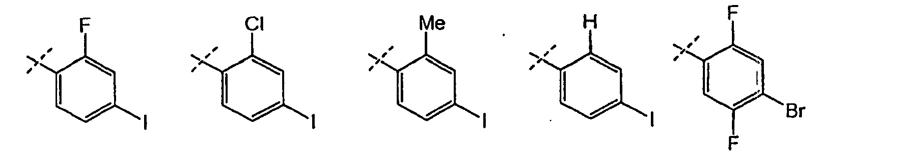 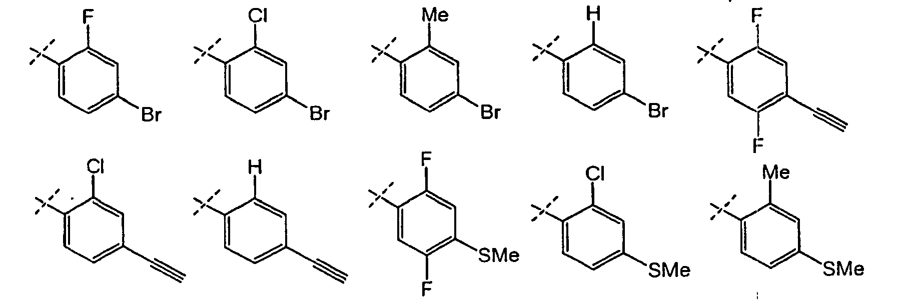 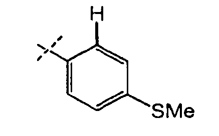 and all other variables are as defined in Formula I, I-a, I-b, I-d, I-f, I-g, II-a, II-b, II-d, II-f, II-g, III-a, III- b, III-d, III-f or III-g or as defined above.

Another embodiment of the present invention includes compounds described in EXAMPLES 5-159 and compounds below:

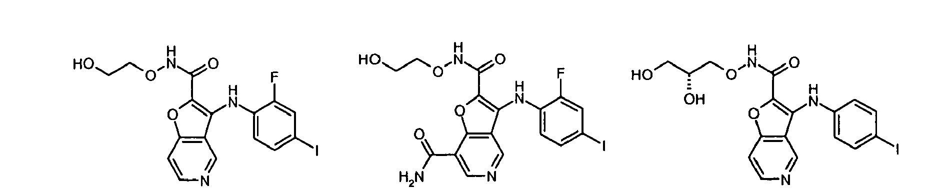

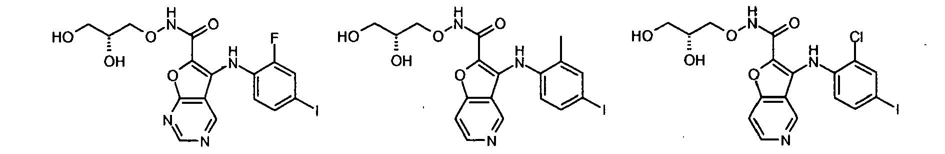

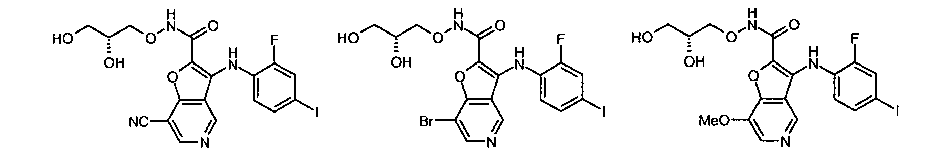

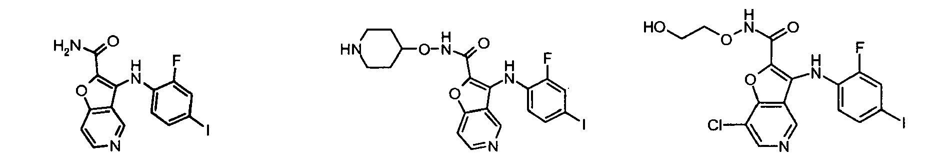

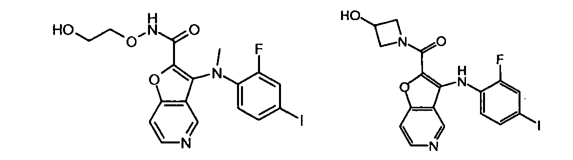

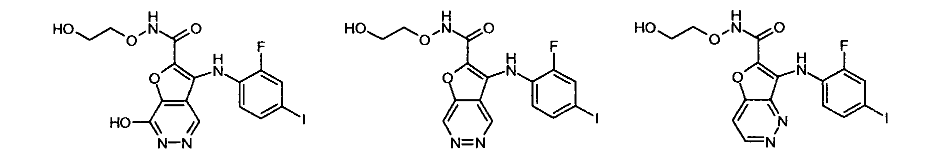

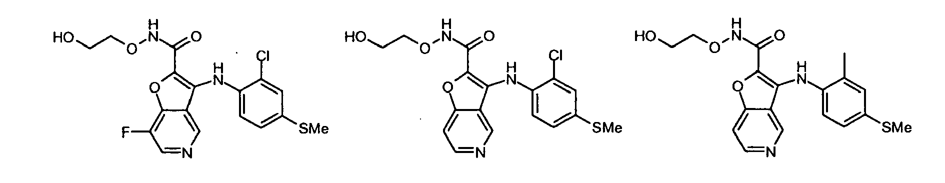

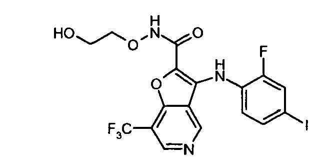

The present compounds are prepared according to the procedures described below in the schemes and examples or by methods known in the art. The starting materials and various intermediates may be obtained from commercial sources, prepared from commercially available compounds, or prepared using well known synthetic methods (for example, those described in WO02/06213, WO 03/077855 and WO03/077914).

For example, 5-azabenzofurans of Formula (I-b), (II-b) or (III-b) may be prepared using the synthetic routes outlined in Schemes 1, 2 and 3.

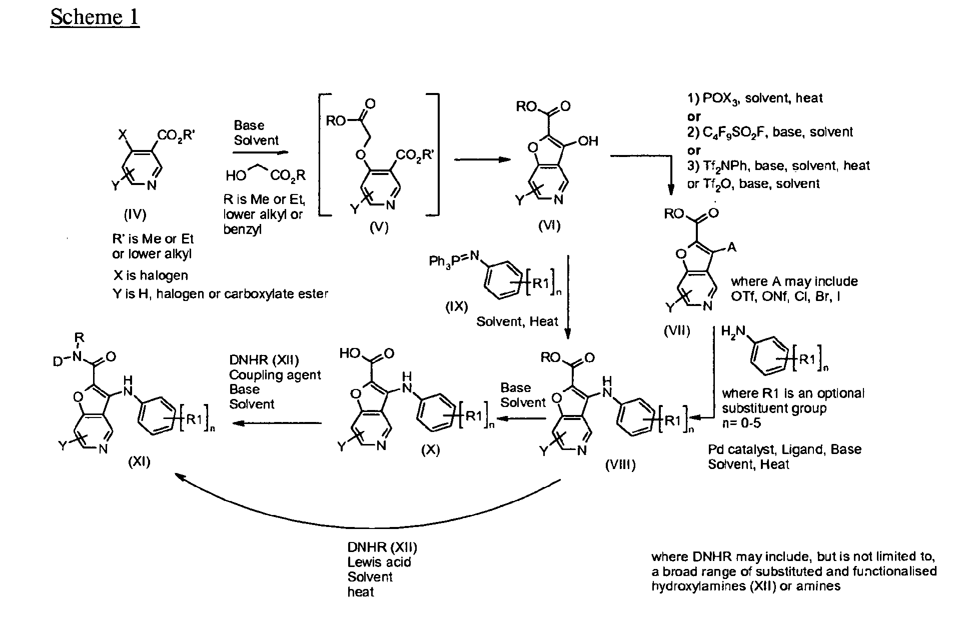

Compounds of formula (IV) may be prepared using published methods described in the literature. They may be reacted with methylglycolate or ethylglycolate in the presence of a base, such as sodium hydride, in a suitable solvent, such as *N,N-*dimethylformamide or 1,2-dimethoxyethane, at a temperature of from -50°C to room temperature, to obtain compounds of formula (VI).

Compounds of formula (VI) may be converted to compounds of formula (VII) by reaction with a halogenating agent such as phosphorus oxybromide, neat or in a suitable solvent such as toluene, at a temperature of from room temperature to 140°C. Alternatively, compounds of formula (VI) may be reacted with nonafluorobutane sulphonyl fluoride in the presence of a base such as diisopropylethylamine and a catalyst such as *N,N-*dimethyl-4-aminopyridine, in a solvent such as dichloromethane at room temperature, with *N-*phenyltrifluoromethanesulfonimide in the presence of a base such as diisopropylethylamine, in a suitable solvent such as 1,2-dimethoxyethane at a temperature from room temperature to the reflux temperature of the solvent. In addition, compounds of formula (VI) may be treated with trifluoromethanesulphonic acid anhydride in the presence of a base such as pyridine in a solvent such as dichloromethane at a temperature of from -20°C to ambient temperature.

Compounds of formula (VIII) may be obtained from compounds of formula (VII) by reaction with an aniline (incorporating appropriate substituents R1), in the presence of a catalyst such as tris(dibenzylideneacetone)dipalladium (0) or palladium acetate, a base such as potassium phosphate, sodium tert-butoxide, 1,8-diazabicyclo[5.4.1]undec-7-ene or cesium carbonate, a ligand such as 9,9'-dimethyl-4,5-bis(diphenylphosphino)xanthene, 2,2'-bis(diphenylphosphino)-1,1'-binaphthyl, 2-dicyclohexylphosphino-2'-(*N,N-*dimethylamino)biphenyl, 2-dicyclohexylphosphino-2',6'-(dimethoxy)biphenyl or tri-butylphosphine in a suitable solvent such as toluene, 1,2-dimethoxyethane, tetrahydrofuran or dioxane, at a temperature of from room temperature to the reflux temperature of the solvent, or under microwave irradiation at a temperature of from 70°C to 150°C.

Alternatively compounds of formula (VIII) can be obtained from compounds of formula (VI) by reaction with compounds of formula (IX) (prepared using published methods described in the literature), in a suitable solvent such as toluene or 1,2-dimethoxyethane, at a temperature of from room temperature to the reflux temperature of the solvent, or under microwave irradiation at a temperature of from 100°C to 180°C.

Compounds of formula (X) can be obtained from compounds of formula (VIII) by reaction with a base such as sodium hydroxide in a protic solvent such as ethanol or methanol, at a temperature of from room temperature up to reflux temperature.
Compounds of formula (X) can be reacted with a functionalised hydroxylamine of formula (XII) (commercially available or prepared according to Scheme 8) or an amine, and a suitable coupling agent, such as *O*-(7-aza-benzo-triazol-1-yl)-*N,N,N',N'*-tetra-methyluronium hexafluoro-phosphate, *N*-(3-dimethylaminopropyl)-*N'*-ethylcarbodiimide hydrochloride or *N,N'*-dicyclohexylcarbodiimide in the presence of *N-*hydroxy-1,2,3-benzotriazole, in the presence of a suitable base such as diisopropylethylamine or triethylamine in an inert solvent, such as tetrahydrofuran, *N,N*-dimethylformamide, or dichloromethane at a temperature of about room temperature, to obtain the compounds of formula (XI). Alternatively, compounds of formula (XI) can be obtained directly from compounds of formula (VIII) by reaction with an amine or hydroxylamine DNHR in the presence of a Lewis acid such as trimethyl aluminium in a solvent such as DCM, at a temperature of from room temperature up to reflux temperature.
Alternatively, compounds of formula (VIII) can be prepared from compounds of formula (XIII), according to Scheme 2.

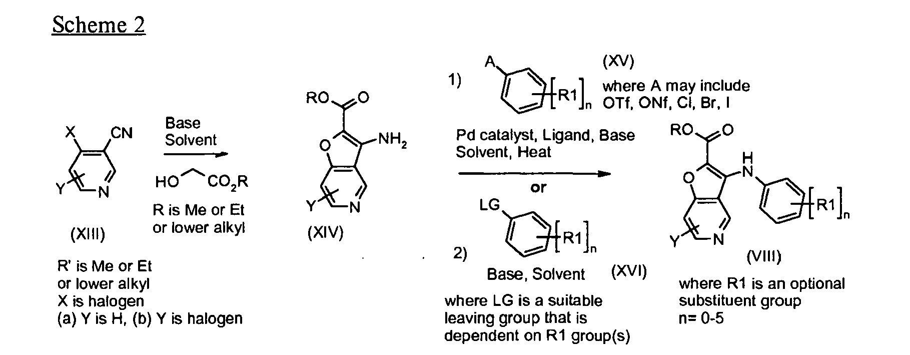

Compounds of formula (XIII) may be prepared using published methods described in the literature. Compounds of general formula (XIV) can be prepared from compounds of formula (XIII) using methods described above for the preparation of compounds of formula (VI) from compounds of formula (IV).

Compounds of formula (VIII) may be obtained from compounds of formula (XIV) by reaction with compounds of formula (XV) (incorporating appropriate substituents R1), using methods described above for the preparation of compounds of formula (VIII) from compounds of formula (VI). Alternatively, compounds of formula (VIII) may be obtained from compounds of formula (XIV) by reaction with compounds of formula (XVI) (incorporating appropriate substituents R1), in the presence of a base such as sodium hydride or lithium hexamethyldisilazane, in a suitable solvent such as tetrahydrofuran or *N,N-*dimethylformamide, at a temperature of from room temperature to 150°C.

Alternatively, compounds of formula (X) can also be prepared from compounds of formula (VII) according to Scheme 3**_**.

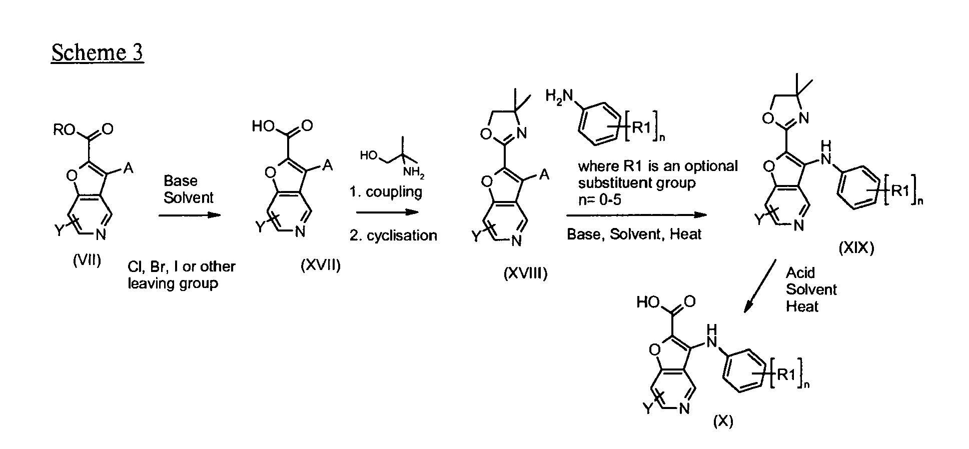

Compounds of formula (VII) can be converted to compounds of formula (XVII) using methods described above for the preparation of compounds of formula (X) from compounds of formula (VIII).

Compounds of formula (XVII) can be coupled to amines such as 2-amino-2-methyl-1-propanol using methods described above for the preparation of compounds of formula (XI) from compounds of formula (X), followed by reaction with an agent such as thionyl chloride or phosphorus oxychloride, neat or in a suitable solvent such as dichloromethane, chloroform or diethyl ether, at a temperature of from room temperature to reflux of the solvent, to afford compounds of formula (XVIII).

Compounds of formula (XIX) may be obtained from compounds of formula (XVIII) by reaction with an aniline (incorporating appropriate substituents R1), in the presence of a catalyst such as tris(dibenzylideneacetone)dipalladium (0) or palladium acetate, a base such as potassium phosphate, sodium tert-butoxide, 1,8-diazabicyclo[5.4.1]undec-7-ene or cesium carbonate, a ligand such as 9,9'-dimethyl-4,5-bis(diphenylphosphino)xanthene, 2,2'-bis(diphenylphosphino)-1,1'-binaphthyl, 2-dicyclohexylphosphino-2'-(*N,N-*dimethylamino)biphenyl, 2-dicyclohexylphosphino-2',6'-(dimethoxy)biphenyl or tri-butylphosphine in a suitable solvent such as toluene, 1,2-dimethoxyethane, tetrahydrofuran or dioxane, at a temperature of from room temperature to the reflux temperature of the solvent, or under microwave irradiation at a temperature of from 70°C to 150°C.

Alternatively, compounds of formula (XIX) may be obtained from compounds of formula (XVIII) by reaction with anilines (incorporating appropriate substituents R1), in the presence of a base such as sodium hydride or lithium hexamethyldisilazane, in a suitable solvent such as tetrahydrofuran or *N,N-*dimethylformamide, at a temperature of from room temperature to 150°C.

Compounds of formula (X) may be obtained from compounds of formula (XIX) by reaction with an acid such as hydrogen chloride, or acetic acid in a suitable solvent such as water, at a temperature of from room temperature to reflux of the solvent.

Furo[2,3-d]pyrimidines of Formula I-f, II-f or III-f may be prepared using the synthetic routes outlined in Scheme 5.

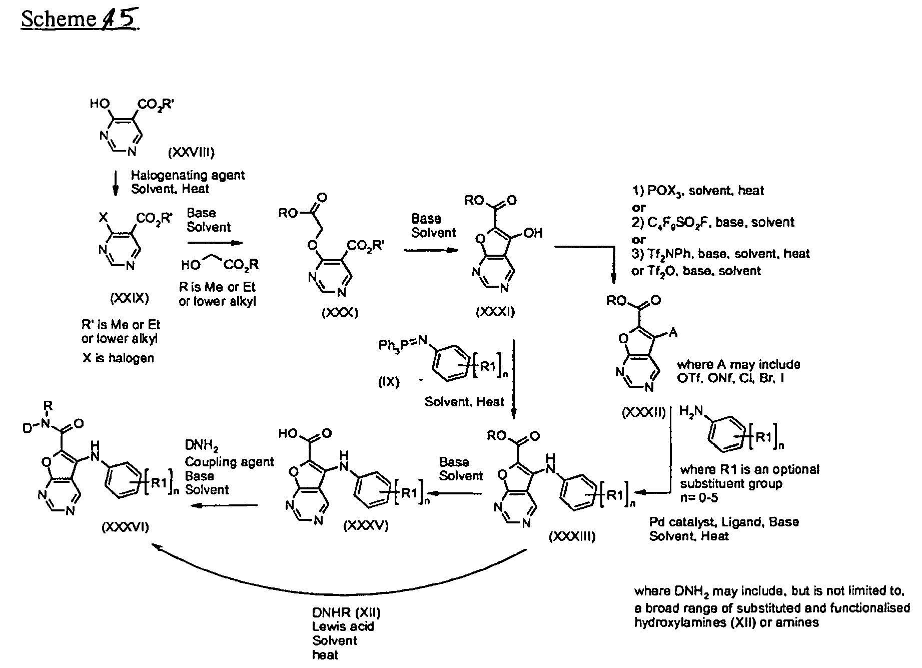

Compounds of formula (XXVIII) may be prepared according to methods described in the literature. They may reacted with a halogenating agent such as phosphorus oxychloride, neat or in a suitable solvent such as toluene, at a temperature from room temperature to reflux, to provide compounds of formula (XXIX).
Compounds of formula (XXXVI) may be obtained from compounds of formula (XXIX) using similar methods to the ones described for the preparation of compounds of formula (XI) from compounds of formula (IV), as shown in Scheme 1.

Furo[3,2-c]pyridazines of formula I-g, II-g, and III-g may be prepared using the synthetic routes outlined in Scheme 6.

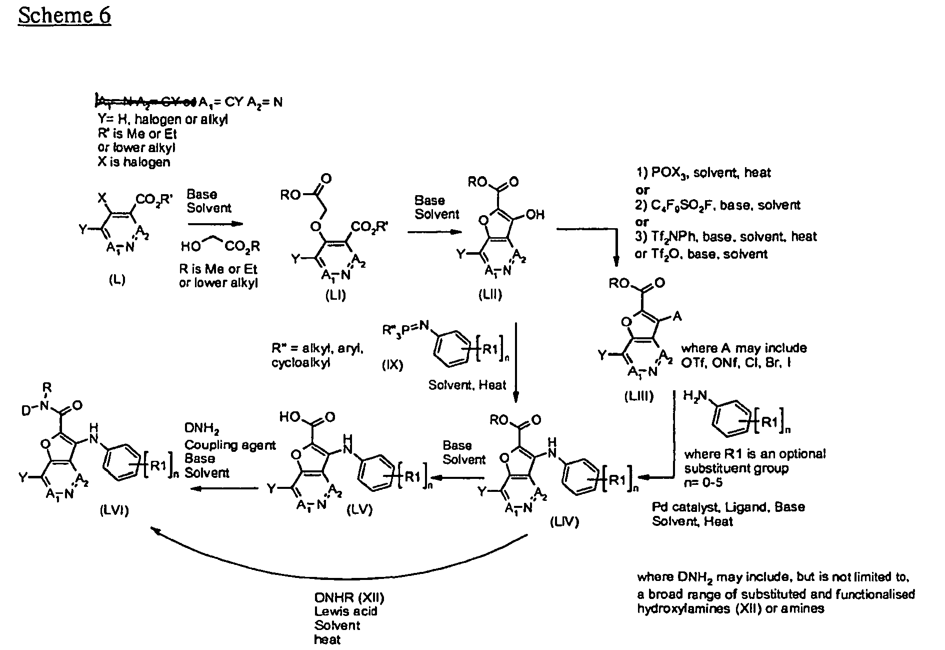

Compounds of formula (L) may be prepared according to methods described in the literature. Compounds of formula (LVI) may be obtained from compounds of formula (L) using similar methods to the ones described for the preparation of compounds of formula (XI) from compounds of formula (IV), as shown in Scheme 1. Alternatively, compounds of formula (LIV) may be prepared according to Scheme 7. 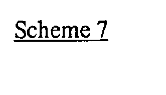 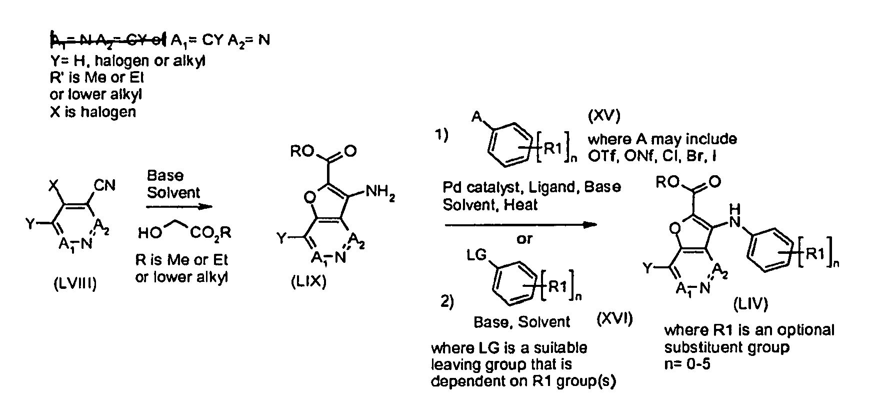 Compounds of formula (LVIII) may be prepared using published methods described in the literature. Compounds of general formula (LIV) can be prepared from compounds of formula (LIX) using methods described above for the preparation of compounds of formula (VIII) from compounds of formula (XIII).

Hydroxylamines of formula (XII) may be prepared using methods described in the literature or the synthetic route outlined in Scheme 8.

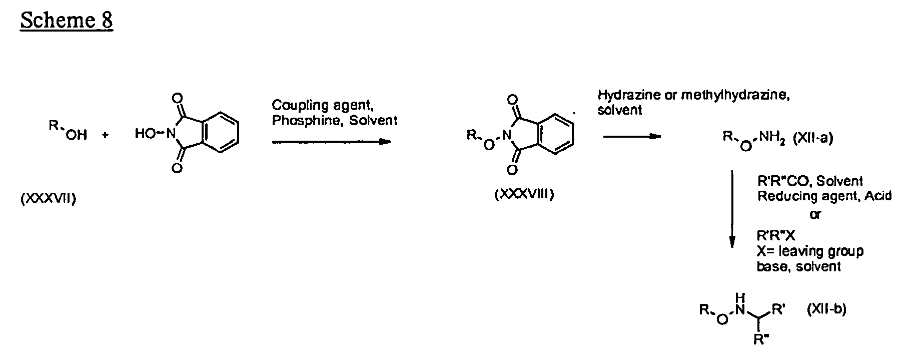

Primary or secondary alcohols of general formula (XXXVII) may be prepared using methods described in the literature. They may be reacted with N-hydroxy phthalimide using a phosphine and coupling reagent such as diethyl azodicarboxylate to provide compounds of general formula (XXXVIII). Compounds of general formula (XXXVIII) may be deprotected using hydrazine or methyl hydrazine to provide hydroxylamines of general formula (XII-a). Compounds of formula (XII-a) may be further modified by reductive amination with aldehydes or ketones using a reducing agent such as sodium triacetoxy borohydride, sodium cyanoborohydride, or borane-pyridine in a solvent such as dichloroethane at a temperature of from ambient temperature to reflux. In addition, compounds of formula (XII-a) may be further modified by alkylation with an alkyl halide in the presence of a base such as triethylamine, in a solvent such as dichloromethane, to provide hydroxylamines of general formula (XII-b).

Anilines of general formula (XXXIX) used in cross-coupling reactions described above may be prepared by using methods described in the literature or according to Scheme 9. 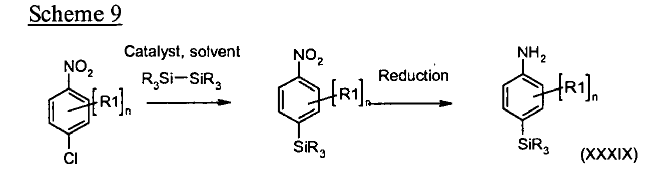 Where R₁ is an optional substituent
group, n= 0-4

Substituted 4-chloro-nitro benzene may be reacted with hexamethyldisilane in a solvent such as xylene using a catalyst such as tetrakis(triphenylphosphine)palladium at a temperature of from room temperature to reflux. The nitro group may be reduced using methods described in the literature such as reaction under an atmosphere of hydrogen at a pressure of from 1 to 5 atmospheres in the presence of a catalyst such as palladium on carbon and in a solvent such as ethanol or ethyl acetate at room temperature.

Trifluoromethanesulfonyl esters of general formula (XL) used in cross-coupling reactions described above may be prepared by using methods described in the literature or according to Scheme 10.

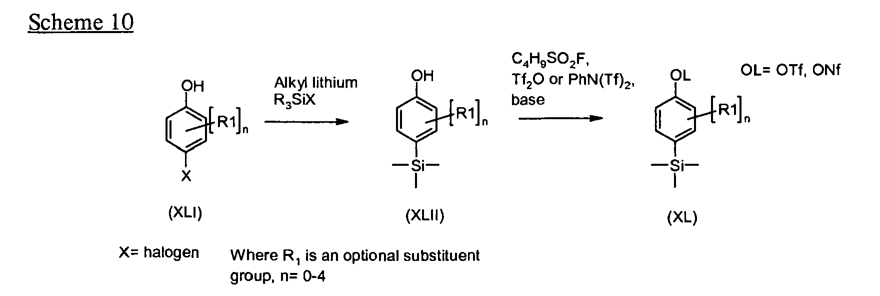

Halo phenols of general structure (XLI) may be reacted with two equivalents of alkylithium reagents such as n-butyl lithium in a solvent such as THF, followed by quenching with trialkylsilyl halide such as trimethylsilyl chloride to give trialkylsilyl phenols (XLII). Trialkylsilyl phenols may be further reacted using literature procedures to give trifluoromethane sulfonates or nonaflates of general structure (XL).

It will be appreciated that where appropriate functional groups exist, compounds of formula (1), (II), (III) or any intermediates used in their preparation may be further derivatised by one or more standard synthetic methods employing substitution, oxidation, reduction, or cleavage reactions. Particular substitution approaches include conventional alkylation, arylation, heteroarylation, acylation, sulfonylation, halogenation, nitration, formylation and coupling procedures.

For example, aryl bromide or chloride groups may be converted to aryl iodides using a Finkelstein reaction employing an iodide source such as sodium iodide, a catalyst such as copper iodide and a ligand such as trans-N,N'-dimethyl-1,2-cyclohexane diamine in a solvent such as 1,4-dioxane and heating the reaction mixture at reflux temperature. Aryl trialkylsilanes may be converted to aryl iodides by treating the silane with an iodide source such as iodine monochloride in a solvent such as dichloromethane with or without Lewis acid such as silver tetrafluoroborate at a temperature from -40°C to reflux.

In a further example primary amine (-NH₂) groups may be alkylated using a reductive alkylation process employing an aldehyde or a ketone and a borohydride, for example sodium triacetoxyborohydride or sodium cyanoborohydride, in a solvent such as a halogenated hydrocarbon, for example 1,2-dichloroethane, or an alcohol such as ethanol, where necessary in the presence of an acid such as acetic acid at around ambient temperature. Secondary amine (-NH-) groups may be similarly alkylated employing an aldehyde.

In a further example, primary amine or secondary amine groups may be converted into amide groups (-NHCOR' or -NRCOR') by acylation. Acylation may be achieved by reaction with an appropriate acid chloride in the presence of a base, such as triethylamine, in a suitable solvent, such as dichloromethane, or by reaction with an appropriate carboxylic acid in the presence of a suitable coupling agent such HATU (O-(7-azabenzotriazol-l-yl)-N,N,N'N'-tetramethylurhexafluorophosphate) in a suitable solvent such as dichloromethane. Similarly, amine groups may be converted into sulphonamide groups (-NHSO₂R' or NR"SO₂R') groups by reaction with an appropriate sulphonyl chloride in the presence of a suitable base, such as triethylamine, in a suitable solvent such as dichloromethane. Primary or secondary amine groups can be converted into urea groups (-NHCONR'R" or -NRCONR'R") by reaction with an appropriate isocyanate in the presence of a suitable base such as triethylamine, in a suitable solvent, such as dichloromethane.

An amine (-NH₂) may be obtained by reduction of a nitro (-NO₂ group, for example by catalytic hydrogenation, using for example hydrogen in the presence of a metal catalyst, for example palladium on a support such as carbon in a solvent such as ethyl acetate or an alcohol e.g. methanol. Alternatively, the transformation may be carried out by chemical reduction using for example a metal, e.g. tin or iron, in the presence of an acid such as hydrochloric acid.

In a further example, amine (-CH₂NH₂) groups may be obtained by reduction of nitriles (-CN), for example by catalytic hydrogenation using for example hydrogen in the presence of a metal catalyst, for example palladium on a support such as carbon, or Raney nickel, in a solvent such as an ether e.g. a cyclic ether such as tetrahydrofuran, at a temperature from -78°C to the reflux temperature of the solvent.

In a further example, amine (-NH₂) groups may be obtained from carboxylic acid groups (-CO₂H by conversion to the corresponding acyl azide (-CON₃), Curtius rearrangement and hydrolysis of the resultant isocyanate (-N=C=O).

Aldehyde groups (-CHO) may be converted to amine groups (-CH₂NR'R")) by reductive amination employing an amine and a borohydride, for example sodium triacetoxyborohydride or sodium cyanoborohydride, in a solvent such as a halogenated hydrocarbon, for example dichloromethane, or an alcohol such as ethanol, where necessary in the presence of an acid such as acetic acid at around ambient temperature.

In a further example, aldehyde groups may be converted into alkenyl groups (-CH=CHR') by the use of a Wittig or Wadsworth-Emmons reaction using an appropriate phosphorane or phosphonate under standard conditions known to those skilled in the art.

Aldehyde groups may be obtained by reduction of ester groups (such as - CO₂Et) or nitriles (-CN) using diisobutylaluminium hydride in a suitable solvent such as toluene. Alternatively, aldehyde groups may be obtained by the oxidation of alcohol groups using any suitable oxidising agent known to those skilled in the art.

Ester groups (-CO₂R') may be converted into the corresponding acid group (-CO₂H) by acid- or base-catalused hydrolysis, depending on the nature of R. If R is t-butyl, acid-catalysed hydrolysis can be achieved for example by treatment with an organic acid such as trifluoroacetic acid in an aqueous solvent, or by treatment with an inorganic acid such as hydrochloric acid in an aqueous solvent.

Carboxylic acid groups (-CO₂H) may be converted into amides (CONHR' or - CONR'R") by reaction with an appropriate amine in the presence of a suitable coupling agent, such as HATU, in a suitable solvent such as dichloromethane.

In a further example, carboxylic acids may be homologated by one carbon (i.e -CO₂H to -CH₂CO₂H) by conversion to the corresponding acid chloride (-COCl) followed by Arndt-Eistert synthesis.

In a further example, -OH groups may be generated from the corresponding ester (e.g. -CO₂R'), or aldehyde (-CHO) by reduction, using for example a complex metal hydride such as lithium aluminium hydride in diethyl ether or tetrahydrofuran, or sodium borohydride in a solvent such as methanol. Alternatively, an alcohol may be prepared by reduction of the corresponding acid (-CO₂H), using for example lithium aluminium hydride in a solvent such as tetrahydrofuran, or by using borane in a solvent such as tetrahydrofuran.

Alcohol groups may be converted into leaving groups, such as halogen atoms or sulfonyloxy groups such as an alkylsulfonyloxy, e.g. trifluoromethylsulfonyloxy or arylsulfonyloxy, e.g. p-toluenesulfonyloxy group using conditions known to those skilled in the art. For example, an alcohol may be reacted with thioyl chloride in a halogenated hydrocarbon (e.g. dichloromethane) to yield the corresponding chloride. A base (e.g. triethylamine) may also be used in the reaction.

In another example, alcohol, phenol or amide groups may be alkylated by coupling a phenol or amide with an alcohol in a solvent such as tetrahydrofuran in the presence of a phosphine, e.g. triphenylphosphine and an activator such as diethyl-, diisopropyl, or dimethylazodicarboxylate. Alternatively alkylation may be achieved by deprotonation using a suitable base e.g. sodium hydride followed by subsequent addition of an alkylating agent, such as an alkyl halide.

Aromatic halogen substituents in the compounds may be subjected to halogen-metal exchange by treatment with a base, for example a lithium base such as n-butyl or t-butyl lithium, optionally at a low temperature, e.g. around -78°C, in a solvent such as tetrahydrofuran, and then quenched with an electrophile to introduce a desired substituent. Thus, for example, a formyl group may be introduced by using N,N-dimethylformamide as the electrophile. Aromatic halogen substituents may alternatively be subjected to metal (e.g. palladium or copper) catalysed reactions, to introduce, for example, acid, ester, cyano, amide, aryl, heteraryl, alkenyl, alkynyl, thio- or amino substituents. Suitable procedures which may be employed include those described by Heck, Suzuki, Stille, Buchwald or Hartwig.

Aromatic halogen substituents may also undergo nucleophilic displacement following reaction with an appropriate nucleophile such as an amine or an alcohol. Advantageously, such a reaction may be carried out at elevated temperature in the presence of microwave irradiation.

The compounds of the present invention are tested for their capacity to inhibit MEK activity and activation (primary assays) and for their biological effects on growing cells (secondary assays) as described below. The compounds having IC₅₀ of less than 10 µM (more preferably less than 5 µM,even more preferably less than 1 µM, most preferably less than 0.5 µM) in the MEK activity assay of Example 1a or 1b, IC₅₀ of less than 5 µM (more preferably less than 0.1 µM, most preferably less than 0.01 µM) in the MEK activation assay of Example 2, EC₅₀ of less than 10 µM (more preferably less than 5 µM, most preferably less than 0.5 µM) in the cell proliferation assay of Example 3, and/or EC₅₀ of less than 10 µM (more preferably less than 1 µM, most preferably less than 0.1 µM in the ERK phosphorylation assay of Example 4, are useful as MEK inhibitors.

The present invention includes a composition (e.g., a pharmaceutical composition) comprising a compound of Formula I (and/or solvates and salts thereof) and a carrier (a pharmaceutically acceptable carrier). The present invention also includes a composition (e.g., a pharmaceutical composition) comprising a compound of Formula I (and/or solvates and salts thereof) and a carrier (a pharmaceutically acceptable carrier), further comprising a second chemotherapeutic agent and/or a second anti-inflammatory agent such as those described herein. The present compositions are useful for inhibiting abnormal cell growth or treating a hyperproliferative disorder in a mammal (e.g., human). The present compositions are also useful for treating inflammatory diseases in a mammal (e.g., human).

The present compounds and compositions are also useful for treating an autoimmune disease, destructive bone disorder, proliferative disorders, infectious disease, viral disease, fibrotic disease or neurodegenerative disease in a mammal (e.g., human). Examples of such diseases/disorders include, but are not limited to, diabetes and diabetic complications, diabetic retinopathy, retinopathy of prematurity, age-related macular degeneration, hemangioma, idiopathic pulmonary fibrosis, rhinitis and atopic dermatitis, renal disease and renal failure, polycystic kidney disease, congestive heart failure, neurofibromatosis, organ transplant rejection, cachexia, stroke, septic shock, heart failure, organ transplant rejection, Alzheimer's disease, chronic or neuropathic pain, and viral infections such as HIV, hepatitis (B) virus (HBV), human papilloma virus (HPV), cytomegalovirus (CMV), and Epstein-Barr virus (EBV). Chronic pain, for purposes of the present invention includes, but is not limited to, idiopathic pain, and pain associated with chronic alcoholism, vitamin deficiency, uremia, hypothyroidism, inflammation, arthritis, and post-operative pain. Neuropathic pain is associated with numerous conditions which include, but arc not limited to, inflammation, postoperative pain, phantom limb pain, burn pain, gout, trigeminal neuralgia, acute herpetic and postherpetic pain, causalgia, diabetic neuropathy, plexus avulsion, neuroma, vasculitis, viral infection, crush injury, constriction injury, tissue injury, limb amputation, arthritis pain, and nerve injury between the peripheral nervous system and the central nervous system.

The present compounds and compositions are also useful for treating pancreatitis or kidney disease (including proliferative glomerulonephritis and diabetes-induced renal disease) in a mammal (e.g., human).

The present compounds and compositions are also useful for the prevention of blastocyte implantation in a mammal (e.g., human).

The compounds of the invention are useful in method of inhibiting abnormal cell growth or treating a hyperproliferative disorder in a mammal (e.g., human) comprising administering to said mammal a therapeutically effective amount of a compound of Formula I (and/or solvates and salts thereof) or a composition thereof. Also included in the uses of the compounds of the invention is a method of treating an inflammatory disease in a mammal (e.g., human) comprising administering to said mammal a therapeutically effective amount of a compound of Formula I (and/or solvates and/or salts thereof) or a composition thereof.

The compounds of the present invention are useful in inbiting abnormal cell growth or treating a hyperproliferative disorder in a mammal (e.g., human) comprising administering to said mammal a therapeutically effetive amount of a compound of Formula I (and/or solvates and salts thereof) or a composition thereof, in combination with a second chemotherapeutic agent such as those described herein. The compoundes of the present invention are also useful in a method of treating an inflammatory disease in a mammal (e.g., human) comprising administering to said mammal a therapeutically effective amount of a compound of Formula I (and/or solvates and/or salts thereof) or a composition thereof, in combination with a second anti-inflammatory agent such as those described herein.

The compounds of the present invention are useful in method of treating an autoimmune disease, destructive bone disorder, proliferative disorders, infectious disease, viral disease, fibrotic disease or neurodegenerative disease in a mammal (e.g., human) comprising administering to said mammal a therapeutically effective amount of a compound of Formula I (and/or solvates and salts thereof) or a composition thereof, and optionally further comprising a second therapeutic agent, Examples of such diseases/disorders include, but arc not limited to, diabetes and diabetic complications, diabetic retinopathy, retinopathy of prematurity, age-related macular degeneration, hemangioma, idiopathic pulmonary fibrosis, rhinitis and atopic dermatitis, renal disease and renal failure, polycystic kidney disease, congestive heart failure, neurofibromatosis, organ transplant rejection, cachexia, stroke, septic shock, heart failure, organ transplant rejection, Alzheimer's disease, chronic or neuropathic pain, and viral infections such as HIV, hepatitis (B) virus (HBV), human papilloma virus (HPV), cytomegalovirus (CMV), and Epstein-Barr virus (EBV).

The compounds of the present invention are useful in a method of treating pancreatitis or kidney disease (including proliferative glomerulonephritis and diabetes-induced renal disease) in a mammal (e.g., human) comprising administering to said mammal a therapeutically effective amount of a compound of Formula I (and/or solvates and salts thereof) or a composition thereof, and optionally further comprising a second therapeutic agent.

The compounds of the present invention are useful in a method for preventing of blastocyte implantation in a mammal (e.g., human) comprising administering to said mammal a therapeutically effective amount of a compound of Formula I (and/or solvates and salts thereof) or a composition thereof, and optionally further comprising a second therapeutic agent.

The present invention includes the present compounds for use in *in vitro, in situ,* and *in vivo* methods of or treastment of mammalian cells, organisms, or associated pathological conditions.

It is also believed that the compounds of the present invention can render abnormal cells more sensitive to treatment with radiation for purposes of killing and/or inhibiting the growth of such cells. Accordingly compounds of this invention are also useful in a method for sensitizing abnormal cells in a mammal (e.g., human) to treatment with radiation which comprises administering to said mammal an amount of a compound of Formula I (and/or solvates and salts thereof) or a composition thereof, which amount is effective is sensitizing abnormal cells to treatment with radiation.

Administration of the compounds of the present invention (hereinafter the "active compound(s)") can be effected by any method that enables delivery of the compounds to the site of action, These methods include oral routes, intraduodenal routes, parenteral injection (including intravenous, subcutaneous, intramuscular, inttravascular or infusion), topical, inhalation and rectal administration.

The amount of the active compound administered will be dependent on the subject being treated, the severity of the disorder or condition, the rate of administration, the disposition of the compound and the discretion of the prescribing physician. However, an effective dosage is in the range of about 0.001 to about 100 mg per kg body weight per day, preferably about 1 to about 35 mg/kg/day, in single or divided doses. For a 70 kg human, this would amount to about 0.05 to 7 g/day, preferably about 0.05 to about 2.5 g/day. In some instances, dosage levels below the lower limit of the aforesaid range may be more than adequate, while in other cases still larger doses may be employed without causing any harmful side effect, provided that such larger doses are first divided into several small doses for administration throughout the day.

The active compound may be applied as a sole therapy or in combination with one or more chemotherapeutic agents, for example those described herein. Such conjoint treatment may be achieved by way of the simultaneous, sequential or separate dosing of the individual components of treatment.

The pharmaceutical composition may, for example, be in a form suitable for oral administration as a tablet, capsule, pill, powder, sustained release formulations, solution, suspension, for parenteral injection as a sterile solution, suspension or emulsion, for topical administration as an ointment or cream or for rectal administration as a suppository. The pharmaceutical composition may be in unit dosage forms suitable for single administration of precise dosages. The pharmaceutical composition will include a conventional pharmaceutical carrier or excipient and a compound according to the invention as an active ingredient. In addition, it may include other medicinal or pharmaceutical agents, carriers, adjuvants, etc.

Exemplary parenteral administration forms include solutions or suspensions of active compounds in sterile aqueous solutions, for example, aqueous propylene glycol or dextrose solutions. Such dosage forms can be suitably buffered, if desired.

Suitable pharmaceutical carriers include inert diluents or fillers, water and various organic solvents. The pharmaceutical compositions may, if desired, contain additional ingredients such as flavorings, binders, excipients and the like. Thus for oral administration, tablets containing various excipients, such as citric acid may be employed together with various disintegrants such as starch, alginic acid and certain complex silicates and with binding agents such as sucrose, gelatin and acacia. Additionally, lubricating agents such as magnesium stearate, sodium lauryl sulfate and talc are often useful for tableting purposes. Solid compositions of a similar type may also be employed in soft and hard filled gelatin capsules. Preferred materials, therefore, include lactose or milk sugar and high molecular weight polyethylene glycols. When aqueous suspensions or elixirs are desired for oral administration the active compound therein may be combined with various sweetening or flavoring agents, coloring matters or dyes and, if desired, emulsifying agents or suspending agents, together with diluents such as water, ethanol, propylene glycol, glycerin, or combinations thereof.

Methods of preparing various pharmaceutical compositions with a specific amount of active compound are known, or will be apparent, to those skilled in this art. For examples, see Remington's Pharmaceutical Sciences, Mack Publishing Company, Ester, Pa., 15.sup.th Edition (1975).

### EXAMPLES

### Abbreviations

- DBU: 1,8-Diazabicyclo[5.4.0]undec-7-ene
- DCM: Dichloromethane
- DIAD: diisopropyl azodicarboxylate
- DIPEA: Diisopropylethylamine
- DMAP: 4-Dimethylaminopyridine
- DMF: Dimethylformamide
- EDCI: 1-Ethyl-3-(3'-dimethylaminopropyl)carbodiimide
- HATU: O-(7-Azabenzotriazol-1-yl)-N,N,N',N'-tetramethyluronium hexafluorophosphate
- HCl: Hydrochloric acid
- HM-N: Isolate® HM-N is a modified form of diatomaceous earth that can efficiently absorb aqueous samples
- HOBt: 1-Hydroxybenzotriazole
- IMS: industrial methylated spirits
- ICI: iodine monochloride
- LDA: Lithium diisopropylamide
- MeOH: Methanol
- NaHC0₃: Sodium bicarbonate
- NaOH: Sodium hydroxide
- Pd(PPh₃)₄: Tetrakis(triphenylphosphine)palladium(0)
- Pd₂dba₃: Tris-(dibenzylideneacetone)dipalladium(0)
- Si-SPE: Pre-packed Isolute® silica flash chromatography cartridge
- Si-ISCO: Pre-packed ISCO® silica flash chromatography cartridge
- THF: Tetrahydrofuran
- Xantphos: 9,9-Dimethyl-4,5-bis(diphenylphosphino)xanthene

General Experimental Conditions

¹H NMR spectra were recorded at ambient temperature using a Varian Unity Inova (400MHz) spectrometer with a triple resonance 5mm probe. Chemical shifts are expressed in ppm relative to tetramethylsilane. The following abbreviations have been used: br = broad signal, s = singlet, d = doublet, dd = double doublet, t = triplet, q = quartet, m = multiplet.

High Pressure Liquid Chromatography - Mass Spectrometry (LCMS) experiments to determine retention times (R_{T}) and associated mass ions were performed using one of the following methods.

Method A: Experiments performed on a Waters Micromass ZQ quadrupole mass spectrometer linked to a Hewlett Packard HP 1100 LC system with diode array detector. This system uses a Higgins Clipeus 5micron C18 100 x 3.0mm column and a 1 ml / minute flow rate. The initial solvent system was 95% water containing 0.1% formic acid (solvent A) and 5% acetonitrile containing 0.1% formic acid (solvent B) for the first minute followed by a gradient up to 5% solvent A and 95% solvent B over the next 14 minutes. The final solvent system was held constant for a further 5 minutes.

Method B: Experiments performed on a Waters Platform LC quadrupole mass spectrometer linked to a Hewlett Packard HP1100 LC system with diode array detector and 100 position autosampler using a Phenomenex Luna C18(2) 30 x 4.6mm column and a 2 ml / minute flow rate. The solvent system was 95% water containing 0.1% formic acid (solvent A) and 5% acetonitrile containing 0.1% formic acid (solvent B) for the first 0.50 minutes followed by a gradient up to 5% solvent A and 95% solvent B over the next 4 minutes. The final solvent system was held constant for a further 0.50 minutes.

Method C: Experiments performed on a PE Sciex API 150 EX quadrupole mass spectrometer linked to a Shimadzu LC-10AD LC system with diode array detector and 225 position autosampler using a Kromasil C18 50 x 4.6mm column and a 3 ml / minute flow rate. The solvent system was a gradient starting with 100% water with 0.05% TFA (solvent A) and 0% acetonitrile with 0.0375% TFA (solvent B), ramping up to 10% solvent A and 90% solvent B over 4 minutes. The final solvent system was held constant for a further 0.50 minutes.

Method D: Experiments performed on a Shimadzu LCMS-2010A liquid chromatography mass spectrometer linked to a Shimadzu LC-10AD VP LC system with diode array detector. Uses a Kromasil 100 5micron C18 50 x 4.6 mm column and a 2.5 ml / minute flow rate. The initial solvent system was 100% water containing 0.05% trifluoroacetic acid (solvent A) and 0% acetonitrile containing 0.05% trifluoroacetic acid (solvent B), followed by a gradient up to 10% solvent A and 90% solvent B over 8 minutes. The final solvent system was held constant for a further 2 minutes.

Method E: Experiments performed on an Agilent Technologies liquid chromatography mass spectrometer linked to an Agilent Technologies Series 1100 LC system with diode array detector. Uses a Zorbax 3.micron SB-C18 30 x 2.6 mm column and a 0.5 ml / minute flow rate. The initial solvent system was 95% water containing 0.05% trifluoroacetic acid (solvent A) and 5% acetonitrile containing 0.0375% trifluoroacetic acid (solvent B), followed by a gradient up to 5% solvent A and 95% solvent B over 9 minutes. The final solvent system was held constant for a further 1 minute.

Microwave experiments were carried out using a Personal Chemistry Emrys Iniatiator™ or Optimizer™, which uses a single-mode resonator and dynamic field tuning, both of which give reproducibility and control. Temperature from 40-250°C can be achieved, and pressures of up to 20bar can be reached.

**EXAMPLE 1a** MEK Assay (MEK activity assay)

Constitutively activated human mutant MEK1 expressed in insect cells is used as source of enzymatic activity at a final concentration in the kinase assay of 62.5nM.

The assay is carried out for 30 minutes in the presence of 50µM ATP using recombinant GST-ERK1 produced in *E.Coli* as substrate. Phosphorylation of the substrate is detected and quantified using HTRF reagents supplied by Cisbio. These consist of an anti-GST antibody conjugated to allophycocyanin (XL665) and an anti-phospho (Thr202/Tyr204) ERK antibody conjugated to europium-cryptate. The anti-phospho antibody recognises ERK1 dually phosphorylated on Thr202 and Tyr204. When both antibodies are bound to ERK1 (i.e. when the substrate is phosphorylated), energy transfer from the cryptate to the allophycocyanin occurs following excitation at 340nm, resulting in fluorescence being emitted that is proportional to the amount of phosphorylated substrate produced. Fluorescence is detected using a multiwell fluorimeter.

Compounds are diluted in DMSO prior to addition to assay buffer and the final DMSO concentration in the assay is 1%.

The IC₅₀ is defined as the concentration at which a given compound achieves 50% inhibition of control. IC₅₀ values are calculated using the XLfit software package (version 2.0.5).

**EXAMPLE 1b** MEK Assay (MEK activity assay)

Constitutively activated human mutant MEK1 expressed in insect cells is used as source of enzymatic activity at a final concentration in the kinase assay of 15nM.

The assay is carried out for 30 minutes in the presence of 50µM ATP using recombinant GST-ERK1 produced in *E.Coli* as substrate. Phosphorylation of the substrate is detected and quantified using HTRF reagents supplied by Cisbio. These consist of an anti-GST antibody conjugated to allophycocyanin (XL665) and an anti-phospho (Thr202/Tyr204) ERK antibody conjugated to europium-cryptate. These are used at a final concentration of 4µg/ml and 0.84µg/ml respectively. The anti-phospho antibody recognises ERK1 dually phosphorylated on Thr202 and Tyr204. When both antibodies are bound to ERK1 (i.e. when the substrate is phosphorylated), energy transfer from the cryptate to the allophycocyanin occurs following excitation at 340nm, resulting in fluorescence being emitted that is proportional to the amount of phosphorylated substrate produced. Fluorescence is detected using a multiwell fluorimeter.

Compounds are diluted in DMSO prior to addition to assay buffer and the final DMSO concentration in the assay is 1%.

The IC₅₀ is defined as the concentration at which a given compound achieves 50% inhibition of control. IC₅₀ values are calculated using the XLfit software package (version 2.0.5).

Compounds of Example 5-18, 20-102, 105-109, 111-118, 120-133, 136-149 and 151-160 exhibited an IC₅₀ of less than 10 µM in the assay described either in Example 1a or 1b, most of these compounds exhibited an IC₅₀ of less than 5 µM.

**EXAMPLE 2** bRaf Assay (MEK activation assay)

Constitutively activated bRaf mutant expressed in insect cells is used as source of enzymatic activity.

The assay is carried out for 30 minutes in the presence of 200µM ATP using recombinant GST-MEK1 produced in *E.Coli* as substrate. Phosphorylation of the substrate is detected and quantified using HTRF, and reagents are supplied by Cisbio. These consist of an anti-GST antibody conjugated to allophycocyanin (XL665) and an anti-phospho (Ser217/Ser221) MEK antibody conjugated to europium-cryptate. The anti-phospho antibody recognises MEK dually phosphorylated on Ser217 and Ser221 or singly phosphorylated on Ser217. When both antibodies are bound to MEK (i.e. when the substrate is phosphorylated), energy transfer from the cryptate to the allophycocyanin occurs following excitation at 340nm, resulting in fluorescence being emitted that is proportional to the amount of phosphorylated substrate produced. Fluorescence is detected using a multiwell fluorimeter.

Compounds are diluted in DMSO prior to addition to assay buffer and the final DMSO concentration in the assay is 1%.

The IC₅₀ is defined as the concentration at which a given compound achieves 50% inhibition of control. IC₅₀ values are calculated using the XLfit software package (version 2.0.5).

In this assay, compounds of Example 5-19 exhibited an IC₅₀ of less than 5 µM.

**EXAMPLE 3** Cell Proliferation Assay

Compounds are tested in a cell proliferation assay using the following cell lines:

HCT116 human colorectal carcinoma (ATCC)

A375 human malignant melanoma (ATCC)

Both cell lines are maintained in DMEM/F12 (1:1) media (Gibco) supplemented with 10% FCS at 37°C in a 5% CO₂ humidified incubator.

Cells are seeded in 96-well plates at 2,000 cells/well and after 24 hours they are exposed to different concentrations of compounds in 0.83% DMSO. Cells are grown for a further 72h, and an equal volume of CellTiter-Glo reagent (Promega) is added to each well. This lyses the cells and generates a luminescent signal proportional to the amount of ATP released (and therefore proportional to the number of cells in the well) that can be detected using a multiwell luminometer.

The EC₅₀ is defined as the concentration at which a given compound achieves 50% inhibition of control. EC₅₀ values are calculated using the XLfit software package (version 2.0.5).

In this assay, compounds of Example 5-13, 15-16, 18, 20-22, 24-25, 28, 31, 35, 38-39, 41, 109, 133-134, 138, 140-141 and 160 exhibited an EC₅₀ of less than 10 µM in either one of the cell lines.

**EXAMPLE 4** Phospho-ERK Cell-Based Assay

Compounds are tested in a cell-based phospho-ERK ELISA using the following cell lines:

HCT116 human colorectal carcinoma (ATCC)

A375 human malignant melanoma (ATCC)

Both cell lines are maintained in DMEM/F12 (1:1) media (Gibco) supplemented with 10% FCS at 37°C in a 5% CO₂ humidified incubator.

Cells are seeded in 96-well plates at 2,000 cells/well and after 24h they are exposed to different concentrations of compounds in 0.83% DMSO. Cells are grown for a further 2 h or 24h, fixed with formaldehyde (2% final) and permeabilised with methanol. Following blocking with TBST-3% BSA, fixed cells are incubated with primary antibody (anti-phospho ERK from rabbit) over-night at 4°C. Cells are incubated with Propidium Iodide (DNA fluorescent dye) and detection of cellular p-ERK is performed using an anti-rabbit secondary antibody conjugated to the fluorescent Alexa Fluor 488 dye (Molecular probes The fluorescence is analysed using the Acumen Explorer (TTP Labtech), a laser-scanning microplate cytometer, and the Alexa Fluor 488 signal is normalised to the PI signal (proportional to cell number).

The EC₅₀ is defined as the concentration at which a given compound achieves a signal half way between the baseline and the maximum response. EC₅₀ values are calculated using the XLfit software package (version 2.0.5).

In this assay, compounds of Example 5-13, 15-16, 18, 20-26, 28-29, 31, 35, 38-39, 41-48, 50,55, 59-61, 68, 70, 73-74, 76, 79, 81-84, 87, 91, 95, 99, 109, 111, 113, 117, 118, 120, 122-124, 126-127, 131, 133, 134, 138-141, 144, 147, 152, and 155-160 exhibited an EC₅₀ of less than 10 µM in either one of the cell lines.

SYNTHESIS OF AZABENZOFURANYL CORES

Ethyl 3-(4-Bromo-2-fluoro-phenylamino)-furo[3,2-c]pyridine-2-carboxylate

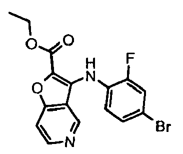

Step1: 4-Chloro-nicotinic acid

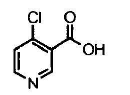

Following the procedures of Guillier et al (1995) J. Org. Chem. 60(2):292-6, to a cold (-78°C) solution of LDA (21 ml, 1.6 M in hexanes, 33.3 mmol) in anhydrous THF (70 ml) was added 4-chloropyridine (5.0 g, 33.3 mmol) under an argon atmosphere. After 1 hour at -78°C, the solution was rapidly poured onto a bed of solid CO₂ contained within a 250 ml conical flask. After allowing to warm to ambient temperature the solution was quenched with water (30 ml). The volatile organic solvents were removed *in vacuo* and the remaining aqueous suspension was extracted with diethyl ether (3 x 100 ml). The aqueous phase was cooled to 0°C and then adjusted to pH 4 by the addition of concentrated hydrochloric acid. The resultant precipitate was aged for 30 minutes then collected by filtration. The solid was washed with cold diethyl ether (10 ml) to afford the title compound as a white solid (3.2 g, 61%).

Step 2: Ethyl 4-chloro-nicotinate

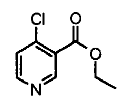

A suspension of 4-chloro-nicotinic acid (3.0 g, 19.0 mmol) in thionyl chloride (50 ml) was heated under reflux for 90 minutes. After cooling to ambient temperature, the solution was concentrated to dryness and then azeotroped with toluene (2 x 50 ml) to afford a solid. The resultant solid was added in portions to a cooled (0°C) solution of ethanol (25 ml) and DIPEA (15 ml). The reaction was stirred at room temperature for 4 hours then concentrated *in vacuo* before water (75 ml) was added. The solution was extracted with ethyl acetate (2 x 75 ml) then the combined organic phases were dried over sodium sulfate then concentrated to give the title compound as a brown oil (3.3 g,94%). 1H NMR (CDCl_{3,} 400MHz) 9.03 (s, 1H), 7.58 (d, J = 5.4 Hz, 1H), 7.41 (dd, J = 5.4 Hz, 0.5 Hz, 1H), 4.45 (q, J = 7.3 Hz, 2H), 1.43 (t, J = 7.3 Hz, 3H).

Step 3: Ethyl 3-hydroxy-furo[3,2-clpyridine-2-carboxylate

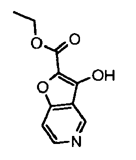

To a cooled (0 °C) solution of ethyl 4-chloro-nicotinate (910 mg, 4.9 mmol) and ethyl glycolate (0.48 ml, 5.1 mmol) in anhydrous DMF (17 ml), under a nitrogen atmosphere, was added sodium hydride (9.8 mmol, 60%, 392 mg). The reaction mixture was stirred for 16 hours (0 °C to room temperature), then acidified by the addition of acetic acid (1.2 ml), and subsequently concentrated to provide a residue. Water (23 ml) was added, and the mixture was stirred for 5 minutes, at which time the resulting brown precipitate was collected by filtration and washed with water 3 x 30 mL to give the title compound as a light brown solid. (875 mg, 86%). 1H NMR (DMSO-D₆, 400MHz) 9.18 (d, J = 1.2 Hz, 1H), 8.60 (d, J = 6.0 Hz, 1H), 7.66 (dd, J = 6.0 Hz, 0.8 Hz, 1H), 4.32 (q, J = 7.2 Hz, 2H), 1.32 (t, J = 7.2 Hz, 3H). LCMS (method B): R_{T} = 1.42 min, M+H⁺ = 208.

Step 4: Ethyl 3-(trifluoromethanesulfonyloxy)-furo[3.2-c]pyriding-2-carboxylate

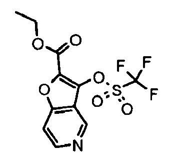

A stirred solution of ethyl 3-hydroxy-furo[3,2-c]pyridine-2-carboxylate (3.15 g, 15.204 mmol), *N,N*-bis(trifluoromethylsulfonyl)aniline (10.08 g, 28.24 mmol) and *N,N-*diisopropylethylamine (11.35 ml, 65.16 mmol) in dimethoxyethane (50 ml) was heated at 95°C for 35 minutes. The reaction was then cooled to room temperature and concentrated under reduced pressure. The residue was then purified by flash chromatography (silica, 120 g column, ISCO, 45 mL/Min, 0-60% Ethyl acetate in hexane in 20 minutes) to afford the title compound as a pale yellow oil / white waxy solid (4.11 g, 79.7%). 1H NMR (CDCl₃, 400MHz) 9.07 (s, 1H), 8.75 (d, 1H), 7.59 (d, 1H), 4.54 (q, 2H), 1.47 (t, 3H). LCMS (5 min., method 2): R_{T} = 2.93 min, M+H⁺ = 339.6.

Step 5: Ethyl 3-(4-Bromo-2-fluoro-phenylamino)-furo[3,2-c]pyridine-2-carboxylate

A suspension of ethyl 3-(trifluoromethanesulfonyloxy)-furo[3,2-c]pyridine-2-carboxylate (4.11 g, 12.11 mmol), 4-bromo-2-fluoroaniline (3.76 g, 19.38 mmol), Pd₂dba₃ (925 mg, 1.01 mmol), Xantphos (591 mg, 1.02 mmol) and K₃PO₄ (4.95 g, 22.61 mmol) in toluene (60 ml) was degassed with bubbling nitrogen for 10 minutes and then heated to 105°C for 24 hours. The reaction mixture was then cooled to room temperature and diluted with ethyl acetate (100 ml). The resultant mixture was then filtered through celite 545 and the celite was washed with an additional 50 ml ethyl acetate. The filtrate was then concentrated and purified by flash chromatography (silica, 120 g column, ISCO, 45 mL/Min, 0-70% Ethyl acetate in hexane in 40 minutes) to afford the title compound as a white solid (2.96 g, 64.5%). 1H NMR (CDC1₃, 400MHz) 8.60 (m, 2H), 7.66 (s, 1H), 7.50 (d,m 1H), 7.39 (d,d, 1H), 7.30 (d,m, 1H), 7.16 (t, 1H), 4.49 (q, 2H), 1.47 (t, 3H). LCMS (5 min., method 2): R_{T} = 2.47 min, M+H⁺ = 378.9.

Ethyl 3-(2-fluoro-4-iodo-phenylamino)-furo[2,3-c]pyridine-2-carboxylate

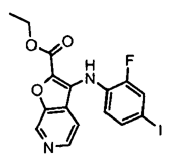

Step 1: 3-Amino-isonicotinic acid

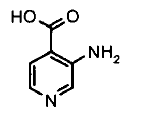

Following the procedures of Zhou et al (2001) Bioorg. Med. Chem. Lett. 9(8):2061-2071, bromine (1.22 ml, 23.9 mmol) was slowly added to a cooled (5°C) 2.5N solution of NaOH (60 ml, 150 mmol) and after stirring for 5 minutes pyrrolo[3,4-c]pyridine-1,3-dione (3.5 g, 23.6 mmol) was added. The temperature was raised to 80°C and the mixture was stirred for 1 hour before being cooled to ambient temperature. Acetic acid (5.9 ml, 98.3 mmol) was cautiously added (N.B.: gas evolution) and the solution stirred for 10 minutes whereby a precipitate formed that was collected by filtration. The solid was washed with water (20 ml) and MeOH (20 ml) then dried to afford the title compound as a yellow solid (2.1 g, 64%).

Step 2: 3-Hydroxy-isonicotinic acid

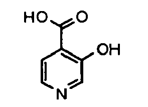

To a suspension of 3-amino-isonicotinic acid (2.1 g, 15.2 mmol) in water (35 ml) was added concentrated sulphuric acid (1.5 ml). The solution was cooled to 5°C and vigorously stirred before a solution of sodium nitrite (1.05 g, 15.2 mmol) in water (10 ml) was added. The suspension was slowly heated to 80°C and held at this temperature for 15 minutes, then cooled to 65°C and acetic acid (1.5 ml) was added. The pH of the solution was adjusted to pH 4.5 by the addition of concentrated ammonia solution (approximately 3.5 ml) then the mixture was placed in the refrigerator overnight. The resultant precipitate was collected by filtration, washed with water (20 ml) and dried under vacuum to afford the title compound as a yellow solid (1.85 g, 88%). 1H NMR (d₄-MeOH, 400 MHz) 8.37 (s, 1H), 8.09 (d, J = 5.5 Hz, 1H), 7.81 (d, J = 5.5 Hz, 1H).

Step 3: Ethyl 3-hydroxy-isonicotinate

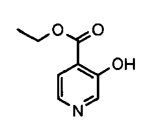

3-Hydroxy-isonicotinic acid (1.83 g, 13.2 mmol) was heated at reflux for 48 hours in a mixture of ethanol (40 ml) and concentrated sulphuric acid (1.0 ml). The mixture was cooled to room temperature and concentrated to give a residue. The residue was dissolved in water (10 ml) and neutralised by the addition of NaHCO₃ (approximately 2 g). The organic components were extracted with DCM (3 x 20 ml), and the combined organic extracts were dried over magnesium sulfate and concentrated to afford the title compound as a yellow oil that solidified on standing (1.87 g, 85%). 1H NMR (d₄-MeOH, 400 MHz) 10.40 (s, 1H), 8.49 (s, 1H), 8.21 (d, J = 5.20 Hz, 1H), 7.62 (d, J = 5.20 Hz, 1H), 4.47 (q, J = 6.44 Hz, 2H), 1.45 (t, J = 6.44 Hz, 3H).

Step 4: Ethyl 3-ethoxycarbonylmethoxy-isonicotinate

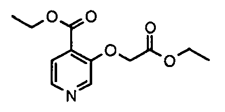

To a cold (5 °C) solution of ethyl 3-hydroxy-isonicotinate (1.67 g, 1.0 mmol), ethyl glycolate (1.15 ml, 12.0 mmol) and triphenylphosphine (3.93 g, 15.0 mmol) in anhydrous THF (50 ml) was added diisopropylazodicarboxylate (2.94 ml, 15.0 mmol) dropwise. The reaction mixture was gradually warmed to ambient temperature then stirred for an additional 1 hour. The solution was concentrated and purified by flash chromatography (Si-SPE, pentane: diethyl ether, gradient 50:50 to 0:100) to afford the title compound as a yellow oil (2.15 g, 85%). LCMS (method B): R_{T} = 2.69 min, M+H⁺ = 254.

Step 5: Ethyl 3-hydroxy-furo[2,3-c]pyridine-2-carboxylate

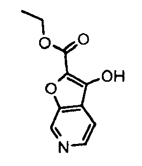

A solution of ethyl 3-ethoxycarbonylmethoxy-isonicotinate (2.1 g. 8.3 mmol) in THF (50 ml) was carefully added to a cold (0°C) solution of potassium tert-butoxide (966 mg, 8.6 mmol) in THF (20 ml). After 30 minutes the reaction mixture was quenched by the addition of acetic acid (10 ml). Evaporation of the solvents afforded a gum that was dissolved in ethyl acetate (50 ml) and washed with water (2 x 10 ml). The organic layer was isolated and dried over anhydrous sodium sulfate. The solution was concentrated to afford the title compound as a yellow solid (1.60 g, 94%). LCMS (method B): R_{T} = 1.89 min, M+H⁺ = 208.

Step 6: Ethyl 3-(nonafluorobutane-1-sulfonyloxy)-furo[2,3-c]pyridine-2-carboxylate

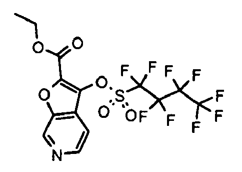

To a stirred suspension of ethyl 3-hydroxy-furo[2,3-c]pyridine-2-carboxylate (1.16 g, 5.60 mmol) in DCM (15 ml) at 0°C was added DIPEA (1.32 ml, 7.5 mmol) followed by nonafluorobutylsulfonyl fluoride (1.25 ml, 6.9 mmol). After 10 minutes the reaction was warmed to room temperature and stirred for an additional 20 hours. The reaction mixture was concentrated, the residue was dissolved in DCM (100 ml) and washed with water (50 ml) followed by 1N NaOH solution (20 ml). The combined organic layer was isolated and dried over sodium sulfate and concentrated *in vacuo.* Purification by flash chromatography (Si-SPE, pentane: diethyl ether, gradient 80:20 to 50:50) afforded the title compound as a white solid (895 mg, 33%). LCMS (method B): R_{T} = 4.34 min, M+H⁺ = 490.

Step 7: Ethyl 3-(4-bromo-2-fluoro-phenylamino)-furo[2,3-c]pyridine-2-carboxylate

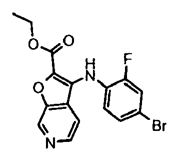

A degassed solution of ethyl 3-(nonafluorobutane-1-sulfonyloxy)-furo[2,3-c]pyridine-2-carboxylate (838 mg, 1.71 mmol), 4-bromo-2-fluoroaniline (423 mg, 2.23 mmol), Pd₂dba₃ (78 mg, 0.09 mmol), Xantphos (99 mg, 0.17 mmol) and DBU (651 µl, 4.28 mmol) in toluene (3.3 ml) was subjected to microwave irradiation at 150°C for 20 minutes. The reaction mixture was concentrated and the resultant residue absorbed onto HM-N before being purified by flash chromatography (Si-SPE, pentane: diethyl ether, gradient 80:20 to 0:100) to afford the title compound as a white solid (369 mg, 57%). LCMS (method B): R_{T} = 3.77 min, M+H⁺ = 380/382.

Step 8: Ethyl 3-(2-fluoro-4-iodo-phenylamino)-furo[2,3-c]pyridine-2-carboxylate

A mixture of ethyl 3-(4-bromo-2-fluoro-phenylamino)-furo[2,3-c]pyridine-2-carboxylate (311 mg, 0.82 mmol), copper(I) iodide (8 mg, 0.04 mmol), sodium iodide (246 mg, 1.64 mmol) and trans-N,N'-dimethyl-1,2-cyclohexane diamine (13 µl, 0.08 mmol) in 1,4-dioxane (0.8 ml) was heated at 115°C for 26 hours under an argon atmosphere. Once the reaction mixture was cooled to room temperature, the mixture was concentrated then purified by flash chromatography (Si-SPE, EtOAc) to afford the title compound as a yellow oil (220 mg, 63%). LCMS (method B): R_{T} = 3.91 min, M+H⁺ = 427.

Ethyl 3-(2-fluoro-4-iodo-phenylamino)-furo[3,2-c]pyridine-2-carboxylate

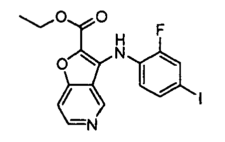

Step 1: 2-Fluoro-4-Trimethylsilanyl-Phenylamine

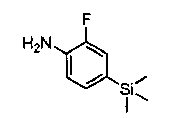

4-Chloro-2-fluoronitrobenzene (6.0g, 34.2 mmol) was added to a 100 mL round bottom flask, followed by hexamethyldisilane (18.9g, 129.0 mmol, 26.4 mL) and xylene (13 mL). The mixture was magnetically stirred while nitrogen was bubbled into the solution via glass pipette for 10 minutes or until the entire solid had dissolved.

Tetrakis(triphenylphosphine)palladium(0) (1.0 g, 0.9 mmol) was added, the flask fitted with a reflux condenser, and the reaction was heated at reflux for 24-48 hours while a slow stream of nitrogen was passed through a rubber septum placed in the top of the condenser. After cooling to room temperature, the reaction mixture was diluted with ethyl ether (40 mL) and filtered through a plug of silica gel (30 mL of SiO₂/ethyl ether slurry packed into a 60 mL fritted glass funnel). The filter cake was washed with ethyl ether (60 mL) and the combined organics were concentrated in vacuo to an orange oil, which was purified by flash chromatography (250 mL silica gel, 98:1:1 hexane-CH₂Cl₂-ethyl ether), yielding the 2-fluoro-4-trimethylsilylnitrobenzene (5.45 g, 75%) as a yellow-orange oil.

The 2-fluoro-4-trimethylsilylnitrobenzene (5.45g, 25.6 mmol) was then dissolved in ethanol (100 mL), transferred to a Parr shaker bottle, flushed with nitrogen, then charged with 10% Pd-C (0.4 g). The reaction mixture was hydrogenated for 1h on the Parr apparatus (45 psi H₂), and then filtered through a plug of Celite. The filter cake was washed with ethanol, and the combined filtrates were concentrated. The resulting residue was purified by flash chromatography (250 mL silica gel, 95:5 hexane-ethyl ether), to afford the title compound as a tan oil (4.31 g, 92%).

Step 2: Ethyl 3-(4-Trimethylsilyl-2-fluoro-phenylamino)-furo[3,2-c]pyridine-2-carboxylate

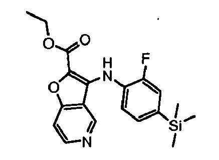

A suspension of ethyl 3-(trifluoromethanesulfonyloxy)-furo[3,2-c]pyridine-2-carboxylate (17.5 g, 51.58 mmol), 2-fluoro-4-trimethylsilanyl-phenylamine (10 g, 54.26 mmol), Pd₂dba₃ (2.98 g, 3.26 mmol), Xantphos (1.94 mg, 3.26 mmol) and K₃PO₄ (15.83 g, 72.34 mmol) in toluene (100 ml) was degassed with bubbling nitrogen for 10 minutes in a 300 mL pressure bottle and then heated to 105°C for 24 hours. The reaction mixture was then cooled to room temperature and diluted with ethyl acetate (200 ml). The resultant mixture was then filtered through celite 545 and the celite was washed with an additional 100 ml ethyl acetate. The filtrate was then concentrated and purified by flash column chromatography (silica, 0-55% Ethyl acetate in hexane) to afford the title compound as a yellow solid (17.9 g, 93.2 %). LCMS (method C): R_{T} = 2.47 min, M+H⁺ = 373. 1H NMR (CDCl₃, 400MHz) 8.66 (d, 1H), 8.57 (d, 1H), 7.52 (s, 1H), 7.45 (d,d, 1H), 7.30 (m, 2H), 4.50 (q, 2H), 1.49 (t, 3H).

Step 3: Ethyl 3-(2-fluoro-4-iodo-phenylaminol-furo[3,2-c]pyridine-2-carboxylate

16.0 g (72.49 mmol) of AgBF₄ was quickly weighed out into a 1000mL round bottom flask and then capped with a rubber septum. The flask was then purged with dry N₂ gas for 10 minutes, after which the flask was cooled to -50°C, while maintaining an inert atmosphere. To this was added 300 ml dry dichloromethane and then the resultant mixture was stirred for 15 minutes at -50°C under nitrogen. To the reaction mixture was then added 9.0 g (24.16 mmol) of ethyl 3-(4-Trimethylsilyl-2-fluoro-phenylamino)-furo[3,2-c]pyridine-2-carboxylate in 75 ml dry dichloromethane and the mixture stirred for 30 mins at -50°C under nitrogen. The color of the reaction was clear yellow. The reaction was then treated with 25 ml IC1 (1.0 M in CH₂Cl₂, 25 mmol) dropwise with stirring over 30 minutes. Addition of IC1 resulted in a precipitate (white/brown, the color of the reaction was yellow - localized red color on contact of IC1 with the reaction, which turned yellow with white ppt). The reaction was stirred at -50°C under nitrogen for 30 minutes. LC/MS showed that the reaction was complete. The reaction was then quenched at -50°C by addition of 200 ml sat. Na₂S₂O₃ solution, followed by 100 ml of water. The mixture was then transferred to a sep. funnel and shaken. The mixture was then filtered through filter paper. The black solid on the filter paper was further rinsed with dichloromethane and then discarded. The filtrate was then transferred to a separatory funnel. This was then quickly extracted with dichloromethane (3 X 100 ml). The combined dichloromethane layers were then washed with 170 mL 4M NH₄OH solution in a sep. funnel. The dichloromethane layer was then separated and bubbled with Nitrogen to remove the ammonia. This was then dried with Magnesium sulfate, filtered and concentrated under reduced pressure to give a yellow solid. The solid was then powdered and triturated with ether (2 X 30 ml) and then dried under vacuum to give 8.90 g of the title product (yellow solid, 86.4%)). LCMS (method C): R_{T} = 2.47 min, M+H⁺ = 427. 1H NMR (CDCl₃, 400MHz) 8.64 (d, 1H), 8.9 (d, 1H), 7.66 (s, 1H), 7.54 (d,d, 1H), 7.46 (d,d,m, 2H), 7.13 (t, 1H), 4.49 (q, 2H), 1.49 (t, 3H).

Ethyl 3-(4-iodo-phenylamino)-furo[3,2-c]pyridine-2-carboxylate

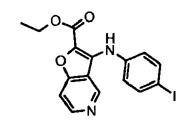

Step 1: Ethyl 3-amino-furo[3,2-c]pyridine-2-carboxylate

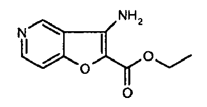

To a stirred mixture of sodium hydride (60% suspension in mineral oil, 6.0 g, 150 mmol) in DMF (160 ml) at -10°C under a nitrogen atmosphere, was added ethyl glycolate (14.5 ml, 150 mmol) over 5 minutes. After 35 minutes the reaction mixture was further cooled to -35°C and a solution of 4-chloronicotinonitrile (6.9 g, 50 mmol) in DMF (40 ml) added over 5 minutes. The reaction mixture was then allowed to warm gradually over 1.5 hours to -5°C, before being quenched with a solution of acetic acid: water (45 ml: 400 ml), and then extracted with ethyl acetate (2 x 200 ml). The separated aqueous phase was basified by addition of solid sodium bicarbonate, and extracted with ethyl acetate (3 x 200 ml). The combined organic extracts were washed with sodium bicarbonate solution (100 ml) and water (2 x100 ml), then the organic phase was isolated, dried (MgSO₄), filtered and evaporated in *vacuo.* Purification of the resultant residue by flash chromatography (Si-SPE, cyclohexane: ethyl acetate, gradient 60:40 to 0:100 then ethyl acetate: methanol, 90:10) afforded the title compound as a pale yellow solid (6.25g, 61%). LCMS (method B): R_{T} = 1.45 min, M+H⁺ = 207.

Step 2: Ethyl 3-(4-iodo-phenylamino)-furo[3,2-c]pyridine-2-carboxylate

A degassed solution of ethyl 3-amino-furo[3,2-c]pyridine-2-carboxylate (206 mg, 1.0 mmol), 1,4-diiodobenzene (3.3 g, 10.0 mmol), Pd₂dba₃ (24 mg, 26 µmol), Xantphos (30 mg, 52 µmol) and potassium phosphate (424 mg, 2.0 mmol) in toluene (10 ml) was stirred and heated to 105°C under an argon atmosphere for 42 hours. The cooled reaction mixture was poured into aqueous ammonium chloride solution and extracted with ethyl acetate (3 x 70 ml). The combined extracts were washed with water (2 x 100 ml) followed by brine (50 ml) before the organic phase was isolated, dried (MgSO₄), filtered and evaporated *in vacuo.* Purification of the resultant residue by flash chromatography (Si-SPE, cyclohexane: ethyl acetate, gradient 100:0 to 60:40) afforded the title compound as an off white solid (100 mg, 24%). LCMS (method B): R_{T} = 3.16 min, M+H⁺ = 409.

Ethyl 3-(2-chloro-4-iodo-phenylamino)-furo[3,2-c]pyridine-2-carboxylate

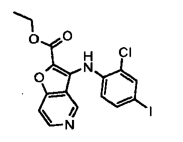

Step 1: Ethyl 3-(4-Bromo-2-chloro-phenylamino)-furo[3,2-c]pyridine-2-carboxylate

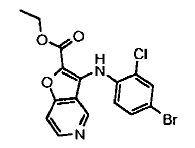

A degassed solution of ethyl 3-(nonafluorobutane-1-sulfonyloxy)-furo[3,2-c]pyridine-2-carboxylate (500 mg, 1.02 mmol), 4-bromo-2-chloroaniline (275 mg, 1.33 mmol), Pd₂dba₃ (47 mg, 0.05 mmol), Xantphos (59 mg, 0.10 mmol) and DBU (388 µl, 2.56 mmol) in toluene (2.0 ml) was subjected to microwave irradiation at 150°C for 10 minutes. The reaction mixture was concentrated and the resultant residue absorbed onto HM-N before being purified by flash chromatography (Si-SPE, cyclohexane: ethyl acetate, gradient 100:0 to 0: 100) to afford the title compound as a white solid (183 mg, 47%). LCMS (method B): R_{T} = 3.54 min, M+H⁺ = 395/397.

Step 2: Ethyl 3-(2-chloro-4-iodo-phenylamino)-furo[3,2-c]pyridine-2-carboxylate

A mixture of ethyl 3-(4-bromo-2-chloro-phenylamino)-furo[3,2-c]pyridine-2-carboxylate (183 mg, 0.46 mmol), copper(I) iodide (4 mg, 0.02 mmol), sodium iodide (139 mg, 0.93 mmol) and trans-N,N'-dimethyl-1,2-cyclohexane diamine (7 µl, 0.04 mmol) in 1,4-dioxane (0.5 ml) was heated at 115°C for 44 hours under an argon atmosphere. The reaction mixture was cooled to room temperature, then additional copper(I) iodide (4 mg, 0.02 mmol) and trans-N,N'-dimethyl-1,2-cyclohexane diamine (7 µl, 0.04 mmol) were added and heating resumed at 115°C for 18 hours under an argon atmosphere. The reaction mixture was then cooled to room temperature, diluted with dichloromethane, and washed with a 10% solution of ammonia in water, water then brine. The organic extract was dried over sodium sulfate, filtered and concentrated *in vacuo* to give a residue that was purified by flash chromatography (Si-SPE, cyclohexane: ethyl acetate, gradient 100:0 to 0:100) to afford the title compound as an off-white solid (115 mg, 57%). LCMS (method B): R_{T} = 3.97 min, M+H⁺ = 443.

Ethyl 3-(2,6-difluoro-4-iodo-phenylamino)-furo[3,2-c]pyridine-2-carboxylate

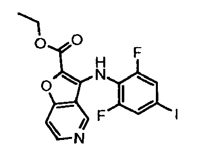

Step 1: Ethyl 3-(4-bromo-2,6-difluoro-phenylamino)-furo[3,2-c]pyridine-2-carboxylate

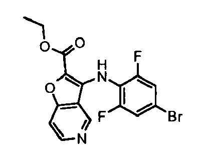

A degassed solution of ethyl 3-(nonafluorobutane-1-sulfonyloxy)-furo[3,2-c]pyridine-2-carboxylate (500 mg, 1.02 mmol), 4-bromo-2,6-difluoroaniline (277 mg, 1.33 mmol), Pd₂dba₃ (47 mg, 0.05 mmol), Xantphos (59 mg, 0.10 mmol) and DBU (388 µl, 2.56 mmol) in toluene (2.0 ml) was subjected to microwave irradiation at 150°C for 10 minutes. The reaction mixture was concentrated and the resultant residue absorbed onto HM-N before being purified by flash chromatography (Si-SPE, cyclohexane: ethyl acetate, gradient 100:0 to 0:100) to afford the title compound as a white solid (89 mg, 22%). LCMS (method B): R_{T} = 3.38 min, M+H⁺ = 397/399.

Step 2: Ethyl 3-(2,6-difluoro-4-iodo-phenylaminol-furo[3,2-c]pyridine-2-carboxylate

A mixture of ethyl 3-(4-bromo-2,6-difluoro-phenylamino)-furo[3,2-c]pyridine-2-carboxylate (165 mg, 0.42 mmol), copper(I) iodide (4 mg, 0.02 mmol), sodium iodide (125 mg, 0.83 mmol) and trans-N,N'-dimethyl-1,2-cyclohexane diamine (7 µl, 0.04 mmol) in 1,4-dioxane (0.5 ml) was subjected to microwave irradiation at 180°C for 15 minutes. Additional copper(I) iodide (4 mg, 0.02 mmol), sodium iodide (60 mg, 0.40 mmol) and trans-N,N'-dimethyl-1,2-cyclohexane diamine (7 µl, 0.04 mmol) were added to the reaction mixture that was re-subjected to microwave irradiation at 180°C for 15 minutes. The reaction mixture was diluted with dichloromethane and washed with a 10% solution of ammonia in water, water then brine. The organic extract was dried over sodium sulfate, filtered and concentrated to give a residue that was purified by flash chromatography (Si-SPE, cyclohexane: ethyl acetate, gradient 100:0 to 0:100) to afford the title compound as an off-white solid (137 mg, 74%). LCMS (method B): R_{T} = 3.48 min, M+H⁺ = 445.

Ethyl 3-(2,5-difluoro-4-iodo-phenylamino)-furo[3.2-c]pyridine-2-carboxylate

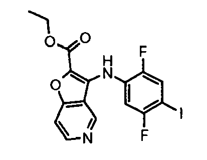

Step 1: Ethyl 3-(4-bromo-2,5-difluoro-phenylamino)-furo[3,2-c]pyridine-2-carboxylate

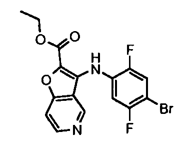

A degassed solution of ethyl 3-(nonafluorobutane-1-sulfonyloxy)-furo[3,2-c]pyridine-2-carboxylate (500 mg, 1.02 mmol), 4-bromo-2,5-difluoroaniline (277 mg, 1.33 mmol), Pd₂dba₃ (47 mg, 0.05 mmol), Xantphos (59 mg, 0.10 mmol) and DBU (388 µl, 2.56 mmol) in toluene (2.0 ml) was subjected to microwave irradiation at 150°C for 10 minutes. The reaction mixture was concentrated and the resultant residue absorbed onto HM-N before being purified by flash chromatography (Si-SPE, cyclohexane: ethyl acetate, gradient 100:0 to 0:100) to afford the title compound as a white solid (231 mg, 57%). LCMS (method B): R_{T} = 3.22 min, M+H⁺ = 397/399.

Step 2: Ethyl 3-(2,5-difluoro-4-iodo-phenylamino)-furo[3,2-c]pyridine-2-carboxylate

A mixture of ethyl 3-(4-bromo-2,5-difluoro-phenylamino)-furo[3,2-c]pyridine-2-carboxylate (222 mg, 0.56 mmol), copper(I) iodide (5 mg, 0.03 mmol), sodium iodide (168 mg, 1.12 mmol) and trans-N,N'-dimethyl-1,2-cyclohexane diamine (10 µl, 0.06 mmol) in 1,4-dioxane (0.5 ml) was heated at 110°C for 18 hours. Additional copper(I) iodide (5 mg, 0.03 mmol), and trans-N,N'-dimethyl-1,2-cyclohexane diamine (10 µl, 0.06 mmol) were added to the reaction mixture that was re-heated at 110°C for 6 hours. The reaction mixture was cooled, diluted with dichloromethane, and washed with a 10% solution of ammonia in water, water then brine. The organic extract was dried over sodium sulfate, filtered and concentrated to give a residue that was purified by flash chromatography (Si-SPE, cyclohexane: ethyl acetate, gradient 100:0 to 0:100) to afford the title compound as a white solid (170 mg, 68%). LCMS (method B): R_{T} = 3.30 min, M+H⁺ = 445.

7-Bromo-3-(2-fluoro-4-iodo-phenylamino)-furo[3,2-c]pyridine-2-carboxylic acid

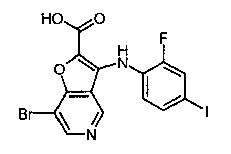

Step 1: Ethyl 7-bromo-3-hydroxy-furo[3,2-c]pyridine-2-carboxylate

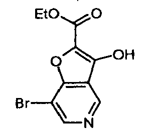

To a solution of ethyl 4,5-dibromonicotinate (2.68 g, 8.67 mmol) and ethyl glycolate (0.90 g, 8.67 mmol) in DMF (25 ml) at 0°C (ice/water), was added sodium hydride (1.04 g, 26 mmol, 60% oil dispersion). The reaction mixture was stirred at 0°C for 15 min before being allowed to warm to room temperature for 2h. The reaction mixture was cooled to 0°C before the addition of 1M HCl (18 ml, 18 mmol). The precipitate was filtered and washed with water to afford the title compound as an off-white solid (2.35 g, 95%). LCMS (method B): R_{T} = 2.96 min, M+H⁺ = 285/287.

Step 2: 3,7-Dibromo-furo[3,2-c]pyridine-2-carboxylic acid

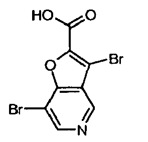

A mixture of ethyl 7-bromo-3-hydroxy-furo[3,2-c]pyridine-2-carboxylate (1.14 g, 4.0 mmol) and phosphorus oxybromide (5.6 g, 19.5 mmol) were heated at 140°C for 2 hours. The reaction mixture was cooled to room temperature before the addition of crushed ice (ca 30 ml). The mixture was neutralised by the addition of solid NaOH before being adjusted to pH 3.0 by the careful addition of 1M HCl. The resultant precipitate was filtered, and then washed with water followed by dichloromethane to afford the title compound as a white solid (1.2 g, 90%). LCMS (method B): R_{T} = 2.62 min, M+H⁺ = 320/322/324.

Step 3: 3,7-Dibromo-furo[3,2-c]pyridine-2-carboxylic acid (2-hydroxy-1,1-dimethyl-ethyl)-amide

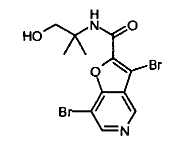

A mixture of 3,7-dibromo-furo[3,2-c]pyridine-2-carboxylic acid (1.2 g, 3.74 mmol) and carbonyl diimidazole (0.85 g, 5.24 mmol) in acetonitrile (18 ml) were heated at 50°C for 2 hours. A further portion of carbonyl diimidazole (0.035 g, 0.5 mmol) was added to the reaction mixture and heating at 50°C continued for 1 hour. After cooling to ambient temperature 2-amino-2-methyl-propan-1-ol (0.30 ml, 3.13 mmol) was added to the solution. The reaction mixture was left to stand at room temperature for 19 hours then heated to 50°C for 1 hour before being concentrated *in vacuo.* Purification of the resultant residue by flash chromatography (Si-SPE, cyclohexane: ethyl acetate, gradient 80:20 to 0:100) afforded the title compound as a pale yellow solid (0.53 g, 72%). LCMS (method B): R_{T} = 2.57 min, M+H⁺ = 391/393/395.

Step 4: 3,7-Dibromo-2-(4,4-dimethyl-4,5-dihydro-oxazol-2-yl)-furo[3,2-c]pyridine

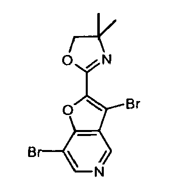

To a solution of 3,7-dibromo-furo[3,2-c]pyridine-2-carboxylic acid (2-hydroxy-1,1-dimethyl-ethyl)-amide (0.53 g, 1.35 mmol) in dichloromethane (10 ml) was added thionyl chloride (0.25 ml, 3.43 mmol). The mixture was stirred at room temperature for 1h, then heated to reflux for 2h, before cooling to 0°C. The mixture was neutralised with 1M NaOH (15ml) and the aqueous layer was extracted with dichloromethane (2 × 15 ml). The organic layer was collected then dried over magnesium sulfate and concentrated *in vacuo* to afford a residue. Purification of the resultant residue by flash chromatography (Si-SPE, dichloromethane then ethyl acetate) afforded the title compound as a pale yellow gum (250 mg, 50%). LCMS (method B): R_{T} = 3.11 min, M+H⁺ = 373/375/377.

Step 5: [7-Bromo-2-(4,4-dimethyl-4,5-dihydro-oxazol-2-yl)-furo[3,2-c]pyridin-3-yl]-(2-fluoro-4-iodo-phenyl)-amine

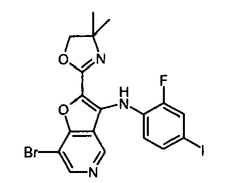

To a solution of 3,7-dibromo-2-(4,4-dimethyl-4,5-dihydro-oxazol-2-yl)-furo[3,2-c]pyridine (250 mg, 0.67 mmol) and 4-iodo-2-fluoroaniline (474 mg, 2 mmol) in THF (2ml) was added lithium hexamethyldisilazide solution in THF (2ml, 1M solution). The reaction mixture was heated at 50°C for 4 hours then cooled to room temperature and diluted with water (15 ml). The aqueous layer was extracted with dichloromethane (2 × 10 ml), and the combined organic extracts dried over MgSO₄ and concentrated *in vacuo* to afford a residue. Purification of the resultant residue by flash chromatography (Si-SPE, cyclohexane: *tert*-butyl-methylether, gradient 1:1 to 1:2) afforded the title compound as a light brown solid (150 mg, 42%). LCMS (method A): R_{T} = 13.97 min, M+H⁺ = 530/532.

Step 6: 7-Bromo-3-(2-fluoro-4-iodo-phenylaminol-furo[3,2-c]pyridine-2-carboxylic acid

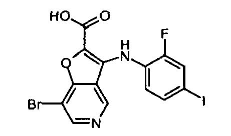

A mixture of [7-bromo-2-(4,4-dimethyl-4,5-dihydro-oxazol-2-yl)-furo[3,2-c]pyridin-3-yl]-(2-fluoro-4-iodo-phenyl)-amine (110 mg, 0.2 mmol) and 1M HCl (2 ml, 2 mmol) was heated at 100°C for 4 hours then cooled and concentrated *in vacuo.* The resultant residue was dissolved in methanol (3 ml) and 2.5M NaOH in methanol was added (0.4 ml, 1 mmol) followed by water (1 ml). The mixture was then heated at 75°C for 1 hour before 1M aqueous NaOH (1 ml, 1 mmol) was added and heating continued for 2 hours. The reaction mixture was concentrated *in vacuo* and the remaining aqueous layer was washed with ethyl acetate (2 × 2 ml). The aqueous layer was then acidified to pH 4 with 1M HCl (∼1.5 ml) and concentrated *in vacuo* to approximately half volume and allowed to stand at room temperature. The resultant precipitate was collected by filtration and washed with water (1 ml) followed by ethyl acetate (1 ml) to afford the title compound as a yellow / brown solid (66 mg, 69%). LCMS (method B): R_{T} = 3.34 min, M+H⁺ = 477/479.

Ethyl 5-(2-fluoro-4-iodo-phenylamino)-furo[2,3-d]pyrimidine-6-carboxylate

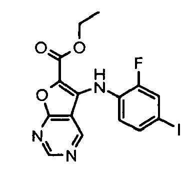

Step 1: Ethyl 4-hydroxy-pyrimidine-5-carboxylate

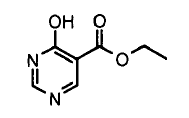

To a pre-formed solution of sodium (1.70 g, 73.9 mmol) in absolute ethanol (300ml) was added 1,3,5-triazine (6.0 g, 74.1 mmol) and diethylmalonate (11.3 ml, 74.1 mmol). The reaction mixture was heated to reflux. After heating for 3h, the reaction mixture was cooled to room temperature and concentrated under reduced pressure to afford a residue. The residue was dissolved in water (300 ml), then cooled to 5°C and acidified by addition of hydrochloric acid (6 ml). The mixture was aged for 48 hours at 5°C and filtered. The resultant solid was washed with water, before being dried under reduced pressure to give the title compound as a beige solid (3.0g, 24%). ¹H NMR (d₆-DMSO, 400 MHz) 8.47 (s, 1H), 8.37 (d, s, 1H), 4.22 (q, J = 7.2 Hz, 2H), 1.26 (t, J = 7.2 Hz, 3H).

Step 2: Ethyl 4-chloro-pyrimidine-5-carboxylate

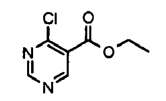

To a suspension of ethyl 4-hydroxy-pyrimidine-5-carboxylate (3.0g, 17.6 mmol) in toluene (35ml) was added diisopropylethylamine (3.4 ml, 19.6mmol) and phosphorous oxychloride (1.8 ml, 19.6 mmol) dropwise under a nitrogen atmosphere. The reaction mixture was heated to 70°C, and stirred for two hours then cooled to 5°C. A 1M aqueous solution of sodium hydroxide (26 ml) was added and the mixture was diluted with water and extracted into ethyl acetate. The organic layer was washed with water, saturated sodium hydrogencarbonate, then dried over sodium sulfate, filtered and concentrated to give the title compound as a brown oil (2.56g, 77%). ¹H NMR (CDCl₃, 400 MHz) 9.13 (s, 1H), 9.08 (s, 1H), 4.47 (q, J = 6.9 Hz, 2H), 1.44 (t, J = 6.9 Hz, 3H).

Step 3: Ethyl 4-ethoxycarbonylmethoxy-pyrimidine-5-carboxylate

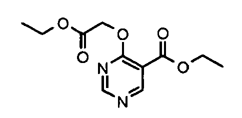

To a suspension of sodium hydride (60% in mineral oil, 602 mg, 15.1 mmol) in anhydrous THF (55 mL) at 5°C under a nitrogen atmosphere was added ethyl glycolate (1.6 ml, 16.5 mmol). The reaction mixture was stirred at 5°C for 30 minutes before a solution of ethyl 4-chloro-pyrimidine-5-carboxylate (2.56 g, 13.8 mmol) in anhydrous THF (20 ml) was added dropwise. The reaction mixture was stirred at 5°C for 30 minutes. Acetic acid (3 ml) was added to the reaction mixture that was then concentrated *in vacuo.* The resultant residue was dissolved in ethyl acetate and washed with water, then brine, before being dried over sodium sulfate and concentrated under reduced pressure to provide a residue. The residue was absorbed on HM-N and purified by flash chromatography (Si-SPE, cyclohexane: ethyl acetate, gradient 100:00 to 40:60) to afford the title compound as a yellow oil (2.67 g, 76%). ¹H NMR (CDCl₃, 400 MHz) 9.05 (s, 1H), 8.82 (s, 1H), 5.05 (s, 2H), 4.41 (q, J = 7.1 Hz, 2H), 4.24 (q, J=7.1 Hz, 2H), 1.40 (t, J = 7.1 Hz, 3H), 1.28 (t, J = 7.1 Hz, 3H).

Step 4: Ethyl 5-hydroxy-furo[2,3-d]lpyrimidine-6-carboxylate

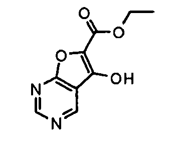

To a solution of ethyl 4-ethoxycarbonylmethoxy-pyrimidine-5-carboxylate (2.12 g, 8.3 mmol) in anhydrous THF (80 ml) at 5°C under an inert atmosphere was added sodium *tert*-butoxide (1.40 g). The reaction mixture was stirred for 30 minutes at 5°C and a 1M solution of hydrochloric acid was added. The mixture was diluted with water and extracted into ethyl acetate. The organic layer was separated and washed with water followed by brine, dried over sodium sulfate, filtered and concentrated under reduced pressure to afford the title compound as a white solid (934 mg, 54%). ¹H NMR (CDCl₃, 400 MHz) 9.22 (s, 1H), 9.14 (s, 1H), 4.51 (q, J = 7.3 Hz, 2H), 1.47 (t, J = 7.3 Hz, 3H).

Step 5: Ethyl 5-trifluoromethanesulfonyloxy-furo[2,3-d]pyrimidine-6-carboxylate

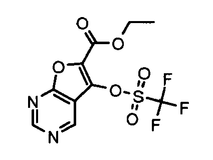

To a solution of ethyl 5-hydroxy-furo[2,3-d]pyrimidine-6-carboxylate (1.2 g, 5.8 mmol) and diisopropylethylamine (1.5 ml, 8.7 mmol) in dimethoxyethane (25 ml) was added *N*-phenyltrifluoromethanesulfonimide (2.3g, 6.4 mmol). The reaction mixture was heated to reflux and stirred for 1 hour, then cooled to room temperature and concentrated under reduced pressure. The residue was dissolved in ethyl acetate then washed with water, saturated aqueous sodium hydrogencarbonate and brine. The organic layer was dried over sodium sulfate, filtered, absorbed on HM-N and purified by flash chromatography (Si-SPE, cyclohexane: ethyl actetate, gradient 100:0 to 50:50) to afford the title compound as a colourless oil (1.5 g, 77%). ¹H NMR (CDCl₃, 400 MHz) 9.23 (s, 2H), 4.54 (q, J = 7.2 Hz, 2H), 1.47 (t, J = 7.2 Hz, 3H).

Step 6: Ethyl 5-(2-fluoro-4-trimethylsilanyl-phenylamino)-furor[2,3-d]pyrimidine-6-carboxylate

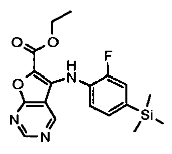

A degassed solution of ethyl 5-trifluoromethanesulfonyloxy-furo[2,3-d]pyrimidine-6-carboxylate(1.5 g, 4.4 mmol), 2-fluoro-4-trimethylsilanyl-phenylamine (888 mg, 4.8 mmol), Pd₂dba₃ (202 mg, 0.22 mmol), Xantphos (127 mg, 0.22 mmol) and K₃PO₄ (1.9 g, 8.8 mmol) in toluene (20 ml) was heated to reflux and stirred for 4 hours under a nitrogen atmosphere. The reaction mixture was cooled to room temperature and filtered through celite, and the filtered cake was washed with ethyl acetate. The organic layer was washed with water then brine, dried over sodium sulfate, filtered and concentrated under reduced pressure. The resultant residue was dissolved in dichloromethane, absorbed on HM-N and purified by flash chromatography (Si-SPE, ethyl acetate: cyclohexane, gradient 0:100 to 40:60) to afford the title compound as an oil that crystallized on standing (1.2 g, 75%). LCMS (method B): R_{T} = 4.39 min, M+H⁺ = 374.

Step 7: Ethyl 5-(2-fluoro-4-iodo-phenylaminol-furo[2,3-d]pyrimidine-6-carboxylate 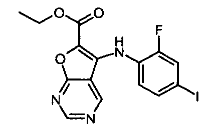 To a solution of ethyl 5-(2-fluoro-4-trimethylsilanyl-phenylamino)-furo[2,3-d]pyrimidine-6-carboxylate(1.2 g, 3.2 mmol) in dichloromethane (10 ml) at 5°C was added a solution of iodine monochloride (674 mg, 4.2 mmol) in dichloromethane (5 ml). The reaction mixture was stirred a 5°C for 1 hour, before a saturated aqueous solution of sodium thiosulfate was added. The organic layer was separated and washed with water then brine, dried over sodium sulfate, filtered and concentrated under reduced pressure. The resultant residue was triturated with hot ethanol, aged overnight at room temperature. The resulting precipitate collected by filtration then washed with cold ethanol before being dried under vacuum to afford the title compound as a white solid (864 mg, 63%). ¹H NMR (CDCl₃, 400 MHz) 9.08 (s, 1H), 8.70 (s, 1H), 7.78 (s, 1H), 7.57 (dd, J=9.6 Hz, 1.9 Hz, 1H), 7.51 (ddd, J=8.4 Hz, 1.7Hz), 1.7Hz), 1H), 7.03 (dd, J=8.2Hz, 8.2 Hz, 1H), 4.49 (q, J = 7.4 Hz, 2H), 1.46 (t, J = 7.4 Hz, 3H).

3-((2-Fluoro-4-iodophenyl)methylaminolfuro[3,2-c]pyridine-2-carboxylic acid ethyl ester

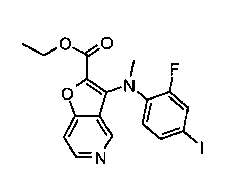

Sodium hydride (60% dispersion in mineral oil, 45 mg, 1.12 mmol) was added portionwise to a stirred solution of 3-(2-fluoro-4-iodo-phenylamino)-furo[3,2-c]pyridine-2-carboxylic acid ethyl ester (430 mg, 1.0 mmol) and iodomethane (310 µL, 4.98 mmol) in DMF (3 mL) under an inert atmosphere. This mixture was stirred for 3 hours, then quenched with brine and extracted with ethyl acetate (3 x 40 mL). The combined organic extracts were washed with brine, dried (MgSO₄), filtered and concentrated *in vacuo.* The resultant residue was purified using flash chromatography (Si-SPE gradient 40:100 to 100:100 ether) to afford the title compound as a yellow solid (57 mg, 13%). LCMS (method B): R_{T} = 3.26min; M+H⁺ 440.

3-(4-Bromo-2-chloro-phenylamino)-furo[3,2-c]pyridine-2-carboxylic acid ethyl ester

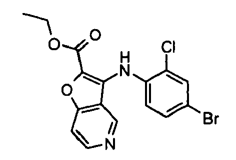

A degassed solution of 3-trifluoromethanesulfonyloxy-furo[3,2-c]pyridine-2-carboxylic acid ethyl ester (300 mg, 0.88 mmol), 4-bromo-2-fluoro aniline (201 mg, 0.97 mmol), Pd₂dba₃ (40 mg, 0.044 mmol), Xantphos (59 mg, 0.044 mmol) and potassium phosphate tribasic (373 mg, 1.76 mmol) in toluene (5 ml) was heated at reflux under an argon atmosphere for 16 hours. The reaction mixture was filtered and concentrated *in vacuo.* The resultant residue was purified by flash chromatography (Si-SPE, pentane: diethyl ether, gradient 80:20 to 50:50) to afford the title compound as a yellow solid (177 mg, 51%). LCMS (method B): R_{T} = 3.76 min, M+H⁺ = 395 / 397.

3-4-Methyl-2-fluoro-phenylamino-furo[3,2-c]pyridine-2-carboxylic acid ethyl ester

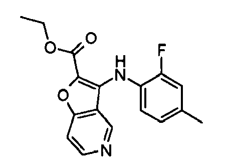

A degassed solution of 3-amino-furo[3,2-c]pyridine-2-carboxylic acid ethyl ester (300 mg, 1.46 mmol), 4-bromo-3-fluorotoluene (277 µl, 2.19 mmol), Pd₂dba₃ (67 mg, 0.073 mmol), Xantphos (84 mg, 0.15 mmol) and potassium phosphate tribasic (620 mg, 2.92 mmol) in toluene (10 ml) was heated at reflux under an argon atmosphere for 16 hours. The reaction mixture was concentrated *in vacuo* and the residue was purified by flash chromatography (Si-SPE, pentane: diethyl ether, gradient 100:0 to 75:25) to afford the title compound as a yellow solid (252 mg, 55%). LCMS (method B): R_{T} = 3.14 min, M+H⁺ = 315.

3-(2-Fluoro-4-methylsulfanyl-phenylamino)-furo[3,2-c]pyridine-2-carboxylic acid ethyl ester

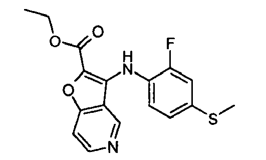

Step 1: 4-Bromo-3-fluoro-benzenethiol

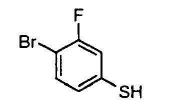

4-Bromo-3-fluoro-benzenesulfonyl chloride (324 µl, 2.19 mmol) was added dropwise to a solution of triphenylphosphine (1.73 g, 6.58 mmol) in a mixture of dimethylformamide (125 µl) and dichloromethane (5 ml). The solution was stirred at room temperature for 16 hours, then 1 M aqueous hydrochloric acid (5 ml) was added and the layers were separated. The organic layer was concentrated *in vacuo* and the resultant residue taken up in 1 M aqueous sodium hydroxide (10 ml). The resulting suspension was filtered through celite ® and the filtrate washed with ether (10 ml × 3), then neutralised by addition of 1 M aqueous hydrochloric acid (10 ml). The solution was extracted with ether (10 ml × 3) and the combined organic extracts were dried (Na₂SO₄) then concentrated *in vacuo* to afford the title compound as a colourless oil (225 mg, 50%). 1H NMR (CDCl₃, 300 MHz) 7.47 (1H, dd, J= 8.4, 7.5 Hz), 7.06 (1H, dd, J= 8.9, 2.2 Hz), 6.93 (1H, ddd, J= 8.4, 2.1, 0.7 Hz), 3.54 (1H, br s).

Step 2: 1-Bromo-2-fluoro-4-methylsulfanyl-benzene

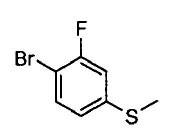

A solution of 4-bromo-3-fluoro-benzenethiol (225 mg, 1.09 mmol) in tetrahydrofuran (3 ml) was cooled to 0°C. Sodium hydride (60% dispersion in mineral oil, 52 mg, 1.31 mmol) was added and the mixture was stirred for 5 minutes. Iodomethane (78 µl, 1.25 mmol) was then added and the mixture was allowed to return to room temperature with stirring over 20 minutes. Dichloromethane (10 ml) was added and the reaction was quenched with 1 M aqueous hydrochloric acid. The layers were separated and the organic layer was washed with water, dried (MgSO₄), then concentrated *in vacuo.* The residue was purified by flash chromatography (Si-SPE, pentane: diethyl ether, gradient 100:0 to 90:10) to afford the title compound as a bright yellow oil (208 mg, 86%). 1H NMR (CDCl₃, 400 MHz) 7.43 (1H, dd, J= 8.4, 7.2), 7.00 (1H, dd, J= 9.4, 2.3), 6.91 (1H, ddd, J=8.4, 2.1, 0.7), 2.48 (3H, s).

Step 3: 3-(2-Fluoro-4-methylsulfanyl-phenylamino)-furo[3,2-c]pyridine-2-carboxylic acid ethyl ester

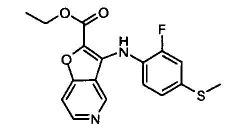

A degassed solution of 3-amino-furo[3,2-c]pyridine-2-carboxylic acid ethyl ester (121 mg, 0.59 mmol), 1-bromo-2-fluoro-4-methylsulfanyl-benzene (195 mg, 0.88 mmol), Pd₂dba₃ (27 mg, 0.030 mmol), Xantphos (34 mg, 0.059 mmol) and potassium phosphate tribasic (250 mg, 1.18 mmol) in toluene (3 ml) was heated at reflux under an argon atmosphere for 60 hours. The reaction mixture was filtered and the filtrate concentrated *in vacuo.* The resultant residue was purified by flash chromatography (Si-SPE, pentane: diethyl ether, gradient 100:0 to 50:50) to afford the title compound as a yellow solid (128 mg, 63%). LCMS (method B): R_{T} = 3.24 min, M+H⁺ = 347.

7-Chloro-3-(2-fluoro-4-iodo-phenylamino)-furo[3,2-c]pyridine-carboxylic acid ethyl ester

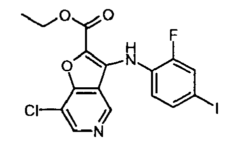

Step 1: 4,5-Dichloropyridine-3-carbaldehyde

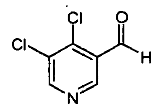

To a solution of diisopropylamine (10.73 ml, 75.9 mmol) in THF (60 ml) at - 40°C, was added n-butyllithium (47.45 ml, 75.9 mmol, 1.6M in hexanes) and the solution was stirred for 15 min at -40°C, before cooling to -70°C. A solution of 3,4-dichloropyridine (10.7 g, 72.3 mmol) in THF (30 ml) was added dropwise to maintain the temperature below -65°C. The reaction was stirred at -70°C for 2 h before the addition of DMF (6.74 ml, 86.8 mmol). The reaction was then stirred at -40°C for 1 h and then allowed to warm to -5°C before the careful addition of saturated ammonium chloride solution (50 ml) with rapid stirring over 3 min. The mixture was then partitioned between saturated ammonium chloride (150 ml) and dichloromethane (150 ml) and the layers separated. The aqueous layer was extracted with dichloromethane (2 × 100 ml) and the combined organic layers were dried over magnesium sulfate, then concentrated *in vacuo.* Purification by flash chromatography (Si-SPE, dichloromethane : ethyl acetate gradient 100:0 to 94:6) afforded the title compound as white waxy solid (8.01 g, 63%).

Step 2: 4,5-Dichloropyridine-3-carbaldehyde oxime

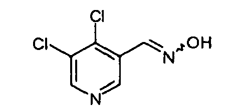

A solution of 4,5-dichloropyridine-3-carbaldehyde (8.01 g, 45.51 mmol) in ethanol (50 ml) was added to a rapidly stirred solution of hydroxylamine hydrochloride (3.48 g, 50.06 mmol) in water (50 ml). The reaction was stirred at r.t. for 45 min then partitioned between ethyl acetate (100 ml) and water (100 ml). The aqueous layer was extracted with ethyl acetate (2 × 50 ml) and the combined organic layers were dried over magnesium sulfate before being concentrated *in vacuo* to afford the title compound as a white solid (8.3 g, 96%).

Step 3: 4,5-Dichloronicotinonitrile

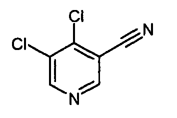

To a suspension of 4,5-dichloropyridine-3-carbaldehyde oxime (7.84 g, 41.05 mmol) in dichloromethane (150 ml) was added carbonyl diimidazole (7.99 g , 49.26 mmol). The mixture was then heated to reflux for 1.5 h before cooling then washing with saturated aqueous sodium bicarbonate (70 ml) and water (70 ml). The organic layer was dried over magnesium sulfate and concentrated *in vacuo.* Purification of the resultant residue by flash chromatography (Si-SPE, cyclohexane : dichloromethane gradient 20:80 to 0:100) afforded the title compound as a white solid (0.53 g, 72%). LCMS (method B): R_{T} = 2.86 min, no ion present.

Step 4: 3-Amino-7-chloro-furo[3,2-c]pyridine-2-carboxylic acid ethyl ester

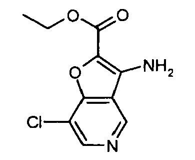

To a solution of ethyl glycolate (1.48 ml, 15.7 mmol) in DMF (15 ml) at -10°C was added sodium hydride (0.63 g, 15.7 mmol, 60% dispersion in oil). The mixture was stirred at this temperature for 35 min then cooled to -40°C. A solution of 3,4-dichloronicotinonitrile (0.906 g, 5.24 mmol) in DMF (5 ml) was added dropwise before allowing the reaction to warm to -15°C for 30 min then -5°C for 1 h. The mixture was poured into 10:1 water/acetic acid (25 ml), diluted with water (25 ml) and extracted with ethyl acetate (2 × 30 ml). The aqueous phase was taken to pH 8 with saturated aqueous sodium bicarbonate then extracted with ethyl acetate (2 × 25 ml). The combined organic layers were dried over magnesium sulfate and concentrated *in vacuo.* Purification of the resultant residue by flash chromatography (Si-SPE, ethyl acetate : triethylamine 98:2) afforded the title compound as a yellow solid (0.60 g, 48%). LCMS (method B): R_{T} = 2.79 min, M+H⁺ = 241, 243.

Step 5: 7-Chloro-3-(2-fluoro-4-trimethylsilanyl-phenylamino)-furo[3,2-c]pyridine-carboxylic acid ethyl ester

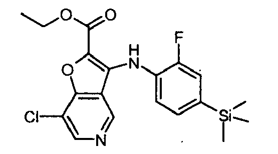

To a solution of 3-amino-7-chloro-furo[3,2-c]pyridine-2-carboxylic acid ethyl ester (4.16 g, 17.3 mmol) in toluene (100 ml) was added caesium carbonate (11.27 g, 34.6 mmol) and the mixture was degassed (argon / vacuum). To this was added trifluoro-methanesulfonic acid 2-fluoro-4-trimethylsilanyl-phenyl ester (7.1 g, 22.5 mmol), Pd₂dba₃ (395 mg, 0.432 mmol) and Xantphos (0.5 g, 0.865 mmol) and the vessel was flushed with argon. The reaction mixture was heated to reflux for 19 h, cooled and poured into saturated ammonium chloride (150 ml). The aqueous layer was extracted with ethyl acetate (3 × 60 ml), the combined organic layers were dried over magnesium sulfate and concentrated in *vacuo.* Purification by flash chromatography (Si-SPE, cyclohexane : dichloromethane gradient 1:0 to 0:1) afforded the title compound as a pale yellow solid (5.13 g, 73%). LCMS (method B): R_{T} = 4.80 min, M+H⁺ = 407, 409.

Step 6: 7-Chloro-3-(2-fluoro-4-iodo-phenylamino)-furo[3,2-c]pyridine-carboxylic acid ethyl ester

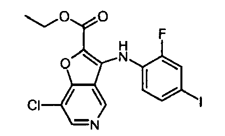

To a solution of 7-chloro-3-(2-fluoro-4-trimethylsilanyl-phenylamino)furo[3,2c]-pyridine-carboxylic acid ethyl ester (250 mg, 0.615 mmol) in dichloromethane (25 ml) at 0°C was added iodine monochloride (1.23 ml, 1.23 mmol, 1M solution in dichloromethane) and the solution was stirred at this temperature for 1 hour. A saturated solution of sodium thiosulfate (5 ml) was added and the mixture was poured into saturated sodium thiosulfate (25 ml). The aqueous layer was extracted with dichloromethane (2 × 25 ml), the combined organic layers were washed with brine, dried over magnesium sulfate and concentrated *in vacuo.* Purification of the resultant residue by flash chromatography (Si-SPE, cyclohexane : dichloromethane gradient 1:0 to 0:1) afforded the title compound as a yellow waxy solid (0.22 g, 78%). LCMS (method B): R_{T} = 4.30 min, M+H⁺ = 461, 463.

7-Cyano-3-(2-fluoro-4-iodo-phenylamino)-furo[3,2-c]pyridine-carboxylic acid ethyl ester

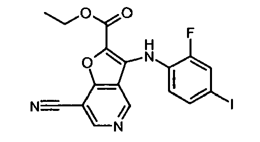

Step 1:7-Cyano-3-(2-fluoro-4-trimethlsyilanyl-phenylamino)-furo[3,2-c]pyridine-carboxylic acid ethyl ester

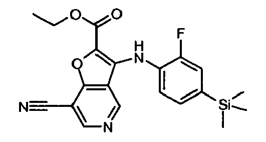

To a solution of 7-chloro-3-(2-fluoro-4-trimethylsilanyl-phenylamino)furo[3,2c]-pyridine-carboxylic acid ethyl ester (0.64 g, 1.57 mmol) in DMF (15 ml) was added zinc (II) cyanide (0.22 g, 12.63 mmol) and the mixture was degassed (argon/vacuum). Pd₂dba₃ (72 mg, 0.079 mmol) and 2-dicyclohexylphosphino-2',6'-dimethoxy-1,1'-biphenyl (S-Phos, 65 mg, 0.158 mmol) were then added and the vessel was flushed with argon, sealed then heated to 150°C under microwave irradiation for 30 minutes. The reaction was cooled, the volatiles removed and the residue azeotroped with toluene (3 × 15 ml). Purification of the resultant residue by flash chromatography (Si-SPE, cyclohexane : dichloromethane gradient 1:0 to 0:1 1 then 10% ethyl acetate in dichloromethane) afforded the title compound as a pale yellow solid (0.46 g, 74%). LCMS (method B): R_{T} = 4.52 min, M+H⁺ = 398.

Step 2: 7-Cyano-3-(2-fluoro-4-iodo-phenylamino)-furo[3,2-c]pyridine-carboxylic acid ethyl ester

To a solution of 7-cyano-3-(2-fluoro-4-trimethylsilanyl-phenylamino)furo[3,2c]-pyridine-carboxylic acid ethyl ester (0.46 g, 1.16 mmol) in dichloromethane (40 ml) at 0°C was added iodine monochloride (2.32 ml, 2.32 mmol, 1M solution in dichloromethane), and the resultant mixture was stirred at this temperature for 30 minutes. A saturated solution of sodium thiosulfate (5 ml) was added and the mixture poured into saturated sodium thiosulfate (35 ml). The aqueous layer was extracted with dichloromethane (2 × 25 ml) and the combined organic layers were washed with brine, dried over magnesium sulfate then concentrated *in vacuo.* Purification of the resultant residue by flash chromatography (Si-SPE, dichloromethane : ethyl acetate gradient 10:0 to 10:1) afforded the title compound as a yellow waxy solid (0.36 g, 69%). LCMS (method B): R_{T} = 4.10 min, M+H⁺ = 452.

3-(2-Fluoro-4-triisopropylsiyloxymethyl-phenylamino)-furo[3,2-c]pyridine-2-carboxylic acid ethyl eater

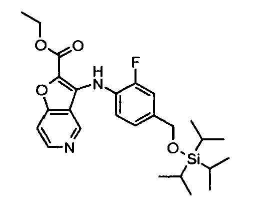

Step 1: (4-Bromo-3-fluoro-benzyloxyl-triisopropyl-silane.

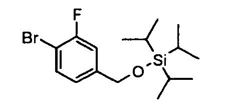

To a solution of (4-bromo-3-fluoro-phenyl)-methanol (410 mg, 2.0 mmol) and imidazole (163 mg, 2.4 mmol) in DMF (10 mL) was added triisopropylsilyl chloride (0.472 mL, 2.2 mmol). The reaction mixture was stirred at room temperature for 18 hours and then partitioned between ethyl acetate and water. The organic layer was isolated, washed with brine, dried (Na₂SO₄), filtered and concentrated *in vacuo.* The resultant residue was purified by flash chromatography (Si-SPE, pentane) to provide the title compound as a colourless oil (643 mg, 89%). 1H NMR (CDCl₃, 400MHz) 7.48 (dd, J=8.1, 7.0 Hz, 1H), 7.16 (d, J = 9.7 Hz, 1H), 6.99 (d, J=8.7 Hz, 1H), 4.78 (s, 2H), 1.04-1.24 (m, 21H).

Step 2: 3-(2-Fluoro-4-triisopropylsilanyloxyl-phenylamino)-furo[3,2-c]pyridine-2-carboxylic acid ethyl ester

A degassed solution of 3-amino-furo[3,2-c]pyridine-2-carboxylic acid ethyl ester (206 g, 1.0 mmol), (4-bromo-3-fluoro-benzyloxy)-triisopropyl-silane (433 mg, 1.2 mmol), Pd₂dba₃ (36 mg, 0.039 mmol), Xantphos (46 mg, 0.08 mmol) and K₃PO₄ (297 mg, 1.4 mmol) in toluene (1 ml) was heated to 110°C then stirred for 4 hours. The reaction mixture was cooled to ambient temperature then diluted with EtOAc and filtered through a pad of celite. The filtrate was concentrated *in vacuo* to give a black oil. The oil was purified by flash chromatography (Si-SPE, MeOH: DCM, gradient 0:100 to 5:95) to provide the title compound as a yellow oil (166 mg, 34%). LCMS (method B): R_{T} = 5.39 min, M+H⁺ = 487.

3-(2-Fluoro-4-methoxy-phenylamino)-furo[3,2-c]pyridine-2-carboxylic acid ethyl ester

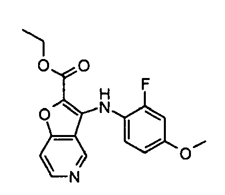

Step 1: 1-Bromo-2-fluoro-4-methoxy-benzene.

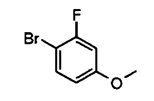

To a solution of 4-bromo-3-fluoro-phenol (500 mg, 2.62 mmol) in anhydrous THF (10 mL) was added sodium hydride (60% dispersion in mineral oil, 115 mg, 2.88 mmol) portionwise. The reaction mixture was stirred for 20 min before iodomethane (0.500 mL, 8.0 mmol). The resultant mixture was stirred at room temperature for 16 hours before being partitioned between EtOAc and water. The organic layer was separated, washed with a saturated solution of sodium hydrogencarbonate followed by brine, dried (Na₂SO₄), filtered and concentrated to give the title compound as a pale yellow oil (518 mg, 96%). 1H NMR (CDCl₃, 400MHz) 7.41 (dd, J=8.8, 8.0 Hz, 1H), 6.69 (dd, J = 10.3, 2.8 Hz, 1H), 6.61 (ddd, J=8.8, 2.8, 1.0 Hz, 1H), 3.79 (s, 3H).

Step 2: 3-(2-Fluoro-4-methoxy-phenylamino)-furo[3,2-c]pyridine-2-carboxylic acid ethyl ester

A degassed solution of 3-amino-furo[3,2-c]pyridine-2-carboxylic acid ethyl ester (206 g, 1.0 mmol), 1-bromo-2-fluoro-4-methoxy-benzene (246 mg, 1.2 mmol), Pd₂dba₃ (46 mg, 0.050 mmol), Xantphos (58 mg, 0.10 mmol) and K₃PO₄ (254 mg, 1.2 mmol) in toluene (5 ml) was heated to 110°C then stirred for 18 hours. The reaction mixture was cooled to ambient temperature then diluted with EtOAc and filtered through a pad of celite. The filtrate was concentrated *in vacuo* to give a black oil. The oil was purified by flash chromatography (Si-SPE, MeOH: DCM, gradient 0:100 to 10:90) to provide the title compound as a yellow oil (130 mg, 39%). LCMS (method B): R_{T} = 2.93 min, M+H⁺ = 331.

Ethyl 3-(4-Bromo-2,5-difluorophenylamino)furo[3,2-c]pyridine-2-carboxylate

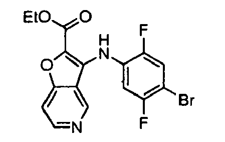

A degassed solution of ethyl 3-(trifluoromethanesulfonyloxy)furo [3,2-c]pyridine-2-carboxylate (678 mg, 2.00 mmol), 4-bromo-2,5-difluoroaniline (670 mg, 3.22 mmol), Pd₂dba₃ (147 mg, 0.160 mmol), Xantphos (97.0 mg, 0.168 mmol) and finely powdered K₃PO₄ (793 mg, 3.74 mmol) in toluene (7.5 ml) was heated in a sealed tube at 105°C overnight. The reaction mixture was cooled to ambient temperature then diluted with ethyl acetate (15 ml) and filtered through a plug of silica gel (15 ml packed in ethyl ether). After washing the filter cake with more ethyl acetate (20 ml), the filtrate was dried over magnesium sulfate and concentrated *in vacuo* to give a brown oil. The residue was purified by flash chromatography (silica gel, using using 5:3:2 hexane-methylene chloride-ethyl ether) to afford the title compound as a tan solid (329 mg, 41%).

3-(2-Fluoro-4-iodo-phenoxy)-furo[3,2-*c*]pyridine-2-carboxylic acid ethyl ester

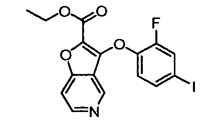

Step 1: 3-(2-Fluoro-4-nitro-phenoxy)-furo[3,2-*c*]pyridine-2-carboxylic acid ethyl ester

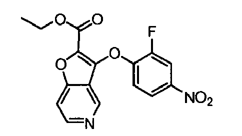

Ethyl 3-hydroxy-furo[3,2-c]pyridine-2-carboxylate (2.70 g, 13.0 mmol), followed by 3,4-difluoro nitrobenzene (2.89 mL, 26.1 mmol), and 18-crown-6 (3.45 g, 13.0 mmol) were added to a suspension of potassium hydride (1.10 g, 27.4 mmol) in DMF (30 mL) at room temperature. The reaction mixture was heated to 100 °C for 2h, then cooled to room temperature and poured into a water / brine mixture. The aqueous layer was extracted 3 times with EtOAc, then the combined organics were washed once with brine, dried over Na₂SO₄ and concentrated. The residue was purified by silica chromatography (30 - 80% EtOAc:Hex) to afford the title compound (442 mg, 10% yield) as a yellow syrup. LCMS (method C): R_{T} = 2.07 min, M+H⁺ = 347. ¹H NMR (CDCl₃, 400MHz) 8.83 (d, *J* = 1.2 Hz, 1H), 8.72 (d, *J =* 6.0 Hz, 1H), 8.17 (dd, *J* = 10.0, 2.4 Hz, 1H), 8.03 (ddd, *J =* 9.2, 2.8, 1.6 Hz, 1H), 7.59 (dd, *J* = 5.6, 0.8 Hz, 1H), 7.08 (dd, *J* = 9.2, 8.0 Hz, 1H), 4.36 (q, *J* = 7.2 Hz, 2H), 1.28 (t, J= 7.2 Hz, 3H).

Step 2: 3-(4-Amino-2-fluoro-phenoxy)-furo[3,2-*c*]pyridine-2-carboxylic acid ethyl ester

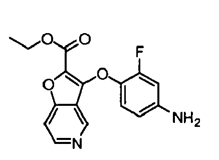

Fe powder (299 mg, 5.36 mmol) was added to a solution of 3-(2-Fluoro-4-nitro-phenoxy)-furo[3,2-c]pyridine-2-carboxylic acid ethyl ester (460 mg, 1.3 mmol) in ethanol (8 mL) and 2N aqueous HCl (8 mL). The reaction mixture was heated to 50 °C, then cooled to room temperature. The unreacted iron was removed with a magnet, then the reaction mixture was concentrated in vacuo. 10 mL each of water and ethanol were added, followed by solid sodium bicarbonate (1.5g). 3.2 g of silica gel was added and the volatiles were removed in vacuo. The residue was purified by silica chromatography (40 - 80% EtOAc:Hex) to afford the title compound (130 mg, 31% yield) as an off-white foam. LCMS (method C): R_{T} = 1.35 min, M+H⁺ = 317. ¹H NMR (CDCl₃, 400MHz) 8.54 (d, *J* = 3:6 Hz, 1H), 8.16 (d, *J* = 0.8 Hz, 1H), 7.45 (dd, *J* = 5.6, 0.8 Hz, 1H), 7.09 (t, *J* = 8.8 Hz, 1H), 6.53 (dd, *J* = 12.4, 2.8 Hz, 1H), 6.44 (ddd, *J* = 8.8, 2.8, 1.6 Hz, 1H), 4.47 (q, *J* = 7.2 Hz, 2H), 3.81 (br, 2H), 1.42 (t, *J* = 7.2 Hz, 3H).

Step 3:3-(2-Fluoro-4-iodo-phenoxy)-furo[3,2-*c*]pyridine-2-carboxylic acid ethyl ester

Sodium nitrite (1.18 mL of a 0.382 M aqueous solution) was added dropwise to a suspension of 3-(4-Amino-2-fluoro-phenoxy)-furo[3,2-*c*]pyridine-2-carboxylic acid ethyl ester (130 mg, 0.41 mmol) in a 2 M aqueous solution of HCl (3.5 mL) at 0 °C. The reaction mixture was stirred for 45 minutes at 0 °C, and then sodium iodide (1.18 mL of a 1.39 M aqueous solution, 1.64 mmol) was added. The reaction mixture was stirred overnight at 0 °C - room temperature. Sodium hydroxide (7 mL of 1N aqueous solution) and Na₂S₂O₃ (5 mL of saturated aqueous solution) were added, and the aqueous layer was extracted three times with CH₂Cl₂. The combined organics were dried over Na₂SO₄, filtered and concentrated. The residue was purified by silica chromatography (30 - 70% EtOAc:Hex) to afford the title compound (60 mg, 30% yield) as a white solid. LCMS (method C): R_{T} = 2.29 min, M+H⁺ = 428. ¹H NMR (CDC1₃, 400MHz) 8.63 (d, *J* = 6.4 Hz, 1H), 8.53 (d, *J* = 1.2 Hz, 1H), 7.58 (dd, *J* = 9.6, 2.0 Hz, 1H), 7.52 (dd, *J* = 6.0, 1.2 Hz, 1H), 7.37 (dt, *J =* 8.8, 1.6 Hz, 1H), 6.86 (t, *J* = 8.4 Hz, 1H), 4.41 (q, *J* = 7.2 Hz, 2H), 1.34 (t, *J =* 7.2 Hz, 3H).

3-(2-Fluoro-4-iodophenylaminol-7-phenylfuro[3,2-c]pyridine-2-carboxylic acid ethyl ester

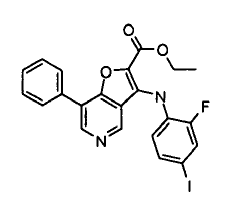

Step 1: 4,5-Dibromonicotinic acid

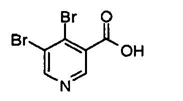

5-Bromonicotinic acid (25.25 g, 125 mmol) was stirred as a solution in dry tetrahydrofuran (500 ml) under nitrogen and cooled to -70°C. The resultant mixture was treated dropwise over 1 hour with lithium diisopropylamide (1.8 M, 144 ml, 260 mmol). After the addition was complete, the solution was stirred for 2.5 hours at - 55°C then cooled to -70°C and treated portionwise over 30 minutes with 1,2-dibromotetrachloroethane (50 g, 154.5 mmol). After stirring for 30 minutes the mixture was allowed to warm to -20°C over 2 hours before the cautious addition water (150 ml). The organic solvent was then removed *in vacuo* and the residue diluted with water (500 ml), then washed with ethyl acetate before acidifying the aqueous layer to pH 3.00 with c. HCl. The precipitated product was collected by filtration and dried at 60°C *in vacuo* to give the title compound (14.2g). The filtrate was extracted with ethyl acetate, the extract washed with water, dried (MgSO₄), filtered and concentrated *in vacuo* to give further title compound (18.6g, total yield 32.8 g, 93%). ¹H NMR (DMSO-d₆, 400MHz) 8.92 (s, 1H), 8.73 (s, 1H).

Step 2: 4,5-Dibromonicotinic acid ethyl ester

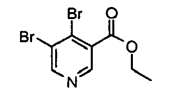

4,5-Dibromonicotinic acid (32.8 g, 116.7 mmol) was stirred as a suspension in acetonitrile (550 ml) at room temperature and treated portionwise with 1,1' - carbonyldiimidazole (29.87 g, 180 mmol) over 10 minutes. The resulting mixture was stirred for 3 hours at room temperature. After this time ethanol (78 ml) was added and stirring continued for a further 48 hours. The solution was then filtered and the filtrate evaporated *in vacuo* to give a light brown oil. The oil was dissolved in ethyl acetate and the solution was washed water followed by brine then dried over magnesium sulfate, filtered and evaporated *in vacuo* to give a brown oil. The oil was purified by flash chromatography (SiO₂ dichloromethane eluent) to give the title compound (20.6 g 57 %) 1H NMR (CDCl₃, 400 MHz) 8.80 (s, 1H), 8.75 (s, 1H), 4.45 (q, 2H J = 7.0 Hz), 1.39 (t, 3H J= 7.0 Hz)

Step 3: 7-Bromo-3-hydroxyfuro[3,2-c]pyridine-2-carboxylic acid ethyl ester

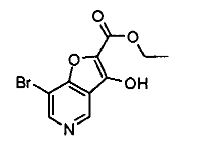

A solution ethyl glycolate (6.30 ml, 66.5 mmol) in dry DMF (50 ml) was added dropwise to a stirred suspension of sodium hydride (8.00 g, 60% dispersion, 200 mmol) in dry DMF (80 ml) whilst cooling to maintain the temperature below 10°C. After the addition, the mixture was stirred for 30 minutes before the dropwise addition of 4,5-dibromonicotinic acid ethyl ester (20.60 g, 66.5 mmol) as a solution in dry DMF (50ml) whilst again maintaining the temperature below 10°C. The resulting dark red/brown solution was allowed to warm to room temperature slowly over 1.5 hours before quenching and acidifying to pH 3.00 with aqueous 1M HCl. The resulting solid precipitate was collected by filtration, the residue was washed with water then cold acetone and dried *in vacuo* at 45°C to give the title compound (11.98 g 63%). 1H NMR (DMSO-d₆, 400MHz) 9.13 (s, 1H), 8.76 (s, 1H), 4.35 (q, 2H J= 7.3 Hz), 1.33 (t, 3H J= 7.3 Hz). LCMS (method B): R_{T} = 2.82 min, M+H⁺ = 286, 288.

Step 4: 7-Bromo-3-trifluoromethanesulfonyloxyfuro[3,2-c]pyridine-2-carboxylic acid ethyl ester

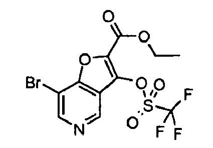

Trifluoromethanesulfonic anhydride (8.32 ml, 49.66 mmol) in dry DCM (70 ml) was added dropwise to a stirred solution of 7-bromo-3-hydroxyfuro[3,2-c]pyridine-2-carboxylic acid ethyl ester (12.80 g, 44.7 mmol) and pyridine (10.88 ml, 128 mmol) in dry DCM (400 ml) at 5-10°C. The resulting mixture was stirred for 1.5 hours at 5-10°C then allowed to warm to room temperature slowly over 3 hours before being left to stand for 16 hours. The mixture was diluted with DCM, washed with 1M aqueous HCl, water, saturated aqueous NaHCO₃ and brine, before being dried over magnesium sulfate, filtered and concentrated *in vacuo* to give light brown oil. The oil was purified by flash chromatography (SiO₂, dichloromethane) to give the title compound as a yellow solid (11.84 g 63%). 1H NMR (CDCl₃, 400 MHz) 8.96 (s, 1H), 8.83 (s, 1H), 4.55 (q, 2H J = 7.2 Hz), 1.47 (t, 3H J= 7.2 Hz).

Step 5: 7-Bromo-3-(2-fluoro-4-trimethylsilanylphenylamino)furo[3,2-c]pyridine-2-carboxylic acid ethyl ester

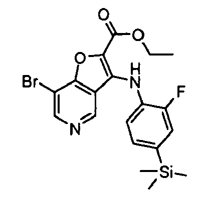

7-Bromo-3-trifluoromethanesulfonyloxyfuro[3,2-c]pyridine-2-carboxylic acid ethyl ester (11.84 g, 28.3 mmol) was stirred in dry toluene (160 ml) with Pd₂(dba)₃ (1.0 g, 1.20 mmol), Xantphos (0.572 g, 1.0 mmol) and potassium phosphate tribasic (11.25 g, 53.75 mmol). The mixture was de-gassed before the addition of a solution 2-fluoro-4-trimethylsilanylphenylamine (5.38 g, 29.54 mmol) in dry toluene (10 ml). The mixture was de-gassed again before heating at 115°C for 4 hours. The reaction mixture was partitioned between ethyl acetate and water then filtered and the layers separated. The organic layer was washed with water then brine, dried over magnesium sulfate, filtered and evaporated *in vacuo* to give a brown solid. The solid was purified by flash chromatography (SiO₂, 30% cyclohexane in DCM) to give the title compound as a pale yellow solid (7.1 g, 55%). 1H NMR (CDCl₃, 400 MHz) 8.66 (s, 1H), 8.50 (s, 1H), 7.73 (s, 1H), 7.27 (m, 3H), 4.50 (q, 2H J = 7.0 Hz), 1.47 (t, 3H J= 7.0 Hz), 0.29 (s, 9H). LCMS (method B): R_{T} = 4.81 min, M+H⁺ = 451,453.

Step 6: 3-(2-Fluoro-4-trimethylsilanylphenylamino)-7-phenylfuro[3,2-c]pyridine-2-carboxylic acid ethyl eater

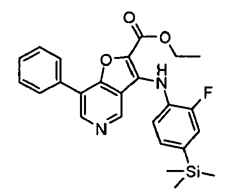

7-Bromo-3-(2-fluoro-4-trimethylsilanylphenylamino)furo[3,2-c]pyridine-2-carboxylic acid ethyl ester (100 mg, 0.22 mmol) was stirred as a suspension at room temperature in ethanol (2 ml) with phenyl boronic acid (30 mg, 0.242 mmol) under argon. After stirring for 20 minutes Pd(OAc)₂ (2 mg, 0.66 µmol), triphenyl phosphine (0.5 mg, 0.002 mmol) and 2M aqueous Na₂CO₃ (130 µl, 0.264 mmol) were added and the reaction mixture de-gassed then heated at reflux under argon for 3 hours. The reaction mixture was cooled to room temperature and diluted with water then extracted with ethyl acetate. The organic layer was washed with water then brine and then dried over magnesium sulfate, filtered and evaporated *in vacuo* to give a yellow solid. This solid was purified by flash chromatography (SiO₂ 30% cyclohexane in DCM) to give the title compound as a pale yellow solid (31 mg, 31%). 1H NMR (CDCl₃, 400 MHz) 8.73 (s, 1H), 8.61 (s, 1H), 7.87 (d, 2H J = 7.80 Hz), 7.72 (s, 1H), 7.59-7.44 (m, 3H), 7.35 - 7.25 (m, 3H), 4.33 (q, 2H J = 7.0 Hz) 1.43 (t, 3H J= 7.0 Hz) 0.30 (s, 9H). LCMS (method B): R_{T} = 4.91 min, M+H⁺ = 449.

Step 7: 3-(2-Fluoro-4-iodophenylamino)-7-phenylfuro[3,2-c]pyridine-2-carboxylic acid ethyl ester

3-(2-Fluoro-4-trimethylsilanylphenylamino)-7-phenylfuro[3,2-c]pyridine-2-carboxylic acid ethyl ester (30 mg, 0.067 mmol) was stirred as a solution in DCM (2 ml) at 0-5°C and treated dropwise with 1M IC1 in DCM (130 µl, 0.13 mmol). The resulting mixture was stirred at 0-5°C for 2 hours before the addition 1M aqueous Na₂S₂O₃ (1 ml). The layers were separated and the organic layer was washed with water, followed by brine, dried over magnesium sulfate, filtered and evaporated *in vacuo* to give the title compound (quantitative). 1H NMR (CDCl₃, 400MHz) 8.75 (s, 1H), 8.58 (s, 1H), 7.87 (d, 2H J = 7.80 Hz), 7.67 (s, 1H), 7.59-7.44 (m, 5H), 7.05 (t, 1H J = 8.50 Hz), 4.47 (q, 2H J = 7.10 Hz), 1.43 (t, 3H J= 7.10 Hz). LCMS (method B): R_{T} = 4.40 min, M+H⁺ = 503.

3-(2-Fluoro-4-iodophenylamino)-7-methylfuro[3,2-c]pyridine-2-carboxylic acid ethyl ester

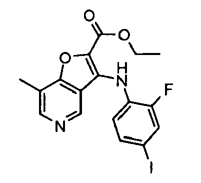

Step 1: 3-(2-Fluoro-4-trimethylsilanylphenylaminol-7-methylfuro[3,2-c]pyridine-2-carboxylic acid ethyl ester

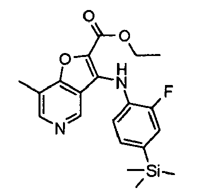

7-Bromo-3-(2-fluoro-4-trimethylsilanylphenylamino)furo[3,2-c]pyridine-2-carboxylic acid ethyl ester (1.0 g, 2.2 mmol) was stirred with potassium carbonate (456.5 mg, 3.3 mmol), tetrakistriphenylphosphine palladium(0) (255 mg, 0.22 mmol) and trimethylboroxine (305 µ1, 2.2 mmol) in dry 1,4-dioxane (5 ml). The reaction mixture was de-gassed before heating at 110°C under argon for 6 hours then cooled to room temperature and left to stand for 16 hours. The reaction mixture was diluted with dichloromethane and water. The organic layer was separated, washed with water flowed by brine, dried over magnesium sulfate, then filtered and evaporated *in vacuo* to give a residue. The crude residue was purified by flash chromatography (SiO₂, 30% cyclohexane in DCM then 1% methanol in DCM) to give the title compound as a pale yellow solid (710 mg, 83%) 1H NMR (CDCl₃ , 400 MHz) 8.50 (s, 1H), 8.3 (s, 1H), 7.70 (s, 1H), 7.32-7.22 (m, 3H), 4.48 (q, 2H J = 7.0 Hz), 2.54 (s, 3H), 1.46 (t, 3H J = 7.0 Hz), 0.29 (s 9H). LCMS (method B): R_{T} = 4.12 min, M+H⁺ = 387.

Step 2: 3-(2-Fluoro-4-iodophenylamino)-7-methylfuro[3,2-c]pyridine-2-carboxylic acid ethyl ester

3-(2-Fluoro-4-trimethylsilanylphenylamino)-7-methylfuro[3,2-c]pyridine-2-carboxylic acid ethyl ester (710 mg, 1.84 mmol) was stirred as a solution in DCM (25 ml) at 0-5°C and treated dropwise with 1M IC1 in DCM (3.5 ml, 3.5 mmol). The resulting mixture was stirred at 0-5°C for 2 hours before the addition 1M aqueous Na₂S₂0₃ (12 mL). The layers were separated and the organic layer was washed with water, brine, then dried over magnesium sulfate, filtered and evaporated *in vacuo* to give a residue. The crude residue was purified by flash chromatography (SiO₂, gradient 0-1% MeOH in DCM) to give the title compound as a pale yellow solid (448 mg, 55%). 1H NMR (CDC1₃ , 400 MHz) 8.47 (s, 1H), 8.39 (s, 1H), 7.65 (s, 1H), 7.52 (dd, 1H J = 9.8, 1.9 Hz), 7.44 (dt, 1H J = 8.4, 1.3 Hz), 7.00 (t, 1H J = 8.5 Hz), 4.48 (q, 2H J = 7.0 Hz), 2.53 (s, 3H), 1.46 (t, 3H J = 7.0 Hz). LCMS (method B): R_{T} = 3.39 min, M+H⁺ = 441.

2-((R)-2,2-Dimethyl-[1,3]dioxolan-4-ylmethoxycarbamoyl)-3-(2-fluoro-4-iodo-phenylamino)-furo[3,2-c]pyfidine-7-carboxylic acid ethyl ester

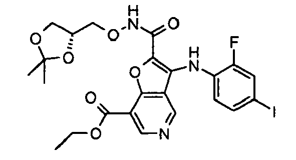

Step 1: 3-Hydroxy-furo[3,2-c]pyridine-2.7-dicarboxylic acid 2-benzyl ester 7-ethyl ester

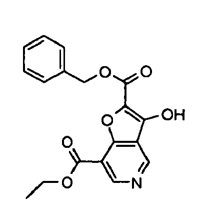

To a solution of 4-chloro-pyridine-3,5-dicarboxylic acid diethyl ester (250 mg, 0.971 mmol) and benzyl glycolate (145 µl, 1.019 mmol) in DMF (5 ml) at 0°C, was added sodium hydride (97 mg, 2.43 mmol, 60% dispersion in mineral oil). The mixture was allowed to warm to room temperature and stirred for 3 hours then quenched by the addition of acetic acid (1 ml). The mixture was then concentrated *in vacuo* and the resulting residue was triturated in water and filtered. The resulting solid was recrystallised from methanol/water to afford the title compound as a pale yellow solid (120 mg, 36%). LCMS (method B): R_{T} = 3.29 min, M+H⁺ = 342.

Step 2: 3-Trifluoromethanesulfonyloxy-furo[3,2-c]pyridine-2,7-dicarboxylic acid 2-benzyl ester 7-ethyl ester

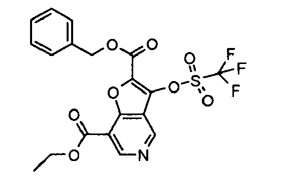

To a solution of 3-hydroxy-furo[3,2-c]pyridine-2,7-dicarboxylic acid 2-benzyl ester 7-ethyl ester (120 mg, 0.352 mmol) and pyridine (85 µl, 1.056 mmol) in dichloromethane (1.5 ml) at 0°C was added trifluoromethanesulfonic anhydride (63 µl, 0.37 mmol) dropwise. The reaction was stirred at room temperature for 90 minutes then partitioned between dichloromethane (30 ml) and O.1M HCl (10 ml). The organic layer was isolated and washed with saturated sodium bicarbonate (10 ml) then brine (10 ml). The isolated organic layer was dried over magnesium sulfate before being concentrated *in vacuo* to afford the title compound as a colourless oil (88 mg, 53%). ¹H NMR (CDC1₃, 400 MHz) 9.27 (1H, s), 9.17 (1H, s), 7.48 (2H, m), 7.38 (3H, m), 5.48 (2H, s), 4.52 (2H, q, J= 7.2 Hz), 1.43 (3H, t, J = 7.2 Hz).

Step 3: 3-(2-Fluoro-4-trimethylsilanyl-phenylamino)-furo[3,2-c]pyridine-2,7-dicarboxylic acid 2-benzyl ester 7-ethyl ester

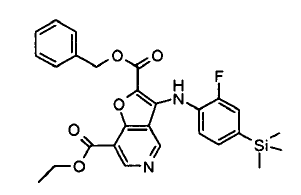

To a solution of 3-trifluoromethanesulfonyloxy-furo[3,2-c]pyridine-2,7-dicarboxylic acid 2-benzyl ester 7-ethyl ester (88 mg, 0.186 mmol) and 2-fluoro-4-trimethylsilanyl-phenylamine (41 mg 0.223 mmol) in toluene (1.5 ml) was added potassium phosphate (55 mg, 0.26 mmol) before the mixture was degassed. Pd₂dba₃ (8.5 mg, 0.0093 mmol) and Xantphos (11 mg, 0.0186 mmol) were added to this mixture and the vessel was flushed with argon. The reaction mixture was heated to reflux for 1.5 hours, cooled and filtered through Celite ® washing with ethyl acetate. The filtrate was washed with saturated sodium bicarbonate (10 ml), then dried over magnesium sulfate and concentrated *in vacuo.* Purification of the resultant residue by flash chromatography (Si-SPE, cyclohexane : t-butyl methyl ether gradient 1:0 to 3:1) afforded the title compound as a pale yellow solid (48 mg, 51%). LCMS (method B): R_{T} = 4.89 min, M+H⁺ = 507.

Step 4: 3-(2-Fluoro-4-trimethylsilanyl-phenylamino)-furo[3,2-c]pyridine-2,7-dicarboxylic acid 7-ethyl ester

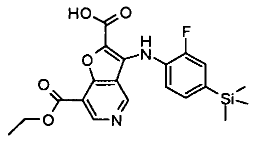

To a solution of 3-(2-fluoro-4-trimethylsilanyl-phenylamino)-furo[3,2-c]pyridine-2,7dicarboxylic acid 2-benzyl ester 7-ethyl ester (48 mg, 0.0949 mmol) in ethyl acetate (2 ml) under nitrogen, was added palladium on carbon (12 mg, 10% palladium on activated charcoal). The suspension was stirred at room temperature for 2 hours under an atmosphere of hydrogen. The reaction mixture was filtered through Celite ®, washing with ethyl acetate, and the filtrate was concentrated *in vacuo* to afford the title product as a colourless oil (34 mg, 86%). LCMS (method B): R_{T} = 4.31 min, M+H⁺ = 417, [M-H]⁻ = 415.

Step 5: 2-((R)-2,2-Dimethyl-[1,3]dioxolan-4-ylmethoxycarbamoyl)-3-(2-fluoro-4-iodo-phenylamino)-furo[3,2-c]pyridine-7-carboxylic acid ethyl ester

To a solution of 2-((R)-2,2-dimethyl-[1,3]dioxolan-4-ylmethoxycarbamoyl)-3-(2-fluoro-4-trimethylsilanyl-phenylamino)-furo[3,2-c]pyridine-7-carboxylic acid ethyl ester (37 mg, 0.068 mmol) in dichloromethane (2 ml) at -5°C was added iodine monochloride (136 µl, 0.136 mmol, 1M solution in dichloromethane) and the solution was stirred at this temperature for 1 hour. A saturated solution of sodium thiosulfate (5 ml) was added and the mixture was poured into saturated sodium thiosulfate (15 ml). The aqueous layer was isolated and extracted with dichloromethane (2 × 25 ml), before the combined organic layers were washed with brine, dried over magnesium sulfate and concentrated *in vacuo.* Purification of the resultant residue by flash chromatography (Si-SPE, dichloromethane : ethyl acetate gradient 1:0 to 0:1 1 then 15% methanol in dichloromethane) afforded the title compound as a yellow waxy solid (29 mg, 71 %). LCMS (method B): R_{T} = 3.92 min, M+H⁺ = 600.

2-Dimethylcarbamoyl-3-(2-fluoro-4-trimethylsilanyl-phenylamino)-furo[3,2-c]pyridine-7-carboxylic acid ethyl ester

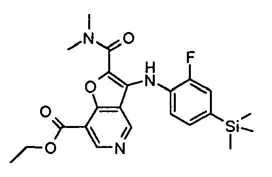

Step 1: 2-Dimethylcarbamoyl-3-hydroxy-furo[3,2-c]pyridine-7-carboxylic acid ethyl ester

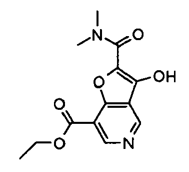

To a solution of 4-chloro-pyridine-3,5-dicarboxylic acid diethyl ester (430 mg, 1.67 mmol) and 2-hydroxy-N,N-dimethyl-acetamide (189 mg, 1.84 mmol) in DMF (7 ml) at 0°C was added sodium hydride (200 mg, 5.01 mmol, 60% dispersion in mineral oil). The resultant mixture was allowed to warm to room temperature and stirred for 2.5 hours. The reaction was quenched by the addition of acetic acid (1 ml). The mixture was then concentrated *in vacuo* and the resulting residue was triturated in water and filtered to afford the title compound as a pale yellow solid (200 mg, 43%). LCMS (method B): R_{T} = 2.73 min, M+H⁺ = 279.

Step 2: 2-Dimethylcarbamoyl-3-trifluoromethanesulfonyloxy-furo[3,2-c]pyridine-7-carboxylic acid ethyl ester 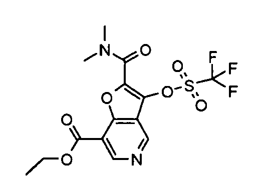 To a solution of 2-dimethylcarbamoyl-3-hydroxy-furo[3,2-c]pyridine-7-carboxylic acid ethyl ester (440 mg, 1.58 mmol) and pyridine (0.38 ml, 4.74 mmol) in dichloromethane (7 ml) at 0°C was added trifluoromethanesulfonic anhydride (0.29 ml, 1.74 mmol) dropwise. The reaction was stirred at room temperature for 120 minutes then partitioned between dichloromethane (50 ml) and 0.1M HCl (20 ml). The organic layer was washed with saturated sodium bicarbonate (20 ml) then brine (20 ml). The combined organic layers were dried over magnesium sulfate before being concentrated *in vacuo* to afford the title compound as a colourless oil (144 mg, 22%). LCMS (method B): R_{T} = 3.39 min, M+H⁺ = 411.

Step 3: 2-Dimethylcarbamoyl-3-(2-fluoro-4-trimethylsilanyl-phenylamino)-furo[3,2-c]pyridine-7-carboxylic acid ethyl ester

To a solution of 2-dimethylcarbamoyl-3-trifluoromethanesulfonyloxyfuro[3,2-c]pyridine-7-carboxylic acid ethyl ester (144 mg, 0.351 mmol) and 2-fluoro-4-trimethylsilanyl-phenylamine (90 mg 0.492 mmol) in toluene (3 ml) was added potassium phosphate (149 mg, 0.70 mmol) before the mixture was degassed. Pd₂dba₃ (16.1 mg, 0.0176 mmol) and Xantphos (20 mg, 0.035 mmol) were added to the reaction mixture and the vessel was flushed with argon. The reaction mixture was then heated to reflux for 3 hours, cooled and filtered through Hyflo, washing with ethyl acetate. The filtrate was washed with saturated sodium bicarbonate (30 ml), the organic layer dried over magnesium sulfate and concentrated *in vacuo.* Purification of the resultant residue by flash chromatography (Si-SPE, cyclohexane : t-butyl methyl ether gradient 3:1 to 1:1) afforded the title compound as a pale yellow solid (84 mg, 54%). LCMS (method B): R_{T} = 4.58 min, M+H⁺ = 444.

7-Fluoro-3-(2-fluoro-4-iodo-phenylamino)-furo[3,2-c]pyridine-2-carboxylic acid ethyl ester

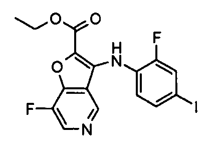

Step 1: 4-Chloro-5-fluoro-nicotinic acid ethyl ester

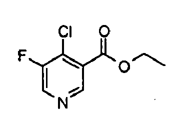

A suspension of 4-chloro-5-fluoro-nicotinic acid (0.36 g, 2.06 mmol) in thionyl chloride (3 ml) was heated at 80°C for 2 hours until most of the solid had dissolved. The reaction mixture was concentrated *in vacuo* and the residue azeotroped with toluene (2 x 20 ml). The resultant residue was dissolved in ethanol (5 ml) and diisopropylethylamine (1.76 ml, 10.31 mmol) and the reaction mixture was stirred at room temperature for 18 hours. The reaction mixture was concentrated *in vacuo,* diluted with ethyl acetate and washed with 0.1M HCl then saturated sodium bicarbonate and brine. The organic layer was dried over magnesium sulfate, filtered and concentrated *in vacuo.* Purification of the resultant residue by flash chromatography (Si-SPE, dichloromethane : ethyl acetate gradient 1:0 to 92:8) afforded the title compound as a colourless oil (415 mg, 99%). ¹H NMR (CDCl₃, 400 MHz) 1.43 (3H, t, J= 7.1 Hz), 4.46 (2H, q, J= 7.1 Hz), 8.60 (1H, d, J = 0.8 Hz), 8.86 (1H, s).

Step 2: 7-Fluoro-3-hydroxy-furo[3,2-c]pyridine-2-carboxylic acid ethyl ester

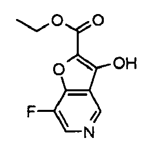

To a solution of 4-chloro-5-fluoro-nicotinic acid ethyl ester (400 mg, 1.975 mmol) and ethyl glycolate (196 µl, 2.074 mmol) in DMF (10 ml) at 0°C, was added sodium hydride (158 mg, 3.95 mmol, 60% dispersion in mineral oil) and the mixture was allowed to warm to room temperature then stirred for 2 hours. The reaction was quenched by the addition of acetic acid (1.5 ml). The mixture was then concentrated *in vacuo* and the resulting residue was triturated in water to afford the title compound as a pale yellow solid (460 mg, quantitative). LCMS (method B): R_{T} = 2.59 min, M+H⁺ = 226.

Step 3: 7-Fluoro-3-trifluoromethanesulfonyloxy-furo[3,2-c]pyridine-2-carboxylic acid ethyl ester

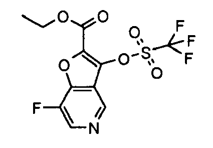

To a solution of 7-fluoro-3-hydroxy-furo[3,2-c]pyridine-2-carboxylic acid ethyl ester (460 mg, 1.97 mmol) and pyridine (0.48 ml, 5.91 mmol) in dichloromethane (10 ml) at 0°C was added trifluoromethanesulfonic anhydride (612 mg, 2.17 mmol) dropwise. The reaction was stirred at room temperature for 90 minutes then partitioned between dichloromethane (50 ml) and 0.1M HCl (20 ml). The organic layer was isolated and washed with saturated sodium bicarbonate (20 ml) then brine (20 ml). The combined organic layers were dried over magnesium sulfate before being concentrated *in vacuo.* Purification of the resultant residue by flash chromatography (Si-SPE, dichloromethane) afforded the title compound as a colourless oil (470 mg, 67%). LCMS (method B): R_{T} = 3.76 min, M+H⁺ = 358.

Step 4: 7-Fluoro-3-(2-fluoro-4-trimethylsiulayl-phenylamino)-frro[3,2-c]pyridine-2-carboxylic acid ethyl ester

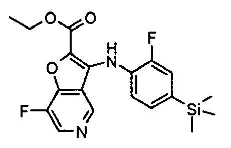

To a solution of 7-fluoro-3-trifluoromethanesulfonyloxy-furo[3,2-c]pyridine-2-carboxylic acid ethyl ester (470 mg, 1.32 mmol) and 2-fluoro-4-trimethylsilanyl-phenylamine (337 mg, 1.84 mmol) in toluene (15 ml) was added potassium phosphate (558 mg, 2.63 mmol) and the mixture was degassed. Pd₂dba₃ (60.5 mg, 0.066 mmol) and Xantphos (76.5 mg, 0.132 mmol) were added to the reaction mixture and the vessel was flushed with argon. The reaction mixture was heated to reflux for 4 hours, cooled and filtered through Hyflo, washing with ethyl acetate. The filtrate was washed with saturated sodium bicarbonate, the organic layer dried over magnesium sulfate and concentrated *in vacuo.* Purification of the resultant residue by flash chromatography (Si-SPE, dichloromethane : ethyl acetate gradient 1:0 to 9:1) afforded the title compound as a pale yellow solid (490 mg, 95%). LCMS (method B): R_{T} = 4.70 min, M+H⁺ = 391.

Step 5: 7-Fluoro-3-(2-fluoro-4-iodo-phenylamino)-furo[3,2-c]pyridine-2-carboxylic acid ethyl ester

To a solution of 7-fluoro-3-(2-fluoro-4-trimethylsilanyl-phenylamino)-furo[3,2-c]pyridine-2-carboxylic acid ethyl ester (490 mg, 1.256 mmol) in dichloromethane (8 ml) at -10°C was added iodine monochloride (2.51 ml, 2.51 mmol, 1M solution in dichloromethane) and the solution was stirred between -10°C and 0°C for 2 h. A saturated solution of sodium thiosulfate (5 ml) was added and the mixture was poured into saturated sodium thiosulfate (15 ml). The aqueous layer was isolated then extracted with dichloromethane (3 × 25 ml) before the combined organic layers were washed with brine, dried over magnesium sulfate and concentrated *in vacuo.* Purification of the resultant residue by flash chromatography (Si-SPE, cyclohexane : ethyl acetate gradient 1:0 to 3:1 then dichloromethane) gave crude material. The crude material was triturated in cyclohexane to afford the title compound as a yellow waxy solid (250 mg, 45%). LCMS (method B): R_{T} = 4.13 min, M+H⁺ = 445.

7-Fluoro-3-(2-fluoro-4-iodo-phenylamino)-furo[3,2-c]pyridine-carboxylic acid ethyl ester

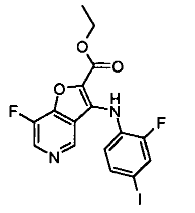

Step 1: 4-Chloro-5-fluoro-pyridine-3-carbaldehyde oxime

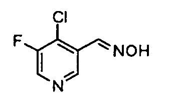

To a cooled (-78°C) solution of 3-fluoro-4-chloro-pyridine (11.0 g, 84 mmol) in THF under nitrogen was added lithium diisopropylamide (1.8 M solution, 47 mL, 84 mmol) dropwise and the resultant solution stirred at -70 to -80°C for 18 hours. DMF (7.68 g, 1.25 eq.) was added dropwise and stirring continued at -78°C for 30 minutes before adding the reaction mixture to ice/2M HCl. The solution was extracted with diethyl ether and the organic layer back-extracted with 2M HCl, the two aqueous solutions held separately. The aqueous extracts were each treated with hydroxylamine hydrochloride (8.76 g, 126 mmol) and adjusted to pH 5 with potassium carbonate. After stirring for 1 hour the mixtures were extracted with ethyl acetate (x 2), the combined organic extracts dried (Na₂SO₄), filtered and concentrated *in vacuo* to afford the title compound as a tan solid (11.07 g, 76%). LCMS (method B): R_{T} = 2.49 min, M+H⁺ 175.

Step 2: 4-Chloro-5-fluoronicotinonitrile

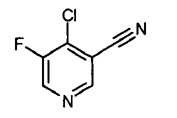

To a suspension of 4-chloro-5-fluoro-pyridine-3-carbaldehyde oxime (6.8 g, 39.0 mmol) in dichloromethane (150 ml) was added carbonyl diimidazole (9.5 g, 58.5 mmol). The mixture was then heated at reflux for 30 minutes then cooled to room temperature, before being washed with saturated aqueous sodium bicarbonate followed by water. The organic layer was dried over sodium sulfate and concentrated *in vacuo* then the resultant residue triturated in diethyl ether / cyclohexane to afford the title compound as a pale yellow solid (4.05 g, 79%). ¹H NMR (CDCl₃ 400 MHz) 8.71 (1H, d, J= 0.4 Hz), 8.70 (1H, s).

Step 3: 3-Amino-7-fluoro-furo[3,2-c]pyridine-2-carboxylic acid ethyl ester

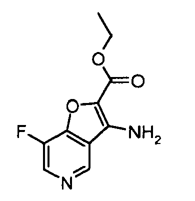

4-Chloro-5-fluoronicotinonitrile (4.0 g, 25.6 mmol) was dissolved in DMF (50 ml) and treated with potassium carbonate (17.8 g, 128 mmol) followed by ethyl glycolate (3.64 ml, 38.4 mmol). The resultant reaction mixture was heated at 80°C for 50 minutes, then cooled to room temperature and diluted with ethyl acetate. The solution was washed with water (x 2), dried (Na₂SO₄), filtered and concentrated *in vacuo.* The resultant solid was triturated in diethyl ether to give the title compound as an off-white solid (3.58 g, 63%). LCMS (method B): R_{T} = 2.65 min, M+H⁺ 225.

Step 4: 7-Fluoro-3-(2-fluoro-4-trimethylsilanyl-phenylamino)- furo[3,2-c]pyridine-2-carboxylic acid ethyl ester

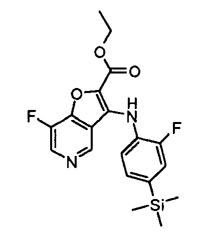

A degassed solution of 3-amino-7-fluoro-furo[3,2-c]pyridine-2-carboxylic acid ethyl ester (2.5 g, 11.1 mmol), trifluoro-methanesulfonic acid 2-fluoro-4-trimethylsilanyl-phenyl ester (4.2 g, 13.3 mmol), Pd₂dba₃ (508 mg, 0.56 mmol), Xantphos (642 mg, 1.12 mmol) and CS₂CO₃ (7.2 g, 22.2 mmol) in toluene (25 ml) was heated at reflux for 1 hour. The reaction mixture was cooled to ambient temperature then filtered through a pad of Celite ® washing with ethyl acetate. The filtrate was concentrated in vacuo and the resultant residue subjected to flash chromatography (Si-SPE, gradient 0-30% ethyl acetate in cyclohexane) to provide a red/orange residue. The residue was triturated in methanol to give the title compound as a yellow solid (2.5 g, 58%). LCMS (method B): R_{T} = 4.71 min, M+H⁺ 391.

Step 5: 7-Fluoro-3-(2-fluoro-4-iodo-phenylamino)-furo[3,2-c]pyridine-carboxylic acid ethyl ester

To a solution of 7-fluoro-3-(2-fluoro-4-trimethylsilanyl-phenylamino)furo[3,2c]-pyridine-carboxylic acid ethyl ester (2.5 g, 6.4 mmol) in dichloromethane (60 ml) at 0°C was added iodine monochloride (2.08 g, 12.8 mmol, solution in dichloromethane) and the solution was stirred and allowed to warm for 45 minutes. The precipitated solid was filtered off, the residue retained, and the filtrate washed with saturated aqueous sodium thiosulfate, dried (Na₂SO₄), filtered and concentrated *in vacuo* to give a residue. The residues from filtration and concentration were combined and triturated in diethyl ether to give a pale tan solid (2.58 g, 91%). LCMS (method B): R_{T} = 4.14 min, M+H⁺ = 445.

4-Chloro-3-(2-fluoro-4-iodo-phenylamino)-furo[3,2-c]pyridine-2-carboxylic acid ethyl ester

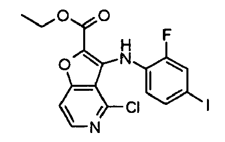

Step 1: 2,4-Dichloro-nicotinic acid ethyl ester

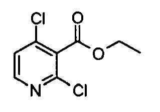

To a solution of diisopropylamine (2.4 ml, 16.9 mmol) in THF (40 ml) at - 78°C under inert atmosphere, was added n-butyllithium (10.6 ml, 16.9 mmol, 1.6M in hexanes) and the solution was stirred for 15 min at -78°C. 2,4-Dichloropyridine (1.8 mL, 16.9 mmol) was added dropwise and the reaction mixture was stirred at -78°C for 2 hours before the addition of ethyl cyanoformate (4.0 ml, 40.4 mmol). The reaction was then stirred at -78°C for 1 hour and then allowed to warm to room temperature. The mixture was then partitioned between water and ethyl acetate and the layers separated. The organic layer was washed with a saturated solution of sodium bicarbonate flowed by brine, dried over magnesium sulfate, then concentrated *in vacuo.* Purification of the resultant residue by flash chromatography (Si-SPE, pentane : diethyl ether gradient 1:0 to 4:1) afforded the title compound as colourless oil (1.6 g, 43%). ¹H NMR (CDCl₃, 400 MHz) 8.34 (1H, d, J= 5.4 Hz), 7.3 (1H, d, J= 5.4 Hz), 4.49 (2H, q, J= 7.1 Hz), 1.43 (3H, t, J= 7.1 Hz).

Step 2: 4-Chloro-3-hydroxy-furo[3,2-c]pyridine-2-carboxylic acid ethyl ester

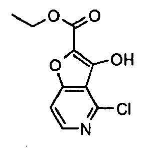

To a solution of 2,4-dichloro-nicotinic acid ethyl ester (1.6 g, 7.3 mmol) and ethyl glycolate (0.72 mL, 7.6 mmol) in DMF at 0°C under an inert atmosphere was added sodium hydride (60% in mineral oil, 584 mg, 14.6 mmol) portionwise. The reaction mixture was stirred at 0°C for 3 hours, quenched by careful addition of acetic acid (ca. 5 mL), diluted with water and extracted into ethyl acetate. The organic layer was separated, washed with water, then brine, dried over sodium sulphate and concentrated to give the title compound as a yellow solid (1.75 g, 100%). LCMS (method B): R_{T} = 2.99 min, M+H⁺ = 242.

Step 3: 4-Chloro-3-trifluoromethanesulfonyloxy-furo[3,2-c]pyridine-2-carboxylic acid ethyl ester

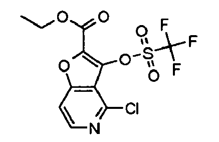

A mixture of 4-chloro-3-hydroxy-furo[3,2-c]pyridine-2-carboxylic acid ethyl ester (1.8 g, 7.5 mmol), N-phenyl-trifluoromethanesulfonimide (5.0 g, 14.0 mmol) and diisopropylethyl amine (5.5 mL, 32.3 mmol) in dimethoxyethane (30 mL) was stirred at 90°C for 48 hours, cooled to room temperature and concentrated under reduced pressure. The resultant residue was purified by flash chromatography (Si-SPE, cyclohexane : ethyl acetate, gradient 1: 0 to 1:1) to afford the title compound as a pale yellow solid (1.06 g, 38%). LCMS (method B): R_{T} = 3.94 min, M+H⁺ = 374.

Step 4: 4-Chloro-3-(2-fluoro-4-trimethylsilanyl-phenylamino)-furo[3,2-c]pyridine-2-carboxylic acid ethyl ester

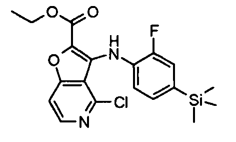

A degassed solution of 4-chloro-3-trifluoromethanesulfonyloxy-furo[3,2-c]pyridine-2-carboxylic acid ethyl ester (810 mg, 2.17 mmol), 2-fluoro-4-trimethylsilanyl-phenylamine (306 mg, 1.67 mmol), Pd₂dba₃ (31 mg, 0.03 mmol), Xantphos (58 mg, 0.10 mmol) and cesium carbonate (817 mg, 2.50 mmol) in toluene (17 ml) was heated at reflux under an argon atmosphere for 16 hours. The reaction mixture was filtered through Celite ® and concentrated *in vacuo.* The resultant residue was purified by flash chromatography (Si-SPE, pentane: diethyl ether, gradient 1: 0 to 0:1) to afford the title compound as a white solid (558 mg, 82%). LCMS (method B): R_{T} = 4.64 min, M+H⁺ = 407.

Step 5: 4-Chloro-3-(2-fluoro-4-iodo-phenylamino)-furo[3,2-c]pyridine-2-carboxylic acid ethyl ester

To a solution of 4-chloro-3-(2-fluoro-4-trimethylsilanyl-phenylamino)-furo[3,2-c]pyridine-2-carboxylic acid ethyl ester (265 mg, 0.65 mmol) in dichloromethane (6.5 ml) at 0°C was added iodine monochloride (1.3 ml, 1.3 mmol, 1M solution in dichloromethane) and the solution was stirred at this temperature for 1 hour. A saturated solution of sodium thiosulfate (5 ml) was added and the mixture was poured into saturated sodium thiosulfate (25 ml). The aqueous layer was extracted with dichloromethane (2 × 25 ml), the combined organic layers were washed with brine, dried over magnesium sulfate and concentrated *in vacuo* to yield the title compound as a yellow solid (239 mg, 80%). LCMS (method B): R_{T} = 4.22 min, M+H⁺ = 461.

3-(2-Fluoro-4-iodo-phenylamino)-4-meth-furo[3,2-c]pyridine-2-arboxylic acid ethyl ester

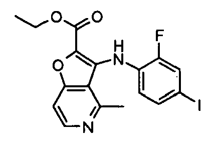

Step 1: 3-(2-Fluoro-4-trimethysilanyl-phenylamino)-4-methylfuro[3,2]pyridine-2-carboxylic acid ethyl ester

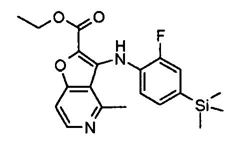

To a solution of 4-chloro-3-(2-fluoro-4-trimethylsilanyl-phenylamino)-furo[3,2-c]pyridine-2-carboxylic acid ethyl ester (406 mg, 1.0 mmol) in dioxane (5 mL) were added trimethylboroxine (0.14 mL, 1.0 mmol), tetrakis(triphenylphosphine)palladium (115 mg, 0.1 mmol) and potassium carbonate (207 mg, 1.5 mmol) before the mixture was degassed and heated at reflux for 6 hours. The reaction mixture was cooled to room temperature and filtered through a pad of Celite ® which was washed with ethyl acetate. The filtrates were combined and concentrated *in vacuo* to give a residue which was purified by flash chromatography (Si-SPE, pentane : diethyl ether 1:0 to 0:1). The title compound was obtained as a pale yellow oil (221 mg, 57%). LCMS (method B): R_{T} = 3.53 min, M+H⁺= 387.

Step 2: 3-(2-Fluoro-4-iodo-phenylamino)-4-methyl-furo[3,2-c]pyridine-2-carboxylic acid ethyl ester

To a solution of 3-(2-fluoro-4-trimethylsilanyl-phenylamino)-4-methylfuro[3,2]pyridine-2-carboxylic acid ethyl ester (215 mg, 0.56 mmol) in dichloromethane (5 ml) at 0°C was added iodine monochloride (1.1 ml, 1.1 mmol, 1M solution in dichloromethane) and the solution was stirred at this temperature for 1 hour. A saturated solution of sodium thiosulfate (5 ml) was added and the mixture was poured into saturated sodium thiosulfate (25 ml). The aqueous layer was extracted with dichloromethane (2 × 25 ml), the combined organic layers were washed with brine, dried over magnesium sulfate and concentrated *in vacuo.* Purification of the resultant residue by flash chromatography (Si-SPE, cyclohexane : dichloromethane gradient 1:0 to 0: 1) afforded the title compound as a yellow solid (241 mg, 98%). LCMS (method B): R_{T} = 2.99 min, M+H⁺ = 441.

3-2-Fluoro-4-methylsulfanyl-phenylamino)-furo[3,2-c]pyridine-2-carboxylic acid ethyl ester

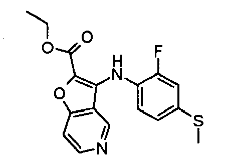

3-Trifluoromethanesulfonyloxy-furo[3,2-c]pyridine-2-carboxylic acid ethyl ester (1.26 g, 3.71 mmol) and 2-fluoro-4-methylsulfanyl-phenylamine (816 mg, 5.20 mmol) were dissolved in toluene (25 ml) and Pd₂(dba)₃ (170 mg, 0.19 mmol) was added, followed by Xantphos (214 mg, 0.37 mmol) and potassium phosphate tribasic (1.57 g, 7.42 mmol). The mixture was thoroughly degassed and purged with argon, then stirred under argon at 120°C for 16 hours. After cooling, the mixture was filtered through Celite ® then concentrated. Purification of the resultant residue by flash chromatography (Si-SPE, ether:pentane gradient 1:4 to 1:0) gave the title compound as a tan solid (770 mg, 60%). LCMS (method B): R_{T} = 3.29, M+H⁺ 347.

7-Fluoro-3-(2-fluoro-4-methylsulfanyl-phenylamino)-furo[3,2- c]idine-2-carboxylic acid ethyl ester

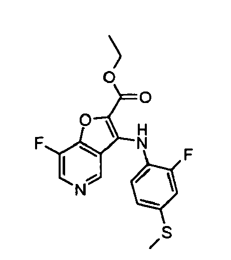

A degassed solution of 3-amino-7-fluoro-furo[3,2-c]pyridine-2-carboxylic acid ethyl ester (0.2 g, 0.89 mmol), 1-bromo-2-fluoro-4-methylsulfanyl-benzene (0.34 g, 1.5 mmol), Pd₂dba₃ (0.041 g, 0.045 mmol), Xantphos (0.052 g, 0.089 mmol) and K₃PO₄ (0.38 g, 1.8 mmol) in toluene (5 ml) was heated at reflux for 18 hours. The reaction mixture was cooled to ambient temperature then filtered through a pad of Hyflo washing with ethyl acetate. The filtrate was concentrated *in vacuo* and the resultant residue subjected to flash chromatography (Si-SPE, gradient 0-10% ethyl acetate in dichloromethane) to provide the title compound as a yellow solid (0.18 g, 55%). LCMS (method B): R_{T} = 3.95 min, M+H⁺ 365.

7-Chloro-3-(2-fluoro-4-methylsulfanyl-phenviamino)-furo[3,2-c]pyridine-2-carboxylic acid ethyl ester

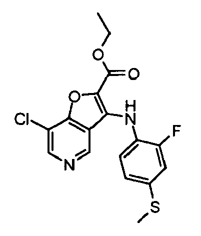

A degassed solution of 3-amino-7-chloro-furo[3,2-c]pyridine-2-carboxylic acid ethyl ester (0.1 g, 0.42 mmol), 1-bromo-2-fluoro-4-methylsulfanyl-benzene (0.16 g, 0.71 mmol), Pd₂dba₃ (0.019 g, 0.021 mmol), Xantphos (0.024 g, 0.042 mmol) and K₃PO₄ (0.18 g, 0.83 mmol) in toluene (2.5 ml) was heated at reflux for 18 hours. The reaction mixture was cooled to ambient temperature then filtered through a pad of Hyflo washing with ethyl acetate. The filtrate was concentrated *in vacuo* and the resultant residue subjected to flash chromatography (Si-SPE, gradient 0-10% ethyl acetate in dichloromethane) to provide the title compound as a pale yellow solid (0.087 g, 54%). LCMS (method B): R_{T} = 4.14 min, M+H⁺ 379.

SYNTHESIS OF REPRESENTATIVE AMINES AND HYDROXYLAMINES

Cyclopropylmethylhydroxylamine Hydrochloride

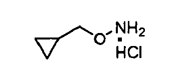

Prepared according to Marquez et al (2005) Synth. Comm. 35(17):2265-2269

O-((R)-2,2-dimethl-[1,3]dioxolan-4-ylmethyl)hydroxylamine

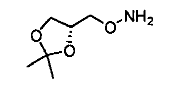

Prepared according to Bailey et al (1991) J. Med. Chem. 34(1):57-65

*O*-(2-Vinyloxy-ethyl)-hydroxylamine

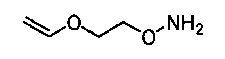

Prepared according to WO 0206213

N-Methyl-O-(2-vinyloxy-ethyl)-hydroxylamine

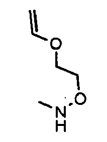

Formaldehyde (37% w/w in water, 80 µL, 1.0 mmol) was added to a cooled (0°C) solution of O-(2-vinyloxy-ethyl)-hydroxylamine (105 mg, 1.0 mmol) in ethanol (1 mL). The mixture was stirred for 30 minutes before addition of pyridinium para-toluene sulfonate (250 mg, 1.0 mmol) and sodium cyanoborohydride (70 mg, 1.1 mmol). The resultant suspension was allowed to warm to ambient temperature and stirred for 20 hours. The solvent was evaporated and the residue diluted with ethyl acetate (25 mL) and washed with brine (20 mL), dried (MgSO₄), filtered, then evaporated to provide the desired product as an oil (84 mg, 71%). 1H NMR (CDC1₃, 400MHz) 6.44-6.55 (m, 1H), 4.98 (s, 1H), 4.16-4.24 (m, 1H), 3.98-4.06 (m, 1H), 3.82-3.96 (m, 4H), 2.59 (s, 3H).

4-(tert-Butyl-dimethyl-silanyloxy)-isoxazolidine

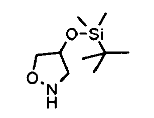

*tert*-Butyl-dimethyl-chlorosilane (0.5 g, 3.21 mmol) was added to a stirred solution of isoxazolidin-4-ol hydrochloride (0.40 g, 3.18 mmol) in DMF (3 mL) and the mixture left to stir at ambient temperature for 2.5 hours. The solvent was evaporated and the residue partitioned between ethyl acetate (50 mL) and water (20 mL). The organic phase was separated, washed with water (3 x 20 mL) then brine (20 mL), dried (MgSO₄), filtered and evaporated to provide the desired product as a colourless oil (0.62 g, 96%). 1H NMR (CDC1₃, 400MHz) 5.52 (s, 1H), 4.60-4.65 (m, 1H), 3.45-3.62 (m, 1H), 3.80-4.05 (m, 1H), 2.80-3.05 (m, 2H), 0.80 (s, 9H), 0.08 (s, 6H).

(S)-3-Aminooxy-pyrrolidine-1-carboxylic acid tert-butylester

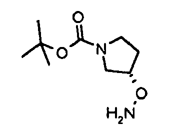

Step 1: (S)-3-(1,3-Dioxo-1,3-dihydro-isoindol-2-yloxy)-pyrrolidine-1-carboxylic acid tert-butyl ester

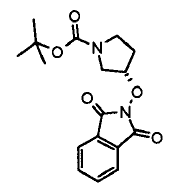

(R)-3-Hydroxy-pyrrolidine-1-carboxylic acid tert-butyl ester (1.37 g, 7.31 mmol) was dissolved in THF (20 mL), 2-hydroxy-isoindole-1,3-dione (1.19 g, 7.31 mmol) and triphenyl phosphine (1.92 g, 7.31 mmol) were added followed by dropwise addition of diisopropyl azodicarboxylate (1.33 mL, 8.04 mmol) over 10 minutes. The reaction mixture was allowed to stir at ambient temperature for 18 hours then the solvent was evaporated. The residue was purified by flash column chromatography (Si-SPE, DCM: EtOAc, gradient 100:0 to 80:20) to provide the title compound as a colourless oil (1.43 g, 59%). 1H NMR (CDC1₃, 400MHz) 7.86 (m, 2H), 7.77 (m , 2H), 4.94-5.02 (m, 1H), 3.66-3.84 (m, 2H), 3.50-3.65 (m, 2H), 2.24-2.32 (m, 1H), 1.93-2.05 (m, 1H), 1.49 (s, 9H).

Step 2: (S)-3-Aminooxy-pyrrolidine-1-carboxylic acid tert-butylester

Methyl hydrazine (0.23 mL, 4.40 mmol) was added dropwise over 5 minutes to a solution of (S)-3-(1,3-dioxo-1,3-dihydro-isoindol-2-yloxy)-pyrrolidine-1-carboxylic acid *tert*-butyl ester (1.43 g, 4.3 mmol) in DCM (12 mL). The mixture was stirred at ambient temperature for 1 hour then evaporated. The residue was suspended in diethyl ether (10 mL) and the solid was filtered. The filtrate was concentrated to provide the title compound as a colourless oil (0.86 g, 99%).1H NMR (CDC1₃, 400MHz) 4.24-4.26 (m, 1H), 3.60-3.66 (m, 1H), 3.44-3.54 (m, 1H), 3.30-3.42 (m, 2H), 2.03-2.12 (m, 1H), 1.84-1.96 (m, 1H), 1.46 (s, 9H).

2-Aminooxy-2-methyl-propan-1-ol hydrochloride

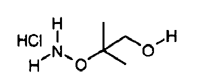

Step 1: 2-(N-Boc-aminooxy)isobutyric acid ethyl ester

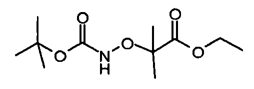

To a solution ofN-Boc-hydroxylamine (5.2 g, 39.05 mmol) in ethanol (100 mL) was added potassium hydroxide (2.6294 g, 46.86 mmol) and stirred at room temperature till the potassium hydroxide dissolved into solution. To this was added 2-Bromoisobutyric acid ethyl ester (6.87 mL, 46.86 mmol) and refluxed overnight. A white precipitate was observed after 1 hour. Reaction cooled to room temperature and then filtered. The white solid was discarded and the filterate was concentrated. The oily residue was partitioned between water (75 mL) and ether (3 X 100 mL). The combined ether layer was dried with sodium sulfate, filtered and the filterate was concentrated to give the title compound as a clear oil (9.543 g, 99%). LCMS (method C): R_{T} = 2.55 min, M+H⁺ = 247.9. 1H NMR (CDC1₃, 400MHz) 4.20 (q, 2H), 1.50 (s , 6H), 1.498 (s, 9H), 1.30 (t, 3H).

Step 2: 2-(N-Boc-aminooxy)-2-methylpropan-1-ol

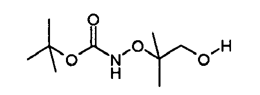

To a solution of 2-(N-Boc-aminooxy)isobutyric acid ethyl ester (2.35 g, 9.5 mmol) in anhydrous ethyl ether (100 mL) at 0°C under nitrogen was added 1.0 M Lithiumtetrahydroaluminate in tetrahydrofuran (17.106 mL, 17 mmol) and stirred at 0°C under nitrogen for 5 hours. To this was added a couple of CO₂ pellets (dry ice), followed by water (25 mL) at 0°C. This was then stirred overnight and let warm to room temperature in the process. The ether layer was decanted off and kept aside. The white solid was triturated with ether and the ether was combined with the ether layer obtained before. The white solid was then discarded. The combined ether layers was dried with sodium sulfate, filtered and concentrated to give the title compound as a white solid ( 1.94 g, 99.5%). 1H NMR (CDC1₃, 400MHz) 3.40 (s, 2H), 1.50 (s , 9H), 1.20 (s, 6H).

Step 3: 2-Aminooxy-2-methyl-propan-1-ol hydrochloride

To a solution of 2-(N-Boc-aminooxy)-2-methylpropan-1-ol (1.94 g, 9.45 mmol) in anhydrous dichloromethane (10 mL) was added 4 M HCl in dioxane (47.26 mL, 200 mmol) at room temperature and stirred for 1 hour. The reaction was concentrated under reduced pressure and the residue triturated with ether (3 X 30 mL) to give the title compound as an oil/white solid (HCl salt). The oil/white solid was dried under vacuum and used as is for the coupling step. (1.10 g, 82.2 %). 1H NMR (DMSO-d₆, 400MHz) 3.58 (s, 2H), 3.48 (s, 2H), 1.34 (s , 6H).

1-Aminooxy-2-methylpropan-2-ol

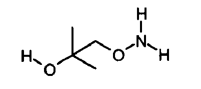

Step1: 2-(2-Hydroxy-2-methyl-propoxy)-isoindole-1,3-dione

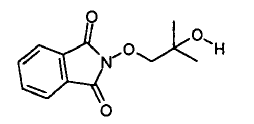

To a solution of N-Hydroxyphthalimide (18.3 g, 112 mmol) and 1,2-Epoxy-3-methyl propane (9.50 mL, 107 mmol) in anhydrous DMF under nitrogen at room temperature was added triethylamine (16.1 mL, 115 mmol). The reaction turned from yellow to dark red. The reaction was then heated to 85°C overnight. The reaction was cooled to room temperature and concentrated under reduced pressure. The residue obtained was partitioned between water (100 mL) and ether (3 X 75 mL). The combined ether layers were washed with water (2 X 50 mL), dried with anhydrous magnesium sulfate, filtered and concentrated to give a yellow oil (26.8 g). This was then treated with dichloromethane (35 mL), which resulted in unreacted N-Hydroxyphthalimide crashing out as a white precipitate. This was filtered off and discarded. The filterate was purified by flash column chromatography (120 g, silica, ISCO, 45 mL/min, 0-10% methanol in dichloromethane in 50 minutes) to give the title compound as a white solid (13.4 g, 53.3 %). LCMS (method C): R_{T} = 1.70 min, M+H⁺ = 236.1 1H NMR (CDC1₃, 400MHz) 7.84 (m, 2H), 7.78 (m, 2H), 4.15 (s, 2H), 1.39 (s, 6H).

Step 2: 1-Aminooxy-2-methlpropan-2-ol

To a solution of 2-(2-Hydroxy-2-methyl-propoxy)-isoindole-1,3-dione (3,70 g, 15.7 mmol) in anhydrous dichloromethane (25 mL) under nitrogen at 0°C was added methyl hydrazine (0.879 mL, 16.50 mmol) and stirred for 2 hours at 0°C. The addition of methyl hydrazine resulted in a pale yellow color followed by a white precipitate. The reaction was filtered after 2 hours at 0°C and the solid was discarded. The filtrate was concentrated under reduced pressure to give the title compound as a pale yellow oil (1.65 g, 100%). LCMS (method C): R_{T} = 0.34 min, M+H⁺ = 106.1.1 H NMR (DMSO-d₆, 400MHz) 3.60 (s, 2H), 1.22 (s , 6H).

3-aminooxy-3-methylbutan-1-ol

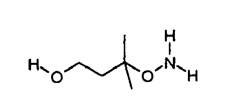

Step 1: 2-(3-Hydroxy-3-methyl-butoxy)-isoindole-1,3-dione 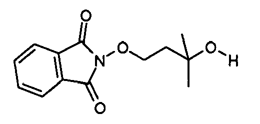 To a solution of N-Hydroxyphthalimide (3.13 g, 19.2 mmol) and 3-Hydroxy-3-methyl butane (2.00 g, 19.2 mmol) in anhydrous dichloromethane under nitrogen at room temperature was added Boron trifluoride etherate (2.43 mL, 19.2 mmol) and stirred overnight. The reaction turned black after 18h with a white precipitate (N-Hydroxyphthalimide). To the reaction was added saturated sodium bicarbonate solution (3 mL) and stirred for 5 minutes at room temperature. The reaction was then filtered and the white solid was discarded. The filtrate was concentrated and the black residue obtained was re-dissolved in 25 mL dichlormethane and filtered. The white solid was discarded and the filtrate was concentrated to give a black residue. This was dissolved in dichloromethane (5 mL) and purified by flash column chromatography (silica, 80 g, ISCO, 30 ml/min, 0-100% ethyl acetate in hexane in 45 minutes) to give the title compound as a yellow oil (228 mg, 4.75%). LCMS (method C): R_{T} = 1.77 min, M+H⁺ = 250.2. 1H NMR (CDC1₃, 400MHz) 7.83 (m, 2H), 7.78 (m, 2H), 3.95 (t, 2H), 2.00 (t, 2H), 1.45 (s, 6H).

Step 2: 3-aminooxy-3-methylbutan-1-ol

To a solution of 2-(3-Hydroxy-3-methyl-butoxy)-isoindole-1,3-dione (228 mg, 0.91 mmol) in anhydrous dichloromethane (2 mL) under nitrogen at 0°C was added methyl hydrazine (0.05 mL, 0.96 mmol) and stirred for 1 hour and let warm to room temperature in the process. The addition of methyl hydrazine resulted in a pale yellow color followed by a white precipitate. The reaction was filtered after 2 hours at 0°C and the solid was discarded. The filtrate was concentrated under reduced pressure to give the title compound as a pale yellow solid (95 mg, 87%). LCMS (method C): R_{T} = 0.34 min, M+H⁺ = 120.1H NMR (DMSO-d₆, 400MHz) 3.75 (t, 2H), 1.83 (t, 2H), 1.24 (s , 6H).

O-Pyridin-2-ylmethyl-hydroxylamine hydrochloride

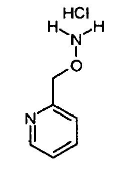

Step 1: N-Boc-aminooxymethyl(pyridine-2-yl)

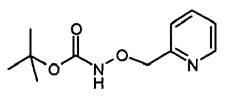

To a solution of N-Boc-hydroxylamine (5.0 g, 37.6 mmol) in ethanol (100 mL) was added potassium hydroxide (4.63 g, 82.61 mmol) and stirred at room temperature till the potassium hydroxide dissolved into solution. To this was added 2-Bromomethylpyridine hydrobromide (11.398 g, 45.06 mmol) and refluxed overnight. A white precipitate was observed after 1 hour. Reaction cooled to room temperature and then filtered. The white solid was discarded and the filterate was concentrated. The oily residue was partitioned between water (75 mL) and ether (3 X 100 mL). The combined ether layer was dried with sodium sulfate, filtered and the filterate was concentrated to give the product as a yellow oil (6.0 g). The oil was dissolved in dichloromethane (10 mL) and purified by flash column chromatography (silica, 120 g, ISCO, 45 mL/min, 0-100 % ethyl acetate in hexane in 40 minutes) to give the title compound as a white solid (1.78g, 21.2%). LCMS (method C): R_{T} = 1.13 min, M+H⁺ = 225.2. 1H NMR (CDC1₃, 400MHz) 8.61 (d,m, 1H), 7.73 (t,d, 1 H), 7.52 (s, 1H), 7.46 (d,t, 1H), 5.01 (s, 2H), 1.50 (s , 9H).

Step 2: O-Pyridin-2-ylmethyl-hydroxylamine hydrochloride

To a solution of N-Boc-aminooxymethyl(pyridine-2-yl) (860 mg, 3.8 mmol) in anhydrous dichloromethane (2 mL) was added 4 M HCl in dioxane (5.06 mL, 20 mmol) at room temperature and stirred for 2 hours. To the reaction was added ether (25 mL) and stirred for 5 minutes. The solvents were decanted off and the residue was treated with ether (25 mL), followed by stirring and then decanting again. This was repeated one more time and the residue (white solid) was dried under vacuum to give the title compound as a white solid ( 688 mg, 91%). LCMS (method C): R_{T} = 0.36 min, M+H⁺ = 125.0. 1H NMR (DMSO-d₆, 400MHz) 8.70 (m, 1H), 8.05 (m, 1H), 7.60 (m, 2H), 5.20 (s, 2H).

O-(1-Phenyl-ethyl)-hydroxylamine hydrochloride

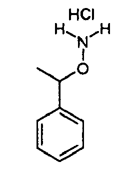

Synthesized from 1-(bromoethyl)benzene following procedures analogous to those used to synthesize O-Pyridin-2-ylmethyl-hydroxylamine hydrochloride.

LCMS (method C): R_{T} = 0.92 min, M+H⁺ = 138.2. 1H NMR (DMSO-d₆, 400MHz) 10.90 (s, 2H), 7.45 (m, 5H), 5.25 (q, 1H), 1.50 (d, 3H).

*O*-[2-(*tert*-Butyl-dimethyl-silanyloxy)-pronyl]-hydroxylamine

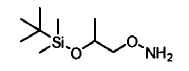

Step 1: (2-Benzyloxy-1-methyl-ethoxy)-*tert*-butyl-dimethyl-silane

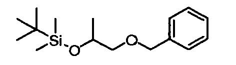

Tert-butyldimethylsilyl chloride (517 mg, 3.43 mmol) was added to a solution of 1-benzyloxy-propan-2-ol (518 mg, 3.12 mmol), imidazole (318 mg, 4.66 mmol), and 4-DMAP (95 mg, 0.78 mmol) in CH₂Cl₂ (3 mL). The reaction mixture was stirred at room temperature for 16 h, then 2g of silica gel was added and the volatiles were removed in vacuo. The residue was purified by silica chromatography (0 - 5% EtOAc:Hex) to afford the title compound (713 mg, 82% yield) as a clear oil.

Step 2: 2-(*tert*-Butyl-dimethyl-silanyloxy)-propan-1-ol

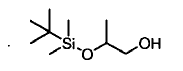

To a solution of (2-Benzyloxy-1-methyl-ethoxy)-*tert*-butyl-dimethyl-silane (640 mg, 2.3 mmol) in ethyl acetate (10 mL) was added 20% Pd on carbon (64 mg). The reaction mixture was evacuated and flushed with H₂, then stirred under an atmosphere of H₂ for 3h. The reaction mixture was then filtered through celite and concentrated to afford the title compound (430 mg, 99% yield) as a clear oil, which was used without further purification in the next step.

Step 3: 2-[2-(*tert*-Butyl-dimethyl-silanyloxy)-propoxy]-isoindole-1.3-dione

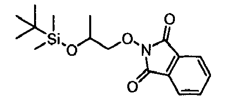

DEAD (0.46 mL, 2.94 mmol) was added dropwise to a solution of 2-(*tert-*Butyl-dimethyl-silanyloxy)-propan-1-ol (430 mg, 2.26 mmol), triphenylphosphine (593 mg, 2.26 mmol), and *N-*hydroxyphthalimide (369 mg, 2.26 mmol) in THF (10 mL) at 0°C. After stirring for 10 minutes at 0 °C, the reaction mixture was brought to room temperature and stirring was continued for a further 48 h. The reaction mixture was filtered through a coarse glass funnel and concentrated in vacuo. The residue was purified by silica chromatography (0 - 40% EtOAc:Hex) to afford the title compound (139 mg, 18% yield) as a white solid.

Step 4: *O*-[2-(*tert-*Butyl-dimethyl-silanlyoxy)-propyl]-hydroxylamine

*N-*methylhydrazine (23 µL, 0.43 mmol) was added to a solution of 2*-*[2-(*tert-*Butyl-dimethyl-silanyloxy)-propoxy]-isoindole-1,3-dione (135 mg, 0.40 mmol) in CH₂CI₂ (3 mL). After stirring for 1h at room temperature, the white precipitate was filtered off and the reaction mixture was concentrated in vacuo to afford the title compound (76 mg, 92% yield) as a yellow oil. ¹H NMR (CDCl₃, 400MHz) 5.48 (br, 2H), 4.04 (m, 1H), 3.58 (dd, 1H), 3.52 (dd, 1H), 1.13 (d, 3H), 0.89 (s, 9H), 0.09 (s, 6H).

*O*-[2-(*tert*-Butyl-dimethyl-silanyloxyloxy)-1-methyl-ethyl]-hydroxylamine

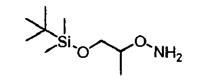

Step 1: 1-(*tert*-Butyl-dimethyl-silanvloxy)-propan-2-ol

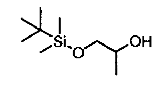

Tert-butyldimethylsilyl chloride (4.1 g, 27 mmol) was added to a solution of propane-1,2-diol (2.0 mL, 27 mmol) and triethylamine (4.93 mL, 35.4 mmol) in CH₂Cl₂. After stirring overnight at room temperature, the reaction mixture was washed one time eachwith 1N aqueous HCl solution, water, and a 1:1 saturated solution of NaHCO₃ and brine. The organic layer was dried over Na₂SO₄, then filtered and concentrated. The crude title compound was used without further purification in the next step.

Step 2: 2-[2-(*tert*-Butyl-dimethyl-silanyloxy)-1-methyl-ethoxy]-isoindole-1,3-dione

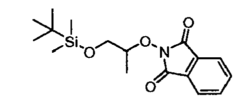

DEAD (1.86 mL, 11.8 mmol) was added dropwise to a solution of 1-(*tert-*Butyl-dimethyl-silanyloxy)-propan-2-ol (1.73 g, 9.09 mmol), triphenylphosphine (2.38 g, 9.09 mmol), and *N-*hydroxyphthalimide (1.48 g, 9.09 mmol) in THF (45 mL) at 0 °C. After stirring for 10 minutes at 0 °C, the reaction mixture was brought to room temperature and stirring was continued for a further 48 h. The reaction mixture was filtered through a coarse glass funnel and concentrated in vacuo. The residue was purified by silica chromatography (0 - 40% EtOAc:Hex) to afford the title compound (1.80 g, 59% yield) as a clear oil.

Step 3: *O*-[2-(*tert*-Butyl-dimethyl-silanyloxy)-1-methyl-ethyl]-hydroxylamine

*N-*methylhydrazine (310 µL, 5.74 mmol) was added to a solution of 2*-*[2*-*(*tert-*Butyl-dimethyl-silanyloxy)-1-methyl-ethoxy]-isoindole-1,3-dione (1.80 g, 5.36 mmol) in CH₂Cl₂ (20 mL). After stirring for 1h at room temperature, the white precipitate was filtered off and the reaction mixture was concentrated in vacuo to afford the title compound (682 mg, 62% yield) as a yellow oil. ¹H NMR (CDCl₃, 400MHz) 5.39 (br, 2H), 3.77 - 3.68 (m, 1H), 3.67 (dd, 1H), 3.61 (dd, 1H), 1.13 (d, 3H), 0.90 (s, 9H), 0.08 (s, 6H).

*O*-(2-Phenyl-1,3-dioxinan-5-yl)-hydroxylamine

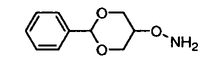

Step 1: 2-(2-Phenyl-1,3-dioxinan-5-yloxy)-isoindole-1,3-dione

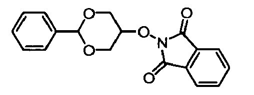

Diethyl Azodicarboxylate (0.85 mL, 5.41 mmol) added dropwise to a solution of 2-Phenyl-1,3-dioxinan-5-ol (750 mg, 4.16 mmol), Triphenylphosphine (1.09 g, 4.16 mmol; ), and N-Hydroxyphthalimide (0.679 g, 4.16 mmol;) in THF (20 mL) at 0 °C. Stirred overnight at 0 °C - room temperature, then concentrated in vacuo. Diluted with CH₂CI₂, then filtered through a Whatman syringe filter. 4 g silica gel added and concentrated in vacuo. The residue was purified by silica chromatography (30 - 70% EtOAc : Hex, flush with 100% EtOAc) to afford the title compound (495 mg, 37% yield) as a white solid.

Step 2: *O*-(2-Phenyl-1,3-dioxinan-5-yl)-hydroxylamine

*N-*methylhydrazine (87 µL, 5.74 mmol) was added to a solution of 2-(2-Phenyl-1,3-dioxinan-5-yloxy)-isoindole-1,3-dione (495 mg, 1.52 mmol) in CH₂Cl₂ (10 mL). After stirring for 3h at room temperature, the white precipitate was filtered off and the reaction mixture was concentrated in vacuo to afford the title compound (272 mg, 92% yield) as a yellow oil. ¹H NMR (CDCl₃, 400MHz) 7.50 - 7.46 (m, 2H), 7.40 - 7.35 (m, 3H), 5.44 (br, 2H), 5.41 (s, 1H) 4.48 - 4.42 (m, 2H), 4.01 - 3.93 (m, 1H), 3.66 - 3.60 (m, 2H).

N-(2-Aminoox-ethyl)-methanesulfonamide

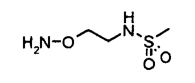

Step 1: [2-(1,3-Dioxo-1,3-dihydro-isoindol-2-yloxy)-ethyl]-carbamic acid tert-butyl ester

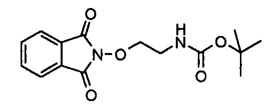

To a suspension of N-(tert-butoxycarbonyl)ethanolamine (5.0 g, 31.0 mmol), N-hydroxyphthalimide (5.1g, 31.0 mmol) and triphenylphosphine (8.5 g, 32.6 mmol) in tetrahydrofuran (30 ml) at 0 °C was added dropwise diisopropyl azodicarboxylate (6.3 ml, 32.6 mmol). The reaction was stirred and allowed to warm to room temperature over 16 hours. The reaction was concentrated *in vacuo* and the product purified by flash chromatography (SiO₂, gradient ethyl acetate:cyclohexane, 20:80 to 30:70) to yield the title compound as an oil (14.2 g). ¹H NMR (CDCl₃, 400 MHz) 7.87-7.85 (2H, m), 7.79-7.77 (2H, m), 4.26 (2H, t, J= 5.5 Hz), 3.47-3.43 (2H, m), 1.47 (9H, s).

Step 2: 2-(2-Amino-ethoxy)-isoindole-1,3-dione

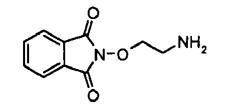

[2-(1,3-Dioxo-1,3-dihydro-isoindo1-2-yloxy)-ethyl]-carbamic acid tert-butyl ester (4.4 g, ~8.6 mmol) was dissolved in hydrochloric acid in dioxane (4N, 20 ml) and stirred at room temperature for 3 hours. The reaction was concentrated *in vacuo.* The resultant residue was dissolved in ethyl acetate (20 ml) and the solution washed with sodium hydroxide solution (20 ml, 1N). The aqueous layer was isolated then extracted with ethyl acetate (2 × 10 ml). The combined organic layers were washed with brine and then dried over magnesium sulfate and concentrated *in vacuo* to yield the title compound as a colourless oil (1.96 g). ¹H NMR (CDCl₃, 400 MHz) 7.85 (2H, dd, J= 5.4, 2.9 Hz), 7.72 (2H, dd, J= 5.4, 3.0 Hz), 3.99-3.97 (2H, m), 3.86-3.83 (2H, m).

Step 3: N-[2-(1,3-Dioxo-1,3-dihydro-isoindol-2-yloxy)-ethyl]-methanesulfonamide

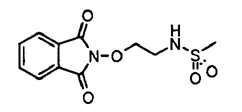

To a solution of 2-(2-amino-ethoxy)-isoindole-1,3-dione (1.96 g, 8.1 mmol) in acetonitrile (20 ml) at 0 °C was added simultaneously methane sulfonylchloride (0.63 ml, 8.1 mmol) and triethylamine (2.3 ml, 16.2 mmol). The reaction was stirred at 0 °C for 1 hour then at room temperature for 1 hour. The reaction was filtered and the filtrate concentrated *in vacuo.* The resultant residue was dissolved in ethyl acetate (20 ml) and washed with water (20 ml). The aqueous layer was isolated then extracted with ethyl acetate (2 × 10 ml). The combined organic layers were washed with brine, dried over magnesium sulfate and concentrated *in vacuo* to yield the title compound as a white solid (1.2 g, 44%). ¹H NMR (CDCl₃, 400 MHz) 7.87 (2H, dd, J= 5.5, 3.1 Hz), 7.79 (2H, dd, J= 5.5, 3.2 Hz), 4.36 (2H, dd, J= 4.82, 4.62 Hz), 3.43-3.47 (2H, m), 3.05 (3H, s).

Step 4: N-(2-Aminoox-ethyl)-methanesulfonamide

To a suspension of N-[2-(1,3-dioxo-1,3-dihydro-isoindol-2-yloxy)-ethyl]-ethanesulfonamide (0.55 g, 1.92 mmol) in dichloromethane (15 ml) was added methylhydrazine (0.1 ml, 1.92 mmol). The reaction was stirred at room temperature for 30 minutes, over which time a white precipitate formed. The reaction was filtered and the filtrate concentrated *in vacuo* to give a residue. The residue was purified by flash chromatography (SiO₂ gradient 1-5% methanol in dichloromethane) to yield the title compound as a white solid (204 mg, 68%). ¹H NMR (CDCl₃, 400 MHz) 3.80 (2H, t, J = 4.9 Hz), 3.39 (2H, t, J= 4.8 Hz), 3.00 (3H, s).

N-Cyclopropylmethyl-O-(2-vinyloxy-ethyl)-hydroxylamine

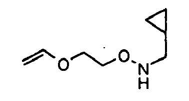

A solution of O-(2-vinyloxy-ethyl)-hydroxylamine (210 mg, 2.0 mmol) and cyclopropane carboxaldehyde (140 mg, 2.0 mmol) in ethanol (2.0 ml) was cooled to 0°C under a nitrogen atmosphere then pyridinium p-toluenesulfonic acid (0.5 g, 2.0 mmol) and sodium cyanoborohydride (0.15 g, 2.2 mmol). The resultant mixture was stirred at ambient temperature for 24 hours. The reaction mixture was diluted with ethyl acetate, washed with water then brine, dried (Na₂SO₄), filtered and concentrated *in vacuo* to give the a colourless oil which was used crude in the subsequent step.

O[1-(Toluene-4-sulfonyl)-1H-imidazol-2-ylmethyl]-hydroxylamine

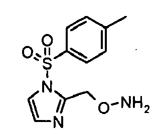

Step 1: 2-[1-(Toluene-4-sulfonyl)-1H-imidazol-2-ylmethoxy]-isoindole-1,3-dione

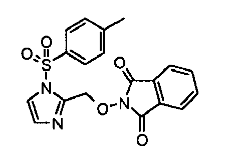

Diisopropyl azodicarboxylate was added dropwise to a cooled (0°C) solution of 2-(hydroxymethyl)-1-(p-tolylsulfonyl)imidazole (0.60 g, 2.4 mmol), triphenylphosphine (0.65 g, 2.5 mmol) and *N-*hydroxyphthalimide (0.39 g, 2.4 mmol) in THF (20 ml). The reaction was stirred and allowed to warm to room temperature over 40 hours. The reaction was concentrated *in vacuo* and the residue dissolved in dichloromethane (20 ml) causing the product to precipitate as a white solid. The product was collected by filtration and washed with dichloromethane (5 ml) to yield the title compound as a white solid (580 mg, 61%). LCMS (method B): R_{T} = 3.46 min, M+H⁺ = 398.

Steep 2: O-[1-(Toluene-4-sulfonyl)-1H-imidazol-2-ylmethyl]-hydroxylamine

Methylhydrazine (40 µl, 0.75 mmol) was added to a solution of 2-[1-(toluene-4-sulfonyl)-1H-imidazol-2-ylmethoxy]-isoindole-1,3-dione (300 mg, 0.75 mmol) in dichloromethane (3 ml) and the reaction stirred at room temperature for 20 minutes. After approximately 10 minutes a white precipitate formed. The reaction was filtered and the filtrate concentrated *in vacuo* to approximately half the volume. Diethyl ether (5 ml) was added causing a white precipitate to form. The reaction was filtered and the filtrated concentrated *in vacuo* to give the title compound as a colourless oil (230 mg, 114%). The product was used without further purification. LCMS (method B): R_{T} = 2.46 min, M+H⁺ = 268.

(3S,4S)-pyrrolidine-3,4-diol hydrochloride

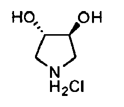

Step 1: (3R,4R)-1-benzyl-3.4-dihydroxydroxlidine-2.5-dione

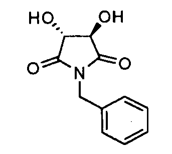

L-(+)-tartaric acid (1.51 g, 10.06 mmol) and benzyl amine (1.08 g, 10.06 mmol) in m-xylene (50 ml) were heated at reflux while water was collected in a Dean-Stark trap. After stirring overnight, the reaction was concentrated. The residue was taken up in minimal THF/EtOH and purified by silica gel flash chromatography (gradient elution, using 3:1 hexane-ethyl acetate, ethyl acetate, and 9:1 ethyl acetate-ethanol) to provide the title compound as a brown solid (0.99 g, 44%).

Step 2: (3S,4S)-1-benzylpyrrolidine-3,4-diol

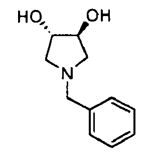

The (3R,4R)-1-benzyl-3,4-dihydroxypyrrolidine-2,5-dione (0.98 g, 4.4 mmol) in THF (20 ml) was added slowly to a stirring solution of LiAlH₄ (4.75 ml, 11.87 mmol of 2.5 M solution in THF) in THF cooled to -5°C. After complete addition, the reaction was warmed to room temperature, then heated at reflux overnight. The reaction was cooled to room temperature, then quenched with saturated aqueous NH₄Cl until further addition produced no more bubbling. The reaction was diluted with ethyl acetate (20 ml), filtered, and the solid washed with ethyl acetate. The combined filtrates were concentrated, and the residue purified by silica flash chromatography (gradient elution, using EtOAc, and 9:1 EtOAc-EtOH) to provide the title compound as a tan solid (0.52 g, 61%).

Step 3: (3S,4S)-pyrrolidine-3,4-diol hydrochloride

The (3S,4S)-1-benzylpyrrolidine-3,4-diol (0.52 g, 2.7 mmol) was dissolved in ethanol (15 ml) and acetic acid (10 ml) and hydrogenated (50 psi H₂) over 10% Pd-C (100 mg) on a Parr apparatus for 6 hours. After filtering through Celite, and washing the filter cake with ethyl acetate, the combined filtrate and washings were concentrated. The residue was diluted with 4N HCl/dioxane (2 ml), methanol (5 ml), then toluene (40 ml) and concentrated. The residue was triturated with ethyl ether to provide the hydrochloride salt of the title compound as a tan solid (0.37 g, 97%). ¹H NMR (D₂O, 400MHz) 4.35 (d, J=3.4 Hz, 2H), 3.54 (dd, J = 12.8 Hz, 3.4 Hz, 2H), 3.30 (d, J = 12.8 Hz, 2H).

3-methylpyrrolidin-3-ol hydrochloride

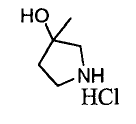

Step 1: tert-butyl 3-hydroxy-3-methylpyrrolidine-1-carboxylate

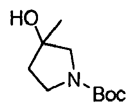

The solution of (0.070 g, 0.38 mmol) tert-butyl 3-oxopyrrolidine-1-carboxylate in anhydrous THF (2 mL) was cooled to -78°C. Then the solution of 1 M methylmagnesium bromide in butyl ether was added dropwise. The reaction was stirred at - 78°C for 4 h and quenched by water (2 mL). After concentrating the reaction in vacuo, the residue was partitioned between ethyl acetate and water. The aqueous layer was extracted once more with ethyl acetate, and the combined organic layers were washed with brine, dried (MgSO₄) and concentrated. The residue was purified by silica flash chromatography (gradient elution, using 1:1 hexane-ethyl acetate and ethyl acetate) to provide the title compound (0.054 g, 70%).

Step 2: 3-methylpyrrolidin-3-ol hydrochloride

To tert-butyl 3-hydroxy-3-methylpyrrolidine-1-carboxylate (0.027 g, 0.13 mmol) was added a 4N HCl/dioxane solution (1 ml) and the mixture was stirred for 2 hours. The solution was concentrated in vacuo. The residue was diluted with toluene (1 ml) and reconcentrated to provide the title compound as a colorless oil (0.018 g, 100%).

(9H-fluoren-9-yl)methyl (3R,4R)-4-hydroxypyrrolidin-3-ylcarbamate hydrochloride

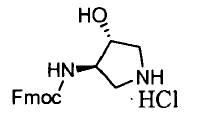

Step 1: (9H-fluoren-9-yl)methyl (3R,4R)-1-(tert-butoxycarbonyl)-4-hydroxypyrrolidin-3-ylcarbamate

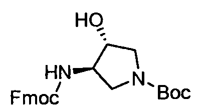

(3R,4R)-tert-butyl 3-amino-4-hydroxypyrrolidine-l-carboxylate (0.05 g, 0.25 mmol) was dissolved in 1,4-dioxane (1 mL), water (1 mL), and toluene (0.3 mL). Then 9-fluorenylmethyl chloroformate (0.077 g, 0.30 mmol) was added slowly followed by sodium bicarbonate (0.083 g, 0.99 mmol). The reaction mixture was stirred at room temperature overnight. After concentrating the reaction in vacuo, the residue was partitioned between ethyl acetate and water. The aqueous layer was extracted once more with ethyl acetate, and the combined organic layers were washed with brine, dried (MgSO₄ and concentrated. The residue was purified by silica flash chromatography (gradient elution, using 1:1 hexane-ethyl acetate and ethyl acetate) to provide the title compound (0.090 g, 90%).

Step 2: (9H-fluoren-9-yl)methyl (3R,4R)-4-hydroxypymolidin-3-ylcarbamate hydrochloride

A 4N HCl/dioxane solution (1 ml) was added to (9H-fluoren-9-yl)methyl (3R,4R)-1-(tert-butoxycarbonyl)-4-hydroxypyrrolidin-3-ylcarbamate and the mixture was stirred for 2 hours. The solution was concentrated in vacuo. The residue was diluted with toluene (1 ml) and reconcentrated to provide the title compound as a colorless oil (0.076 g, 100%).

(2R,3R)-2-(hydroxymethyl)pyrrolidin-3-ol hydrochloride

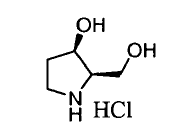

Step 1: (2S.3R)-1-(tert-butoxycarbonyl)-3-hydroxypyrrolidine-2-carboxylic acid

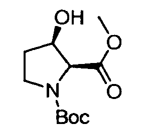

The (2S,3R)-1-(tert-butoxycarbonyl)-3-hydroxypyrrolidine-2-carboxylic acid (1.76 g, 7.63 mmol) and NaHCO₃ (1.28 g, 15.3 mmol) were suspended in DMF (10 ml). Methyl iodide (2.37 ml, 5.41 g, 38.13 mmol) was added to the mixture, which was then heated at 50°C overnight. After concentrating the reaction in vacuo, the residue was partitioned between ethyl acetate and water. The aqueous layer was extracted once more with ethyl acetate, and the combined organic layers were washed with brine, dried (MgSO₄) and concentrated. The residue was purified by silica flash chromatography (gradient elution, using 1:1 hexane-ethyl acetate and ethyl acetate) to provide the title compound as a colorless oil (1.66 g, 89%).

Step 2: (2R,3R)-tert-butyl 3-hydroxy-2-(hydroxymethyl)pyrrolidine-1-carboxylate

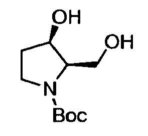

To a stirring solution of the (2S,3R)-1-(tert-butoxycarbonyl)-3-hydroxypyrrolidine-2-carboxylic acid (0.36 g, 1.47 mmol) in THF (5 ml) was added LiCI (0.19 g, 4.4 mmol) followed by NaBH₄ (0.17 g, 4.4 mmol). After addition of ethanol (10 ml), the resulting mixture was stirred at room temperature overnight. The reaction flask was placed in an ice bath, and the cooled milky white solution was acidified to pH 2-3 with 37% HCl. The solution was concentrated, and the residue was partitioned between ethyl acetate and water. The aqueous layer was extracted with ethyl acetate, and the combined organic layers were washed with brine, dried (MgSO₄) and concentrated. Purification of the resulting oil by flash chromatography (silica gel, using ethyl acetate) provided 0.30 g (95%) of the title compound as a colorless oil.

Step 3: (2R,3R)-2-(hydroxymethyl)pyrrolidin-3-ol hydrochloride

4N HCl/dioxane solution (5 ml) was added to (2R,3R)-tert-butyl 3-hydroxy-2-(hydroxymethyl)pyrrolidine-1-carboxylate and the mixture was stirred for 2 hours. The solution was concentrated in vacuo. The residue was diluted with toluene (5 ml) and reconcentrated to provide the title compound as a colorless oil (0.21 g, 100%).

(3R,5R)-5-(Hydroxymethyl)pyrrolidin-3-ol hydrochloride

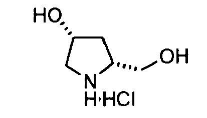

Step 1: (2R,4R)-1-((benzyloxylcarbonyl)-4-hydroxypyrrolidine-2-carboxylic acid

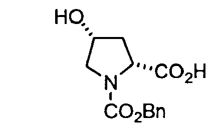

cis-4-Hydroxy-D-proline (1.0 g, 7.63 mmol) and NaHCO₃ (1.6 g, 19.05 mmol) were dissolved in H₂O (16 ml), then a solution of benzyl chloroformate (1.25 ml, 1.49 g, 8.76 mmol) in toluene (4 ml) was added dropwise over a period of 15 minutes. After stirring at room temperature for 16 hours, the two phases were separated. Excess benzyl chloroformate was removed from the aqueous phase by washing with ether (4x5 ml). Acidification of the aqueous phase to pH 2 with concentrated HCl caused the oily product to precipitate and this was extracted into ethyl acetate by repeated washings (3x5 ml) of the aqueous layer. The combined organic layers were dried (MgSO₄ and concentrated to give the title compound as a viscous oil (2.02 g, 100%).

Step 2: (2R, 4R)-1-Benzyl 2-methyl4-hydroxypyrrolidine-1,2-dicarboxylate

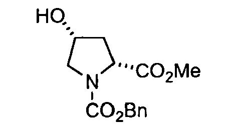

The (2R,4R)-1-((benzyloxy)carbonyl)-4-hydroxypyrrolidine-2-carboxylic acid (2.02 g, 7.63 mmol) and NaHCO₃ (1.28 g, 15.3 mmol) were suspended in DMF (10 ml). Methyl iodide (2.37 ml, 5.41 g, 38.13 mmol) was added to the mixture, which was then stirred and heated at 50°C overnight. After concentrating the reaction under reduced pressure, the residue was partitioned between ethyl acetate and water. The aqueous layer was extracted once more with ethyl acetate, and the combined organic layers were washed with brine, dried (MgSO₄) and concentrated. The residue was purified by silica flash chromatography (gradient elution, using 1:1 hexane-ethyl acetate and ethyl acetate) to provide the title compound as a colorless oil (1.9 g, 89%).

Step 3: (2R,4R)-Benzyl 4-hydroxy-2-(hydroxymethyl)pyrrolidine-1-carboxylate

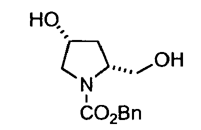

To a stirring solution of the (2R, 4R)-1-benzyl 2-methyl 4-hydroxypyrrolidine-1,2-dicarboxylate (0.41 g, 1.47 mmol) in THF (5 ml) was added LiCl (0.19 g, 4.4 mmol) followed by NaBH₄ (0.17 g, 4.4 mmol). After addition of ethanol (10 ml), the resulting mixture was stirred at room temperature overnight. The reaction flask was placed in an ice bath, and the cooled milky white solution was acidified to pH 2-3 with 37% HCl. The solution was concentrated, and the residue was partitioned between ethyl acetate and water. The aqueous layer was extracted with ethyl acetate, and the combined organic layers were washed with brine, dried (MgSO₄) and concentrated. Purification of the resulting oil by flash chromatography (silica gel, using ethyl acetate) provided the title compound (0.35 g, 95%) as a colorless oil.

Step 4: (3R,5R)-5-(hydroxymethyl)pyrrolidin-3-ol hydrochloride

(2R,4R)-benzyl 4-hydroxy-2-(hydroxymethyl)pyrrolidine-1-carboxylate (0.35 g, 1.4 mmol) was dissolved in ethanol (30 ml) and transferred into a Parr shaker bottle. After adding 10% Pd-C (0.07 g), the mixture was shaken under an atmosphere of hydrogen at 50 psi for 0.5 h on the Parr apparatus. The catalyst was removed by filtration through Celite. The filter cake was washed with ethanol and the combined filtrate and washings were concentrated in vacuo to yield a colorless oil. For ease of handling, the amine was converted to the hydrochloride salt. A 4N HCl/dioxane solution (1 ml) was added to the residue, along with enough methanol (∼1 ml) to completely dissolve the residue. After complete mixture, the solvent was evaporated under reduced pressure. The solid was diluted with toluene (20 ml) and reconcentrated. Finally, the solid was triturated with ether, the ether was discarded, and the solid was dried in vacuo to yield 0.186 g (87%) of the title compound as a pink solid.

(3R, 5S)-5-(Hydroxymethyl)pyrrolidin-3-ol hydrochloride

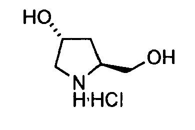

To N-Boc-trans-4-hydroxy-L-prolinol (0.422 g, 1.94 mmol) was added a 4N HCl/dioxane solution (5 ml) and the mixture was swirled for 1 hour. The solution was concentrated in vacuo. The residue was diluted with toluene (10 ml) and reconcentrated. The resulting white solid was triturated with ethyl ether, the ether was discarded, and the solid dried under vacuum to provide 0.29 g (97%) of the title compound as a colorless white solid. ¹H NMR (D₂O 400 MHz) 4.65-4.67 (m, 1H), 3.99-4.06 (m, 1H), 3.93 (dd, J=12.5Hz, 3.6 Hz, 1H), 3.71 (dd, J=12.5 Hz, 6.9 Hz, 1H), 3.44 (dd, J=12.7 Hz, 3.8 Hz, 1H), 3.32 (d, J=12.7 Hz, 1H), 2.11-2.17 (m, 1H), 1.92-1.98 (m, 1H).

tert-Butyl (3R,5R)-5-(hydroxymethyl)pyrrolidin-3-ylcarbamate

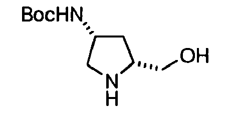

Step 1: (2R, 4S)-1-benzyl 2-methyl 4-hydroxypyrrolidine-1,2-dicarboxylate

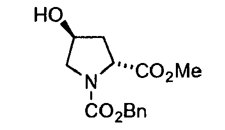

To a stirred solution of the (2R, 4R)-1-benzyl 2-methyl 4-hydroxypyrrolidine-1,2-dicarboxylate (1.45 g, 5.2 mmol), triphenylphosphine (4.59 g, 17.5 mmol), and p-nitrobenzoic acid (2.6 g, 15.6 mmol) in dry benzene (10 ml) at room temperature was added diethylazodicarboxylate dropwise (2.57 ml, 17.5 mmol). The solution was then stirred at room temperature for 6 h, whereupon the volatile components were removed in vacuo and the residue purified by flash chromatography (silica gel, hexane-ethyl ether 1:1, and again with hexane-ethyl ether-methylene chloride 2:1:1). This residue was dissolved in methanol (10 ml), K₂CO₃ was added (0.02 g, 0.14 mmol) and the mixture was stirred for 1 h at room temperature. After removing the volatile components in vacuo, the residue was purified by flash chromatography (silica gel, gradient elution, using ethyl ether to ethyl acetate) to provide the title compound as a colorless oil (0.33 g, 23%).

Step 2: (3S,5R)-1-((Benzyloxy)carbonyl)-5-(methoxycarbonyl)pyrrolidin-3-yl 4-methylbenzenesulfonate

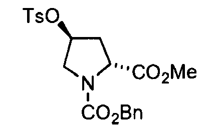

(2R,4S)-1-benzyl 2-methyl 4-hydroxypyrrolidine-1,2-dicarboxylate (0.33 g, 1.18 mmol) and DMAP (0.43 g, 3.55 mmol) were dissolve in chloroform and the mixture cooled to -5°C in an ice-ethanol bath. p-Toluenesulfonyl chloride (0.45 g, 2.24 mmol) was added and the reaction was stirred while warming to room temperature for 2 hours. After quenching with water (0.6 ml) and vigorous stirring for 10 minutes, the layers were separated and the aqueous layer extracted with methylene chloride (2x). The organic layers were dried (MgSO₄) filtered through a plug of silica gel (7 ml packed in ethyl ether), eluted with ethyl ether and concentrated. The residue was purified by flash chromatography (gradient elution, using hexane-ethyl ether 1:1 to ethyl ether) to provide the title compound as a colorless oil (0.49 g, 95%).

Step 3: (2R,4R)-1-benzyl 2-methyl 4-azidopyrrolidine-1,2-dicarboxylate

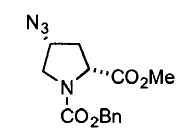

Sodium azide (0.33 g, 5.07 mmol) was added to the (3S,SR)-1-((benzyloxy)carbonyl)-5-(methoxycarbonyl)pyrrolidin-3-yl 4-methylbenzenesulfonate (0.49 g, 1.13 mmol) in DMF (8 ml) and the mixture heated at 50°C overnight. After concentrating in vacuo, the residue was partitioned between ethyl ether and water. The aqueous layer was extracted with ethyl ether, and the combined organic layers were dried (MgSO₄) and concentrated. Purification of the resulting oil by flash chromatography (silica gel, using ethyl ether) provided 0.33 g (97%) of the title compound as a colorless oil.

Step 4: (3R,5R)-1-((benaloxy)carbonyl)-5-(methoxycarbonyl)pyrrolidin-3-ylcarbamate

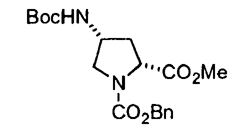

Triphenylphosphine (0.33 g, 1.15 mmol) was added to a solution of the (2R,4R)-1-benzyl 2-methyl 4-azidopyrrolidine-1,2-dicarboxylate (0.33 g, 1.08 mmol) in THF (4 ml) and water (2 ml). After stirring at 50 °C overnight, sodium bicarbonate (0.23 g, 2.71 1 mmol) was added, followed by di-tert-butyl dicarbonate (0.47 g, 2.17 mmol) and stirring was continued at 50°C for another 4 h. Volatiles were removed under reduced pressure, and the residue was partitioned between ethyl ether and water. The aqueous layer was extracted with ethyl ether, and the combined organic layers were dried (MgSO₄) and concentrated. Purification of the resulting oil by silica gel flash chromatography (gradient elution, using hexane-ethyl ether 1:1 to 3:7) provided 0.258 g (64%) of the title compound as a colorless oil.

Step 5: tert-butyl (3R,5R)-1-((benzyloxy)carbonyl)-5-(hydroxymethyl)pyrrolidin-3-ylcarbamate

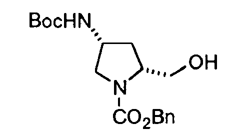

To a stirring solution of the tert-butyl (3R,5R)-1-((benzyloxy)carbonyl)-5-(methoxycarbonyl)pyrrolidin-3-ylcarbamate (0.16 g, 0.42 mmol) in THF (1.5 ml) was added LiCl (0.054 g, 1.27 mmol) and NaBH₄ (0.048 g, 1.27 mmol). After addition of ethanol (3 ml), the resulting mixture was stirred at room temperature overnight, and then quenched with water (1 ml). The solution was concentrated, and the residue was partitioned between ethyl acetate (20 ml) and water (3 ml). The aqueous layer was extracted with ethyl acetate (2x 2ml), and the combined organic layers were washed with brine, dried (MgSO₄) and concentrated. Purification of the resulting oil by flash chromatography (silica gel, using ethyl ether) provided 0.11 g (74%) of the title compound as a colorless oil.

Step 6: tert-butyl (3R,5R)-5-(hydroxymethyl)pyrrolidin-3-ylcarbamate

tert-Butyl (3R,5R)-1-((benzyloxy)carbonyl)-5-(hydroxymethyl)pyrrolidin-3-ylcarbamate (0.11 g, 0.31 mmol) was dissolved in ethanol (20 ml) and transferred to a Parr shaker bottle. After adding 10% Pd-C (0.030 g), the mixture was shaken under an atmosphere of hydrogen at 50 psi for 0.5 h on the Parr apparatus. The catalyst was removed by filtration through Celite. The filter cake was washed with ethanol and the combined filtrate and washings were concentrated in vacuo to yield the title compound as a colorless oil (0.07 g, 100%).

(2R,3S)-2-(Hydroxymethyl)pyrrolidin-3-ol hydrochloride

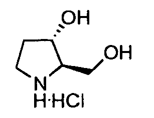

Step 1: (2S,3S)-1-(tert-butoxycarbonyl)-3-hydroxypyrrolidine-2-carboxylic acid

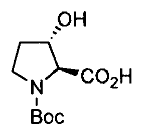

Trans-3-hydroxy-L-proline (2.62 g, 20.0 mmol) and sodium bicarbonate (5.04 g, 60 mmol) were dissolved in water (20 ml). Dioxane was added (20 ml) followed by di-tert-butyl-dicarbonate (8.72 g, 40 mmol). Stirring was continued at room temperature overnight. The reaction was concentrated, and the residue was partitioned between ethyl ether (10 ml) and water (30 ml). The aqueous layer was washed once more with ether, and the organic layers were discarded. Gradual acidification of the aqueous phase with concentrated HCl caused the oily product to precipitate and this was extracted into ethyl acetate by repeated washings (3x10 ml) of the aqueous layer. The combined organic layers were washed with brine, dried (MgSO₄) and concentrated to provide the title compound as a viscous oil (4.17 g, 90%).

Step 2: (25,3S)-1-tert-Butyl-2-methyl 3-hydroxypyrrolidine-1,2-dicarboxylate

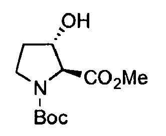

The (2S,3S)-1-(tert-butoxycarbonyl)-3-hydroxypyrrolidine-2-carboxylic acid (4.2 g, 18.2 mmol) and NaHCO₃ (3.1 g, 36.3 mmol) were suspended in DMF (20 ml). Methyl iodide (5.7 ml, 12.9 g, 91.0 mmol) was added to the mixture, which was then heated at 50°C overnight. After concentrating the reaction in vacuo, the residue was partitioned between ethyl acetate and water. The aqueous layer was extracted once more with ethyl acetate, and the combined organic layers were washed with brine, dried (MgSO₄) and concentrated. The residue was purified by silica flash chromatography (gradient elution, using 1:1 hexane-ethyl ether to ethyl ether) to provide the title compound as a colorless oil (3.7 g, 82%).

Step 3: (2R,3S)-tert-butyl 3-hydroxy-2-(hydroxymethyl)pyrrolidine-1-carboxylate

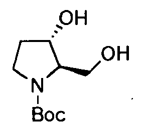

The (2S,3S)-1-tert-butyl-2-methyl 3-hydroxypyrrolidine-1,2-dicarboxylate (0.54g, 2.20 mmol) was dissolved in THF (8.0 ml). Lithium chloride (0.28 g, 6.60 mmol) and sodium borohydride (0.25 g, 6.60 mmol) were added, then ethanol (16.0 ml). The reaction was stirred overnight, then quenched with water (4 mL) and concentrated. The residue was partitioned between ethyl acetate and water. The aqueous layer was extracted with ethyl acetate. The organic layers were combined, washed with brine, and dried (MgSO₄). Flash chromatography (50 ml silica gel, gradient elution, using ethyl acetate to 9:1 ethyl acetate-ethanol) yielded 0.5 grams (100%) of the title compound as a colorless solid.

Step 4: (2R,3S)-2-(hydroxymethyl)pyrrolidin-3-ol hydrochloride

To (2R,3S)-tert-butyl 3 -hydroxy-2-(hydroxymethyl)pyrrolidine-1-carboxylate (0.50 g, 2.30 mmol) was added a 4N HCl/dioxane solution (6 ml) and the mixture was swirled for 2 hours. The solution was concentrated in vacuo. The residue was diluted with toluene (20 ml) and reconcentrated to provide the title compound as a colorless oil (0.36 g, 100%).

(2R,3R,4S)-2-(Hydroxymethyl)pyrrolidine-3,4-diol hydrochloride

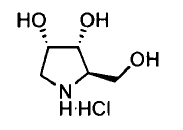

Step 1: (S)-1-(tert-butoxycarbonyl)-2,5-dihydro-1H-pyrrole-2-carboxylic acid

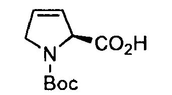

3,4-Dehydro-L-Proline (1.0 g, 8.8 mmol) was dissolved in H₂O (9.0 ml) and sodium bicarbonate (2.23 g, 26.5 mmol). Dioxane (9.0 ml) was added followed by di-tert-butyldicarbonate (3.86 g, 17.7 mmol). The reaction was stirred overnight, then concentrated. The residue was partitioned between ethyl ether (20 ml) and water (25 ml), and the layers were separated. The aqueous layer was diluted with ethyl acetate (20 ml) and the mixture was slowly acidified with concentrated HCl while stirring the mixture vigorously to extract the precipitate into the organic layer. After acidification to - pH2, and further extraction with ethyl acetate, the aqueous layer was saturated with salt and extracted once more with ethyl acetate. The combined organic layers were washed with brine, dried (MgSO₄) and concentrated to yield the title compound (2.0 g, 100%) as a viscous, colorless oil.

Step 2: (S)-1-tert-butyl-2-methyl 2H-pyrrole-1,2(5H)-dicarboxylate

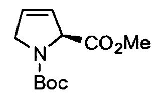

The (S)-1-(tert-butoxycarbonyl)-2,5-dihydro-1H-pyrrole-2-carboxylic acid (0.85 g, 4.0 mmol) was dissolved in ethyl ether (10 ml) and methanol (10 ml), then cooled to -5°C in an ice/ethanol bath. Trimethylsilyldiazomethane (4.4 ml of 2.0M solution in hexane, 8.8 mmol) was added dropwise. After stirring overnight, volatiles were removed under reduced pressure. The residue was partitioned between ethyl ether (20 ml) and water (5 ml), and the layers were separated. The organic layer was washed with saturated NaHCO₃ and brine, then dried (MgSO₄), filtered through a silica gel plug (7 ml) with ethyl ether, and concentrated, yielding the title compound as a colorless oil (0.821 g, 911%).

Step 3: (2S,3R,4S)-1-tert-Bulyl 2-methyl 3,4-dihydroxypyrrolidine-1,2-dicarboxylate

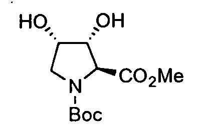

(S)-1-tert-Butyl-2-methyl 2H-pyrrole-1,2(5H)-dicarboxylate (0.83 g, 3.65 mmol) was dissolved in tert-butyl alcohol (15 ml), tetrahydrofuran (4 ml), and water (1.3 ml). Osmium tetraoxide (0.37 ml of 100 mg/ml solution in tert-butyl alcohol, 0.15 mmol) was added, followed by N-methylmorpholine N-oxide (0.51 g, 4.4 mmol). The reaction was stirred at room temperature for 5 hours, and then diluted with saturated sodium thiosulfate (5 ml), ethyl acetate (15 ml) and water (5 ml). After separation of layers, the organic layer was washed once more with sodium thiosulfate, then with brine, dried (MgSO₄), and filtered through a silica gel plug (7 ml) with ethyl acetate (75 ml) and concentrated. The oil was taken up in minimal ethyl ether/methylene chloride and purified by flash chromatography (gradient elution, using hexane-ethyl acetate 3:7 to ethyl acetate) to provide the title compound as a colorless oil (0.83 g, 87%).

Step 4: 3aR,4S,6aS)-5-tert-Butyl 4-methyl tetrahydro-2,2-dimethyl-[1,3]dioxolo[4,5-c]pyrrole-4,5-dicarboxylate

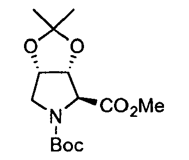

The (2S,3R,4S)-1-tert-butyl 2-methyl 3,4-dihydroxypyrrolidine-1,2-dicarboxylate (0.426 g, 1.63 mmol) was dissolved in 2,2-dimethoxypropane (10 ml). Pyridinium p-toluenesulfonate (0.02 g, 0.08 mmol) was added and the reaction was stirred at room temperature overnight. TLC showed reaction to be almost complete. More 2,2-dimethoxypropane (5 ml) was added and the mixture was heated with a heat gun until the mixture boiled and the total volume was reduced by about 1/4 (took about 5 minutes). The reaction was diluted with ethyl ether(10 ml) and the solution was extracted with saturated NaHCO₃ and brine, then it was dried (MgSO₄), filtered and concentrated, providing a pale yellow oil. Flash chromatography (70 ml silica gel, gradient elution, using hexane-ethyl ether 25:15 to hexane-ethyl ether 1:1) yielded the title compound (0.402 g, 82%) as colorless oil.

Step 5: (3aR,4R,6aS)-tert-butyl tetrahydro-4-(hydroxymethyl)-2,2-dimethyl [1,3]dioxolo[4,5-c]pyrrole-5-carboxylate

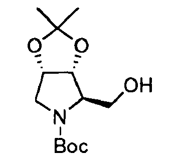

The (3aR,4S,6aS)-5-tert-butyl 4-methyl tetrahydro-2,2-dimethyl-[1,3]dioxolo[4,5-c]pyrrole-4,5-dicarboxylate (0.40 g, 1.3 mmol) was dissolved in THF (5.0 ml) and treated sequentially with lithium chloride (0.17 g, 4.0 mmol), sodium borohydride (0.15 g, 4.0 mmol) and ethanol (10 ml). The reaction was stirred overnight, then quenched with water (3 mL) and concentrated. The residue was partitioned between ethyl acetate and water. After extracting the aqueous layer once more with ethyl acetate, the combined organic layers were washed with brine and dried (MgSO₄). Flash chromatography (50 ml silica gel, gradient elution, using hexane-ethyl ether 6:4 to ethyl ether) yielded 0.36 grams (99%) of the title compound as a colorless oil.

Step 6: (2R,3R,4S)-2-(hydroxymethyl)pyrrolidine-3,4-diol hydrochloride

The (3aR,4R,6aS)-tert-butyl tetrahydro-4-(hydroxymethyl)-2,2-dimethyl-[1,3]dioxolo[4,5-c]pyrrole-5-carboxylate (0.36 g, 1.3 mmol) was dissolved in 4N HCl/dioxane (5 mL) and water (0.5 ml) and the reaction was swirled at room temperature for 2 hours. Then volatiles were removed under reduced pressure to yield a pink oil. This residue was diluted with toluene (20 mL) and reconcentrated to a solid which was triturated with ethyl ether. The ether was discarded and the solid dried in vacuo. Yield was 200 mg (90%) of the title compound as a pink solid. ¹H NMR (D₂O 400 MHz) 4.37-4.39 (m, 1H), 4.21(dd, J=8.6 Hz, 4.1 Hz, 1H), 3.98 (dd, J=12.7 Hz, 3.5 Hz, 1H), 3.83 (dd, J=12.5Hz, 6.0 Hz, 1H), 3.62 (ddd, J = 8.6 Hz, 6.0 Hz, 3.5 Hz, 1H), 3.50 (dd, J = 13.0 Hz, 4.1 Hz, 1H), 3.37 (dd, J=13.0 Hz, 2.0 Hz, 1H).

(2R,3S,4S)-2-(Hydroxymethyl)pyrrolidine-3,4-diol hydrochloride

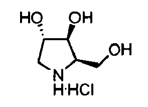

Step 1:

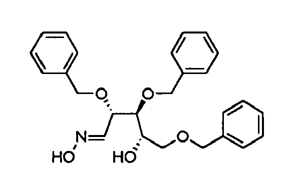

The 2,3,5-tri-O-benzyl-β-L-arabinose (0.5g, 1.19 mmol) was dissolved in ethanol (5 ml) with heating. Sodium bicarbonate (249 mg, 2.96 mmol) in water (2.5 ml) was added, followed by hydroxylamine hydrochloride (247 mg, 3.55 mmol). The heterogeneous mixture was stirred at room temperature for 5 hours. Then more hydroxylamine hydrochloride (100 mg, 1.44 mmol) and sodium bicarbonate (100 mg, 1.19 mmol) was added and the reaction stirred overnight. More sodium bicarbonate (0.084 g, lmmol) was added and the mixture was heated to boiling for 5 minutes. After cooling to room temperature, the reaction was concentrated. The resulting oil was triturated with THF (20 ml) until solids were a fine powder. The solids were filtered off and the filtrate was concentrated. The residue was purified by flash chromatography (hexane:ethyl acetate 3:1) to yield 0.45 grams (87%) of the product as a colorless oil.

Step 2:

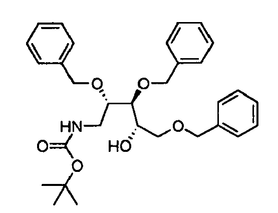

A solution of the oxime of 2,3,5-tri-O-benzyl-β-L-arabinose (0.45 g, 1.0 mmol) in dry ethyl ether (5 ml) was added dropwise to a solution of LiAlH₄ (0.75 mL of 2.5 M in THF, 1.85 mmol). The mixture was stirred for an additional 2 hours at room temperature after the addition. Ethyl acetate (1.7 ml) was added slowly to decompose the excess LiAlH₄, followed by 0.75 mL of 4N NaOH solution. The resulting cloudy suspension was filtered through a bed of Celite, and the Celite cake was washed thoroughly with ether and ethyl acetate. The filtrate and washings were concentrated to provide a viscous oil. The oil was taken up in ethyl acetate (20 ml) and washed with saturated NaHCO₃ and brine, then dried (MgSO₄) and concentrated to provide the crude amine as a pale yellow oil (0.44 g, 100%). This amine (0.44 g, 1.0 mmol) was dissolved in THF (3 ml) and water (1.5 ml). Sodium bicarbonate (0.22 g, 2.6 mmol) was added followed by di-tert-butyldicarbonate (0.46 g, 2.1 mmol). The reaction was stirred overnight, then concentrated. The residue was partitioned between ethyl ether (20 ml) and water (10 ml), and the layers were separated. The aqueous layer was extracted once more with ethyl ether, and the combined organic layers were washed with brine, dried (MgS0₄) and concentrated. Flash chromatography (silica gel, hexane-ethyl ether 1:1) yielded 0.29 g (52%) of the Boc amine as a colorless oil.

Step 3:

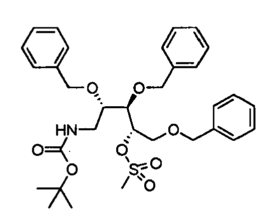

The alcohol (0.29 g, 0.55 mmol) and triethylamine (0.13 mL, 0.96 mmol) were dissolved in methylene chloride (2.0 ml) and cooled to -5°C in an ice-ethanol bath. Methanesulfonyl choride (64 µL, 0.83 mmol) was added and the reaction was stirred for 2 hours. After quenching with water (0.2 ml) the mixture was stirred for 30 minutes. The layers were separated, the aqueous layer was washed with methylene chloride and the combined organic layers were dried (MgSO₄) and concentrated. The residue was purified by flash chromatography (hexane-ethyl ether-methylene chloride 2:1:1) to yield the title compound (0.30 g, 91%).

Step 4:

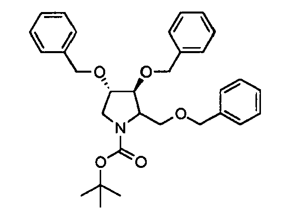

The N-Boc-O-mesylate compound (0.247 g, 0.412 mmol) was dissolved in DMF (2.0 mL), then sodium hydride (0.023 grams of 60% oil dispersion) was added directly to the solution and the cloudy mixture was stirred at room temperature for 2.5 hours. TLC showed the reaction to be complete. The reaction was diluted with ethyl ether (8 ml) and filtered directly through a plug of silica gel (7 ml packed in ethyl ether), and the filter cake was washed with ether. After removing volatiles under reduced pressure, the resulting residue was purified by silica gel flash chromatography, (hexane-ethyl ether-methylene chloride 3:1:1) to yield 0.196 g (95%) of the pyrrolidine product as colorless oil.

Step 5: (2R,3S,4S)-tert-Butyl 3,4-dihydroxy-2-(hydroxymethyl)pyrrolidine-1-carboxylate

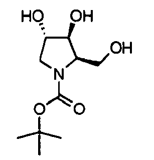

The tri-O-benzyl pyrrolidine (0.24 g, 0.47 mmol) was dissolved in methanol (50 ml) and added to a Parr shaker bottle. After flushing with nitrogen, 10% Pd-C (150 mg) was added and the mixture was hydrogenated on the Parr apparatus at 50 psi H₂ for 4 hours. The reaction mixture was filtered through a pad of Celite, the filter cake washed with methanol, and the solution was concentrated. Purification of the resulting residue by flash chromatography (silica gel, gradient elution, using ethyl acetate to ethyl acetate-ethanol 9:1) gave the triol product (0.103 g, 95%) as a colorless oil.

Step 6: (2R,3S,4S)-2-(Hydroxymethyl)pyrrolidine-3.4-diol hydrochloride

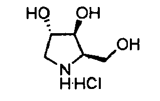

The N-Boc-pyrrolidine (0.103 g, 0.442 mmol) was dissolved in 4N HCl/dioxane (3 mL) and swirled at room temperature for 1.5 hour. The reaction was concentrated in vacuo to yield a colorless oil. The residue was diluted with toluene (20 mL), reconcentrated, then triturated with ethyl ether to try to induce crystallization. The residue solidified, the ether was discarded, and the solid was dried under vacuum to provide the crude title compound as a white solid (75 mg, 100%). ¹H NMR (D₂O 400 MHz) 4.39 (d, J = 4.3 Hz, 1H), 4.32 (app s, 1H), 3.99-4.05 (m, 1H), 3.87-3.93 (m, 2H), 3.66 (dd, J = 13.0 Hz, 4.3 Hz, 1H), 3.30 (d, J=13.0 Hz, 1H).

**EXAMPLE 5:** 3-(2-Fluoro-4-iodo-phenylamino)-furo[3,2-c]pyridine-2-carboxylic acid ((R)-2,3-dihydroxy-propoxy)-amide

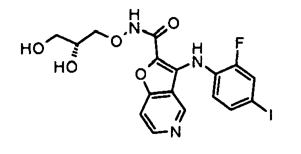

Step 1: 3-(2-Fluoro-4-iodo-phenylamino)-furor[3,2-c]pyridine-2-carboxylic acid ((R)-2,2-dimethyl-[1,3]dioxolan-4-ylmethoxy)-amide

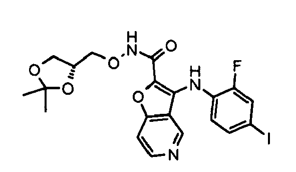

A mixture of ethyl 3-(2-fluoro-4-iodo-phenylamino)-furo[3,2-c]pyridine-2-carboxylate (60 mg, 0.15 mmol), 1N aqueous sodium hydroxide solution (0.18 ml, 0.18 mmol) and methanol (2 ml) were heated at 65°C for 30 minutes. The reaction mixture was concentrated then azeotroped with toluene (3x10 ml) to give a solid residue. The solid residue was dissolved in anhydrous THF (2 ml) and O-((R)-2,2-dimethyl-[1,3]dioxolan-4-ylmethyl)hydroxylamine (35 mg, 0.23 mmol), EDCI (28 mg, 0.15 mmol), HOBt (22 mg, 0.16 mmol) and DIPEA (61 µl, 0.35 mmol) were added. After stirring overnight at 40°C, the residue was absorbed onto HM-N and purified by flash chromatography (Si-SPE, diethyl ether: MeOH, gradient 100:0 to 90:10) to afford the title compound as a yellow foam (30 mg, 48%). LCMS (method B): R_{T}= 3.10 min, M+H⁺ = 528.

Step 2: 3-(2-Fluoro-4-iodo-phenylamino)-furo[3,2-c]pyridine-2-carboxylic acid ((R)-2,3-dihydroxy-propoxy)-amide

3-(2-Fluoro-4-iodo-phenylamino)-furo[3,2-c]pyridine-2-carboxylic acid ((R)-2,2-dimethyl-[1,3]dioxolan-4-ylmethoxy)-amide (30 mg, 0.06 mmol) was dissolved in methanol (0.5 ml) and loaded onto an Isolate® SCX-2 cartridge (5g). The cartridge was then washed with methanol (15 ml) and the desired product was subsequently eluted using2M NH₃ in MeOH and the eluant was collected and concentrated to give a residue. The residue was absorbed onto HM-N and purified by flash chromatography (Si-SPE, diethyl ether: MeOH, gradient 100:0 to 80:20) to afford the title compound as a white solid (18 mg, 64%). LCMS (method A): R_{T}= 5.80 min, M+H⁺ = 488. 1H NMR (d₄-MeOH, 400MHz) 8.53 (d, J = 5.9 Hz, 1H), 8.49 (d, J = 1.0 Hz, 1H), 7.62 (dd, J = 5.9 Hz, 1.0 Hz, 1H), 7.60 (dd, J = 10.3 Hz, 2.0 Hz, 1H), 7.51 (dd, J = 8.5 Hz, 2.0 Hz, 1H), 7.06 (t, J = 8.5 Hz, 1.0 Hz, 1H), 4.10 (m, 1H), 3.96 (m, 2H), 3.63 (m, 2H).

**EXAMPLE** 6: 3-(2-Fluoro-4-bromo-phenylamino)-furo[3,2-c]pyridine-2-carboxylic acid ((R)-2,3-dihydroxy-propoxy)-amide

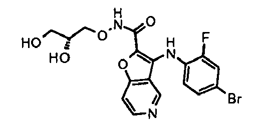

Step 1: 3-(2-Fluoro-4-bromo-phenylamino)-furo[3,2-c]pyridine-2-carboxylic acid ((R)-2,2-dimethyl-[1,3]dioxolan-4-ylmethoxy)-amide

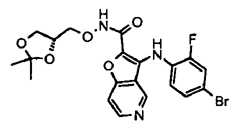

A mixture of ethyl 3-(2-fluoro-4-bromo-phenylamino)-furo[3,2-c]pyridine-2-carboxylate (400 mg, 1.06 mmol), 1N aqueous sodium hydroxide solution (1.11 ml, 1.11 mmol) and methanol (10 ml) were heated at 65°C for 30 minutes. The reaction mixture was concentrated *in vacuo* then azeotroped with toluene (3x10 ml) to give a solid residue. The solid residue was dissolved in anhydrous THF (10 ml) and O-((R)-2,2-dimethyl-[1,3]dioxolan-4-ylmethyl)hydroxylamine (255 mg, 2.12 mmol), EDCI (254 mg, 1.32 mmol), HOBt (200 mg, 1.48 mmol) and DIPEA (556 µl, 3.18 mmol) were added. After stirring overnight at ambient temperature, the reaction mixture was concentrated *in vacuo* to afford a yellow residue. The resultant residue was dissolved in ethyl acetate (30 ml), washed with water (30 ml) followed by brine (30 ml) before the organic layer was isolated then dried over sodium sulfate and concentrated *in vacuo* to afford a yellow oil. The oil was purified by flash chromatography (Si-SPE, pentane: ethyl acetate, gradient 50:50 to 0:100) to afford the title compound as an off-white foam (370 mg, 73%). 1H NMR (CDCl₃, 400 MHz) 9.22 (s, 1H), 7.95 (s, 1H), 7.67 (m, 1H), 7.51 (m, 1H), 7.45-7.31 (m, 3H), 7.13 (t, J = 8.4 Hz, 1H), 4.49 (m, 1H), 4.08-4.26 (m, 3H), 3.89 (m, 1H), 1.49 (s, 3H), 1.40 (s, 3H).

Step 2: 3-(2-Fluoro-4-bromo-phenylamino)-furo[3,2-c]pyridine-2-carboxylic acid ((R)-2.3-dihydroxy-propoxy)-amide

To 3-(2-Fluoro-4-bromo-phenylamino)-furo[3,2-c]pyridine-2-carboxylic acid ((R)-2,2-dimethyl-[1,3]dioxolan-4-ylmethoxy)-amide (50 mg, 0.10 mmol) was added a solution of 4N HCl in methanol (1 ml) then the reaction mixture was stirred at ambient temperature for 30 minutes. Water (10 ml) and ethyl acetate (10 ml) were added to the reaction mixture and the organic layer was isolated. The resultant organic phase was washed with saturated NaHCO₃ solution (10 ml) then dried over sodium sulfate before being concentrated *in vacuo* to produce a residue. The residue was loaded onto an Isolate® SCX-2 cartridge (5g). The cartridge was then washed with methanol (15 ml) before the desired product was eluted using 2M ammonia in MeOH and the eluant was collected then concentrated to give a residue. The residue was purified by flash chromatography (Si-SPE, DCM: MeOH, gradient 100:0 to 93:7) to afford the title compound as a white solid (27 mg, 59%). LCMS (method A): R_{T} = 5.55 min, M+H⁺ = 440/442. 1H NMR (d₄-MeOH, 400MHz) 8.52 (s, 1H), 8.44 (s, 1H), 7.60 (d, J = 5.9 Hz, 1H), 7.44 (dd, J = 8.8 Hz, 2.2 Hz, 1H), 7.32 (m, 1H), 7.19 (m, 1H), 4.06 (m, 1H), 3.91 (m, 2H), 3.59 (m, 2H).

**EXAMPLE 7:** 3-(4-Ethynyl-2-fluoro-phenylamino)-furo[3,2-c]pyridine-2-carboxylic acid ((R)-2,3-dihydroxy-propoxy)-amide

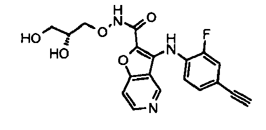

A mixture of 3-(2-fluoro-4-bromo-phenylamino)-furo[3,2-c]pyridine-2-carboxylic acid ((R)-2,2-dimethyl-[1,3]dioxolan-4-ylmethoxy)-amide (100 mg, 0.21 mmol), trimethylsilyl acetylene (288 µl, 2.08 mmol) and PdCl₂(PPh₃)₂ (7.3 mg, 0.01 mmol) in triethylamine (3.0 ml) were subjected to microwave irradiation at 150°C for 10 minutes. The reaction mixture was then diluted with ethyl acetate (5 ml) and the resultant solution was washed with water (10 ml) followed by brine (5 ml) then the organic layer was dried over sodium sulfate and concentrated *in vacuo* to give a residue. The residue was dissolved in methanol (3 ml) and potassium carbonate (58 mg, 0.42 mmol) was added, and the reaction mixture was stirred for 1 hour at ambient temperature. The reaction mixture was then evaporated to dryness and the resultant residue dissolved in ethyl acetate (20 ml). The organic phase was washed with water (10 ml) followed by brine (10 ml) and dried over sodium sulfate the concentrated *in vacuo* to produce a residue. The resultant residue was dissolved in methanol (0.5 ml) and loaded onto an Isolate® SCX-2 cartridge (5g). The cartridge was washed with methanol (15 ml) before the desired product was eluted using 2M triethylamine in MeOH, and the eluant was collected then concentrated to afford the title compound as a white solid (19 mg, 24%). LCMS (method A): R_{T}= 5.78 min, M+H⁺= 386. 1H NMR (d₄-MeOH, 400MHz) 8.51 (m, 2H), 7.60 (d, J = 5.9 Hz, 1H), 7.31 (dd, J =11.3 Hz, 1.8 Hz, 1H), 7.23 (dd, J = 8.4 Hz, 1.6 Hz, 1H), 7.06 (t, J = 8.5 Hz, 1H), 4.07 (m, 1H), 3.92 (m, 2H), 3.55 (m, 2H), 3.49 (s, 1H).

**EXAMPLE 8:** 3-(2-Fluoro-4-iodo-phenylamino)-furo[3,2-c]pyridine-2-carboxylic acid cyclopropylmethoxy-amide 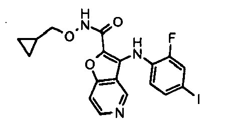 A mixture of ethyl 3-(2-fluoro-4-iodo-phenylamino)-furo[3,2-c]pyridine-2-carboxylate (87 mg, 0.20 mmol), 1N aqueous sodium hydroxide solution (0.21 ml, 0.21 mmol) and methanol (2 ml) were heated at 65°C for 60 minutes. The reaction mixture was concentrated *in vacuo* then azeotroped with toluene (3x10 ml) to give a solid residue. The solid residue was dissolved in anhydrous THF (5 ml) and cyclopropylmethylhydroxylamine hydrochloride (49 mg, 0.40 mmol), EDCI (48 mg, 0.25 mmol), HOBt (36 mg, 0.27 mmol) and DIPEA (140 µl, 0.80 mmol) were added. After stirring overnight at 40°C, the residue was absorbed onto HM-N and purified by flash chromatography (Si-SPE, diethyl ether: MeOH, gradient 98:2 to 95:5) to afford the title compound as an off-white solid (31 mg, 33%). 1H NMR (CDCl₃, 400MHz) 8.84 (s, 1H), 8.62 (s, 1H), 8.59 (d, J = 5.9 Hz, 1H), 7.99 (s, 1H), 7.51 (dd, J = 9.7 Hz, 1.5Hz,, 1H), 7.43 (d, J = 8.6 Hz, 1H), 7.38 (d, J = 5.9 Hz, 1H), 6.98 (t, J = 8.4 Hz, 1H), 3.90 (d, J = 7.5 Hz, 2H), 1.20 (m, 1H), 0.66 (m, 2H), 0.37 (m, 2H).

**EXAMPLE 10:** 3-(2-Fluoro-4-iodo-phenylamino)-furo[3,2-*c*]pyridine-2-carboxylic acid (2-hydroxy-ethoxy)-amide

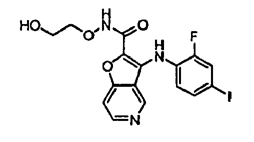

Step 1: 3-(2-Fluoro-4-iodo-phenylamino)-furo[3,2-c]pyddine-2-carboxylic acid (2-vinyloxy-ethoxy)-amide

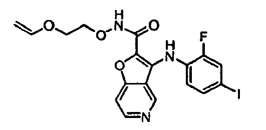

To a solution of Ethyl 3-(2-fluoro-4-iodo-phenylamino)-furo[3,2-c]pyridine-2-carboxylate (6.50 g, 15.2 mmol) in THF (92 mL) and methanol (31 mL) was added a 1.0 M aqueous solution of sodium hydroxide (31 mL, 31 mmol). The reaction mixture was heated to 65°C for 1.5 h and then cooled to room temperature and concentrated in vacuo. The resulting residue was azeotroped with toluene (3 x 75 mL), and then suspended in THF (75 mL). *O*-(2-Vinyloxy-ethyl)-hydroxylamine (1.86 g, 18.0 mmol), *N-N*-diisopropylethylamine (10.4 mL, 60.0 mmol), EDCI (5.75 g, 30.0 mmol), and HOBt (4.46 g, 33.0 mmol) were then added sequentially, and the reaction mixture stirred 18 hours at room temperature. 18.9 g silica gel was then added and the mixture was concentrated in vacuo. The residue was purified by silica gel chromatography (0-7% Methanol:CH₂Cl₂) to afford the title compound as a pale yellow solid: 4.40 g, 60%. LCMS (method C): R_{T} = 2.11 min, M+H⁺ = 484. 1H NMR (CDCl₃, 400MHz) 8.97 (s, 1H), 8.63 (s, 1H), 8.60 (d, J = 5.6 Hz, 1H), 7.98 (s, 1H), 7.52 (dd, J = 9.6, 2.0 Hz, 1H), 7.44 (m, 1H), 7.39 (dd, J = 6.0, 1.2 Hz, 1H), 7.00 (t, J = 8.8 Hz, 1H), 6.56 (dd, J = 14.4, 6.8 Hz, 1 H), 4.34 (m, 2H), 4.28 (dd, J = 14.0, 2.0 Hz, 1 H), 4.12 (dd, J = 6.4, 2.0 Hz, 1 H), 4.03 (m, 2 H).

3-(2-Fluoro-4-iodo-phenylamino)-furo[3,2-*c*]pyridine-2-carboxylic acid (2-hydroxy-ethoxy)-amide

To a suspension of 3-(2-Fluoro-4-iodo-phenylamino)-furo[3,2-c]pyridine-2-carboxylic acid (2-vinyloxy-ethoxy)-amide (4.40 g, 9.10 mmol) in a mixture of methanol (14.3 mL) and ethanol (51.9 mL) was added a 1.0 M aqueous solution of hydrochloric acid (18.2 mL, 18.2 mmol) at 0°C. After addition was complete, the reaction mixture was brought to room temperature and stirred for 1.5 h. Solid sodium bicarbonate (4.75 g, 56.5 mmol) was then added portionwise, and stirring was continued for 15 minutes. Silica gel (14 g) was added and the mixture concentrated in vacuo. The residual solid was purified by silica gel chromatography (0-10% methanol:CH₂Cl₂) to afford the title compound as a pale yellow solid: 4.12 g, 91%. LCMS (method C): R_{T}= 1.61 min, M+H⁺= 458. 1H NMR (CDCl₃, 400MHz) 8.84 (s, 1H), 8.61 (s, 1H), 8.60 (s, 1H), 7.94 (s, 1H), 7.53 (dd, J = 9.6, 2.0 Hz, 1H), 7.43 (m, 1H), 7.38 (dd, J = 6.0, 1.2 Hz, 1H), 7.00 (t, J = 8.4 Hz, 1H), 4.30 (b, 1H), 4.11 (m, 2 H), 3.83 (b, 2 H).

**EXAMPLE 11:** 3-(2-Fluoro-4-iodo-phenylamino)-furo[3,2-*c*]pyridine-2-carboxylic acid (2-vinyloxy-ethoxy)-amide

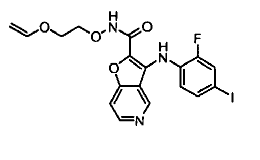

**EXAMPLE 12:** 3-(4-Iodo-phenylamino)-furo[3,2-c]pyridine-2-carboxylic acid ((R)-2,3-dihydroxy-propoxy)-amide

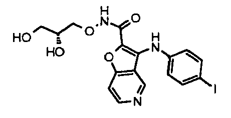

Step 1: 3-(4-Iodo-phenylamino)-furo[3,2-c]pyridine-2-carboxylic acid ((R)-2,2-dimethyl-[1,3]dioxolan-4-ylmethoxy)-amide

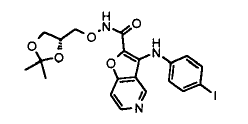

A mixture of ethyl 3-(4-iodo-phenylamino)-furo[3,2-c]pyridine-2-carboxylate (100 mg, 0.24 mmol), 1N aqueous sodium hydroxide (260 µl) and ethanol (4 ml) was heated at 65°C for 3 hours. The reaction mixture was condensed and azeotroped with toluene (3 x 20 ml) to give a solid residue. The solid residue was dissolved in anhydrous THF (7 ml), to which EDCI (57 mg, 0.30 mmol) and HOBt (45 mg, 0.33 mmol) were added and the mixture stirred for 30 minutes before O-((R)-2,2-dimethyl)-[1,3]dioxolan-4-ylmethyl)hydroxylamine (71 mg, 0.48 mmol) and DIPEA (125 µl, 0.72 mmol) were finally added. After stirring for 16 hours at ambient temperature, the residue was absorbed onto HM-N and purified by flash chromatography (Si-SPE, cyclohexane: ethyl acetate, gradient 50:50 to 0:100) to afford the title compound as an off white solid (103 mg, 84%). LCMS (method B): R_{T} = 2.86 min, M+H⁺ = 510.

Step 2:3-(4-Iodo-phenylamino)-furo[3,2-c]pyridine-2-carboxylic acid ((R)-2,3-dihydroxy-propoxy)-amide

3-(4-Iodo-phenylamino)-furo[3,2-c]pyridine-2-carboxylic acid ((R)-2,2-dimethyl-[1,3]dioxolan-4-ylmethoxy)-amide (100 mg, 0.19 mmol) was dissolved in methanol and subjected to chromatography (Isolute® SCX-2, EtOAc then EtOAc: MeOH: Et₃N, 89:10:1). The resultant residue was absorbed onto HM-N and purified by flash chromatography (Si-SPE, dichloromethane: MeOH, gradient 100:0 to 90:10) to afford the title compound as a pale yellow solid (38 mg, 42%). LCMS (method A): R_{T} = 6.16 min, M+H⁺ = 470. 1H NMR (d₄-MeOH, 400MHz) 8.52 (d, J = 5.9 Hz, 1H), 8.48 (s, 1H), 7.67 (d, J = 8.8 Hz, 2H), 7.60 (dd, J = 6.0Hz, 0.8 Hz, 1H), 7.00 (d, J = 8.7 Hz, 2H), 4.09 (dd, J = 9.9 Hz, 3.4 Hz, 1H), 3.93 - 4.00 (m, 2H), 3.61 - 3.64 (m, 2H).

**EXAMPLE 13**: 3-(2-Chloro-4-iodo-phenylamino)-furo[3,2-c]pyridine-2-carboxylic acid ((R)-2,3-dihydroxy-propoxy)-amide

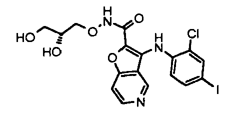

Step 1: 3-(2-Chloro-4-iodo-phenylamino)-furo[3,2-c]pyridine-2-carboxylic acid ((R)-2,2-dimethyl-[1,3]dioxolan-4-ylmethoxy)-amide

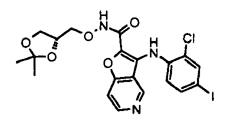

A mixture of ethyl 3-(2-chloro-4-iodo-phenylamino)-furo[3,2-c]pyridine-2-carboxylate (115 mg, 0.26 mmol), 1N aqueous sodium hydroxide solution (0.27 ml, 0.27 mmol) and industrialized methylated spirits (3.0 ml) were heated at 65°C for 60 minutes. The reaction mixture was concentrated then azeotroped with toluene (3 x 10 ml) to give a solid residue. The resultant solid residue was dissolved in anhydrous THF (5 ml), to which O-((R)-2,2-dimethyl-[1,3]dioxolan-4-ylmethyl)hydroxylamine (75 mg, 0.51 mmol), EDCI (65 mg, 0.34 mmol), HOBt (49 mg, 0.36 mmol) and DIPEA (175 µl, 1.02 mmol) were added. The reaction mixture was stirred for 48h, and was then concentrated under reduced pressure. The resultant residue was absorbed onto HM-N and purified by flash chromatography (Si-SPE, dichloromethane: methanol, gradient 100:0 to 95:5) to afford the title compound as a yellow oil (119 mg, 84%). LCMS (method B): R_{T} = 3.14 min, M+H⁺ = 544.

Step 2: 3-(2-Chloro-4-iodo-phenylamino)-furo[3,2-c]pyridine-2-carboxylic acid ((R)-2,3-dihydroxypropoxy)-amide

3-(2-Chloro-4-iodo-phenylamino)-furo[3,2-c]pyridine-2-carboxylic acid ((R)-2,2-dimethyl-[1,3]dioxolan-4-ylmethoxy)-amide (119 mg, 0.22 mmol) was dissolved in methanol (5.0 ml) and loaded onto an Isolute® SCX-2 cartridge (5g). The cartridge was then washed with methanol (15 ml) and the desired product was subsequently eluted using 2M NH₃ in MeOH. The eluant was collected and concentrated to give a residue. The residue was absorbed onto HM-N and purified by flash chromatography (Si-SPE, dichloromethane: methanol, gradient 100:0 to 90:10) to afford the title compound as a white solid (20 mg, 18%). LCMS (method A): R_{T} = 7.02 min, M+H⁺ = 504. 1H NMR (d₄-MeOH, 400MHz) 8.52 (d, J= 6.2 Hz, 1H), 8.52 (d, J = 0.9 Hz, 1H), 7.81 (d, J = 2.0 Hz, 1H), 7.61 (dd, J = 6.2 Hz, 0.9 Hz, 1H), 7.58 (dd, J = 8.5 Hz, 2.0 Hz, 1H), 7.01 (d, J = 8.5 Hz, 1H), 4.09-4.05 (m, 1H), 3.98-3.88 (m, 2H), 3.60-3.58 (m, 2H).

**EXAMPLE 14:** 3-(2,6-Difluoro-4-iodo-phenylamino)-furo[3,2-c]pylidine-2-carboxylic acid ((R)-2,3-dihydroxy-propoxy)-amide

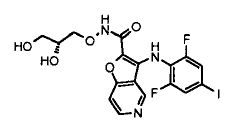

Step 1: 3-(2,6-Difluoro-4-iodo-phenylamino)-furo[3,2-c]pyridine-2-carboxylic acid (R)-2,2-dimeth1-[1,3]dioxolan-4-ylmethoxy)-amide

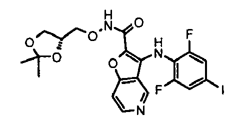

A mixture of ethyl 3-(2,6-difluoro-4-iodo-phenylamino)-furo[3,2-c]pyridine-2-carboxylate (137 mg, 0.31 mmol), 1N aqueous sodium hydroxide solution (0.32 ml, 0.32 mmol) and industrialized methylated spirits (5.0 ml) were heated at 65°C for 60 minutes. The reaction mixture was concentrated then azeotroped with toluene (2x10 ml) to give a solid residue. The solid residue was dissolved in anhydrous THF (5 ml) before O-((R)-2,2-dimethyl-[1,3]dioxolan-4-ylmethyl)hydroxylamine (89 mg, 0.61 mmol), EDCI (77 mg, 0.40 mmol), HOBt (58 mg, 0.43 mmol) and DIPEA (213 µl, 1.22 mmol) were added. The reaction mixture was stirred for 16 hours and concentrated under reduced pressure. The residue was absorbed onto HM-N and purified by flash chromatography (Si-SPE, dichloromethane: methanol, gradient 100:0 to 95:5) to afford the title compound as a yellow oil (63 mg, 37%). LCMS (method B): R_{T} = 2.87 min, M+H⁺ = 546.

Step 2: 3-(2,6-Difluoro-4-iodo-phenylamino)-furo[3,2-c]pyridine-2-carboxylic acid ((R)-2,3-dihydroxy-propoxy)-amide

3-(2,6-Difluoro-4-iodo-phenylamino)-furo[3,2-c]pyridine-2-carboxylic acid ((R)-2,2-dimethyl-[1,3]dioxolan-4-ylmethoxy)-amide (63 mg, 0.11 mmol) was dissolved in methanol (4.0 ml) and loaded onto an Isolute® SCX-2 cartridge (5g). The cartridge was then washed with methanol (15 ml) and the desired product was subsequently eluted using 2M NH₃ in MeOH. The eluant was collected and concentrated to give a residue. The resultant residue was absorbed onto HM-N and purified by flash chromatography (Si-SPE, dichloromethane: methanol, gradient 100:0 to 90:10) to afford the title compound as a white solid (17 mg, 31%). LCMS (method A): R_{T} = 5.97 min, M+H⁺ = 506. 1H NMR (d₄-MeOH, 400MHz) 8.48 (d, J= 6.0 Hz, 1H), 8.26 (s, 1H), 7.55 (d, J = 6.0 Hz, 1H), 7.54-7.49 (m, 2H), 4.08-4.05 (m, 1H), 3.96-3.87 (m, 2H), 3.60-3.58 (m, 2H).

**EXAMPLE 15**: 3-(2,5-Difluoro-4-iodo-phenylamino)-furo[3,2-c]pyridine-2-carboxylic acid ((R)-2,3-dihydroxy-propoxy)-amide

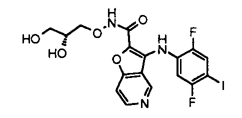

Step 1: 3-(2,5-Difluoro-4-iodo-phenylamino)-furo[3,2-c]pyridine-2-carboxylic acid ((R)-2,2-dimethyl-[1,3]dioxolan-4-ylmethoxy)-amide

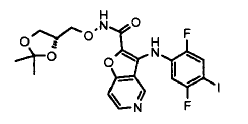

A mixture of ethyl 3-(2,5-difluoro-4-iodo-phenylamino)-furo[3,2-c]pyridine-2-carboxylate (163 mg, 0.37 mmol), 1N aqueous sodium hydroxide solution (0.38 ml, 0.38 mmol) and industrialized methylated spirits (4.0 ml) were heated at 65°C for 30 minutes. The reaction mixture was concentrated *in vacuo* then azeotroped with toluene (2 x 10 ml) to give a solid residue. The solid residue was dissolved in anhydrous THF (5 ml) before O-((R)-2,2-dimethyl-[1,3]dioxolan-4-ylmethyl)hydroxylamine (106 mg, 0.72 mmol), EDCI (91 mg, 0.470 mmol), HOBt (69 mg, 0.51 mmol) and DIPEA (250 µl, 1.45 mmol) were added. The reaction mixture was stirred for 16 hours and concentrated under reduced pressure. The residue was absorbed onto HM-N and purified by flash chromatography (Si-SPE, dichloromethane: methanol, gradient 100:0 to 95:5) to afford the title compound as a yellow oil (152 mg, 76%). LCMS (method B): R_{T} = 3.01 min, M+H⁺ = 546.

Step 2: 3-(2,5-Difluoro-4-iodo-phenylamino)-furo[3,2-c]pyridine-2-carboxylic acid ((R)-2,3-dihydroxy-propoxy)-amide

3-(2,5-Difluoro-4-iodo-phenylamino)-furo[3,2-c]pyridine-2-carboxylic acid ((R)-2,2-dimethyl-[1,3]dioxolan-4-ylmethoxy)-amide (145 mg, 0.27 mmol) was dissolved in methanol (5.0 ml) and loaded onto an Isolute® SCX-2 cartridge (5g). The cartridge was then washed with methanol (15 ml) and the desired product was subsequently eluted using 2M NH₃ in MeOH. The eluant was collected and concentrated to give a residue. The residue was absorbed onto HM-N and purified by flash chromatography (Si-SPE, dichloromethane: methanol, gradient 100:0 to 90:10) to afford the title compound as a white solid (75 mg, 56%). LCMS (method A): R_{T} = 6.49 min, M+H⁺= 506. 1H NMR (d₄-MeOH, 400MHz) 8.61 (d, J= 0.9 Hz, 1H), 8.52 (d, J=6.1 Hz, 1H), 7.61 (dd, J = 6.1 Hz, 0.9 Hz, 1H), 7.59 (dd, J=10.1Hz, 5.7 Hz, 2H), 6.94 (dd, J=8.4 Hz, 7.5 Hz, 1H), 4.07-4.03 (m, 1H), 3.96-3.87 (m, 2H), 3.63-3.55 (m, 2H).

**EXAMPLE 16**: 4-{[3-(2-Fluoro-4-iodo-phenylaminol-furo[3,2-c]pyridine-2-carbonyl]aminooxy}-piperidine-1-carboxylic acid tert-butyl ester

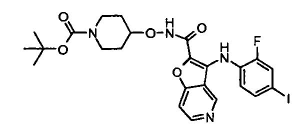

A mixture of 3-(2-fluoro-4-iodo-phenylamino)-furo[3,2-c]pyridine-2-carboxylic acid (398 mg, 1 mmol), HOBT (190 mg, 1.4 mmol) and EDCI (240 mg, 1.25 mmol) in THF (5 ml) was stirred for lh. To this mixture was added DIPEA (530 µl, 3.0 mmol) and 4-aminooxy-piperidine-1-carboxylic acid *tert*-butyl ester (432 mg, 2.0 mmol). After stirring overnight, the reaction mixture was concentrated *in vacuo,* diluted with ethyl acetate (30 ml) and washed with saturated NaHCO₃ solution (10 ml). The organic layer was isolated, dried over sodium sulfate and concentrated *in vacuo.* Purification of the resultant residue by flash chromatography (Si-SPE, dichloromethane: methanol, gradient 100% to 95:5) afforded the title compound as a pale yellow foam (285 mg, 47%). LCMS (method A): R_{T}= 10.43 min, M+H⁺= 597. 1H NMR (CDCl₃, 400MHz) 11.86 (s, 1H), 8.15 (s, 1H), 7.66 (dd, J = 10.8 Hz, 2.0 Hz, 1H), 7.61 (d, J = 8.6 Hz, 1H), 7.51 (m, 1H), 7.43 (dd, J = 8.8 Hz, 1H), 7.32 (d, J = 7.7 Hz, 1H), 7.27 (m, 1H), 6.86 (t, J = 8.8 Hz, 1H), 4.88 (d (br), J = 3.4 Hz, 1H), 4.60 (t (br), J = 5.5 Hz, 1H), 3.17 (ddd, J= 13.6, 8.8.3.7 Hz, 2H), 1.97-2.03 (m, 2H), 1.7-1.81 (m, 2H), 1.63 (s, 9H).

**EYAMPLE 17:** 3-(2-Fluoro-4-iodo-phenylamino)-furo[3,2-c]pyridine-2-carboxylic acid (2-morpholin-4-yl-ethoxy)-amide

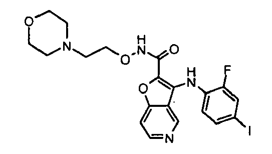

A mixture of 3-(2-fluoro-4-iodo-phenylamino)-furo[3,2-c]pyridine-2-carboxylic acid (199 mg, 0.5 mmol), HOBt (95 mg, 0.7 mmol) and EDCI (125 mg, 0.65 mmol) in THF (2 ml) was stirred for lh. To this mixture was added DIPEA (270 µl, 1.5 mmol) and O-(2-(tetrahydropyran-4-yl)ethyl)hydroxylamine (146 mg, 1.0 mmol). After stirring overnight, the solution was concentrated *in vacuo,* diluted with ethyl acetate (30 ml) and washed with saturated NaHCO₃ solution (10 ml). The organic layer was isolated, dried over sodium sulfate and concentrated *in vacuo.* Purification of the resultant residue by flash chromatography (Si-SPE, dichloromethane: methanol, gradient 100:0 to 95:5) afforded the title compound as a foam which crystallised from ether/dichloromethane (75 mg, 28%). LCMS (method A): R_{T} = 5.67 min, M+H⁺ = 527. 1H NMR (CDCl₃, 400MHz) 8.77 (s, 1H), 8.63 (d, J = 0.7 Hz, 1H), 8.59 (d, J = 5.8Hz, 1H), 7.99 (s, 1H), 7.51 (dd, J = 9.7 Hz, 2.0 Hz, 1H), 7.42-7.46 (m, 1H), 7.37 (dd, J = 6.0, 1.2 Hz, 1H), 6.99 (t, J = 8.5 Hz, 1H), 4.20-4.12 (m, 2H), 3.92-3.80 (m, 2H), 3.17 (ddd, J = 13.6, 8.8, 3.7 Hz, 2H), 2.03-1.97 (m, 2H), 1.81-1.7 (m, 2H), 1.68-1.61 (m, 2H).

**EYAMPLE 18:** 7-Bromo-3-(2-fluoro-4-iodo-phenylamino)-furo[3,2-c]pyridine-2-carboxylic acid ((R)-2,3-dihydroxy-propoxy)amide

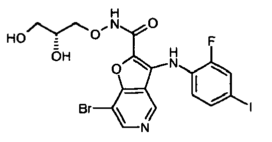

Step 1: 7-Bromo-3-(2-fluoro-4-iodo-phenylamino)-furo[3,2-c]pyridine-2-carboxylic acid ((R)-2,2-dimethyl-[1,3]dioxolan-4-ylmethoxy)-amide

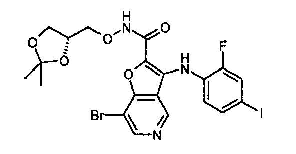

A mixture of 7-bromo-3-(2-fluoro-4-iodo-phenylamino)-furo[3,2-c]pyridine-2-carboxylic acid (50 mg, 0.21 mmol) and carbonyl diimidazole (35 mg, 0.21 mmol) in acetonitrile (2 ml) was heated at 50°C for 4 hours. The reaction was then treated with a solution of O-((R)-2,2-dimethyl-[1,3]dioxolan-4-ylmethyl)-hydroxylamine (46 mg, 0.36 mmol) in acetonitrile (1 ml) and heated at 80°C for 3.5 hours before cooling and allowing to stand at room temperature. The reaction mixture was filtered, washed with ethyl acetate before the filtrate was collected and concentrated *in vacuo.* Purification of the resultant residue by flash chromatography (Si-SPE, dichloromethane: ethyl acetate, gradient 1:0 to 4:1 to 0:1, then methanol) afforded the title compound as a light brown solid (11 mg, 20%). LCMS (method B): R_{T} = 3.55 min, M+H⁺ = 606/608.

Step 2: 7-Bromo-3-(2-fluoro-4-iodo-phenylamino)-furo[3,2-c]pyridine-2-carboxylic acid ((R)-2,3-dihydroxy-propoxy)amide

7-Bromo-3-(2-fluoro-4-iodo-phenylamino)-furo[3,2-c]pyridine-2-carboxylic acid ((R)-2,2-dimethyl-[1,3]dioxolan-4-ylmethoxy)-amide (40mg, 0.066mmol) was dissolved in 0.067M methanolic HCl (2.79ml, 0.198mmol) and stirred at room temperature for 40 mins. The reaction mixture was concentrated *in vacuo* then azeotroped with toluene (2 × 15ml). The resultant residue was dissolved in IMS (4 ml), and then potassium carbonate was added, before stirring at room temperature for 4 mins. The reaction mixture was filtered and washed with IMS, before the filtrate was evaporated *in vacuo* to afford a solid. The resultant solid was triturated with acetonitrile to afford the desired product as a cream solid (29 mg, 77%). LCMS (Method A): R_{T} = 9.06 min, M+H⁺ = 566/568. ¹H NMR (CD₃OD): 8.68 (1H, s, br), 8.42 (1H, s, br), 7.61 (1H, dd, J = 10.2, 1.9Hz), 7.52 (1H, m), 7.07 (1H, t, J = 8.6Hz), 4.12 (1H, dd, J = 10.0, 3.4Hz), 3.99 (1H, dd, J = 10.0, 6.8Hz), 3.96 (1H, m), 3.63 (2H, m).

**EYAMPLE 19:** 5-(2-Fluoro-4-iodo-phenylamino)-furo[2,3-d]pyrimidine-6-carboxylic acid ((R)-2,3-dihydroxy-propoxy)-amide

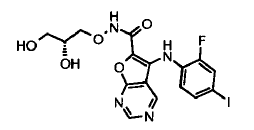

Step 1: 5-(2-fluoro-4-iodo-phenylamino)-furo[2,3-d]pyrimidine-6-carboxylic acid ((R)-2,2-dimethyl-[1,3]dioxolan-4-ylmethoxy)-amide

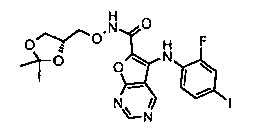

A mixture of ethyl 5-(2-fluoro-4-iodo-phenylamino)-furo[2,3-d]pyrimidine-6-carboxylate (300 mg, 0.70 mmol), 1N aqueous sodium hydroxide solution (0.75 ml, 0.75 mmol) and industrialized methylated spirits (8.0 ml) were heated at 65°C for 30 minutes. The reaction mixture was concentrated then azeotroped with toluene (3 x 10 ml) to give a solid residue. The solid residue was dissolved in anhydrous THF (5 ml) and O-((R)-2,2-dimethyl-[1,3]dioxolan-4-ylmethyl)hydroxylamine (106 mg, 0.72 mmol), EDCI (91 mg, 0.47 mmol), HOBt (69 mg, 0.51 mmol) and DIPEA (250 µl, 1.45 mmol) were added. The reaction mixture was stirred for 18h before being concentrated under reduced pressure. The resultant residue was absorbed onto HM-N and purified by flash chromatography (Si-SPE, dichloromethane: methanol, gradient 100:0 to 95:5) to afford the title compound (124 mg, 67%). LCMS (method B): R_{T} = 3.46 min, M+H⁺ = 529.

Step 2: 5-(2-Fluoro-4-iodo-phenylamino)-furo[2,3-d]pyrimidine-6-carboxylic acid ((R)-2,3-dihydroxy-propoxy)-amide

To a suspension of 5-(2-fluoro-4-iodo-phenylamino)-furo[2,3-d]pyrimidine-6-carboxylic acid ((R)-2,2-dimethyl-[1,3]dioxolan-4-ylmethoxy)-amide (124 mg, 0.23 mmol) in methanol (5.0 ml) was added concentrated hydrochloric acid (10 drops) and the mixture was stirred for 1 hour. The reaction mixture was then concentrated under reduced pressure, dissolved in methanol (5ml), and potassium sodium carbonate (~200 mg) was added. This mixture was stirred for 5 minutes, absorbed on HM-N and purified by flash chromatography (Si-SPE, dichloromethane: methanol, gradient 100:0 to 0:100) to afford the title compound as a white solid (60 mg, 54%). LCMS (method A): R_{T} = 7.85 min, M+H⁺ = 489. 1H NMR (d₆-DMSO, 400MHz) 8.96 (s, 1H), 8.77 (s, 1H), 7.64 (dd, J =10.7 Hz, 1.9 Hz, 1H), 7.43 (ddd, J = 8.5 Hz, 1.9 Hz, 0.9 Hz, 1H), 6.91 (dd, J = 8.5 Hz, 8.5 Hz, 1H), 3.86-3.71 (m, 3H), 3.40-3.30 (m, 4H).

**EXAMPLE 97**: 3-(2-Fluoro-4-iodophenylamino)furo[3,2-c]pyridine-2-carboxylic acid (1-methylpiperidin-4-yloxy) amide

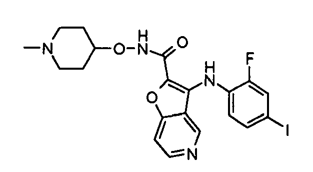

A mixture of 3-(2-fluoro-4-iodo-phenylamino)-furo[3,2-c]pyridine-2-carboxylic acid(piperidin-4-yloxy)-amide (80 mg, 0.16 mmol), pyridinium p-toluenesulfonic acid (40 mg, 0.16 mmol) and formaldehyde (0.05 ml, 10M aq. soln., 0.48 mmol) was suspended in methanol (0.5 ml) and was stirred under argon for 16 hours. Sodium cyanoborohydride (30 mg, 0.47 mmol) was added and the resulting solution was stirred for lh. The solvent was evaporated and the resultant residue partitioned between ethyl acetate and sodium bicarbonate, the organic layer was isolated, washed with brine, dried (Na₂SO₄) and evaporated. The resultant product was purified by flash chromatography (Si-SPE 2M methanolic ammonia: DCM gradient 0:100 to 10:100) to afford the product as a pale yellow solid (50 mg, 61% yield). LCMS (method A): R_{T} = 5.49min; M+H⁺ 511; 1H NMR (CDCl₃) 1.88 (2H, m), 2.08 (2H, m), 2.22 (2H, m), 2.31 (3H, s), 2.78 (2H, m), 4.08 (1H, m), 6.98 (1H, t, J = 8.5Hz), 7.37 (1H, dd, J = 5.9, 1.0Hz), 7.44 (1H, m), 7.51 (1H, dd, J = 9.8, 1.9Hz), 7.99 (1H, s), 8.59 (1H, d, J = 5.9Hz), 8.63 (1H, d, J = 1.0Hz).

**EXAMPLE 98:** 3-(2-Fluoro-4-iodo-phenylamino)furo[3,2-c]pyridine-2-carboxylic acid (2-dimethylaminoethoxy) amide

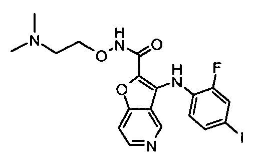

A mixture of 3-(2-fluoro-4-iodo-phenylamino)-fur o[3,2-c]pyridine-2-carboxylic acid (2-methylamino-ethoxy)-amide (45 mg, 99 mmol), formaldehyde (8 µL, 107 mmol), pyridinium p-toluenesulfonic acid (25 mg, 97 mmol) in ethanol (1 mL) was stirred at 0-5°C for 30min. Sodium cyanoborohydride (7 mg, 106 mmol) was added and the solution was stirred for 30 min at room temperature. HCl (1M, 200 µL) was added and the solution was loaded onto an Isolate® SCX-2 cartridge (2 g) eluting with methanolic ammonia. The appropriate fractions were combined and concentrated to provide a residue which was further purified by flash chromatography (Si-SPE gradient 2M methanolic ammonia: DCM 0:100 to 10:100) to afford the product as a white foam. (23 mg). LCMS (method A): R_{T} = 5.12main M+H⁺ 484; 1H NMR (CD₃OD) 2.70 (6H, s), 3.11 (2H, m), 4.15 (2H, m), 6.80 (1H, t, J = 8.7Hz), 7.42 (1H, m), 7.53 (1H, dd, J = 10.6, 1.9Hz), 7.61 (1H, dd, J = 5.9, 0.9Hz), 8.47 (1H, d, J = 5.9Hz), 8.56 (1H, d, J = 0.9Hz).

**EXAMPLE 99:** (2-Fluoro-4-iodo-phenylamino)-N-tert-butoxyfuro[3,2-c]pyridine-2-carboxamide 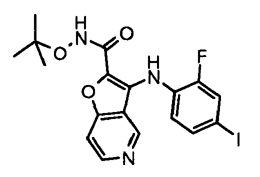 A mixture of 3-(2-fluoro-4-iodo-phenylamino)-furo[3,2-c]pyridine-2-carboxylic acid ethyl ester (251 mg, 0.59 mmol), 1N aqueous NaOH solution (1.77 ml, 1.77 mmol), methanol (15 ml) and tetrahydrofuran (15 ml) was refluxed for 2 hours. The reaction mixture was concentrated *in vacuo* then the resultant residue was azeotroped with toluene (3 x 15 ml) to give a solid residue. The solid residue was triturated with ether (3x10 ml), and the ether layers were discarded. The resultant solid residue was dried under vacuum. The solid residue was then dissolved in anhydrous DMF (5 ml) before O-tertbutylhydroxylamine hydrochloride (58 mg, 0.56 mmol), HATU (270 mg, 0.71 mmol) and DIPEA (470 µl, 2.36 mmol) were added. After stirring for 18 hours at ambient temperature the solvent was evaporated and the resultant residue was purified by preparative HPLC to afford the title compound as a white solid (80 mg, 23%). LCMS (method E): R_{T} = 2.30 min, M+H⁺ = 470. ¹H NMR (CDCl₃, 400MHz) 8.82 (d, 1H), 8.69 (s, 1H), 8.39 (s, 1H), 8.15 (s, 1H), 7.73 (d, 1H), 7.59 (dd, 1H), 7.51 (d,m, 1H), 6.85 (t, 1H), 1.4 (2, 1H).

**EXAMPLE 100:** (3-(2-fluoro-4-iodophenylamino)furo[3,2-c]pyridin-2-yl)(1-oxothiazolidin-3-yl)methanone

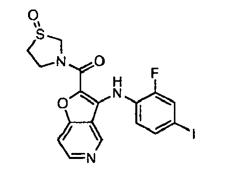

(3-(2-Fluoro-4-iodophenylamino)furo[3,2-c]pyridin-2-yl)(thiazolidin-3-yl)methanone was dissolved in methanol (1 ml) and THF (1 ml) and cooled to -5°C. Oxone (21 mg, 0.035 mmol) in water (0.5 ml) was added, and the reaction was stirred and warmed to room temperature over 1 hour. The reaction was diluted with ethyl acetate (3 ml) and water (1 ml) and solids decanted. The layers were separated, the aqueous layer extracted with ethyl acetate, and the combined organic layers were washed with sat, aqueous NaHCO₃, and brine, dried (MgSO₄), and concentrated.. The resulting oil was purified by reverse phase HPLC to provide the TFA salt of the title compound as a yellow solid (6.8 mg). LCMS (method D): R_{T} = 2.45 min M+H⁺ = 486.

**EXAMPLE 101:** (3-(2-fluoro-4-iodophenylamino)furo[3,2-c]pyridin-2-yl)(1,1-dioxothiomorpholin-4-yl)methanone

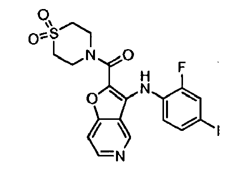

(3-(2-Fluoro-4-iodophenylamino)furo [3,2-c]pyridin-2-yl)(thiomorpholin-4-yl)methanone (19 mg, 0.039 mmol) was dissolved in methanol (1 ml) and THF (1 ml) and cooled to -5°C. Oxone (30 mg, 0.049 mmol) in water (0.5 ml) was added, and the reaction was stirred while warming to room temperature over 1 hour. The reaction was diluted with ethyl acetate (3 ml) and water (1 ml) and solids decanted. The layers were separated, the aqueous layer extracted with ethyl acetate, and the combined organic layers were washed with saturated aqueous NaHCO_{3,} and brine, dried (MgSO₄), and concentrated.. The resulting oil was purified by reverse phase HPLC to provide the TFA salt of the title compound as a yellow solid (1.5 mg). LCMS (method E) R_{T} = 4.17, M+H⁺ = 516.

**EXAMPLE 102:** (3-(2,5-difluoro-4-(4-pyrazolyl)phenylamino)furo[3,2-c]pyridin-2-yl)((R)-3-hydroxypyrrolidin-1-yl)methanone 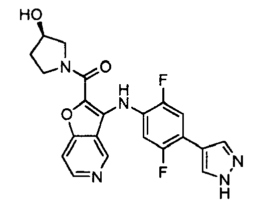 A degassed solution of (3-(4-bromo-2,5-difluorophenylamino)furo[3,2-c]pyridin-2-yl)((R)-3-hydroxypyrrolidin-1-yl)methanone (53 mg, 0.12 mmol), 1-Boc-pyrazole-4-boronic acid pinacol ester (53 mg, 0.18 mmol), Pd(PPh₃)₄ (7.0 mg, 0.0061 mmol), and Na₂CO₃ (29 mg, 0.27 mmol) in dimethoxyethane (2.0 ml), ethanol (0.7 ml), and water (0.7 ml) was heated at reflux overnight. The reaction mixture was cooled to ambient temperature, filtered, and the solid was washed with ethyl acetate and dried to provide the crude product as a tan solid (41 mg, 80%). LCMS (method D): R_{T} = 1.44 min M+H⁺ = 426). ¹H NMR (DMSO-d₆, 400MHz) 8.77-8.82 (m, 2H), 8.66-8.68 (m, 1H), 8.07 (app s, 2H), 7.91-7.93 (m, 1 H), 7.73-7.78 (m, 1 H), 7.10-7.15 (m, 1 H), 4.32-4.41 (m, 1H), 3.89-4.04(m, 2 H), 3.76-3.80 (m, 1 H), 3.45-3.62 (m, 2 H), 1.80-2.02 (m, 2 H).

**EXAMPLE 104:** 2-Dimethylcarbamoyl-3-(2-fluoro-4-iodo-phenylamino)-furo[3,2-c]pyridine-7-carboxylic acid ethyl ester

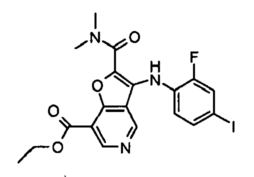

To a solution of 2-dimethylcarbamoyl-3-(2-fluoro-4-trimethylsilanyl-phenylamino)furo[3,2-c]pyridine-7-carboxylic acid ethyl ester (84 mg, 0.19 mmol) in dichloromethane (3 ml) at -10°C was added iodine monochloride (0.38 ml, 0.38 mmol, 1M solution in dichloromethane) and the solution was stirred at this temperature for 1 hour. A saturated solution of sodium thiosulfate (5 ml) was added and the mixture was poured into saturated sodium thiosulfate (15 ml). The aqueous layer was isolated then extracted with dichloromethane (2 × 25 ml), before the combined organic layers were washed with brine, dried over magnesium sulfate and concentrated *in vacuo.* Purification of the resultant residue by flash chromatography (Si-SPE, dichloromethane : t-butyl methyl ether gradient 1:0 to 1:3) afforded the title compound as a yellow waxy solid (87 mg, 92%). LCMS (method B): R_{T} = 3.97 min, M+H⁺= 498.

**EXAMPLE 105:** 3-(2-Fluoro-4-iodo-phenylamino)-7-hydroxymethyl-furo[3,2-c]pyridine-2-carboxylic acid dimethylamide

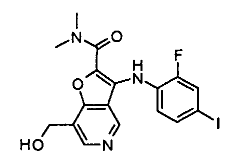

To a solution of 2-dimethylcarbamoyl-3-(2-fluoro-4-iodo-phenylamino)-furo[3,2-c]pyridine-7-carboxylic acid ethyl ester (96 mg, 0.193 mmol) in THF (4 ml) at -40°C was added dropwise lithium triethylborohydride (0.41 ml, 0.41 mmol, 1M solution in THF). The resulting mixture was stirred at -40°C for 30 minutes before quenching the reaction with the addition of saturated ammonium chloride (20 ml). The aqueous layer was isolated then extracted with dichloromethane (3 × 20 ml) before the combined organic layers were dried over magnesium sulfate and concentrated *in vacuo.* Purification of the resultant residue by flash chromatography (Si-SPE, dichloromethane : ethyl acetate gradient 1:0 to 0:1 1 then ethyl acetate : methanol 85:15) afforded crude material. The crude material was triturated in methanol to afford the title compound as a white solid (34 mg, 39%). ¹H NMR (DMSO-D₆, 400 MHz) 3.07 (6H, s, br), 4.83 (2H, d, J = 5.6 Hz), 5.47 (1H, t, J = 5.6 Hz), 6.87 (1H, t, J = 8.7 Hz), 7.43 (1H, m), 7.66 (1H, dd, J = 10.8, 2.0 Hz), 8.54 (2H, m), 8.56 (1H, s). LCMS (method A): R_{T} = 6.74 min, M+H⁺ = 456.

**EXAMPLE 106:** 3-(2-Fluoro-4-iodo-phenylamino)-7-phenoxymethyl-furor[3,2-c]pyridine-2-carboxylic acid dimethylamide

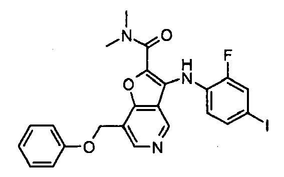

To a solution of 3-(2-fluoro-4-iodo-phenylamino)-7-hydroxymethyl-furo[3,2-c]pyridine-2-carboxylic acid dimethylamide (34 mg, 0.075 mmol) and triphenylphosphine (20 mg, 0.075 mmol) in THF (3 ml) was added phenol (7.75 mg, 0.083 mmol) and DIAD (18.5 µl, 0.094 mmol) and the mixture was stirred at room temperature for 21 hours. The reaction mixture was diluted with ethyl acetate (40 ml) washed with 1M NaOH (15 ml) and brine (15 ml). The organic layer was isolated, dried over magnesium sulfate and concentrated *in vacuo.* Purification of the resultant residue by flash chromatography (Si-SPE, dichloromethane : ethyl acetate gradient 1:0 to 0:1 1 then ethyl acetate : methanol 85:15) afforded crude material. Repurification by flash chromatography (Si-SPE, ethyl acetate : dichloromethane : cyclohexane 1:4:1) afforded the title compound as a white solid (12 mg, 30%). ¹H NMR (CDCl₃, 400 MHz) 3.26 (6H, s, br), 5.37 (2H, s), 6.94 (1H, t, J = 8.6 Hz), 7.02 (3H, m), 7.33 (2H, m), 7.41 (1H, m), 7.49 (1H, dd, J = 9.9, 1.9 Hz), 8.55 (1H, s), 8.66 (2H, apparent s). LCMS (method A): R_{T} = 12.30 min, M+H⁺= 532.

**EXAMPLE 107:** 7-chloro-3-(2-fluoro-4-iodo-phenylamino)-furo[3,2-c]pyridine-2-carboxylic acid sodium salt

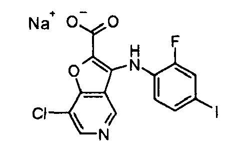

To a suspension of 7-chloro-3-(2-fluoro-4-iodo-phenylamino)-furo[3,2-c]pyridine-carboxylic acid ethyl ester (100 mg, 0.277 mmol) in methanol (7 ml) was added 1M NaOH (0.32 ml, 0.32 mmol) and the mixture was stirred at 55°C for 3 hours. The resultant suspension was cooled to room temperature and stirred for 17 hours then heated at 55°C for 2.5 hours. The reaction mixture was concentrated *in vacuo* and the residue azeotroped with toluene (2 x 20 ml). The resultant residue was then triturated in water and filtered to afford the title compound as a white solid (74 mg, 75%). ¹H NMR (CD₃OD, 400 MHz) 6.95 (1H, t, J = 8.6 Hz), 7.44 (1H, ddd, J = 8.4, 1.8, 1.2 Hz) 7.54 (1H, dd, J=10.4, 1.9 Hz), 8.48 (1H, s), 8.49 (1H, s). LCMS (method A): R_{T}= 10.57 min, M+H⁺= 433.

**EXAMPLE 108:** 7-Chloro-3-(2-fluoro-4-iodo-phenylamino)-furo[3,2-c]pyridine-2-carboxylic acid amide

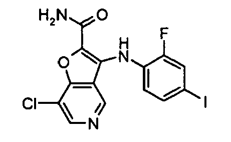

To a solution of 7-chloro-3-(2-fluoro-4-iodo-phenylamino)-furo[3,2-c]pyridine-2-carboxylic acid sodium salt (60 mg, 0.110 mmol) and ammonium chloride (17.5 mg, 0.33 mmol) in DMF (1.5 ml) was added HATU (84 mg, 0.22 mmol) and diisopropylethylamine (75 µl, 0.44 mmol) and the resulting solution was stirred at room temperature for 3 hours. The reaction mixture was diluted with ethyl acetate and washed with water then saturated sodium bicarbonate and then brine. The organic layer was dried over magnesium sulfate, filtered and concentrated *in vacuo.* The resultant residue was triturated in acetonitrile to afford the title compound as a yellow solid (14 mg, 30%). ¹H NMR (CDCl₃, 400 MHz) 5.69 (1H, s, br), 6.30 (1H, s, br), 6.98 (1H, t, J = 8.4 Hz), 7.46 (1H, m), 7.53 (1H, dd, J = 9.7, 1.9 Hz), 8.03 (1H, s), 8.46 (1H, s), 8.56 (1H, s). LCMS (method A): R_{T}=10.46 min, M+H⁺ = 432.

**EXAMPLE 109:** 3-(2-Fluoro-4-iodo-phenylamino)-furo[3,2-cl]pyridine-2-carboxylic acid (2-methanesulfonylamino-ethoxyl-amide 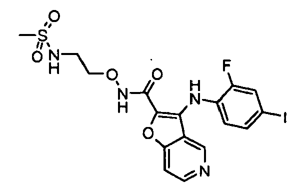 N-(2-Aminooxy-ethyl)-methanesulfonamide (116 mg, 0.75 mmol), 3-(2-fluoro-4-iodo-phenylamino)-furo[3,2-c]pyridine-2-carboxylic acid (300 mg, 0.75 mmol), EDC (159 mg, 0.83 mmol), HOBT (112 mg, 0.83 mmol) and DIPEA (0.13 ml, 0.75 mmol) were suspended in THF-(5 ml) before DMF (5 drops) was added. The reaction was stirred at room temperature for 16 hours. The reaction was concentrated *in vacuo* and the residue dissolved in ethyl acetate (20 ml) and washed with aqueous saturated sodium bicarbonate solution (20 ml). The aqueous layer was washed with ethyl acetate (2×10 ml) and the combined organic extracts were washed with brine and dried over magnesium sulfate and concentrated *in vacuo.* The resultant residue was subjected to flash chromatography (SiO₂, gradient 0-10% methanol in dichloromethane) to yield the title compound as a light yellow solid (120 mg, 49%). ¹H NMR (CDCl₃, 400 MHz) 8.95 (1H, s), 8.61-8.59 (2H, m), 7.91 (1H, s), 7.54 (1H, dd, J = 9.6, 1.9 Hz), 7.48 (1H, dt, J = 8.4, 1.3 Hz), 7.37 (1H, dd, J = 5.9, 0.8 Hz), 7.03 (1H, t, J = 8.4 Hz), 6.09-6.06 (1H, m), 4.17-4.15 (2H, m), 3.47-3.43 (2H, m), 3.04 (3H, s). LCMS (method A): R_{T} = 7.34 min, M+H⁺ = 535.

**EXAMPLE 138:** 7-Chloro-3-(2-fluoro-4-iodo-phenylamino)-furo[3,2-c]pyridine-2- carboxylic acid ((R)-2,3-dihydroxy-propoxy)-amide

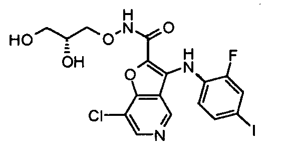

Step 1: 7-Chloro-3-(2-fluoro-4-iodo-phenylamino)-furo[3,2-c]pyridine-2-carboxylic acid 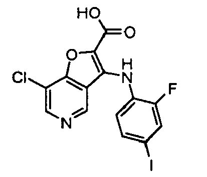 A suspension of 7-chloro-3-(2-fluoro-4-iodo-phenylamino)-furo[3,2-c]pyridine-2-carboxylic acid ethyl ester (150 mg, 0.33 mmol) in IMS (10 ml) was treated with was treated with sodium hydroxide (1M aqueous solution, 0.832 ml) and the reaction mixture heated at 60°C for 3 hours. The resultant mixture was allowed to cool then concentrated *in vacuo,* the crude residue treated with water and the mixture adjusted to pH 5 with acetic acid. The resultant suspension was filtered, the residue collected and dried *in vacuo* to give the title compound as a yellow solid (105 mg, 74%). 1H NMR (DMSO-d₆ 400 MHz) 8.63 (1 H, s), 8.45 (1 H, s), 7.69 (1 H, d, J = 10.28 Hz), 7.49 (1 H, d, J = 8.22 Hz), 7.03 (1 H, s).

Step 2: 7-Chloro-3-(2-fluoro-4-iodo-phenylamino)-furo[3,2-c]pyridine-2-carboxylic acid ((R)-2,2-dimethyl-[1,3]dioxolan-4-ylmethoxy)-amide

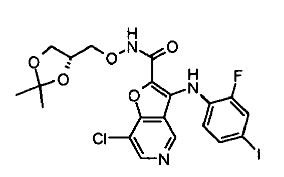

A suspension of 7-chloro-3-(2-fluoro-4-iodo-phenylamino)-furo[3,2-c]pyridine-2-carboxylic acid (177 mg, 0.41 mmol) in dry dichloromethane (6 ml) under an atmosphere of nitrogen was cooled to 0°C and treated with DMF (1 drop) and oxalyl chloride (0.102 ml, 1.16 mmol). The reaction mixture was stirred for 1 hour then the solvent removed *in vacuo.* The resultant residue was re-suspended in dichloromethane and treated dropwise with a solution of O-((R)-2,2-dimethyl-[1,3]dioxolan-4-ylmethyl)-hydroxylamine (101 mg, 0.69 mmol) and DIPEA (0.172 ml, 1.21 mmol) in dichloromethane (4 ml) before being stirred for 3 hours. The reaction mixture was washed (water, brine), dried (Na₂SO₄), filtered and concentrated *in vacuo* to give the title compound as a yellow foam (207 mg, 90%). The foam was used in the subsequent step without further analysis or purification.

Step 3: 7-Chloro-3-(2-fluoro-4-iodo-phenylamino)-furor[3,2-c]pyridine-2-carboxylic acid ((R)-2,3-dihydroxy-propoxy)-amide

A solution of 7-chloro-3-(2-fluoro-4-iodo-phenylamino)-furo[3,2-c]pyridine-2-carboxylic acid ((R)-2,2-dimethyl-[1,3]dioxolan-4-ylmethoxy)-amide (280 mg, 0.49 mmol) in methanol/ c.HCl (0.14 ml c.HCl_{(aq)} in 25 ml methanol) was stirred and room temperature until TLC showed no starting material remaining. The reaction mixture was concentrated *in vacuo* and the residue treated with dichloromethane (11 ml) and triethylamine (0.210 ml), stirring for 20 minutes before re-concentrating *in vacuo.* The resultant solid residue was subjected to reverse phase HPLC (Phenomenex Luna 5 C18, 0.1% HCO₂H in water on a gradient of acetonitrile) to give the title compound as a pale yellow solid (168 mg, 66%). LCMS (method A): R_{T} = 8.87 min, M+H⁺= 522. (CD₃OD 400 MHz) 3.61 (1H, dd, J = 11.4, 5.3Hz), 3.65 (1H, dd, J = 11.4, 5.1Hz), 3.94 (1H, m), 3.99 (1H, dd, J = 10.0, 6.8Hz), 4.11 (1H, dd, J = 10.0, 3.5Hz), 7.07 (1H, t, J = 8.6Hz), 7.52 (1H, m), 7.61 (1H, dd, J = 10.2, 1.9Hz), 8.38 (1H, s), 8.56 (1H, s).

**EXAMPLE 139:** 7-Fluoro-3-(2-fluoro-4-iodophenylamino)-furo[3,2-c]pyridine-2-carboxylic acid (2-hydroxyethoxy)amide

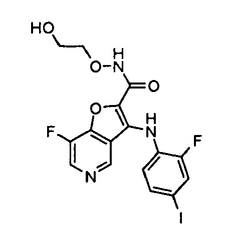

Step 1: 7-Fluoro-3-(2-fluoro-4-iodophenylamino)-furo[3,2-c]pyridine-2-carboxylic acid

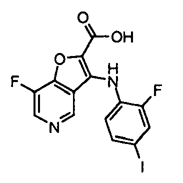

7-Fluoro-3-(2-fluoro-4-iodophenylamino)-furo[3,2-c]pyridine-2 carboxylic acid ethyl ester (4.00 g, 9.0 mmol) was stirred as a suspension in ethanol (150 ml) at room temperature before being treated with 1M NaOH and heated at 60°C for 2 hours. The solvent was removed *in vacuo* and the residue diluted with water (50 ml) and acidified to pH 4 with glacial acetic acid. The resulting solid precipitate was collected by filtration, washed with water and dried at 40 °C under vacuum (P₂O₅ to give the title compound (3.75 g quant.). LCMS (method B) R_{T} 3.51 min M+H⁺ 417.

Step 2: 7-Fluoro-3-(2-fluoro-4-iodophenylamino)-furo[3,2-c]pyridine-2-carboxylic acid (2-vinyloxyethoxy)amide

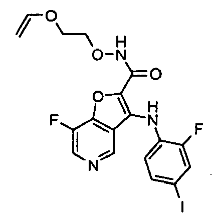

7-Fluoro-3-(2-fluoro-4-iodophenylamino)-furo[3,2-c]pyridine-2 carboxylic acid (3.75 g, 9.0 mmol) was stirred as a suspension in dry DCM (100 ml) under argon at 0 °C, and treated dropwise with oxalyl chloride (2.24 ml, 25.7 mmol), maintaining the temperature below 5°C. The resulting mixture was stirred for a further 1 hour before concentration *in vacuo.* The residue was re-suspended in dry DCM (100 ml) under argon at 0 °C and treated dropwise with a solution O-(2-vinyloxyethyl)hydroxylamine (1.40 g, 14.3 - -mmol) and diisopropylethylamine (4.70 ml, 3.48g, 27 mmol) in DCM (20 ml). The resulting solution was stirred and allowed to warm to room temperature over 3 hours before being washed with water then saturated saline, dried (MgSO₄), filtered and evaporated *in vacuo* to give a residue which was subjected to flash chromatography (SiO₂, gradient 0-10% ethyl acetate in dichloromethane) to give the title compound as a yellow solid (2.41 g 53%). LCMS (Method B) R_{T} 3.82 min M+H⁺ 502.

Step 3: 7-Fluoro-3-(2-fluoro-4-iodophenylamino)-furo[3,2-c]pyridine-2-carboxylic acid (2-hydroxyethoxy)amide

7-Fluoro-3-(2-fluoro-4-iodophenylamino)-furo[3,2-c]pyridine-2 carboxylic acid (2-vinyloxyethoxy)amide (2.41 g, 4.80 mmol) was suspended in ethanol (100 ml) and concentrated hydrochloric acid (2.0 ml) added. The mixture was stirred at room temperature for 1 hour before neutralizing by the addition saturated aqueous sodium hydrogencarbonate solution. The solvent was then removed *in vacuo,* and the resultant residue was dissolved in DCM, washed water, dried (MgSO₄) filtered and evaporated *in vacuo* to give a crude residue which was subjected to flash chromatography (SiO₂, gradient 0 to 2% methanol in dichloromethane) followed by recrystallisation (aqueous methanol) to give the title compound as yellow needles (0.837 g, 36%). LCMS (method A): R_{T} 9.15 min, M+H⁺ 476; 1H NMR (DMSO-d₆, 400 MHz) 3.60 (2 H, m), 3.88-3.93 (2H, m), 4.70 (1 H, br s), 7.02 (1H, t, J = 8.68 Hz), 7.46-7.49 (1H, m), 7.68 (1H, dd, J = 10.55, 1.92 Hz), 8.36 (1H, s), 8.40 (1H, s), 8.64 (1H, d, J = 2.58 Hz), 11.95 (1 H, s).

**EXAMPLES 20-96 and 111-159**

Compounds in Tables 2, 3, and 4 were prepared by general methods outlined below:

Amides and hydroxamates were prepared from the appropriate acid by using the coupling general method described below. In some cases the intermediate acid was not isolated, the coupling reaction performed on the crude carboxylate salt produced by following the saponification general method.

Saponification general method

A mixture of carboxylic acid ester, 1N aqueous NaOH (1-2 eq.) and EtOH was heated at 70°C for 1 hour. The reaction mixture was concentrated *in vacuo* and azeotroped with toluene to give the crude carboxylate salt.

Coupling general method

The appropriate carboxylic acid or carboxylate salt was suspended in anhydrous THF before the appropriate hydroxylamine or amine (1-4eq.), EDCI (1-2eq.) or HATU (1-2 eq.), HOBt (1-2eq.) and DIPEA (2-4eq.) were added. In some cases DMF was added as a co-solvent to improve solubility. After stirring at ambient temperature until the reaction was complete (LCMS/TLC), the reaction mixture was concentrated *in vacuo.* The resultant residue was dissolved in ethyl acetate and washed with water before the organic layer was isolated, dried over sodium sulfate, then concentrated *in vacuo* and purified by one of the general purification methods described below. If necessary, any protecting groups were then removed using one of the deprotection conditions described below.

Deprotection general methods

Method A: Aqueous HCl (1N or 2N) was added to a mixture of the protected substrate in an appropriate solvent at ambient temperature. The mixture was stirred until analysis (TLC/LCMS) showed complete consumption of starting material. The reaction mixture was neutralized, concentrated *in vacuo* and subjected to purification.

Method B: A solution of the substrate in methanol was loaded onto an Isolute® SCX-2 cartridge. The cartridge was then washed with methanol before the desired product was eluted using 2M ammonia in MeOH and the eluent collected then concentrated to give a residue. The residue was subjected to purification.

Method C: TBAF in THF was added to a solution of the silyl ether, the mixture stirred at ambient temperature until analysis (TLC/LCMS) showed complete consumption of starting material. The reaction mixture was concentrated *in vacuo* and subjected to purification.

Method D: TFA was added to the substrate either neat or as a solution in DCM. The reaction mixture was stirred at ambient temperature until analysis (TLC/LCMS) showed complete consumption of starting material. The reaction mixture was concentrated *in vacuo,* and subjected to purification.

Method E: A 20% solution of piperidine in DME was added to the substrate. The reaction mixture was stirred at ambient temperature until analysis (TLC/LCMS) showed complete consumption of starting material. The reaction mixture was then concentrated.

Method F: A 4N HCl solution in dioxane was added to the substrate. The reaction mixture was stirred at ambient temperature until analysis (TLC/LCMS) showed complete consumption of starting material. The reaction mixture was then concentrated.

Method G: An aliquot (3 mol equivalents) of freshly prepared HCl in methanol solution [concentrated HCl (0.14 ml) in methanol (25 ml)] was added to the coupled substrate at ambient temperature. The mixture was stirred until analysis (TLC/LCMS) showed complete consumption of starting material. The contents were evaporated to dryness and the residue was dissolved in dichloromethane and treated with triethylamine (3 mol equivalents) at room temperature for 10 min. The mixture was then concentrated *in vacuo* and the residue subjected to purification.

Purification general methods

Method A: Si-SPE or Si-ISCO, ethyl acetate/cyclohexane gradient

Method B: Si-SPE or Si-ISCO, ethyl acetate/DCM gradient

Method C: Si-SPE or Si-ISCO, methanol/DCM gradient

Method D: Si-SPE or Si-ISCO, methanol/ethyl acetate gradient

Method E: reversed phase HPLC Phenomenex Luna 5 phenyl/hexyl, 0.1% TFA in water on a gradient of methanol

Method F: reversed phase HPLC Phenomenex Luna 5 phenyl/hexyl, 0.1% TFA in water on a gradient of acetonitrile

Method G: reversed phase HPLC Phenomenex Luna 5 phenyl/hexyl, 0.1% HCO2H in water on a gradient of methanol

Method H: reversed phase HPLC Phenomenex Luna 5 phenyl/hexyl, 0.1% HCO2H in water on a gradient of acetonitrile

Method I: A solution of the substrate in methanol was loaded onto an Isolute® SCX-2 cartridge. The cartridge was then washed with methanol before the desired product was eluted using 2M ammonia in MeOH.

Method J: Si-SPE or Si-ISCO, ethyl acetate/hexane gradient.

Method K: reversed phase HPLC Sunfire C18, 0.05% TFA in water on a gradient of acetonitrile.

Method L: Si-SPE or Si-ISCO, ethanol/ethyl acetate gradient.

Method M: Si-SPE, ether/pentane gradient then methanol/ether gradient

Deviations from general methods:
¹ Triturated in hot methanol; ² recrystallised from ethyl acetate; ³ triturated in diethyl ether; ⁴ recrystallised from diethyl ether; ⁵ recrystallised from 5% MeOH in CHCl₃; ⁶Si-SPE ether/pentane then methanol in ether eluent; ⁷ triturated in ethyl acetate, ⁸ ester saponification with lithium hydroxide, ⁹ A C 18 column used; ¹⁰ reaction carried out in DMF; ¹¹ chloroform/methanol recrystallisation; ¹² trituration in acetonitrile; ¹³ DMF used as reaction co-solvent; ¹⁴ recrystallisation in methanol; ¹⁵ final elution with 10% methanol in ethyl acetate; ¹⁶ Reaction mixture heated at 55°C; ¹⁷ triturated in diethyl ether/DCM.

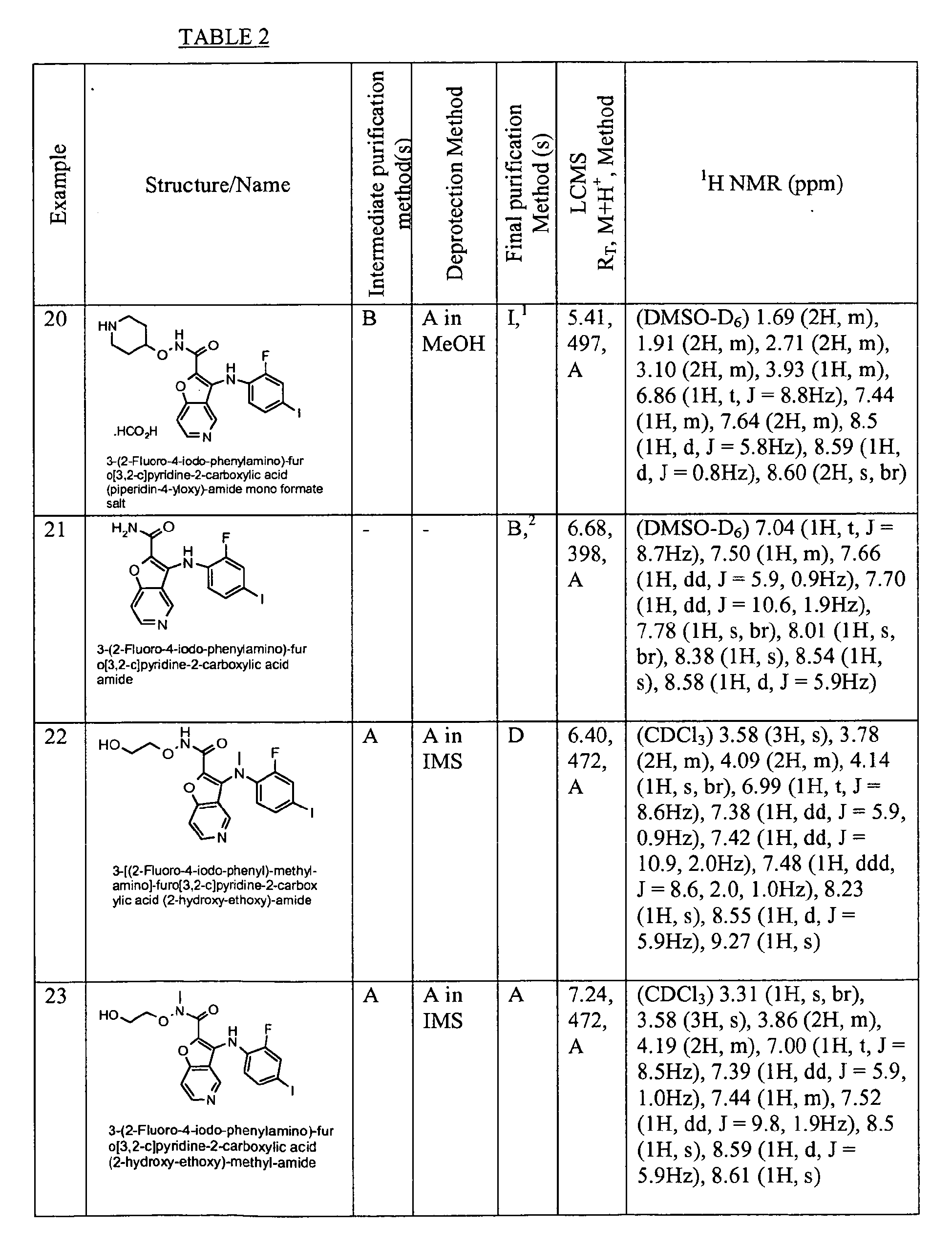

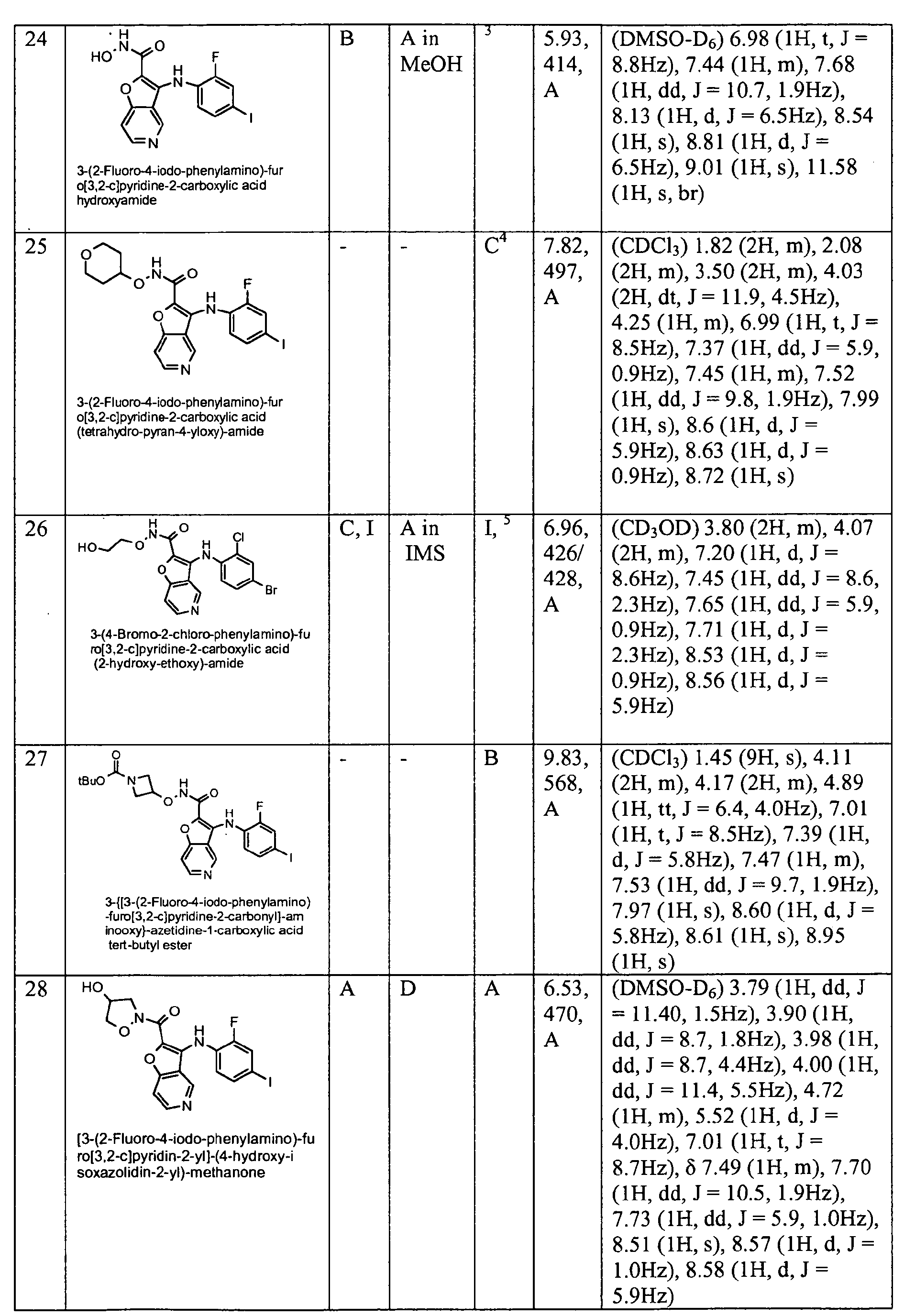

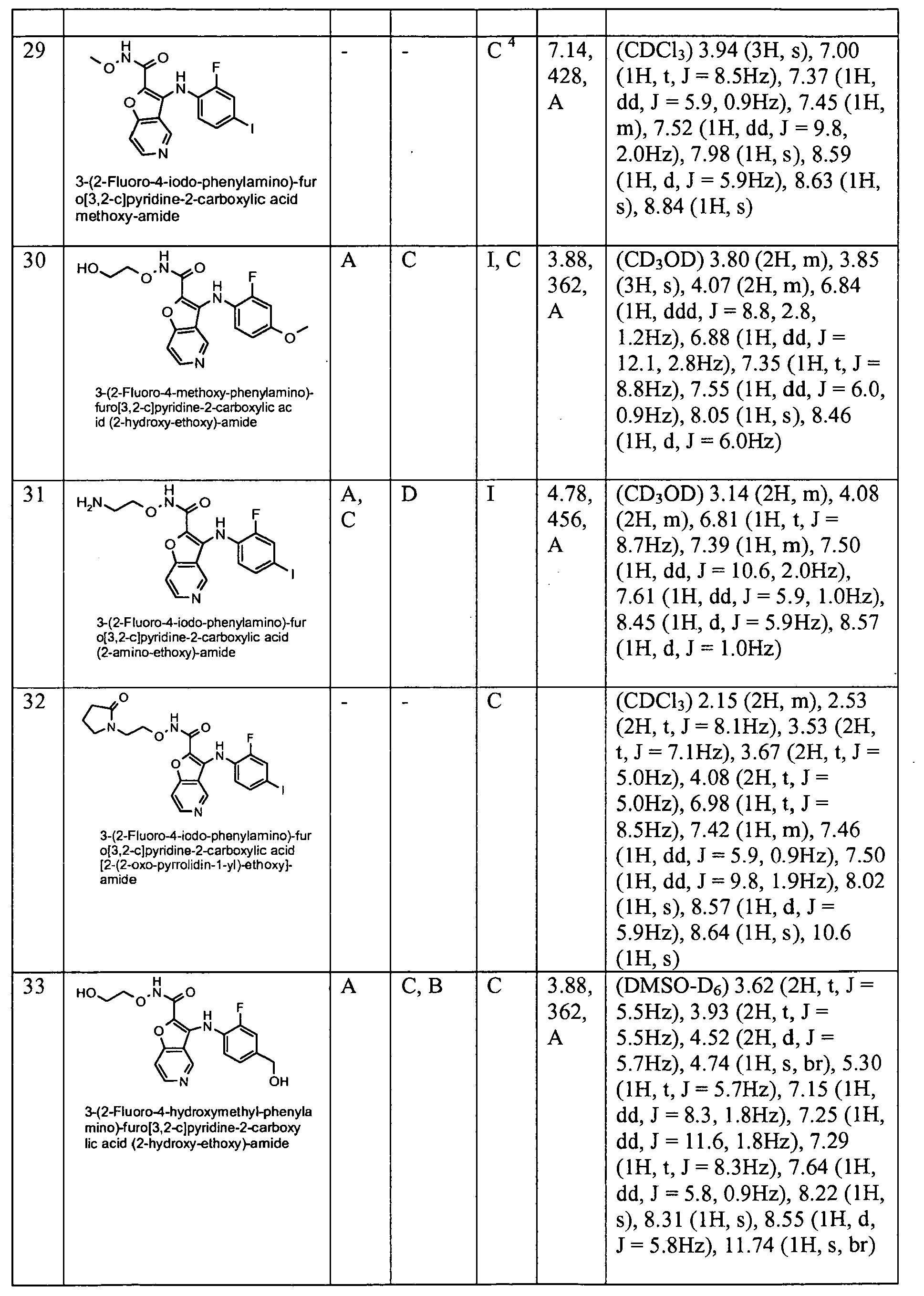

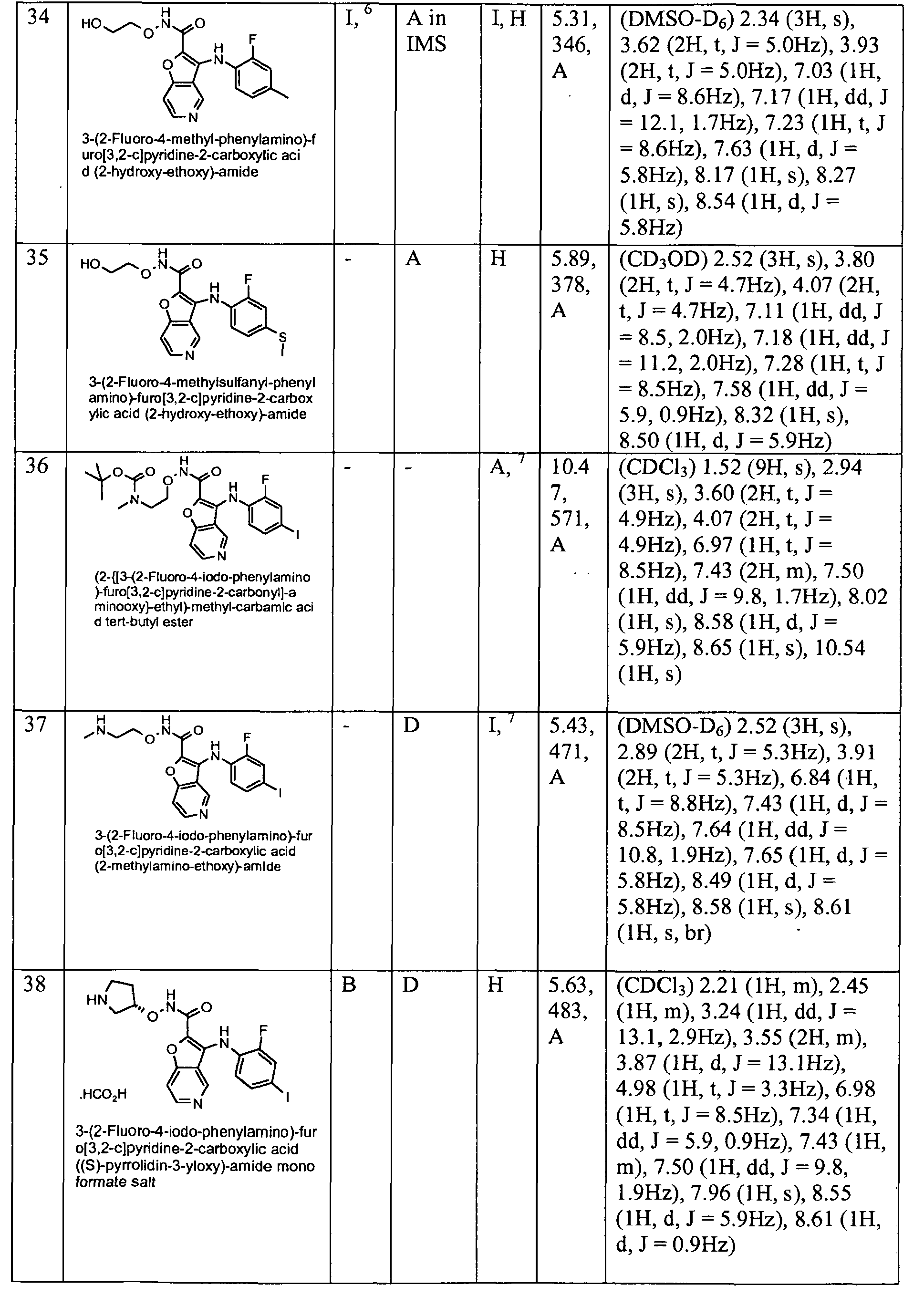

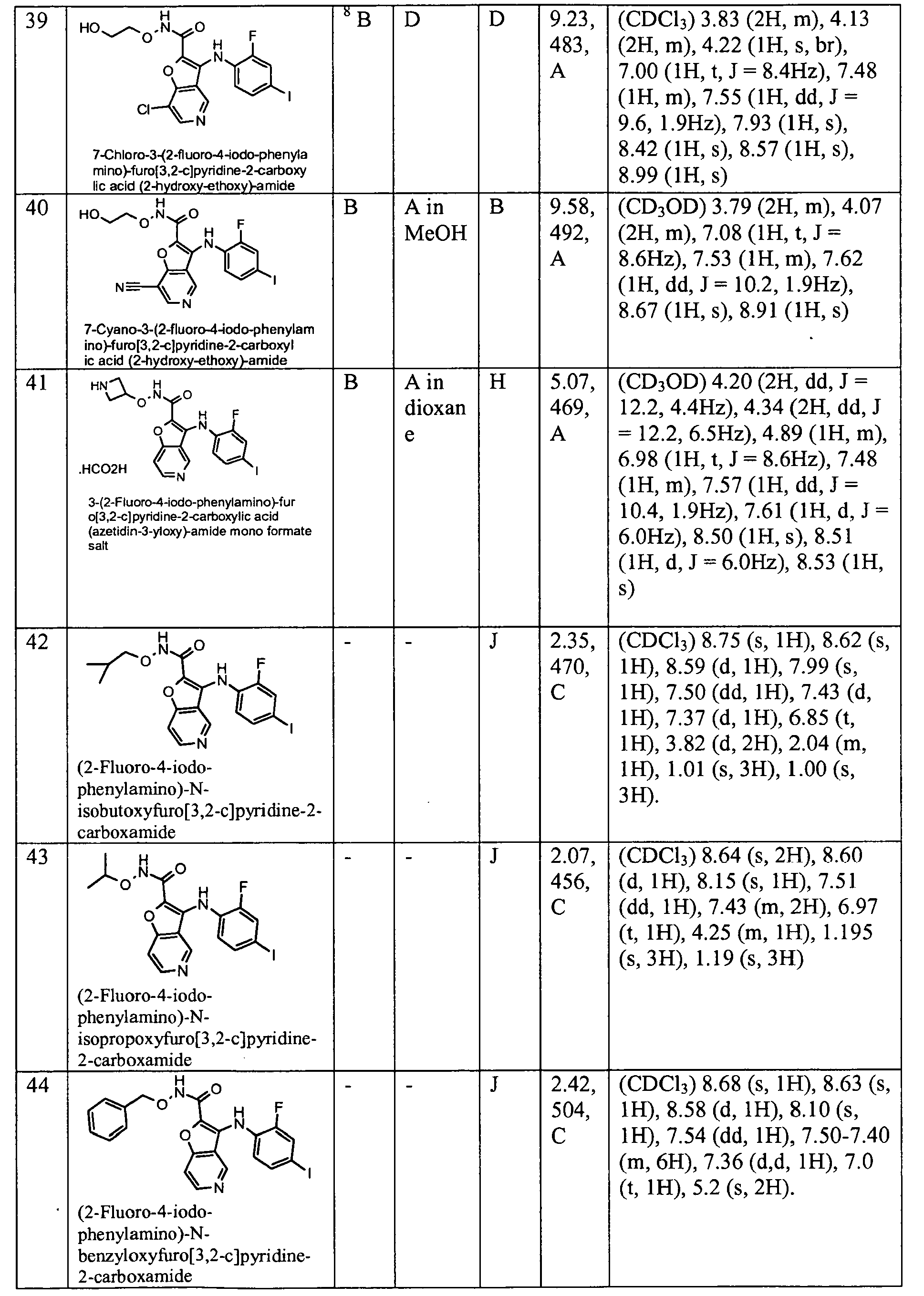

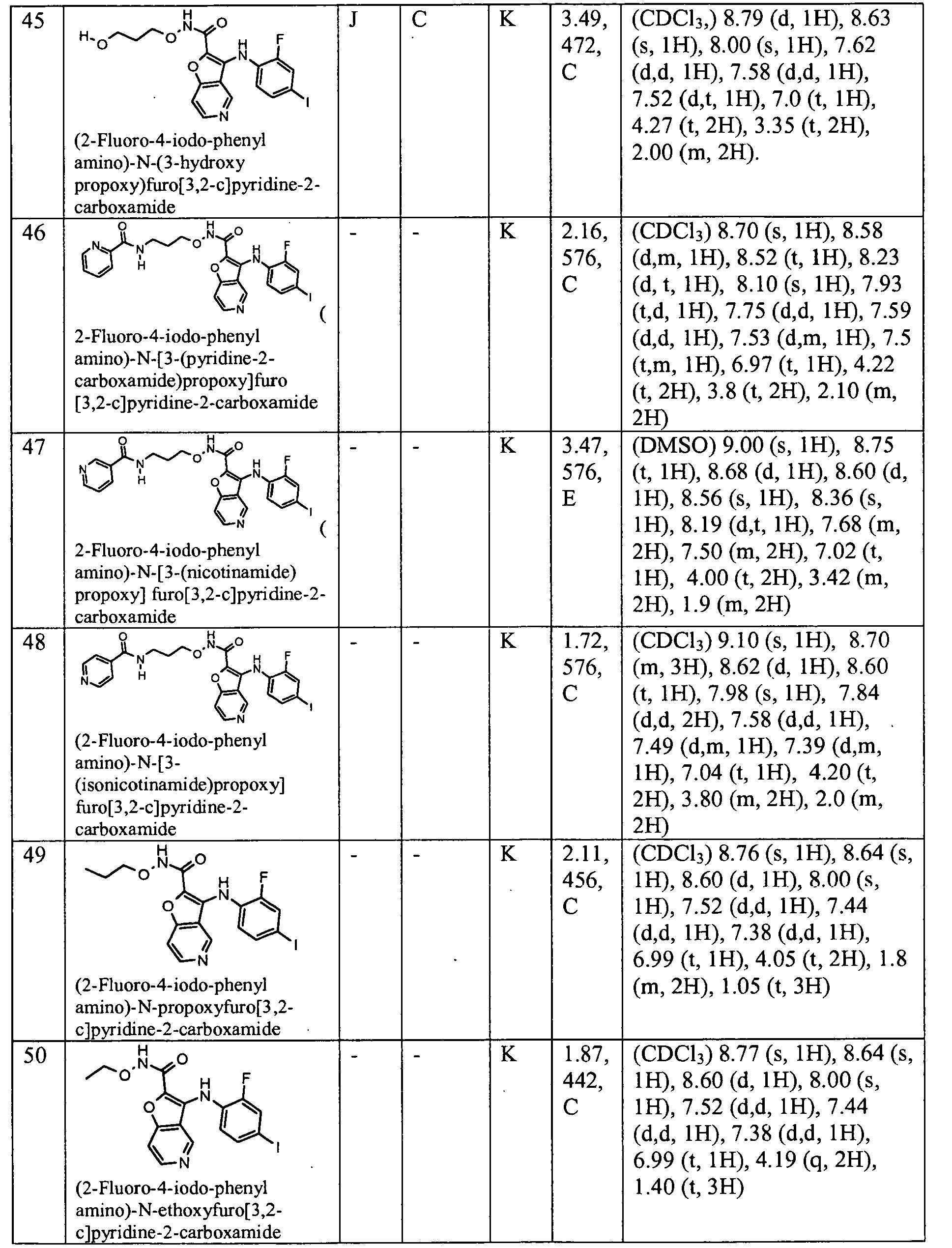

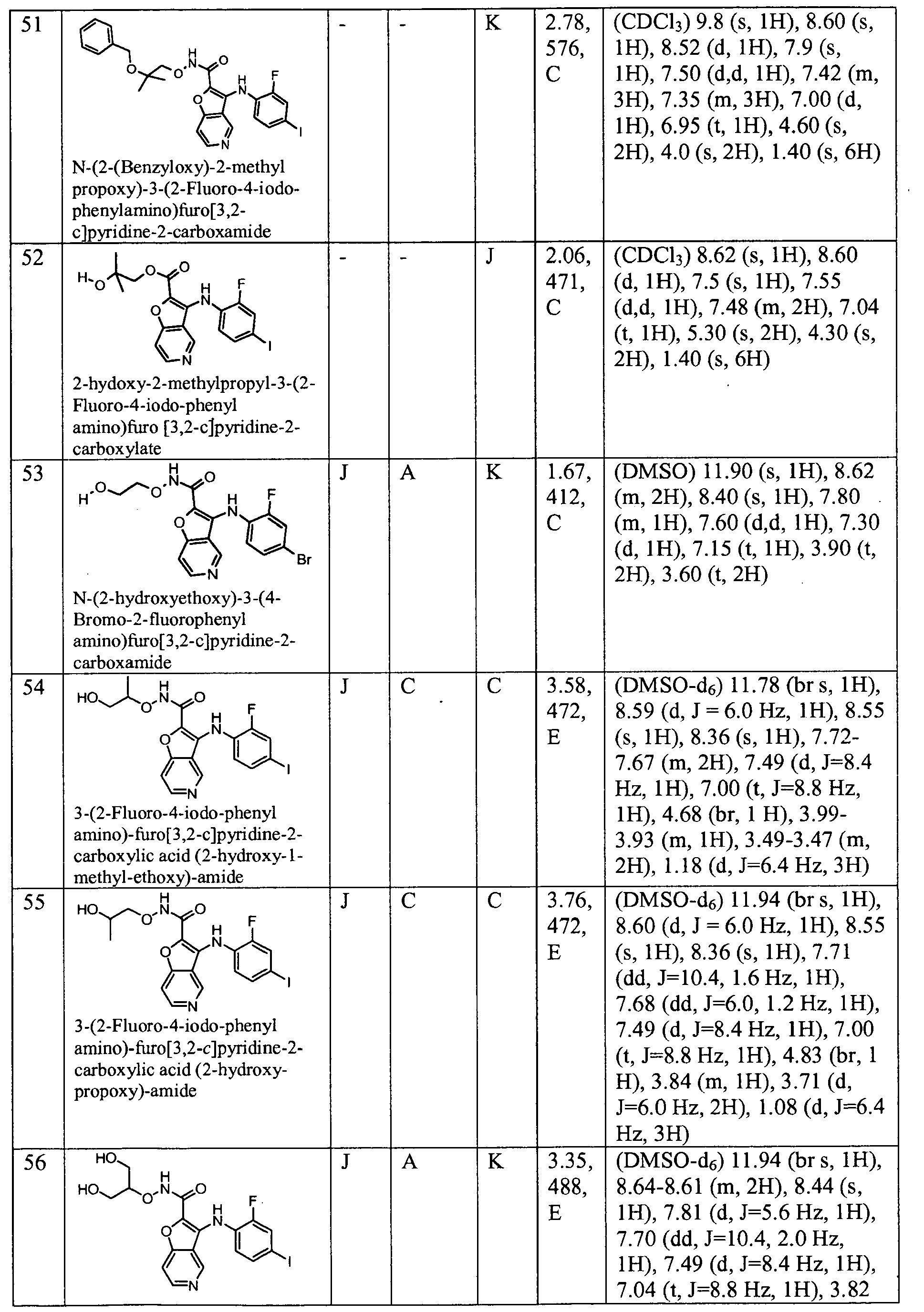

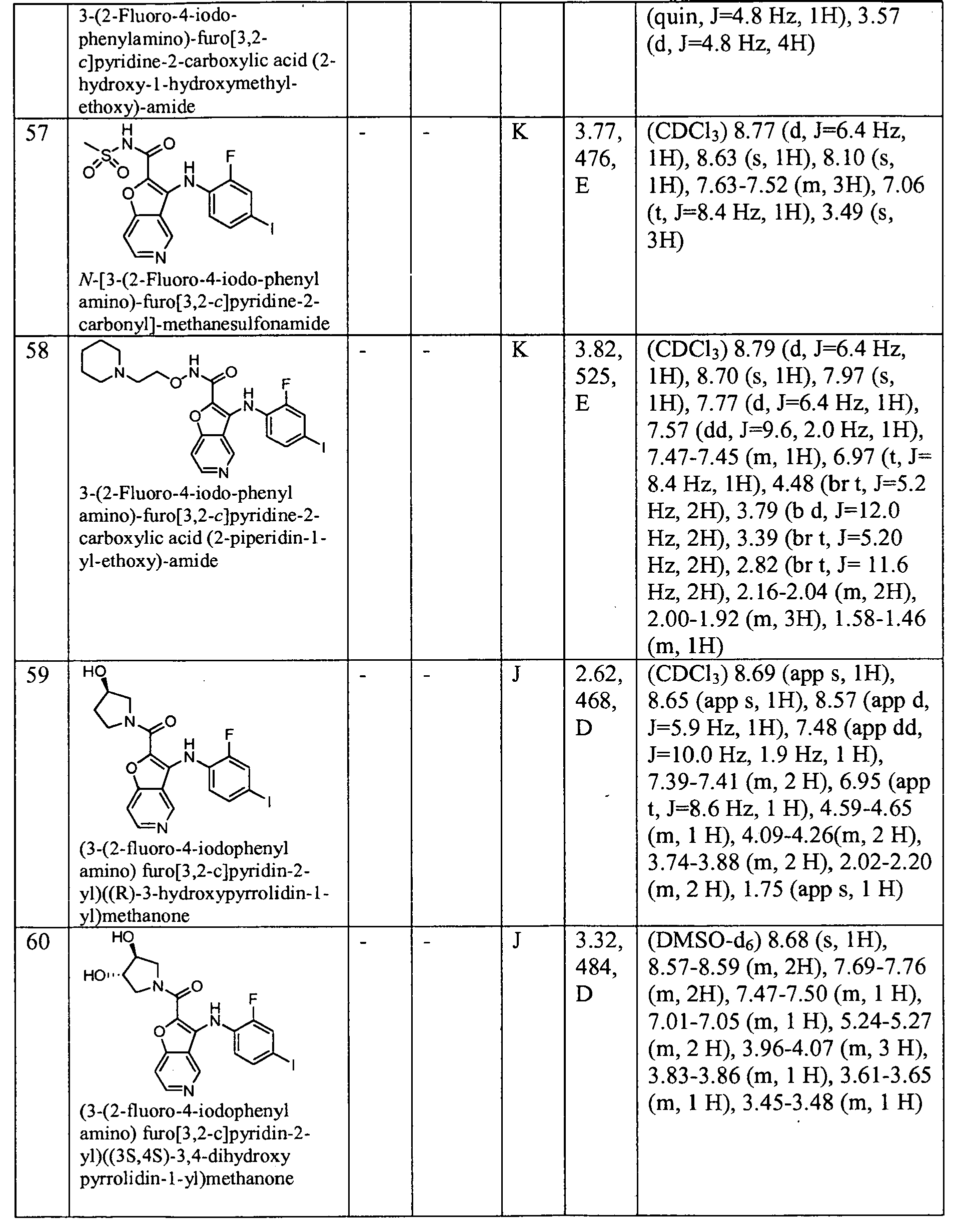

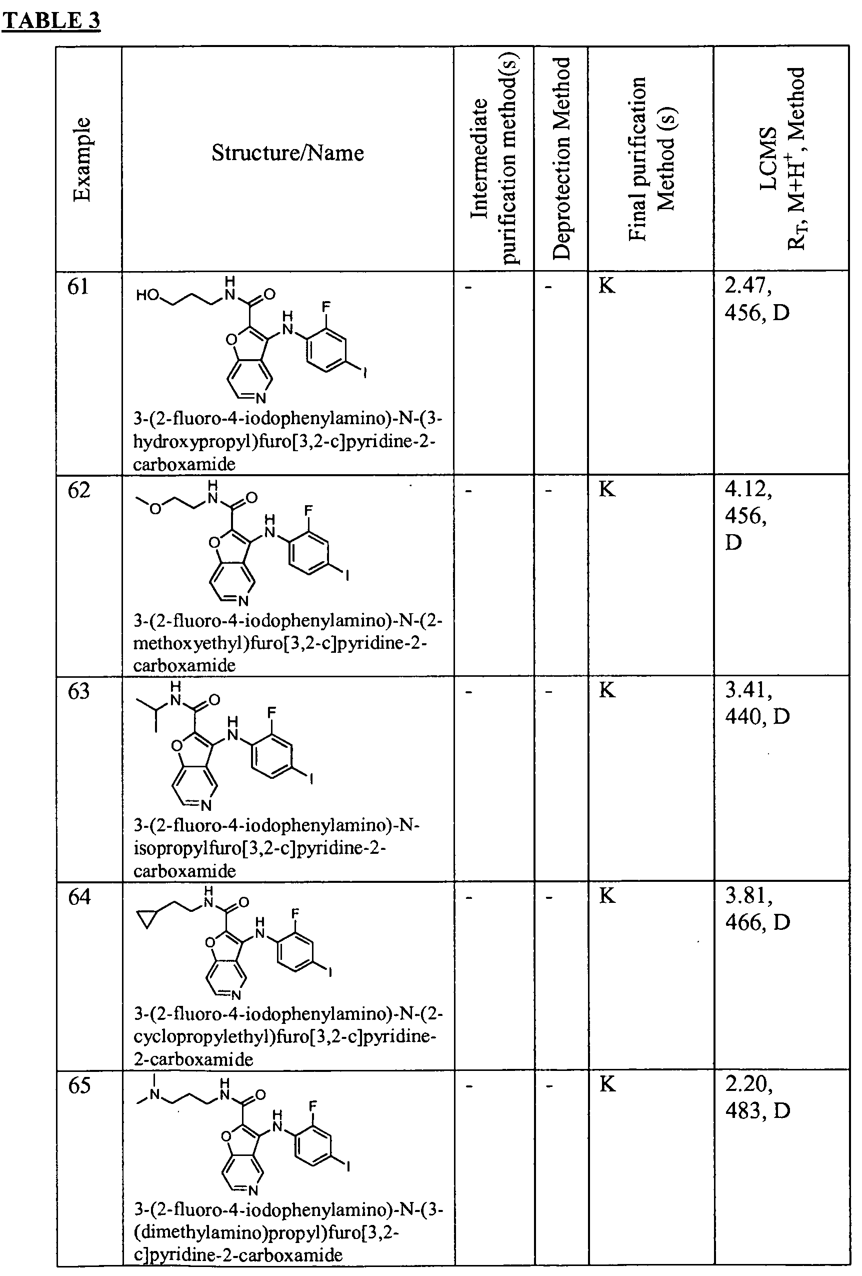

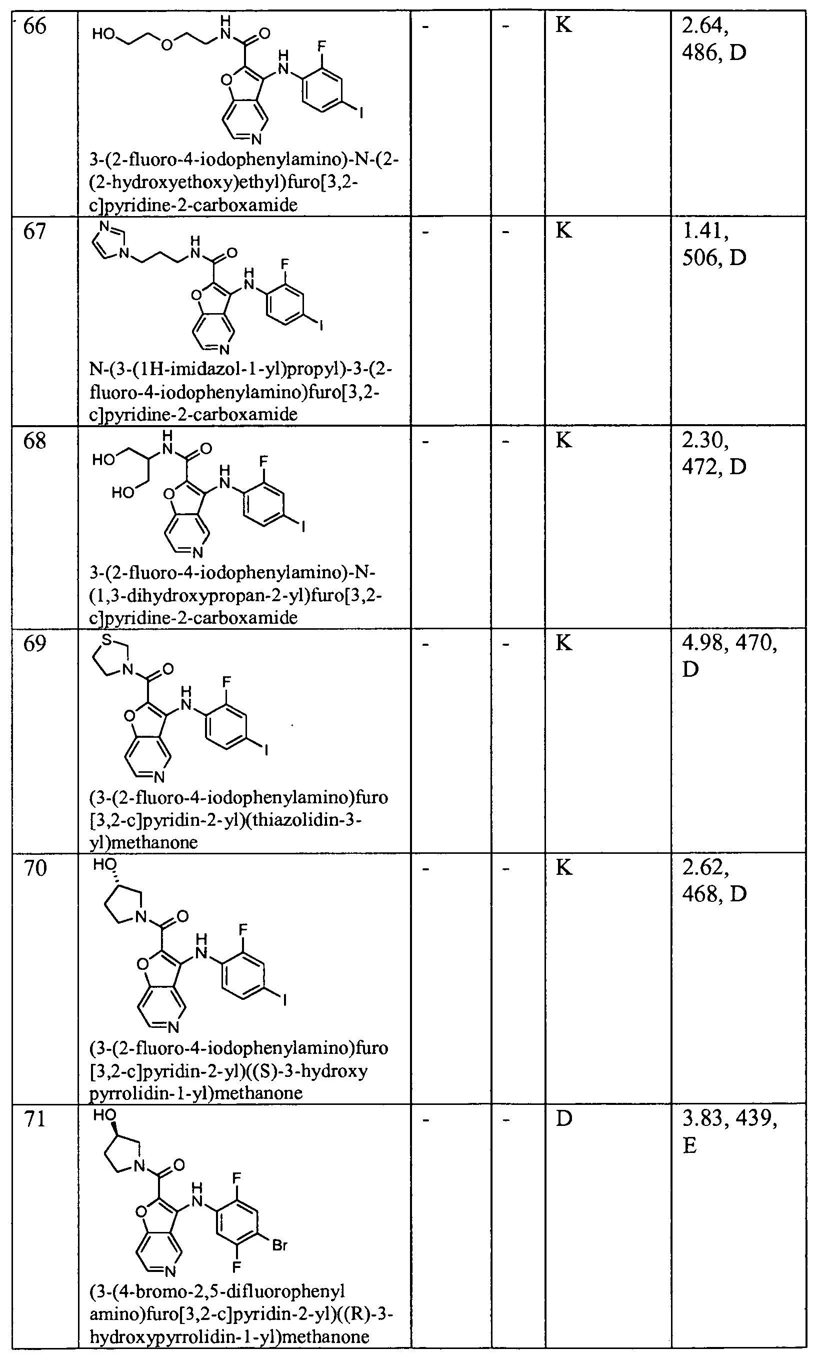

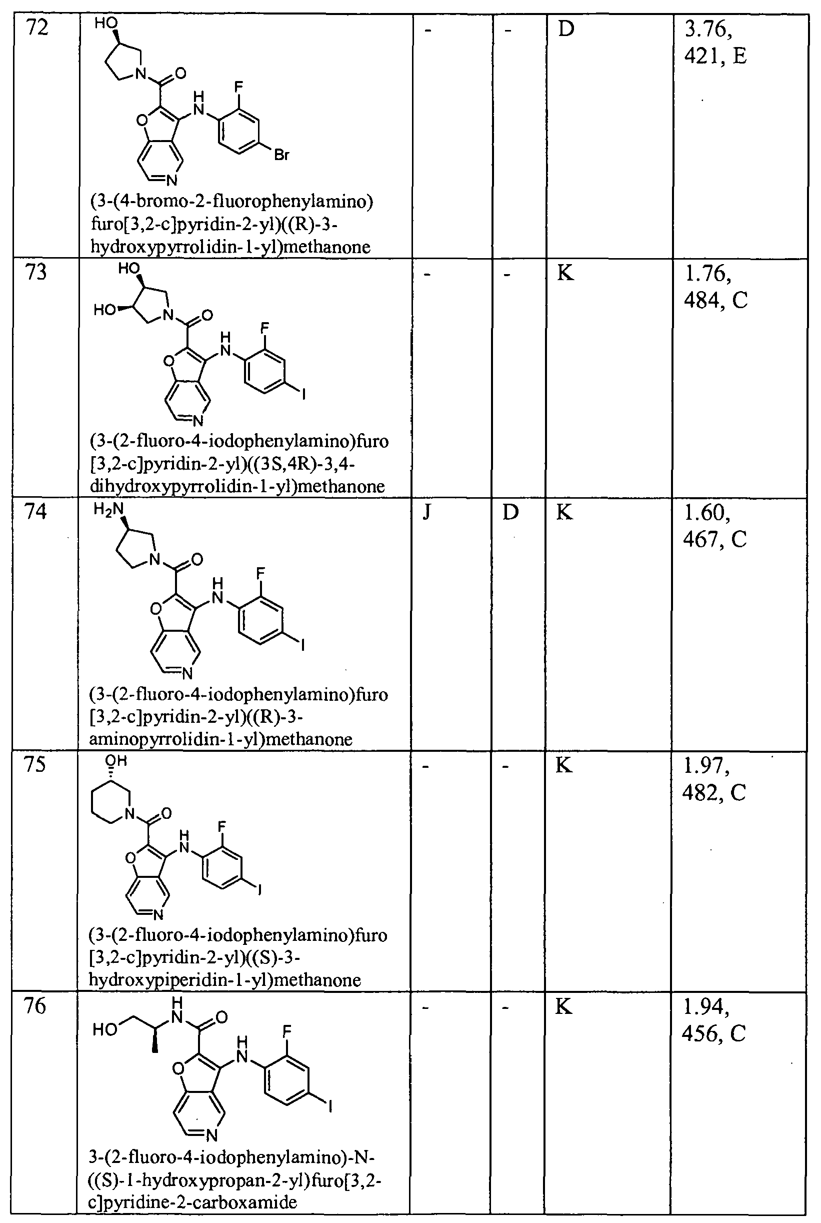

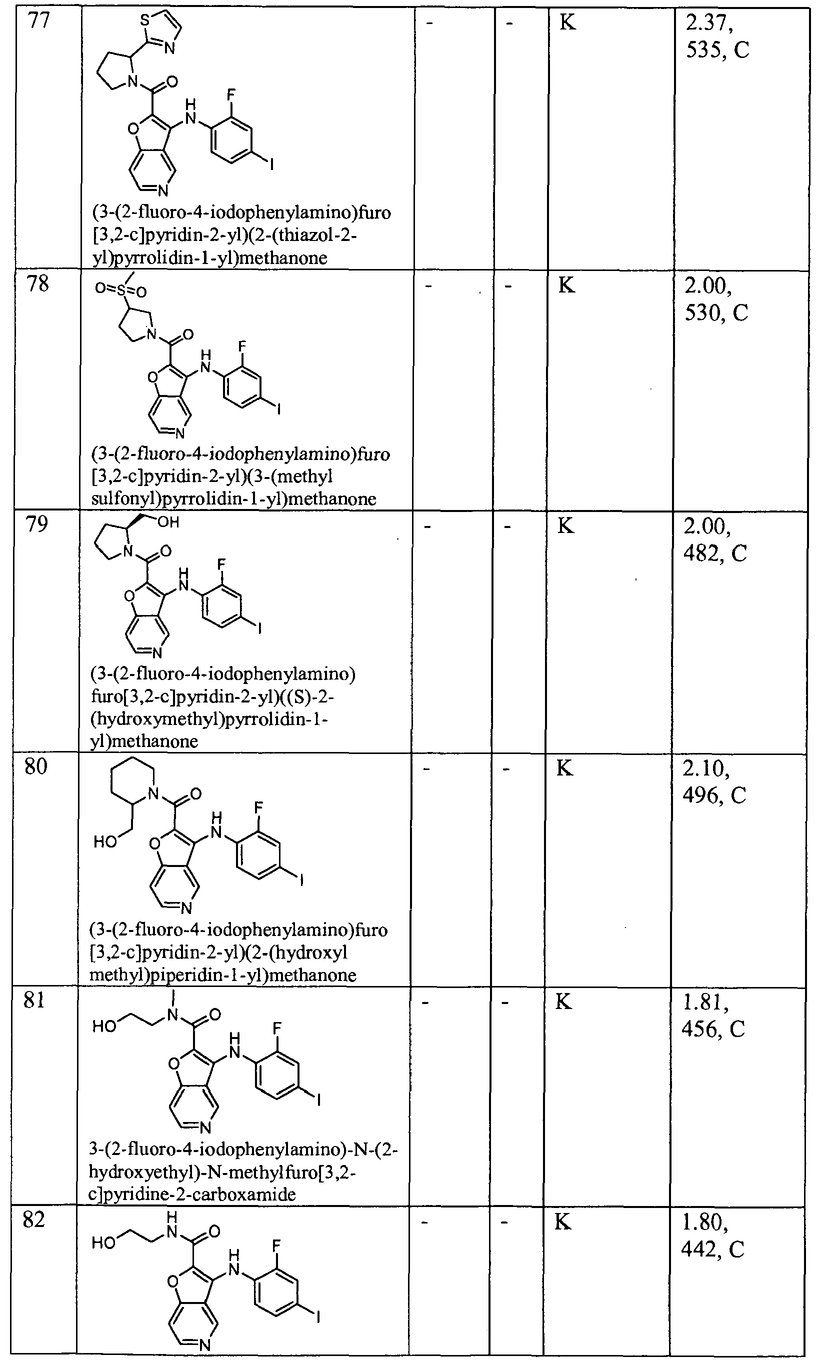

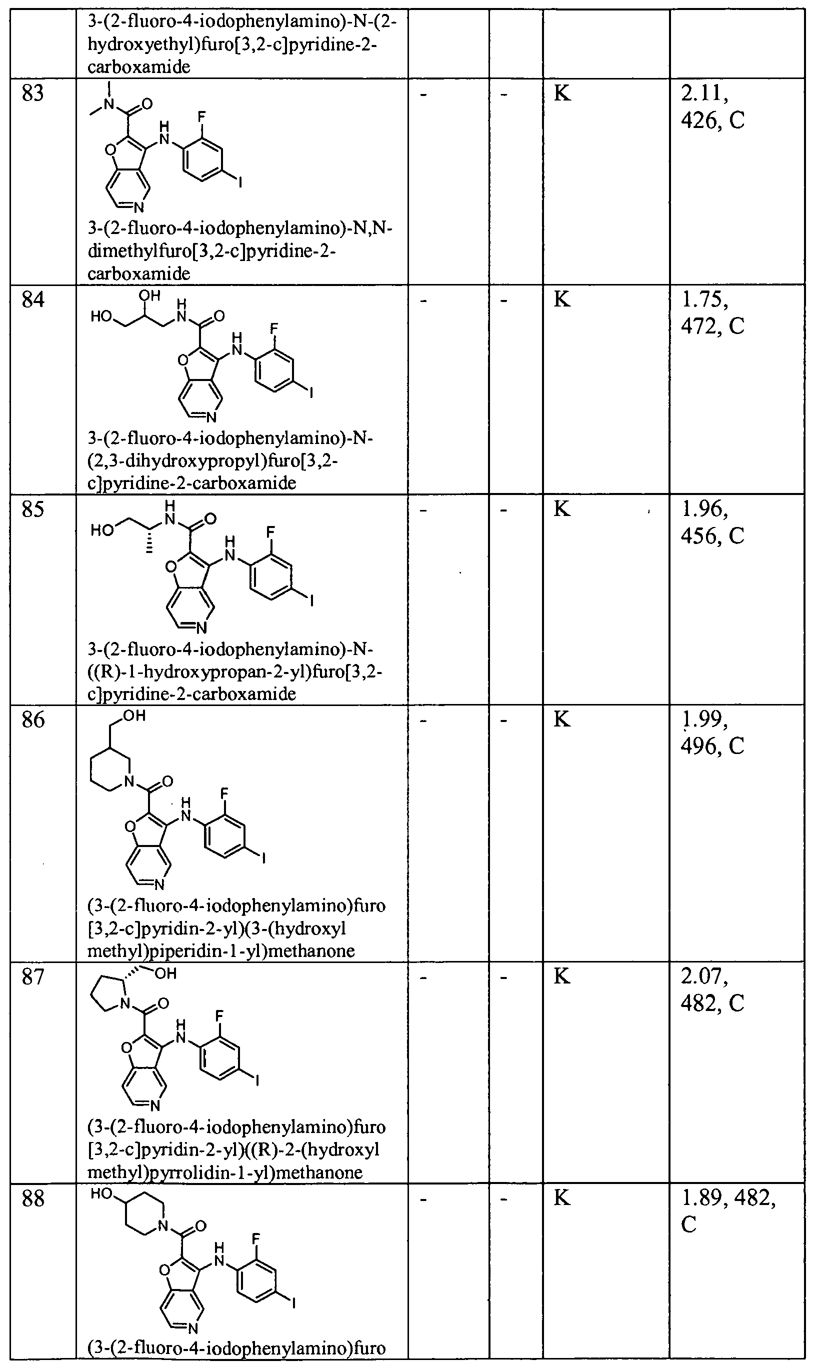

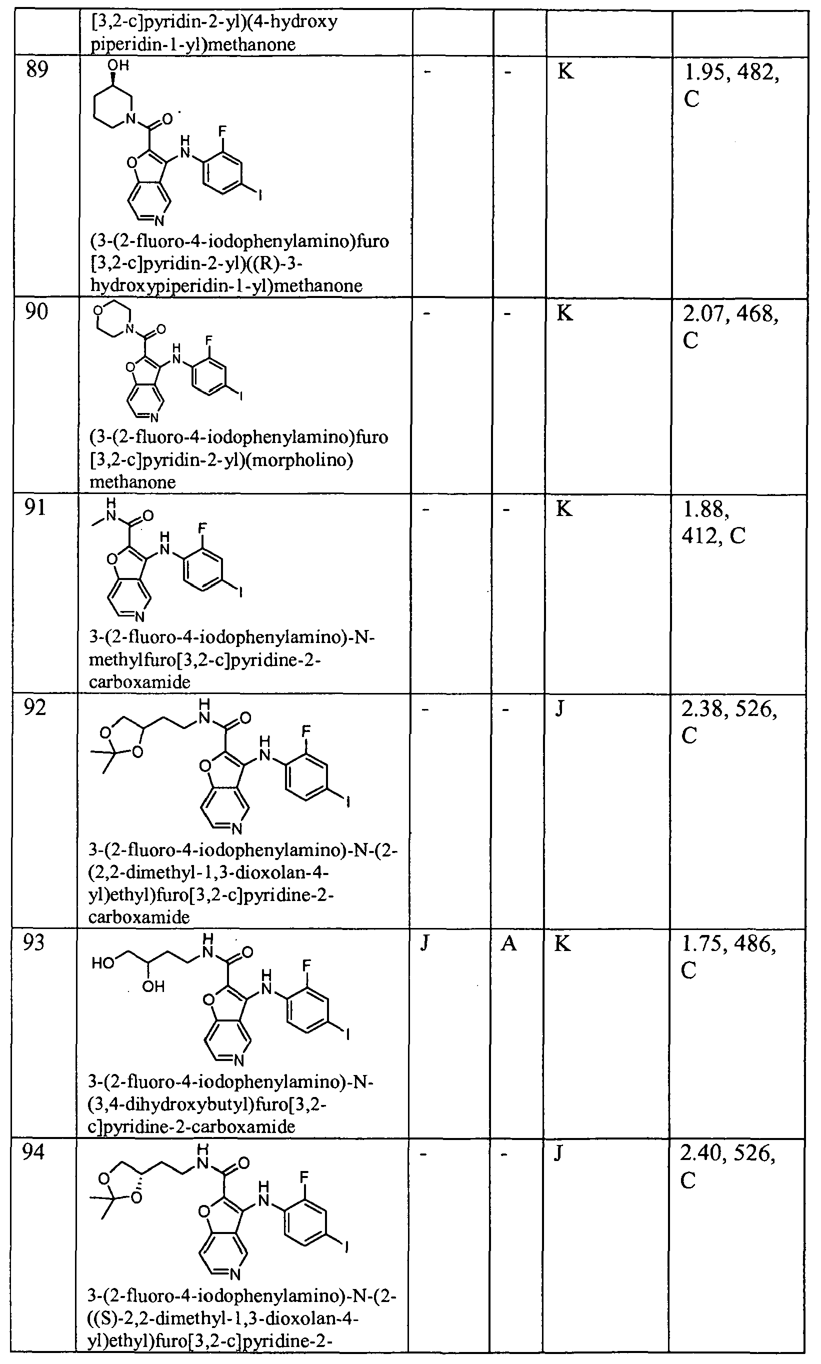

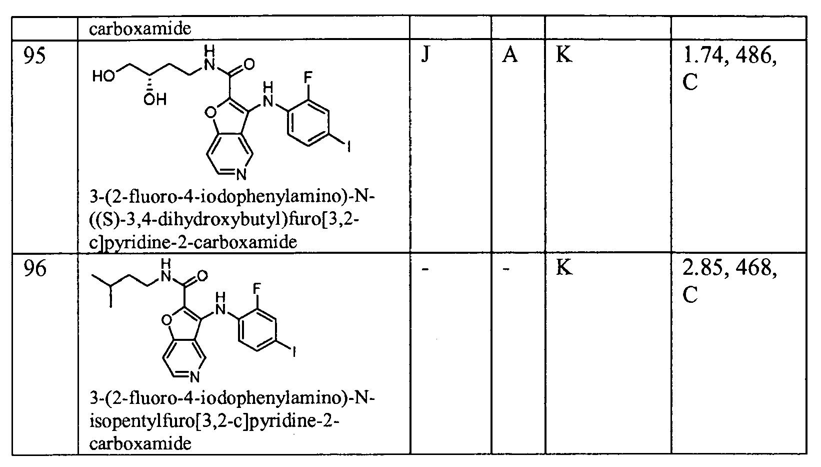

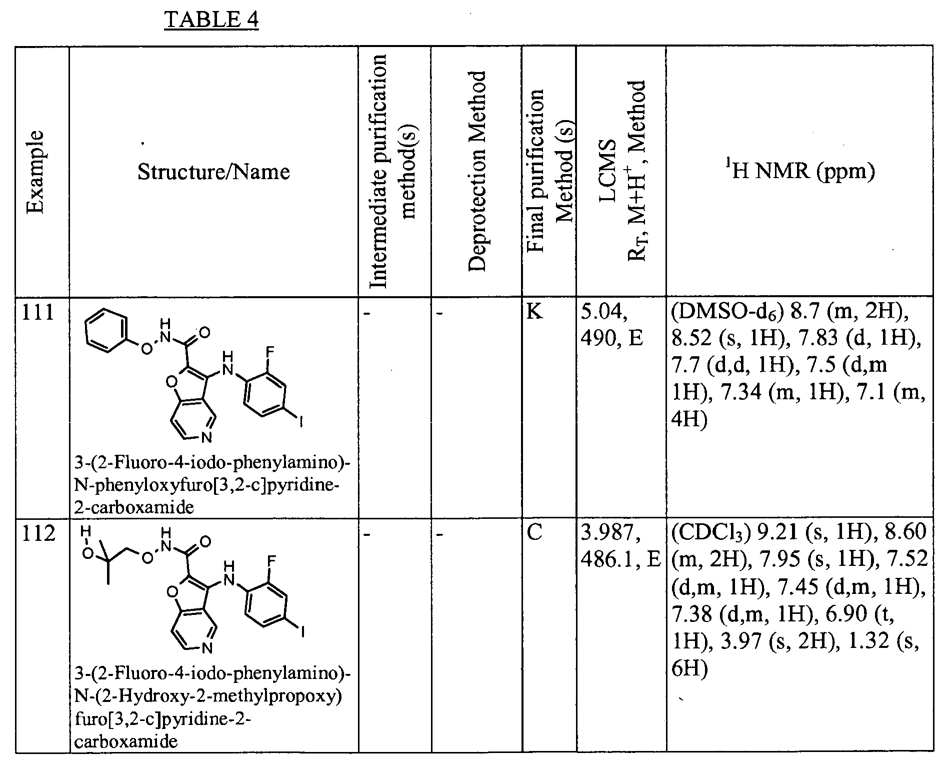

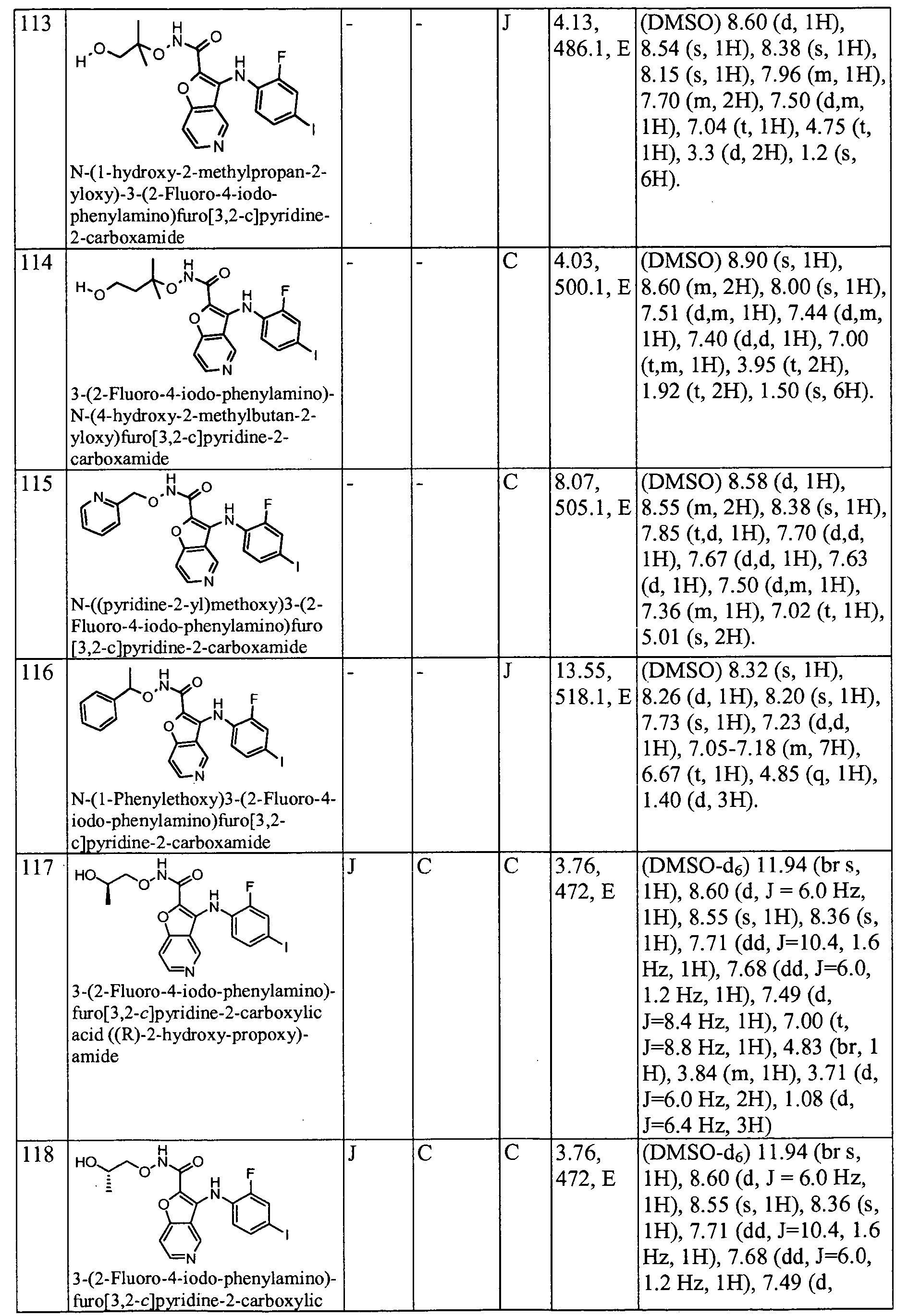

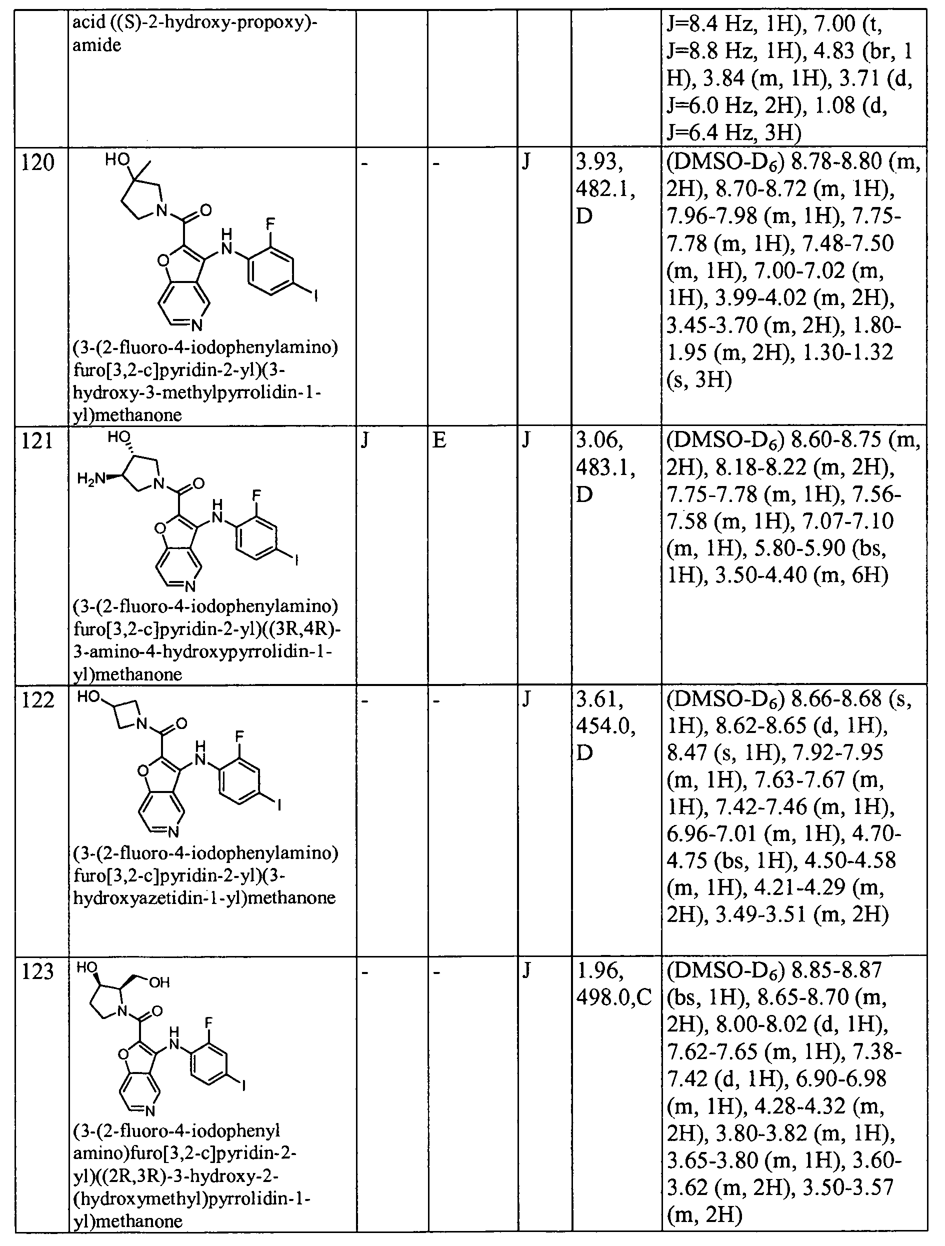

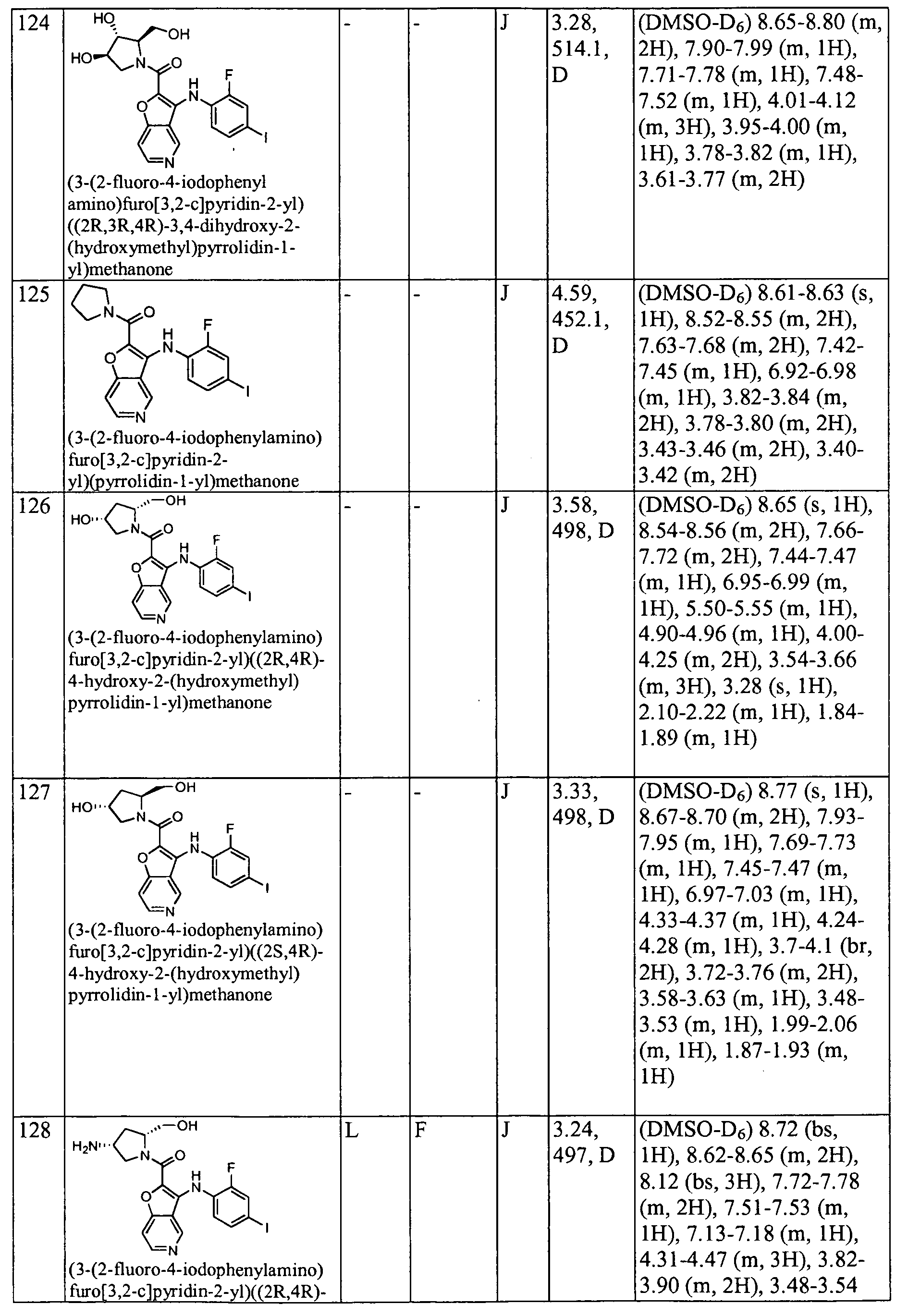

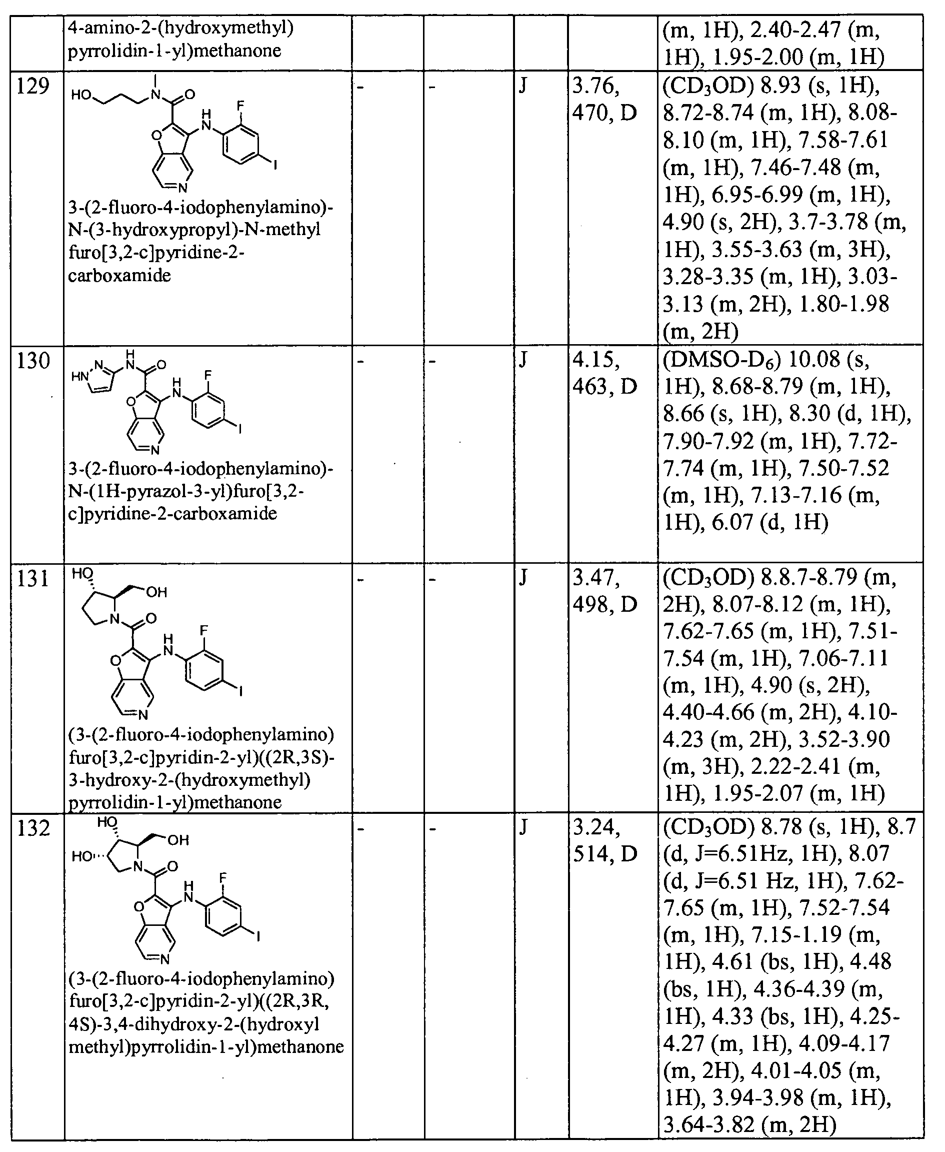

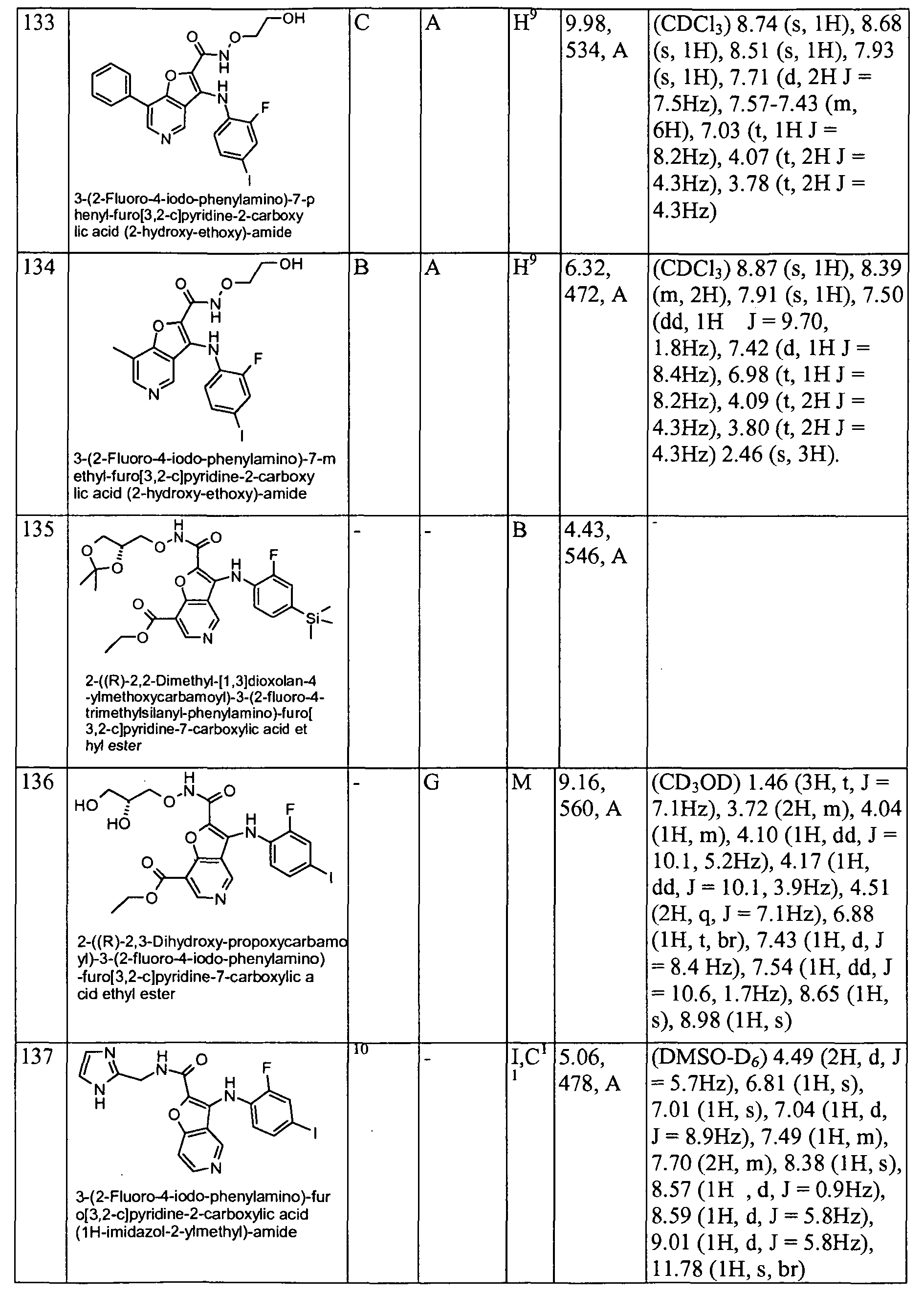

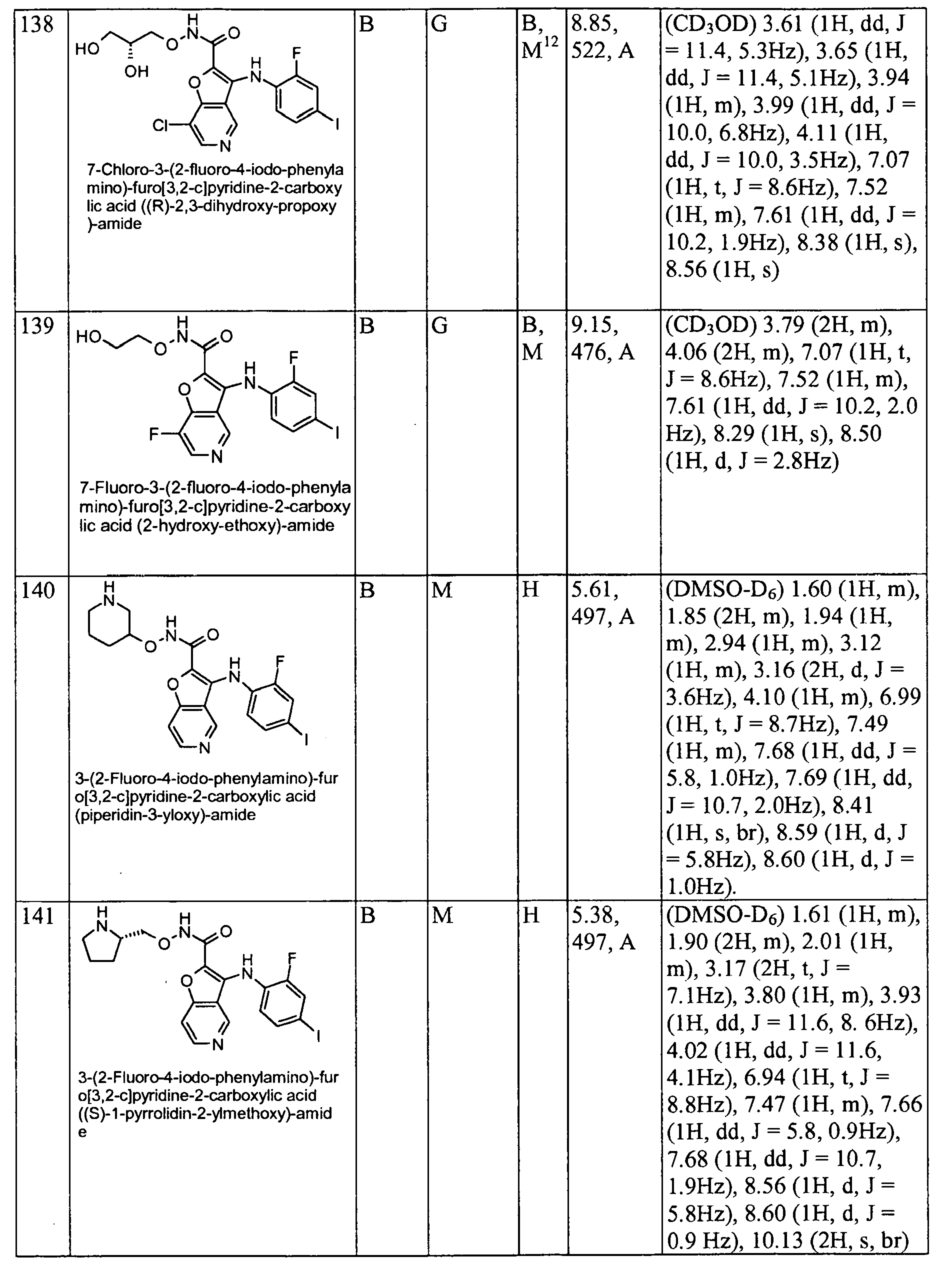

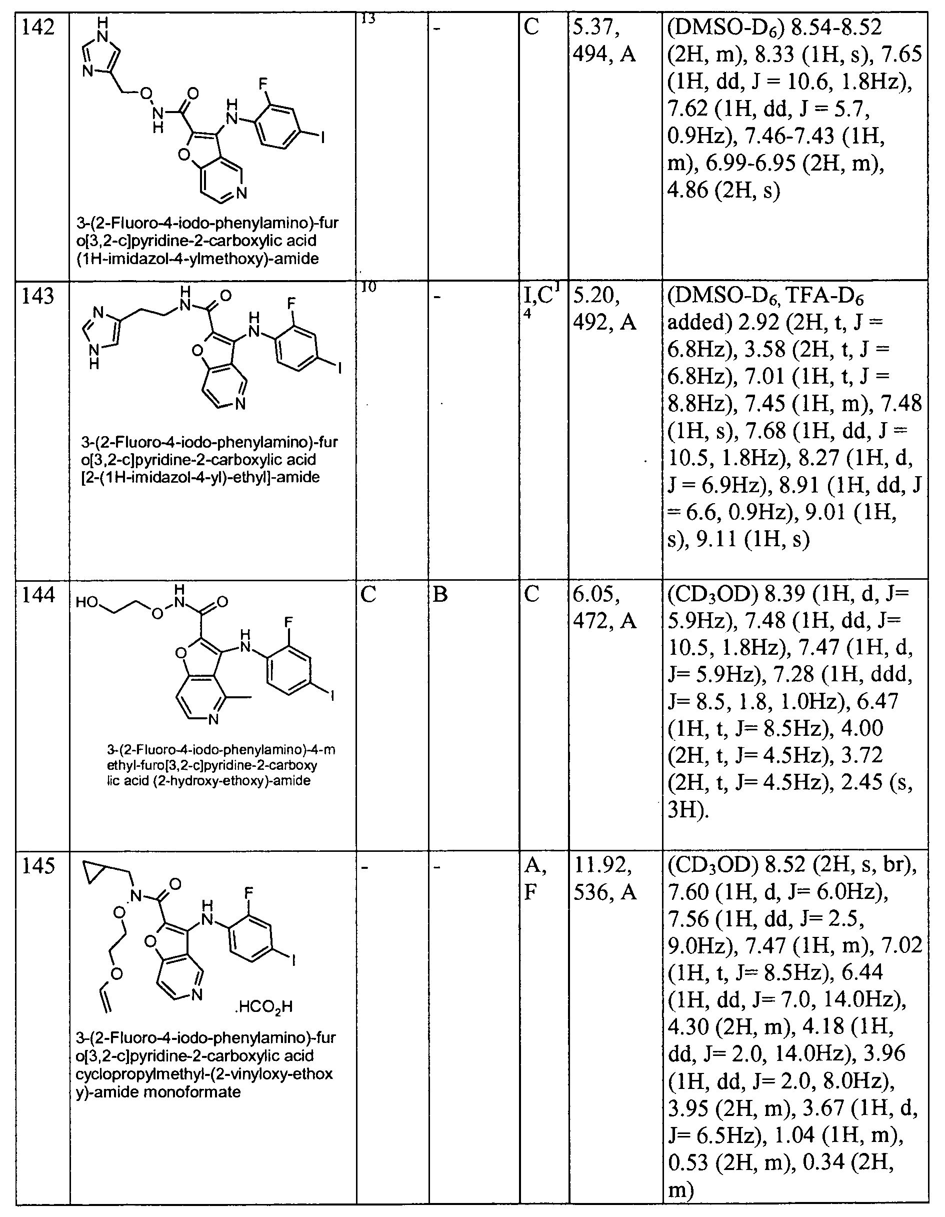

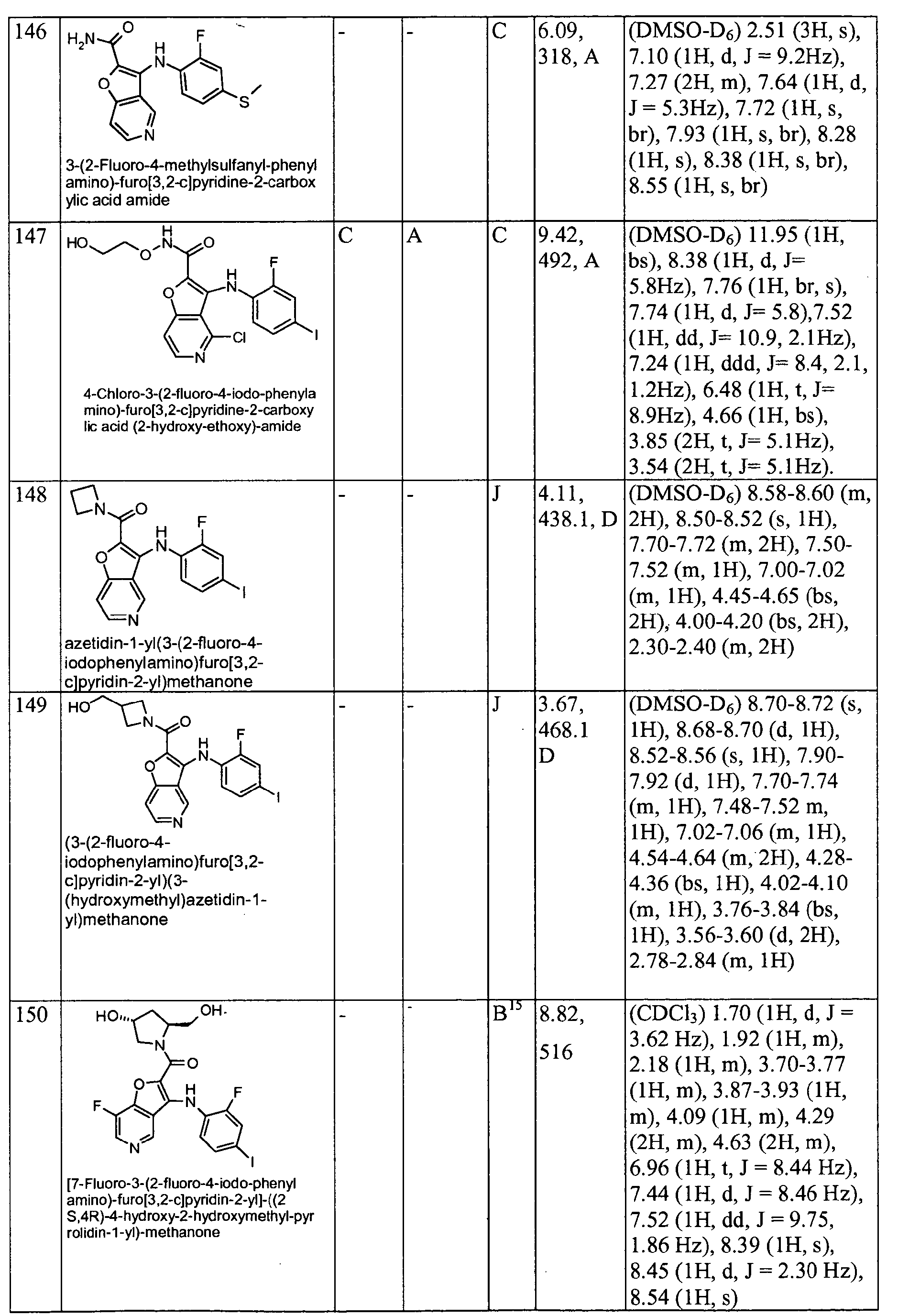

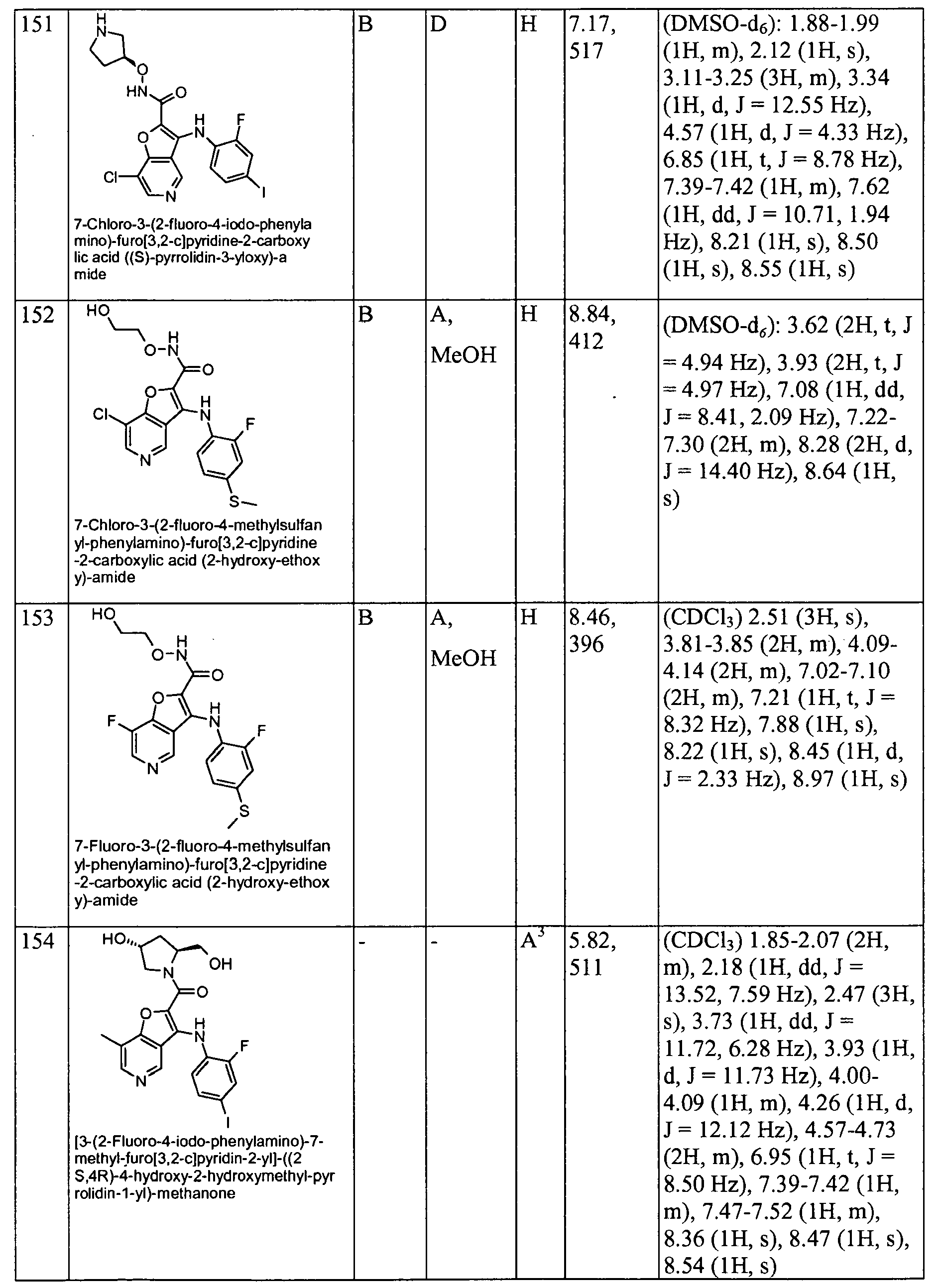

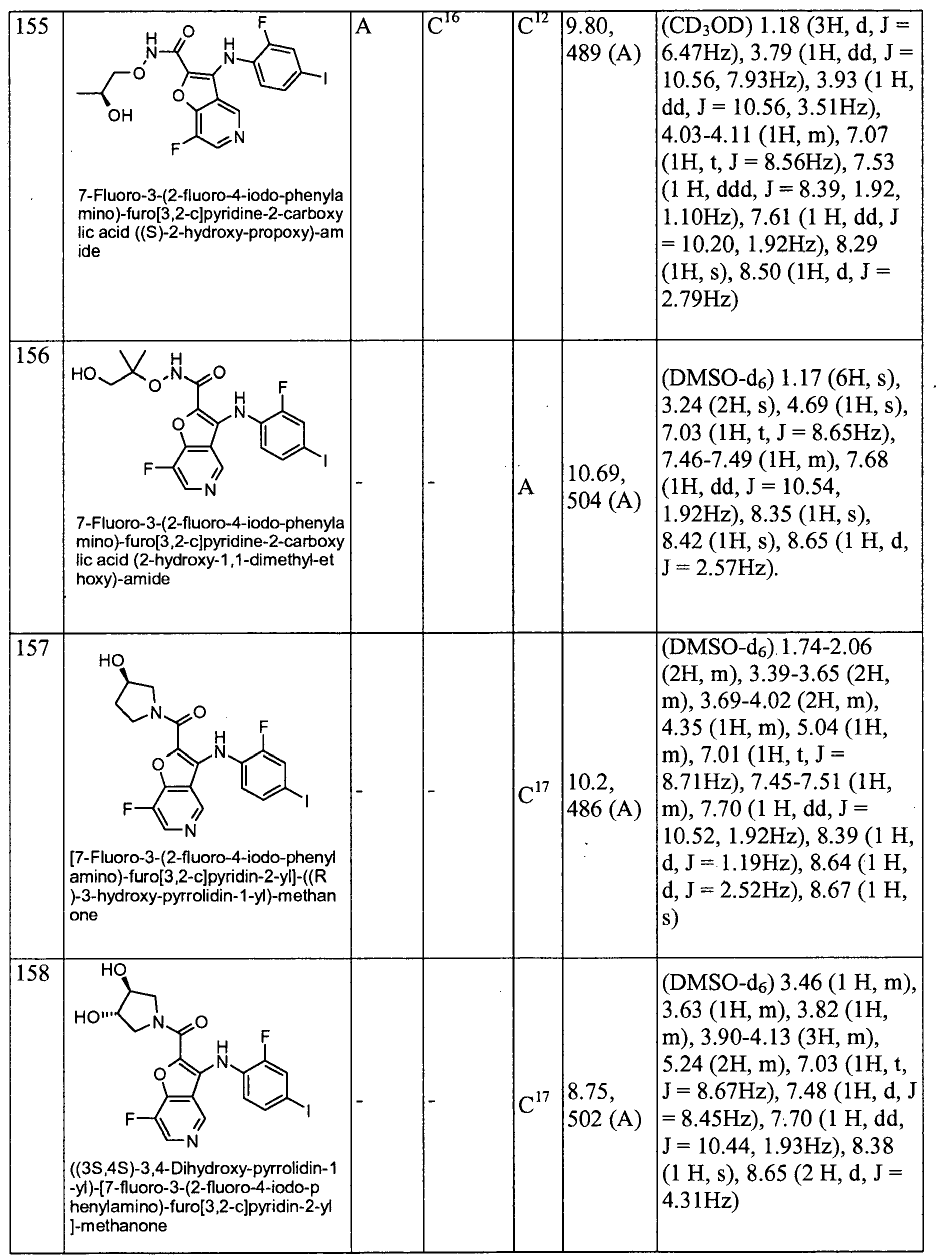

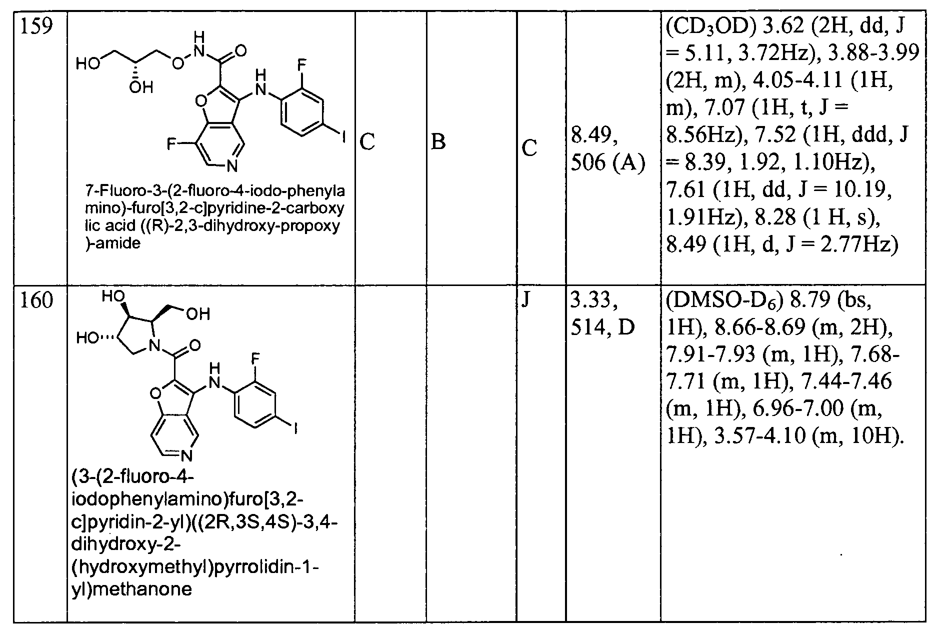

## Claims

1. A compound selected from Formula I: 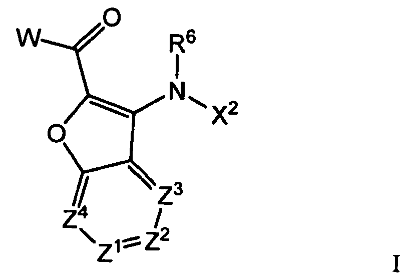 and solvates and salts thereof, wherein:
Z¹ is CR¹;
Z² is CR² or N;
Z³ is CR³ or N;
Z⁴ is CR⁴ or N;
where one or two of Z², Z³, and Z⁴ are N;
R¹, R², R³ and R⁴ are independently selected from H, halo, CN, CF₃, -OCF₃, -NO₂, -(CR¹⁴R¹⁵)ₙC(=Y)R¹¹, -(CR¹⁴R¹⁵)ₙC(=Y)OR¹¹, -(CR¹⁴R¹⁵)ₙC(=Y)NR¹¹R¹², -(CR¹⁴R¹⁵)ₙNR¹¹R¹², -(CR¹⁴R¹⁵)ₙOR¹¹, -(CR¹⁴R¹⁵)ₙNR¹²C(=Y)R¹¹, C₁-C₁₂ alkyl, carbocyclyl, heterocyclyl, aryl, and heteroaryl;
W is 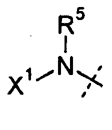 or 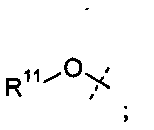
R⁵ and R⁶ are independently selected from H or C₁-C₁₂ alkyl;
X¹ is selected from R¹¹, -OR¹¹, -S(O)R¹¹, and -S(O)₂R¹¹; when X¹ is R¹¹ or -OR¹¹, R¹¹ or -OR¹¹ of X¹ and -R⁵ are optionally taken together with the nitrogen atom to which they are attached to form a 4-7 membered saturated or unsaturated ring having 0-2 additional heteroatoms selected from O, S and N, wherein said ring is optionally substituted with one or more groups selected from halo, CN, CF₃, -OCF₃, -NO₂, oxo, -(CR¹⁹R²⁰)ₙNR¹⁶R¹⁷, -(CR¹⁹R²⁰)ₙOR¹⁶, -(CR¹⁹R²⁰)ₙS(O)R¹⁶, -(CR¹⁹R²⁰)ₙS(O)₂R¹⁶, and R²¹;
X² is aryl;
R¹¹, R¹² and R¹³ are independently H, C₁-C₁₂ alkyl, C₂-C₈ alkenyl, C₂-C₈ alkynyl, carbocyclyl, heterocyclyl, aryl, or heteroaryl;
R¹⁴ and R¹⁵ are independently selected from H, C₁-C₁₂ alkyl;
m and n are independently selected from 0, 1, 2, 3, 4, 5, or 6;
Y is independently O, NR¹¹, or S;
wherein each said alkyl, alkenyl, alkynyl, carbocyclyl, heterocyclyl, aryl and heteroaryl of R¹, R², R³, R⁴, R⁵, R⁶, X¹, X², R¹¹, R¹², R¹³, R¹⁴, and R¹⁵ is independently optionally substituted with one or more groups independently selected from halo, CN, CF₃, -OCF₃, -NO₂, oxo, -Si(C₁-C₆ alkyl), -(CR¹⁹R²⁰)ₙC(=Y')R¹⁶, -(CR¹⁹R²⁰)ₙC(=Y')OR¹⁶, -(CR¹⁹R²⁰)ₙC(=Y')NR¹⁶R¹⁷, -(CR¹⁹R²⁰)ₙNR¹⁶R¹⁷, -(CR¹⁹R²⁰)ₙOR¹⁶, -(CR¹⁹R²⁰)ₙ-SR¹⁶, -(CR¹⁹R²⁰)ₙNR¹⁶C(=Y')R¹⁷, -(CR¹⁹R²⁰)ₙ NR¹⁶C(=Y')OR¹⁷, -(CR¹⁹R²⁰)ₙ NR¹⁸C(=Y')NR¹⁶R¹⁷, -(CR¹⁹R²⁰)ₙNR¹⁷SO₂R¹⁶, -(CR¹⁹R²⁰)ₙOC(=Y')R¹⁶, -(CR¹⁹R²⁰)ₙOC(=Y')OR¹⁶, -(CR¹⁹R²⁰)ₙOC(=Y')NR¹⁶R¹⁷and R²¹;
each R¹⁶, R¹⁷ and R¹⁸ is independently H, C₁-C₁₂ alkyl, C₂-C₈ alkenyl, C₂-C₈ alkynyl, carbocyclyl, heterocyclyl, aryl, or heteroaryl, wherein said alkyl, alkenyl, alkynyl, carbocyclyl, heterocyclyl, aryl, or heteroaryl is optionally substituted with one or more groups selected from halo, CN, -OCF₃, CF₃, -NO₂, C₁-C₆ alkyl, -OH, -O(C₁-C₆ alkyl), -NH₂, -NH(C₁-C₆ alkyl), -N(C₁-C₆ alkyl)₂, -CO₂H, -CO₂(C₁-C₆ alkyl),;
R¹⁹ and R²⁰ are independently selected from H, C₁-C₁₂ alkyl;
R²¹ is C₁-C₁₂ alkyl, C₂-C₈ alkenyl, C₂-C₈ alkynyl, carbocyclyl, heterocyclyl, aryl, or heteroaryl, wherein each member of R²¹ is optionally substituted with one or more groups selected from halo, oxo, CN, -OCF₃, CF₃, -NO₂ C₁-C₆ alkyl, -OH, -O(C₁-C₆ alkyl), -NH₂, -NH(C₁-C₆ alkyl), -N(C₁-C₆ alkyl)₂, -CO₂H, -CO₂(C₁-C₆ alkyl);
each Y' is independently O, NR²², or S; and
R²² is H or C₁-C₁₂ alkyl.

2. The compound of claim 1 selected from Formulae I-b, I-f and I-g:

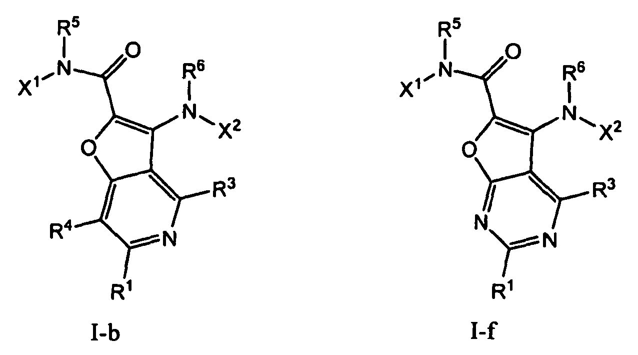

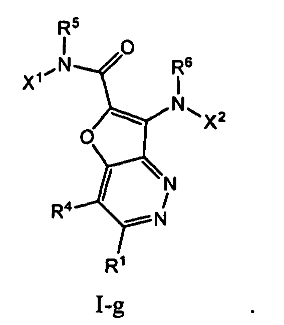

3. The compound of claim 2 wherein X¹ is selected from:

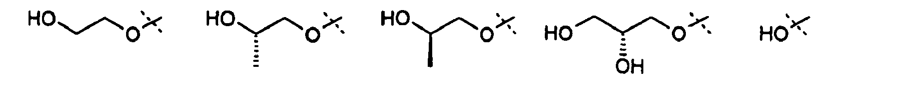

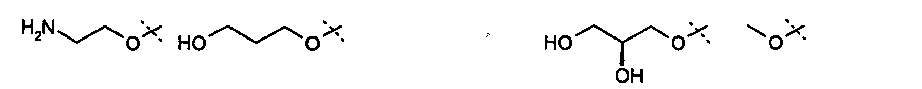

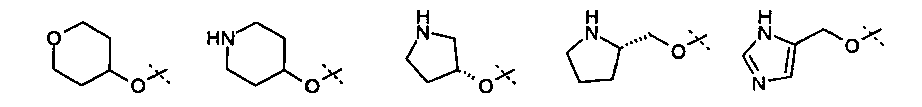

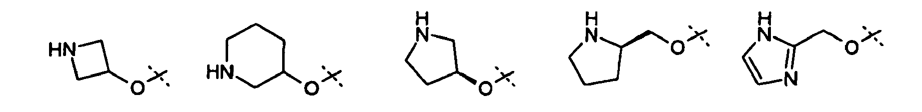

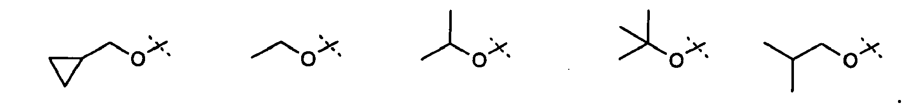

4. The compound of claim 2 wherein X¹ is selected from:

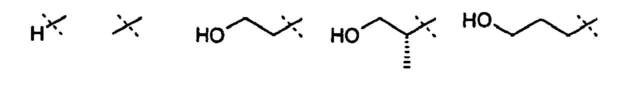

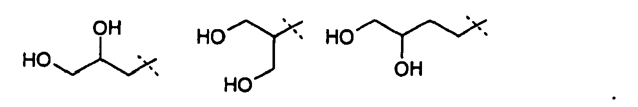

5. The compound of claim 2 wherein X¹ is R¹¹, and R¹¹ and R⁵ are taken together with the nitrogen atom to which they are attached to form:

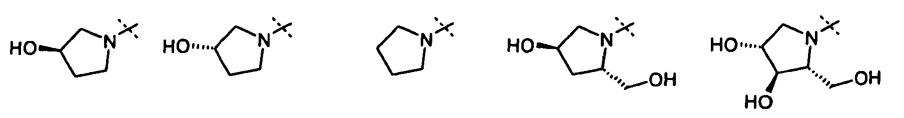

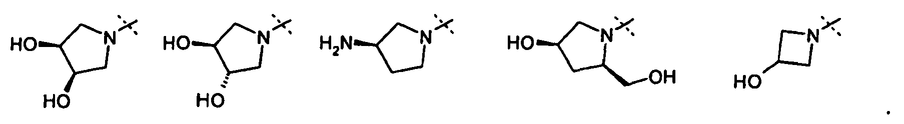

6. The compound of claim 2 wherein X² is:

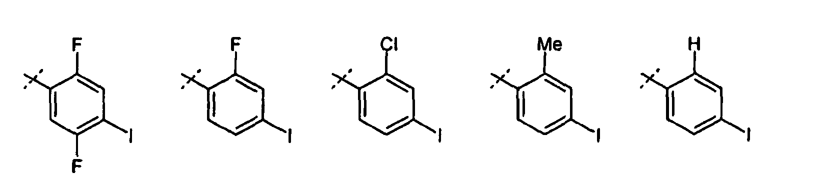

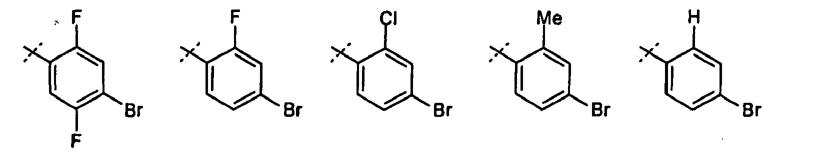

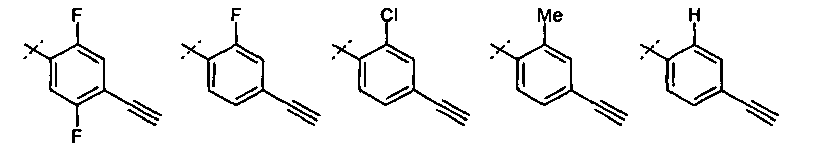

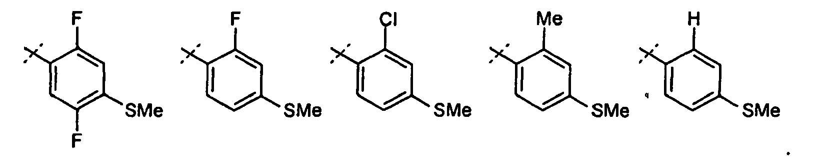

7. The compound of claim 2 wherein R¹ is selected from H, CH₃, CF₃, CN, -NR¹¹R¹², -OR¹¹, and Cl.

8. The compound of claim 2 wherein R³ is selected from H, CH₃, F, or CF₃.

9. The compound of claim 2 wherein R⁴ is selected from CF₃, Br, Cl, CN, -NR¹¹R¹², -OR¹¹, and -C(=O)NR¹¹R¹².

10. The compound of claim 9 wherein R⁴ is selected from Cl, Br, Me, Et, F, CHF-2, CF₃ or -OH.

11. The compound of claim 2 wherein R⁵ is H or methyl.

12. The compound of claim 2 wherein R⁶ is H or methyl.

13. The compound of claim 1 wherein W is OR¹¹.

14. The compound of claim 13 wherein W is OH.

15. A compound selected from
3-(2-Fluoro-4-iodo-phenylamino)-furo[3,2-c]pyridine-2-carboxylic acid ((R)-2,3-dihydroxypropoxy)-amide;
3-(2-Fluoro-4-bromo-phenylamino)-furo[3,2-c]pyridine-2-carboxylic acid ((R)-2,3-dihydroxy-propoxy)-amide;
3-(4-Ethynyl-2-fluoro-phenylamino)-furo[3,2-c]pyridine-2-carboxylic acid ((R)-2,3-dihydroxy-propoxy)-amide;
3-(2-Fluoro-4-iodo-phenylamino)-furo[3,2-c]pyridine-2-carboxylic acid cyclopropylmethoxy-amide;
3-(2-Fluoro-4-iodo-phenylamino)-furo[2,3-c]pyridine-2-carboxylic acid ((R)-2,3-dihydroxypropoxy)-amide;
3-(2-Fluoro-4-iodo-phenylamino)-furo[3,2-*c*]pyridine-2-carboxylic acid (2-hydroxy-ethoxy)-amide;
3-(2-Fluoro-4-iodo-phenylamino)-furo[3,2-*c*]pyridine-2-carboxylic acid (2-vinyloxy-ethoxy)-amide;
3-(4-Iodo-phenylamino)-furo[3,2-c]pyridine-2-carboxylic acid ((R)-2,3-dihydroxy-propoxy)-amide;
3-(2-Chloro-4-iodo-phenylamino)-furo[3,2-c]pyridine-2-carboxylic acid ((R)-2,3-dihydroxypropoxy)-amide;
3-(2,6-Difluoro-4-iodo-phenylamino)-furo[3,2-c]pyridine-2-carboxylic acid ((R)-2,3-dihydroxy-propoxy)-amide;
3-(2,5-Difluoro-4-iodo-phenylamino)-furo[3,2-c]pyridine-2-carboxylic acid ((R)-2,3-dihydroxy-propoxy)-amide;
4-{[3-(2-Fluoro-4-iodo-phenylamino)-furo[3,2-c]pyridine-2-carbonyl]aminooxy}-piperidine-1-carboxylic acid tert-butyl ester;
3-(2-Fluoro-4-iodo-phenylamino)-furo[3,2-c]pyridine-2-carboxylic acid (2-morpholin-4-yl-ethoxy)-amide;
7-Bromo-3-(2-fluoro-4-iodo-phenylamino)-furo[3,2-c]pyridine-2-carboxylic acid ((R)-2,3-dihydroxy-propoxy)amide;
5-(2-Fluoro-4-iodo-phenylamino)-furo[2,3-d]pyrimidine-6-carboxylic acid ((R)-2,3-dihydroxy-propoxy)-amide;
3-(2-Fluoro-4-iodophenylamino)furo[3,2-c]pyridine-2-carboxylic acid (1-methylpiperidin-4-yloxy) amide;
3-(2-Fluoro-4-iodo-phenylamino)furo[3,2-c]pyridine-2-carboxylic acid (2-dimethylaminoethoxy) amide;
(2-Fluoro-4-iodo-phenylamino)-N-tert-butoxyfuro[3,2-c]pyridine-2-carboxamide ; (3-(2-fluoro-4-iodophenylamino)furo[3,2-c]pyridin-2-yl)(1-oxothiazolidin-3-yl)methanone;
(3-(2-fluoro-4-iodophenylamino)furo[3,2-c]pyridin-2-yl)(1,1-dioxothiomorpholin-4-yl)methanone;
(3-(2,5-difluoro-4-(4-pyrazolyl)phenylamino)furo[3,2-c]pyridin-2-yl)((R)-3-hydroxypyrrolidin-1-yl)methanone;
2-Dimethylcarbamoyl-3-(2-fluoro-4-iodo-phenylamino)-furo[3,2-c]pyridine-7-carboxylic acid ethyl ester;
3-(2-Fluoro-4-iodo-phenylamino)-7-hydroxymethyl-furo[3,2-c]pyridine-2-carboxylic acid dimethylamide;
3-(2-Fluoro-4-iodo-phenylamino)-7-phenoxymethyl-furo[3,2-c]pyridine-2-carboxylic acid dimethylamide;
7-chloro-3-(2-fluoro-4-iodo-phenylamino)-furo[3,2-c]pyridine-2-carboxylic acid sodium salt;
7-Chloro-3-(2-fluoro-4-iodo-phenylamino)-furo[3,2-c]pyridine-2-carboxylic acid amide;
3-(2-Fluoro-4-iodo-phenylamino)-furo[3,2-c]pyridine-2-carboxylic acid (2-methanesulfonylamino-ethoxy)-amide;
7-Chloro-3-(2-fluoro-4-iodo-phenylamino)-furo[3,2-c]pyridine-2- carboxylic acid ((R)-2,3-dihydroxy-propoxy)-amide;
7-Fluoro-3-(2-fluoro-4-iodophenylamino)-furo[3,2-c]pyridine-2-carboxylic acid (2-hydroxyethoxy)amide;
(2-Fluoro-4-iodo-phenylamino)-N-isobutoxyfuro[3,2-c]pyridine-2-carboxamide;
(2-Fluoro-4-iodo-phenylamino)-N-isopropoxyfuro[3,2-c]pyridine-2-carboxamide;
(2-Fluoro-4-iodo-phenylamino)-N-benzyloxyfuro[3,2-c]pyridine-2-carboxamide;
(2-Fluoro-4-iodo-phenyl amino)-N-(3-hydroxy propoxy)furo[3,2-c]pyridine-2-carboxamide;
(2-Fluoro-4-iodo-phenyl amino)-N-[3-(pyridine-2-carboxamide)propoxy]furo [3,2-c]pyridine-2-carboxamide;
(2-Fluoro-4-iodo-phenyl amino)-N-[3-(nicotinamide) propoxy] furo[3,2-c]pyridine-2-carboxamide;
(2-Fluoro-4-iodo-phenyl amino)-N-[3-(isonicotinamide)propoxy] furo[3,2-c]pyridine-2-carboxamide;
(2-Fluoro-4-iodo-phenyl amino)-N-propoxyfuro[3,2-c]pyridine-2-carboxamide;
(2-Fluoro-4-iodo-phenyl amino)-N-ethoxyfuro[3,2-c]pyridine-2-carboxamide;
N-(2-(Benzyloxy)-2-methyl propoxy)-3-(2-Fluoro-4-iodo-phenylamino)furo[3,2-c]pyridine-2-carboxamide;
2-hydoxy-2-methylpropyl-3-(2-Fluoro-4-iodo-phenyl amino)furo [3,2-c]pyridine-2-carboxylate;
N-(2-hydroxyethoxy)-3-(4-Bromo-2-fluorophenyl amino)furo[3,2-c]pyridine-2-carboxamide;
3-(2-Fluoro-4-iodo-phenyl amino)-furo[3,2-c]pyridine-2-carboxylic acid (2-hydroxy-1-methyl-ethoxy)-amide;
3-(2-Fluoro-4-iodo-phenyl amino)-furo[3,2-*c*]pyridine-2-carboxylic acid (2-hydroxypropoxy)-amide;
3-(2-Fluoro-4-iodo-phenylamino)-furo[3,2-*c*]pyridine-2-carboxylic acid (2-hydroxy-1-hydroxymethyl-ethoxy)-amide;
*N*-[3-(2-Fluoro-4-iodo-phenyl amino)-furo[3,2-c]pyridine-2-carbonyl]-methanesulfonamide; 3-(2-Fluoro-4-iodo-phenyl amino)-furo[3,2-c]pyridine-2-carboxylic acid (2-piperidin-1-ylethoxy)-amide;
(3-(2-fluoro-4-iodophenyl amino) furo[3,2-c]pyridin-2-yl)((R)-3-hydroxypyrrolidin-1-yl)methanone;
(3-(2-fluoro-4-iodophenyl amino) furo[3,2-c]pyridin-2-yl)((3S,4S)-3,4-dihydroxy pyrrolidin-1-yl)methanone;
3-(2-fluoro-4-iodophenylamino)-N-(3-hydroxypropyl)furo[3,2-c]pyridine-2-carboxamide;
3-(2-fluoro-4-iodophenylamino)-N-(2-methoxyethyl)furo[3,2-c]pyridine-2-carboxamide;
3-(2-fluoro-4-iodophenylamino)-N-isopropylfuro[3,2-c]pyridine-2-carboxamide;
3-(2-fluoro-4-iodophenylamino)-N-(2-cyclopropylethyl)furo[3,2-c]pyridine-2-carboxamide;
3-(2-fluoro-4-iodophenylamino)-N-(3-(dimethylamino)propyl)furo[3,2-c]pyridine-2-carboxamide;
3-(2-fluoro-4-iodophenylamino)-N-(2-(2-hydroxyethoxy)ethyl)furo[3,2-c]pyridine-2-carboxamide;
N-(3-(1H-imidazol-1-yl)propyl)-3-(2-fluoro-4-iodophenylamino)furo[3,2-c]pyridine-2-carboxamide;
3-(2-fluoro-4-iodophenylamino)-N-(1,3-dihydroxypropan-2-yl)furo[3,2-c]pyridine-2-carboxamide;
(3-(2-fluoro-4-iodophenylamino)furo [3,2-c]pyridin-2-yl)(thiazolidin-3-yl)methanone;
(3-(2-fluoro-4-iodophenylamino)furo [3,2-c]pyridin-2-yl)((S)-3-hydroxy pyrrolidin-1-yl)methanone;
(3-(4-bromo-2,5-difluorophenyl amino)furo[3,2-c]pyridin-2-yl)((R)-3-hydroxypyrrolidin-1-yl)methanone;
(3-(4-bromo-2-fluorophenylamino) furo[3,2-c]pyridin-2-yl)((R)-3-hydroxypyrrolidin-1-yl)methanone;
(3-(2-fluoro-4-iodophenylamino)furo [3,2-c]pyridin-2-yl)((3S,4R)-3,4-dihydroxypyrrolidin-1-yl)methanone;
(3-(2-fluoro-4-iodophenylamino)furo [3,2-c]pyridin-2-yl)((R)-3-aminopyrrolidin-1-yl)methanone;
(3-(2-fluoro-4-iodophenylamino)furo [3,2-c]pyridin-2-yl)((S)-3-hydroxypiperidin-1-yl)methanone; 3-(2-fluoro-4-iodophenylamino)-N-((S)-1-hydroxypropan-2-yl)furo[3,2-c]pyridine-2-carboxamide;
(3-(2-fluoro-4-iodophenylamino)furo [3,2-c]pyridin-2-yl)(2-(thiazol-2-yl)pyrrolidin-1-yl)methanone;
(3-(2-fluoro-4-iodophenylamino)furo [3,2-c]pyridin-2-yl)(3-(methyl sulfonyl)pyrrolidin-1-yl)methanone;
(3-(2-fluoro-4-iodophenylamino) furo[3,2-c]pyridin-2-yl)((S)-2-(hydroxymethyl)pyrrolidin-1-yl)methanone;
(3-(2-fluoro-4-iodophenylamino)furo [3,2-c]pyridin-2-yl)(2-(hydroxyl methyl)piperidin-1-yl)methanone;
3-(2-fluoro-4-iodophenylamino)-N-(2-hydroxyethyl)-N-methylfuro[3,2-c]pyridine-2-carboxamide;
3-(2-fluoro-4-iodophenylamino)-N-(2-hydroxyethyl)furo[3,2-c]pyridine-2-carboxamide;
3-(2-fluoro-4-iodophenylamino)-N,N-dimethylfuro[3,2-c]pyridine-2-carboxamide;
3-(2-fluoro-4-iodophenylamino)-N-(2,3-dihydroxypropyl)furo[3,2-c]pyridine-2-carboxamide;
3-(2-fluoro-4-iodophenylamino)-N-((R)-1-hydroxypropan-2-yl)furo[3,2-c]pyridine-2-carboxamide;
(3-(2-fluoro-4-iodophenylamino)furo [3,2-c]pyridin-2-yl)(3-(hydroxyl methyl)piperidin-1-yl)methanone;
(3-(2-fluoro-4-iodophenylamino)furo [3,2-c]pyridin-2-yl)((R)-2-(hydroxyl methyl)pyrrolidin-1-yl)methanone;
(3-(2-fluoro-4-iodophenylamino)furo [3,2-c]pyridin-2-yl)(4-hydroxy piperidin-1-yl)methanone;
(3-(2-fluoro-4-iodophenylamino)furo [3,2-c]pyridin-2-yl)((R)-3-hydroxypiperidin-1-yl)methanone;
(3-(2-fluoro-4-iodophenylamino)furo [3,2-c]pyridin-2-yl)(morpholino) methanone;
3-(2-fluoro-4-iodophenylamino)-N-methylfuro[3,2-c]pyridine-2-carboxamide;
3-(2-fluoro-4-iodophenylamino)-N-(2-(2,2-dimethyl-1,3-dioxolan-4-yl)ethyl)furo[3,2-c]pyridine-2-carboxamide;
3-(2-fluoro-4-iodophenylamino)-N-(3,4-dihydroxybutyl)furo[3,2-c]pyridine-2-carboxamide;
3-(2-fluoro-4-iodophenylamino)-N-(2-((S)-2,2-dimethyl-1,3-dioxolan-4-yl)ethyl)furo[3,2-c]pyridine-2-carboxamide;
3-(2-fluoro-4-iodophenylamino)-N-((S)-3,4-dihydroxybutyl)furo[3,2-c]pyridine-2-carboxamide;
3-(2-fluoro-4-iodophenylamino)-N-isopentylfuro[3,2-c]pyridine-2-carboxamide;
3-(2-Fluoro-4-iodo-phenylamino)-N-phenyloxyfuro[3,2-c]pyridine-2-carboxamide;
3-(2-Fluoro-4-iodo-phenylamino)-N-(2-Hydroxy-2-methylpropoxy) furo[3,2-c]pyridine-2-carboxamide;
N-(1-hydroxy-2-methylpropan-2-yloxy)-3-(2-Fluoro-4-iodo-phenylamino)furo[3,2-c]pyridine-2-carboxamide;
3-(2-Fluoro-4-iodo-phenylamino)-N-(4-hydroxy-2-methylbutan-2-yloxy)furo[3,2-c]pyridine-2-carboxamide;
N-((pyridine-2-yl)methoxy)3-(2-Fluoro-4-iodo-phenylamino)furo [3,2-c]pyridine-2-carboxamide;
N-(1-Phenylethoxy)3-(2-Fluoro-4-iodo-phenylamino)furo[3,2-c]pyridine-2-carboxamide;
3-(2-Fluoro-4-iodo-phenylamino)-furo[3,2-*c*]pyridine-2-carboxylic acid ((R)-2-hydroxypropoxy)-amide;
3-(2-Fluoro-4-iodo-phenylamino)-furo[3,2-*c*]pyridine-2-carboxylic acid ((S)-2-hydroxypropoxy)-amide;
(3-(2-fluoro-4-iodophenylamino) furo[3,2-c]pyridin-2-yl)(3-hydroxy-3-methylpyrrolidin-1-yl)methanone;
(3-(2-fluoro-4-iodophenylamino) furo[3,2-c]pyridin-2-yl)((3R,4R)-3-amino-4-hydroxypyrrolidin-1-yl)methanone;
(3-(2-fluoro-4-iodophenylamino) furo[3,2-c]pyridin-2-yl)(3-hydroxyazetidin-1-yl)methanone;
(3-(2-fluoro-4-iodophenyl amino)furo[3,2-c]pyridin-2-yl)((2R,3R)-3-hydroxy-2-(hydroxymethyl)pyrrolidin-1-yl)methanone;
(3-(2-fluoro-4-iodophenyl amino)furo[3,2-c]pyridin-2-yl) ((2R,3R,4R)-3,4-dihydroxy-2-(hydroxymethyl)pyrrolidin-1-yl)methanone;
(3-(2-fluoro-4-iodophenylamino) furo[3,2-c]pyridin-2-yl)(pyrrolidin-1-yl)methanone;
(3-(2-fluoro-4-iodophenylamino) furo[3,2-c]pyridin-2-yl)((2R,4R)-4-hydroxy-2-(hydroxymethyl) pyrrolidin-1-yl)methanone;
(3-(2-fluoro-4-iodophenylamino) furo[3,2-c]pyridin-2-yl)((2S,4R)-4-hydroxy-2-(hydroxymethyl) pyrrolidin-1-yl)methanone;
(3-(2-fluoro-4-iodophenylamino) furo[3,2-c]pyridin-2-yl)((2R,4R)-4-amino-2-(hydroxymethyl) pyrrolidin-1-yl)methanone;
3-(2-fluoro-4-iodophenylamino)-N-(3-hydroxypropyl)-N-methyl furo[3,2-c]pyridine-2-carboxamide;
3-(2-fluoro-4-iodophenylamino)-N-(1H-pyrazol-3-yl)furo[3,2-c]pyridine-2-carboxamide;
(3-(2-fluoro-4-iodophenylamino) furo[3,2-c]pyridin-2-yl)((2R,3S)-3-hydroxy-2-(hydroxymethyl) pyrrolidin-1-yl)methanone;
(3-(2-fluoro-4-iodophenylamino) furo[3,2-c]pyridin-2-yl)((2R,3R, 4S)-3,4-dihydroxy-2-(hydroxyl methyl)pyrrolidin-1-yl)methanone;
azetidin-1-yl(3-(2-fluoro-4-iodophenylamino)furo[3,2-c]pyridin-2-yl)methanone;
(3-(2-fluoro-4-iodophenylamino)furo[3,2-c]pyridin-2-yl)(3-(hydroxymethyl)azetidin-1-yl)methanone;
(3-(2-fluoro-4-iodophenylamino)furo[3,2-c]pyridin-2-yl)((2R,3S,4S)-3,4-dihydroxy-2-(hydroxymethyl)pyrrolidin-1-yl)methanone; 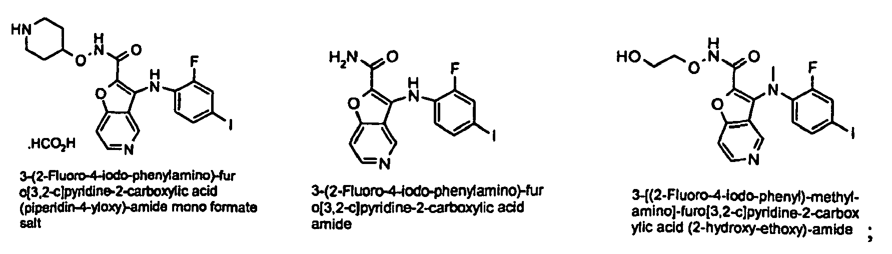 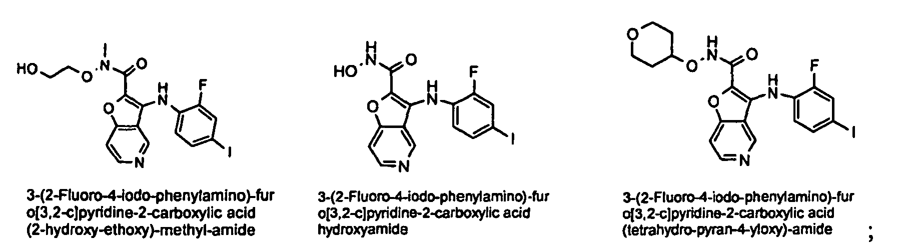 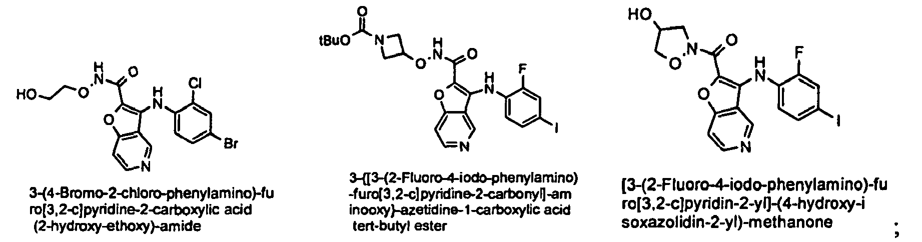 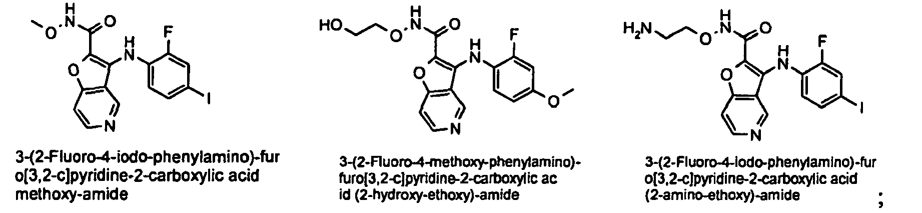 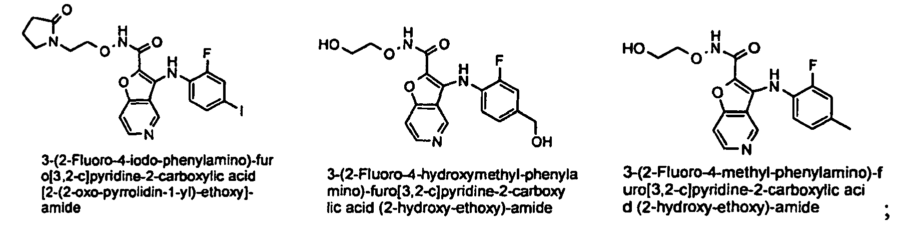 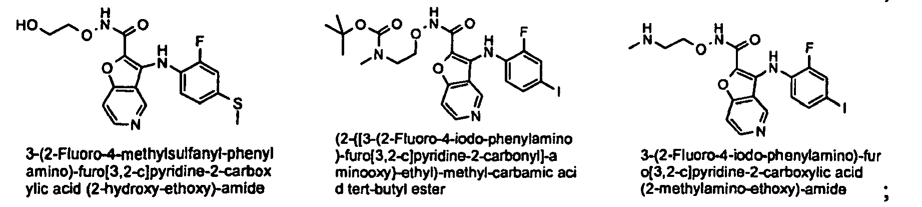 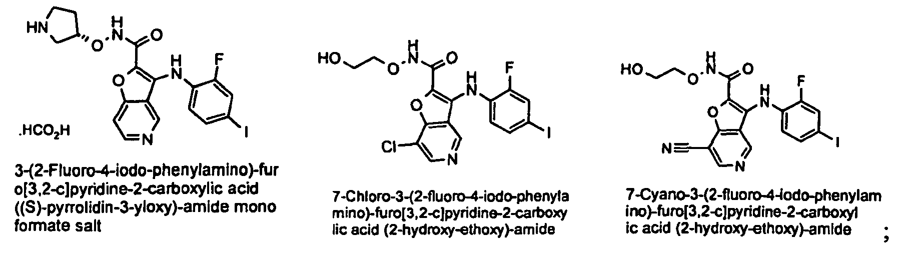 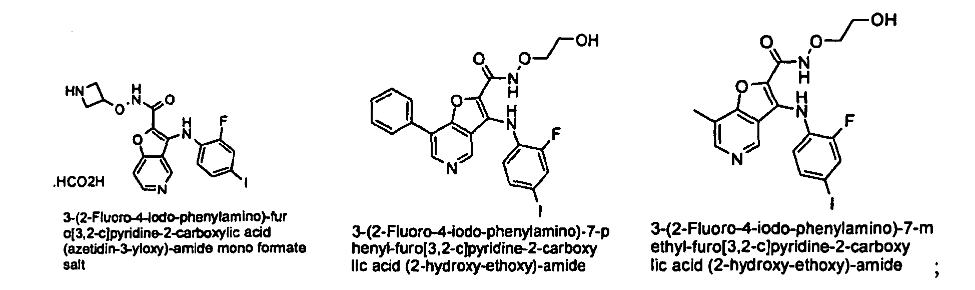 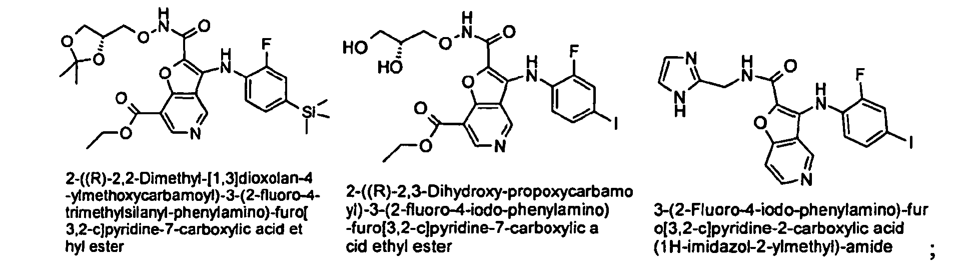 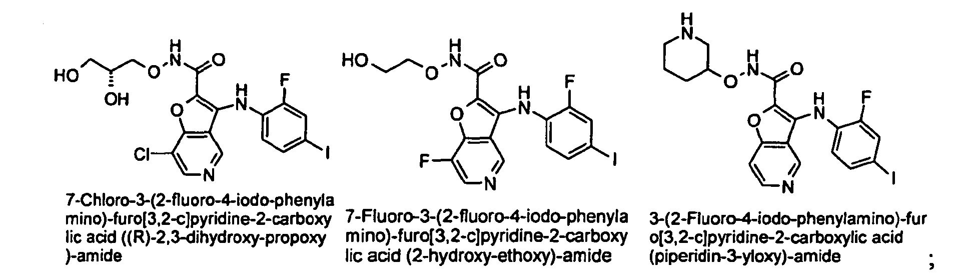 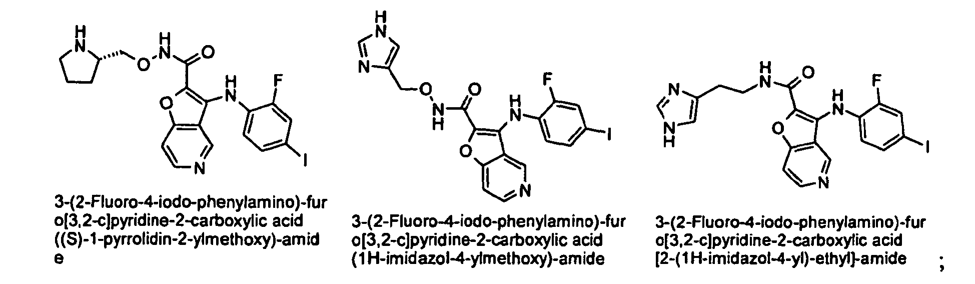 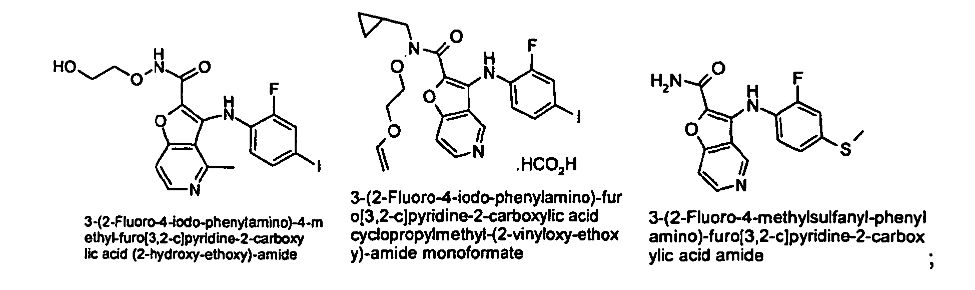 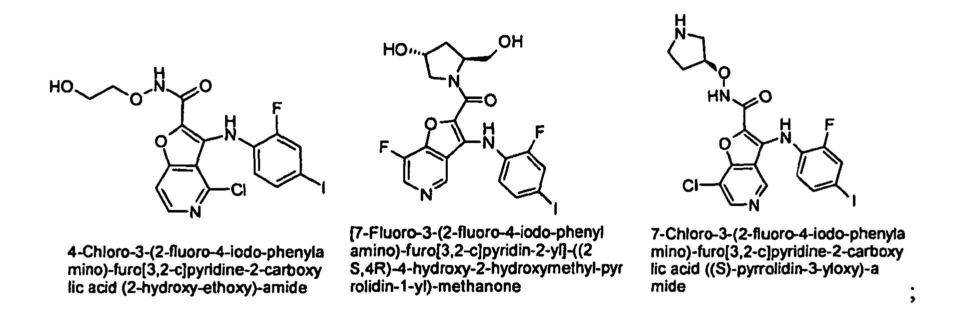 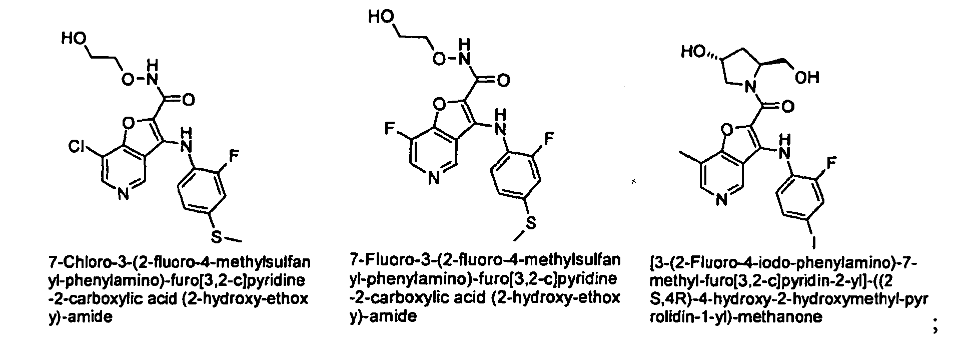 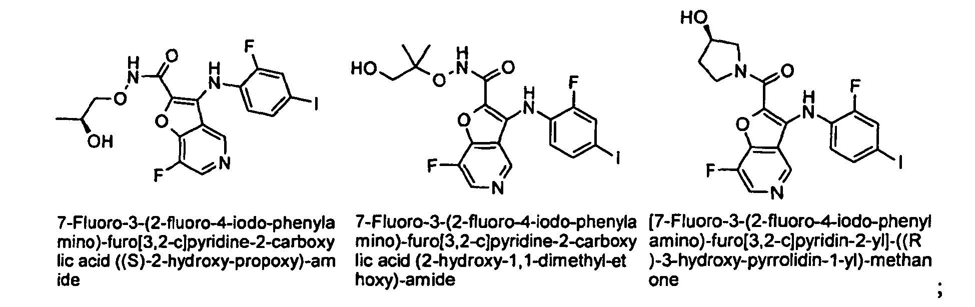 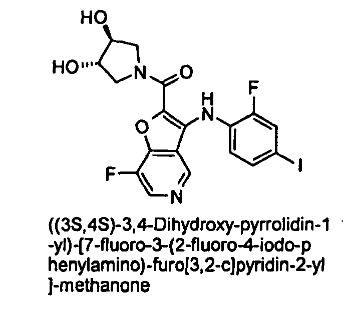 and

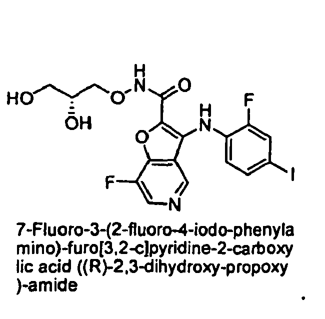

16. A pharmaceutical composition comprising a compound of any one of claims 1-15, and a pharmaceutically acceptable carrier.

17. The pharmaceutical composition of claim 16, further comprising a second chemotherapeutic agent or a second anti-inflammatory agent.

18. A compound of any one of claims 1 to 15 for use in a method of treatment, wherein the method is for inhibiting abnormal cell growth or treating a hyperproliferative disorder in a mammal.

19. Use of a compound of any one of claims 1 to 15, and optionally a second chemotherapeutic agent, in the manufacture of a medicament for inhibiting abnormal cell growth or treating a hyperproliferative disorder in a mammal.

20. A compound of any one of claims 1 to 15 for use in a method of treatment, wherein the method is for treating an inflammatory disease in a mammal.

21. Use of a compound of any one of claims 1 to 15, and optionally a second anti-inflammatory agent, in the manufacture of a medicament for treating an inflammatory disease in a mammal.

22. A compound of any one of claims 1 to 15 for use in a method of treatment, wherein the method is for treating an autoimmune disease, destructive bone disorder, proliferative disorders, infectious disease, viral disease, fibrotic disease, neurodegenerative disease, pancreatitis or kidney disease in a mammal.

23. Use of a compound of any one of claims 1 to 15, and optionally a second therapeutic agent, in the manufacture of a medicament for treating an autoimmune disease, destructive bone disorder, proliferative disorders, infectious disease, viral disease, fibrotic disease, neurodegenerative disease, pancreatitis or kidney disease in a mammal.

24. The use of claim 19, 21 or 23, wherein said second chemotherapeutic or anti-inflammatory or therapeutic agent is for sequential or consecutive administration to said mammal.

## Patentansprüche

1. Verbindung, ausgewählt aus Formel I: 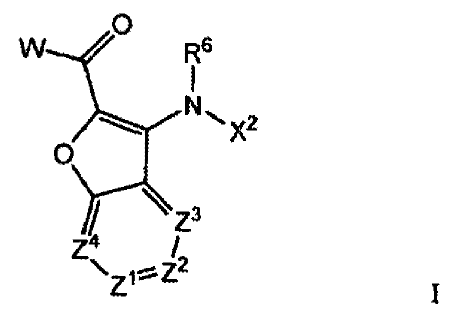 und Solvaten und Salzen davon, worin:
Z¹ CR¹ ist;
Z² CR² oder N ist;
Z³ CR³ oder N ist;
Z⁴ CR⁴ oder N ist;
worin eines oder zwei aus Z², Z³ und Z⁴ N sind;
R¹, R², R³ und R⁴ unabhängig voneinander aus H, Halogen, CN, CF₃, -OCF₃, -NO₂ -(CR¹⁴R¹⁵)ₙC(=Y)R¹¹, -(CR¹⁴R¹⁵)ₙC(=Y)OR¹¹, -(CR¹⁴R¹⁵)ₙC(=Y)NR¹¹R¹², -(CR¹⁴R¹⁵)ₙNR¹¹R¹², -(CR¹⁴R¹⁵)ₙOR¹¹, -(CR¹⁴R¹⁵)ₙNR¹²C(=Y)R¹¹, C₁-C₁₂-Alkyl, Carbocyclyl, Heterocyclyl, Aryl und Heteroaryl ausgewählt sind; 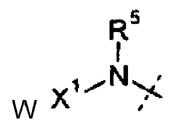 oder 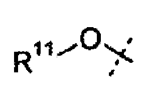 ist;
R⁵ und R⁶ unabhängig voneinander aus H oder C₁-C₁₂-Alkyl ausgewählt sind;
X¹ aus R¹¹, -OR¹¹, -S(O)R¹¹ und -S(O)₂R¹¹ ausgewählt ist; wenn X¹ R¹¹ oder -OR¹¹ ist, dann bilden R¹¹ oder -OR¹¹ von X¹ und -R⁵ gegebenenfalls zusammen mit dem Stickstoffatom, an das sie gebunden sind, einen 4- bis 7-gliedrigen gesättigten oder ungesättigten Ring mit 0-2 zusätzlichen Heteroatomen, die aus O, S und N ausgewählt sind, wobei der Ring gegebenenfalls mit einer oder mehreren aus Halogen, CN, CF₃, -OCF₃, -NO₂ OXO, -(CR¹⁹C²⁰)ₙNR¹⁶N¹⁷, -(CR¹⁹R²⁰)ₙOR¹⁶, -(CR¹⁹R²⁰)ₙS(O)R¹⁶, -(CR¹⁹R²⁰)ₙS(O)₂R¹⁶ und R²¹ ausgewählten Gruppen substituiert ist;
X² Aryl ist;
R¹¹, R¹² und R¹³ unabhängig voneinander H, C₁-C₁₂-Alkyl, C₂-C₈-Alkenyl, C₂-C₈-Alkinyl, Carbocyclyl, Heterocyclyl, Aryl oder Heteroaryl sind;
R¹⁴ und R¹⁵ unabhängig voneinander aus H und C₁-C₁₂-Alkyl ausgewählt sind;
m und n unabhängig voneinander aus 0, 1, 2, 3, 4, 5 und 6 ausgewählt sind;
Y unabhängig aus O, NR¹¹ oder S ausgewählt ist;
worin das Alkyl, Alkenyl, Alkinyl, Carbocyclyl, Heterocyclyl, Aryl und Heteroaryl von R¹, R², R³, R⁴, R⁵, R⁶, X¹, X², R¹¹, R¹², R¹³, R¹⁴ und R¹⁵ jeweils unabhängig voneinander gegebenenfalls mit einer oder mehreren Gruppen substituiert sind, die aus Halogen, CN, CF₃, -OCF₃, -NO₂, OXO, -Si(C₁-C₆-Alkyl), -(CR¹⁹R²⁰)ₙC(=Y')R¹⁶, -(CR¹⁹R²⁰)ₙC(=Y')OR¹⁶, -(CR¹⁹R²⁰)ₙC(=Y')NR¹⁶R¹⁷, -(CR¹⁹R²⁰)ₙNR¹⁶R¹⁷, -(CR¹⁹R²⁰)ₙOR¹⁶, -(CR¹⁹R²⁰)ₙ-SR¹⁶, -(CR¹⁹R²⁰)ₙNR₁₆C(=Y')R¹⁷, -(CR¹⁹R²⁰)ₙNR¹⁶C(=Y')OR¹⁷, -(CR¹⁹R²⁰)ₙNR¹⁸C(=Y')NR¹⁶R¹⁷, -(CR¹⁹R²⁰)ₙNR¹⁷SO₂R¹⁶, -(CR¹⁹R²⁰)ₙOC(=Y')R¹⁶, -(CR¹⁹R²⁰)ₙOC(=Y')OR¹⁶, (CR¹⁹R²⁰)ₙOC(=Y')NR¹⁶R¹⁷ und R²¹ ausgewählt sind;
R¹⁶, R¹⁷ und R¹⁸ unabhängig voneinander H, C₁-C₁₂-Alkyl, C₂-C₈-Alkenyl, C₂-C₈-Alkinyl, Carbocyclyl, Heterocyclyl, Aryl oder Heteroaryl sind, worin das Alkyl, Alkenyl, Alkinyl, Carbocyclyl, Heterocyclyl, Aryl oder Heteroaryl gegebenenfalls mit einer oder mehreren Gruppen substituiert sind, die aus Halogen, CN, -OCF₃, CF₃, -NO₂, C₁-C₆-Alkyl, -OH, -O(C₁-C₆-Alkyl), -NH₂, -NH(C₁-C₆-Alkyl), -N(C₁-C₆-Alkyl)₂, -CO₂H, -CO₂(C₁-C₆-Alkyl) ausgewählt sind;
R¹⁹ und R²⁰ unabhängig voneinander aus Hund C₁-C₁₂-Alkyl ausgewählt sind;
R²¹ C₁-C₁₂-Alkyl, C₂-C₈-Alkenyl, C₂-C₈-Alkinyl, Carbocyclyl, Heterocyclyl, Aryl oder Heteroaryl ist, worin jedes Element von R²¹ gegebenenfalls mit einer oder mehreren Gruppen substituiert ist, die aus Halogen, Oxo, CN, -OCF₃, CF₃, -NO₂, C₁-C₆-Alkyl, -OH, -O(C₁-C₆-Alkyl), -NH₂, -NH(C₁-C₆-Alkyl), -N(C₁-C₆-Alkyl)₂, -CO₂H, -CO₂(C₁-C₆-Alkyl) ausgewählt sind;
die Y' jeweils unabhängig voneinander O, NR²² oder S sind; und
R²² H oder C₁-C₁₂-Alkyl ist.

2. Verbindung nach Anspruch 1, ausgewählt aus den Formeln I-b, I-f und I-g:

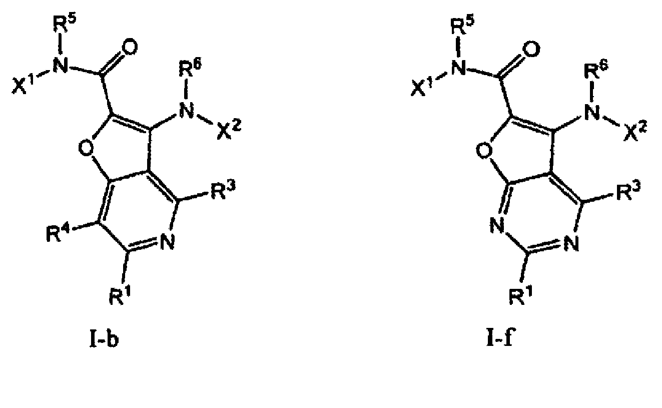

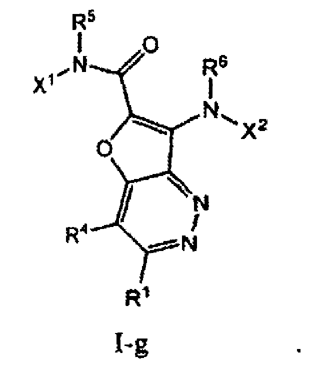

3. Verbindung nach Anspruch 2, worin X¹ aus Folgendem ausgewählt ist:

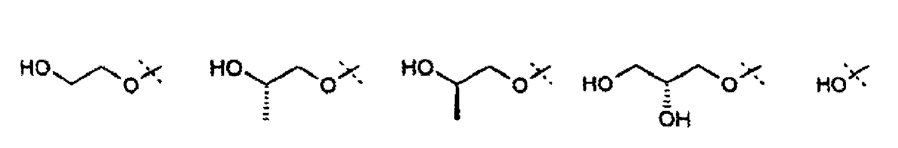

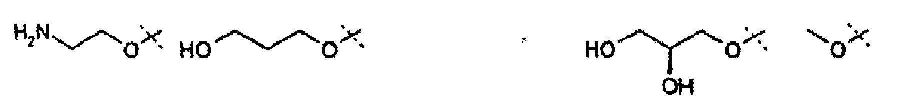

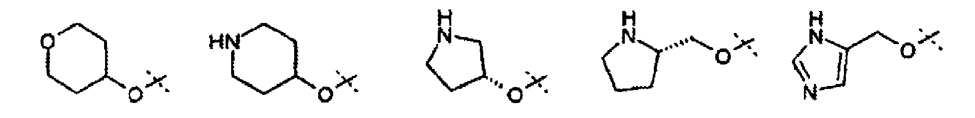

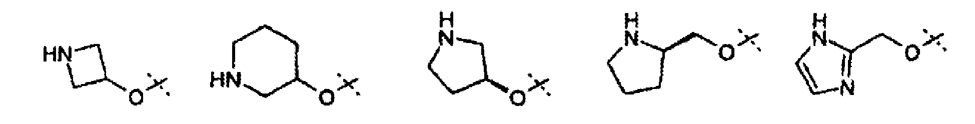

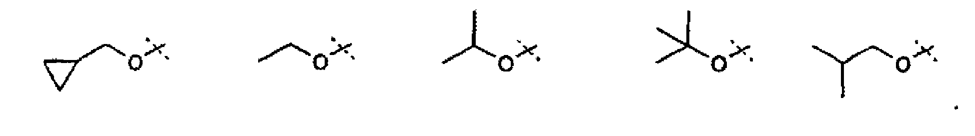

4. Verbindung nach Anspruch 2, worin X¹ aus Folgendem ausgewählt ist:

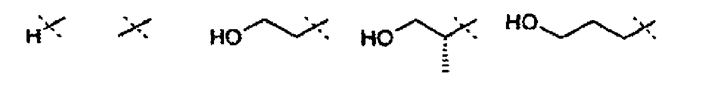

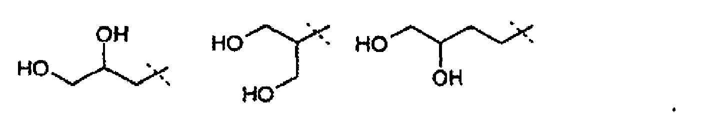

5. Verbindung nach Anspruch 2, worin X¹ R¹¹ ist und R¹¹ und R⁵ zusammen mit dem Stickstoffatom, an das sie gebunden sind, Folgendes bilden:

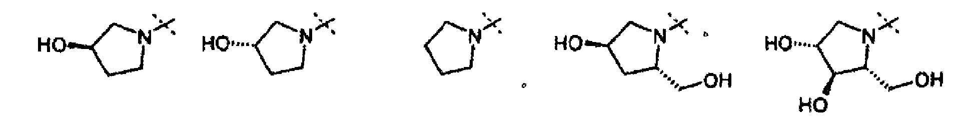

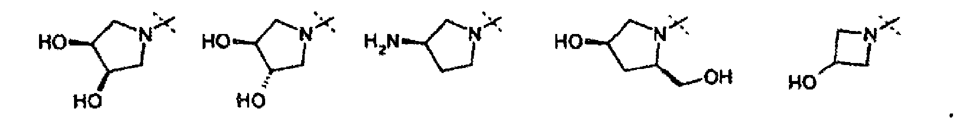

6. Verbindung nach Anspruch 2, worin X² Folgendes ist:

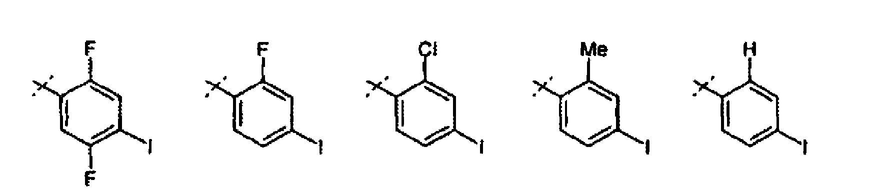

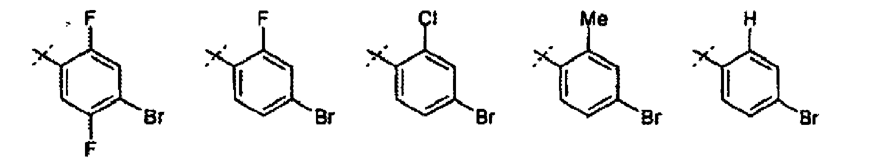

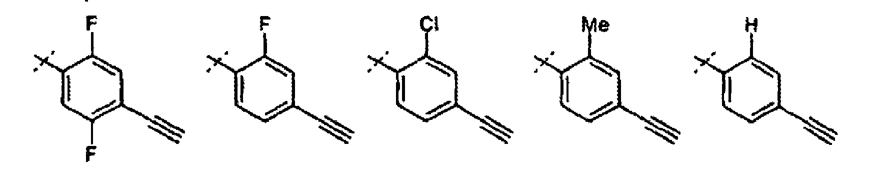

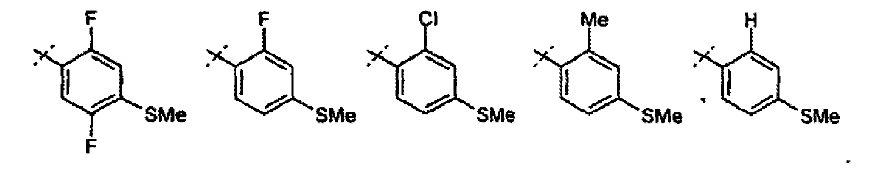

7. Verbindung nach Anspruch 2, worin R¹ aus H, CH₃, CF₃, CN, -NR¹¹R¹², -OR¹¹ und Cl ausgewählt ist.

8. Verbindung nach Anspruch 2, worin R³ aus H, CH₃, F und CF₃ ausgewählt ist.

9. Verbindung nach Anspruch 2, worin R⁴ aus CF₃, Br, Cl, CN, -NR¹¹R¹², -OR¹¹ und -C(=O)NR¹¹R¹² ausgewählt ist.

10. Verbindung nach Anspruch 9, worin R⁴ aus Cl, Br, Me, Et, F, CHF₂, CF₃ oder -OH ausgewählt ist.

11. Verbindung nach Anspruch 2, worin R⁵ H oder Methyl ist.

12. Verbindung nach Anspruch 2, worin R⁶ H oder Methyl ist.

13. Verbindung nach Anspruch 1, worin WOR¹¹ ist.

14. Verbindung nach Anspruch 13, worin W OH ist.

15. Verbindung, ausgewählt aus:
3-(2-Fluor-4-iodphenylamino)furo[3,2-c]pyridin-2-carbonsäure-((R)-2,3-dihydroxypropoxy)amid;
3-(2-Fluor-4-bromphenylamino)furo[3,2-c]pyridin-2-carbonsäure-((R)-2,3-dihydroxypropoxy)amid;
3-(4-Ethinyl-2-fluorphenylamino)furo[3,2-c]pyridin-2-carbonsäure-((R)-2,3-dihydroxypropoxy)amid;
3-(2-Fluor-4-iodphenylamino)furo[3,2-c]pyridin-2-carbonsäurecyclopropylmethoxyamid;
3-(2-F luor-4-iodphenylamino)furo[2,3-c]pyridin-2-carbonsäure-((R)-2,3-dihydroxypropoxy)amid;
3-(2-Fluor-4-iodphenylamino)furo[3,2-c]pyridin-2-carbonsäure(2-hydroxyethoxy)amid;
3-(2-Fluor-4-iodphenylamino)furo[3,2-c]pyridin-2-carbonsäure(2-vinyloxyethoxy)amid;
3-(4-lodphenylamino)furo[3,2-c]pyridin-2-carbonsäure-((R)-2,3-dihydroxypropoxy)-amid;
3-(2-Chlor-4-iodphenylamino)furo[3,2-c]pyridin-2-carbonsäure-((R)-2,3-dihydroxypropoxy)amid;
3-(2,6-Difluor-4-iodphenylamino)furo[3,2-c]pyridin-2-carbonsäure-((R)-2,3-dihydroxypropoxy)amid;
3-(2,5-Difluor-4-iodphenylamino)furo[3,2-c]pyridin-2-carbonsäure-((R)-2,3-dihydroxypropoxy)amid;
4-{[3-(2-Fluor-4-iodphenylam ino)furo[3,2-c]pyridin-2-carbonyl]am inooxy}piperidin-1-carbonsäure-tert-butylester;
3-(2-Fluor-4-iodphenylamino)furo[3,2-c]pyridin-2-carbonsäure-(2-morpholin-4-ylethoxy)amid;
7-Brom-3-(2-fluor-4-iodphenylamino)furo[3,2-c]pyridin-2-carbonsäure-((R)-2,3-dihydroxypropoxy)amid;
5-(2-Fluor-4-iodphenylamino)furo[2,3-d]pyrimidin-6-carbonsäure-((R)-2,3-dihydroxypropoxy)amid;
3-(2-Fluor-4-iodphenylamino)furo[3,2-c]pyridin-2-carbonsäure-(1-methylpiperidin-4-yl-oxy)amid;
3-(2-Fluor-4-iodphenylamino)furo[3,2-c]pyridin-2-carbonsäure-(2-dimethylaminoethoxy)amid;
(2-Fluor-4-iodphenylamino)-N-tert-butoxyfuro[3,2-c]pyridin-2-carboxamid;
(3-(2-Fluor-4-iodphenylamino)furo[3,2-c]pyridin-2-yl)-(1-oxothiazolidin-3-yl)methanon;
(3-(2-Fluor-4-iodphenylamino)furo[3,2-c]pyridin-2-yl)-(1,1-dioxothiomorpholin-4-yl)methanon;
(3-(2,5-Difluor-4-(4-pyrazolyl)phenylamino)furo[3,2-c]pyridin-2-yl)((R)-3-hydroxypyrrolidin-1-yl)methanon;
2-Dimethylcarbamoyl-3-(2-fluor-4-iodphenylamino)furo[3,2-c]pyridin-7-carbonsäureethylester;
3-(2-Fluor-4-iodphenylamino)-7-hydroxymethylfuro[3,2-c]pyridin-2-carbonsäuredimethylamid;
3-(2-Fluor-4-iodphenylamino)-7-phenoxymethylfuro[3,2-c]pyridin-2-carbonsäuredimethylamid;
7-Chlor-3-(2-fluor-4-iodphenylamino)furo[3,2-c]pyridin-2-carbonsäurenatriumsalz;
7-Chlor-3-(2-fluor-4-iodphenylamino)furo[3,2-c]pyridin-2-carbonsäureamid;
3-(2-Fluor-4-iodphenylamino)furo[3,2-c]pyridin-2-carbonsäure-(2-methansulfonylaminoethoxy)amid;
7-Chlor-3-(2-fluor-4-iodphenylamino)furo[3,2-c]pyridin-2-carbonsäure-((R)-2,3-dihydroxypropoxy)amid;
7-Fluor-3-(2-fluor-4-iodphenylamino)furo[3,2-c]pyridin-2-carbonsäure-(2-hydroxyethoxy)amid;
(2-Fluor-4-iodphenylamino)-N-isobutoxyfuro[3,2-c]pyridin-2-carboxamid;
(2-Fluor-4-iodphenylamino)-N-isopropoxyfuro[3,2-c]pyridin-2-carboxamid;
(2-Fluor-4-iodphenylamino)-N-benzyloxyfuro[3,2-c]pyridin-2-carboxamid;
(2-Fluor-4-iodphenylamino)-N-(3-hydroxypropoxy)furo[3,2-c]pyridin-2-carboxamid;
(2-Fluor-4-iodphenylamino)-N-[3-(pyridin-2-carboxamid)propoxy]furo[3,2-c]pyridin-2-carboxamid;
(2-Fluor-4-iodphenylamino)-N-[3-(nicotinamid)propoxy]furo[3,2-c]pyridin-2-carboxamid;
(2-Fluor-4-iodphenylamino)-N-[3-(isonicotinamid)propoxy]furo[3,2-c]pyridin-2-carboxamid;
(2-Fluor-4-iodphenylamino)-N-propoxyfuro[3,2-c]pyridin-2-carboxamid;
(2-Fluor-4-iodphenylamino)-N-ethoxyfuro[3,2-c]pyridin-2-carboxamid;
N-(2-(Benzyloxy)-2-methylpropoxy)-3-(2-fluor-4-iodphenylamino)furo[3,2-c]pyridin-2-carboxamid;
2-Hydoxy-2-methylpropyl-3-(2-fluor-4-iodphenylamino)furo[3,2-c]pyridin-2-carboxylat;
N-(2-Hydroxyethoxy)-3-(4-brom-2-fluorphenylamino)furo[3,2-c]pyridin-2-carboxamid;
3-(2-Fluor-4-iodphenylamino)furo[3,2-c]pyridin-2-carbonsäure-(2-hydroxy-1-methylethoxy)amid;
3-(2-Fluor-4-iodphenylamino)furo[3,2-c]pyridin-2-carbonsäure-(2-hydroxypropoxy)-amid;
3-(2-Fluor-4-iodphenylamino)furo[3,2-c]pyridin-2-carbonsäure-(2-hydroxy-1-hydroxymethylethoxy)amid;
N-[3-(2-Fluor-4-iodphenylamino)furo[3,2-c]pyridin-2-carbonyl]methansulfonamid;
3-(2-Fluor-4-iodphenylamino)furo[3,2-c]pyridin-2-carbonsäure-(2-piperidin-1-yl-ethoxy)amid;
(3-(2-Fluor-4-iodphenylamino)furo[3,2-c]pyridin-2-yl)((R)-3-hydroxypyrrolidin-1-yl)methanon;
(3-(2-Fluor-4-iodphenylamino)furo[3,2-c]pyridin-2-yl)((3S,4S)-3,4-dihydroxypyrrolidin-1-yl)methanon;
3-(2-Fluor-4-iodphenylamino)-N-(3-hydroxypropyl)furo[3,2-c]pyridin-2-carboxamid;
3-(2-Fluor-4-iodphenylamino)-N-(2-methoxyethyl)furo[3,2-c]pyridin-2-carboxamid;
3-(2-Fluor-4-iodphenylamino)-N-isopropylfuro[3,2-c]pyridin-2-carboxamid;
3-(2-Fluor-4-iodphenylamino)-N-(2-cyclopropylethyl)furo[3,2-c]pyridin-2-carboxamid;
3-(2-Fluor-4-iodphenylamino)-N-(3-(dimethylamino)propyl)furo[3,2-c]pyridin-2-carboxamid;
3-(2-Fluor-4-iodphenylamino)-N-(2-(2-hydroxyethoxy)ethyl)furo[3,2-c]pyridin-2-carboxamid;
N-(3-(1H-Imidazol-1-yl)propyl)-3-(2-fluor-4-iodphenylamino)furo[3,2-c]pyridin-2-carboxamid;
3-(2-Fluor-4-iodphenylamino)-N-(1,3-dihydroxypropan-2-yl)furo[3,2-c]pyridin-2-carboxamid;
(3-(2-Fluor-4-iodphenylamino)furo[3,2-c]pyridin-2-yl)(thiazolidin-3-yl)methanon;
(3-(2-Fluor-4-iodphenylamino)furo[3,2-c]pyridin-2-yl)((S)-3-hydroxypyrrolidin-1-yl)methanon;
(3-(4-Brom-2,5-difluorphenylamino)furo[3,2-c]pyridin-2-yl)((R)-3-hydroxypyrrolidin-1-yl)methanon;
(3-(4-Brom-2-fluorphenylamino)furo[3,2-c]pyridin-2-yl)((R)-3-hydroxypyrrolidin-1-yl)-methanon;
(3-(2-Fluor-4-iodphenylamino)furo[3,2-c]pyridin-2-yl)((3S,4R)-3,4-dihydroxypyrrolidin-1-yl)methanon;
(3-(2-Fluor-4-iodphenylamino)furo[3,2-c]pyridin-2-yl)((R)-3-aminopyrrolidin-1-yl)methanon;
(3-(2-Fluor-4-iodphenylamino)furo[3,2-c]pyridin-2-yl)((S)-3-hydroxypiperidin-1-yl)methanon;
3-(2-Fluor-4-iodphenylamino)-N-((S)-1-hydroxypropan-2-yl)furo[3,2-c]pyridin-2-carboxamid;
(3-(2-Fluor-4-iodphenylamino)furo[3,2-c]pyridin-2-yl)-(2-(thiazol-2-yl)pyrrolidin-1-yl)-methanon;
(3-(2-Fluor-4-iodphenylamino)furo[3,2-c]pyridin-2-yl)-(3-(methylsulfonyl)pyrrolidin-1-yl)methanon;
(3-(2-Fluor-4-iodphenylamino)furo[3,2-c]pyridin-2-yl)((S)-2-(hydroxymethyl)pyrrolidin-1-yl)methanon;
(3-(2-Fluor-4-iodphenylamino)furo[3,2-c]pyridin-2-yl)-(2-(hydroxylmethyl)piperidin-1-yl)methanon;
3-(2-Fluor-4-iodphenylamino)-N-(2-hydroxyethyl)-N-methylfuro[3,2-c]pyridin-2-carboxamid;
3-(2-Fluor-4-iodphenylamino)-N-(2-hydroxyethyl)furo[3,2-c]pyridin-2-carboxamid;
3-(2-Fluor-4-iodphenylamino)-N,N-dimethylfuro[3,2-c]pyridin-2-carboxamid;
3-(2-Fluor-4-iodphenylamino)-N-(2,3-dihydroxypropyl)furo[3,2-c]pyridin-2-carboxamid;
3-(2-Fluor-4-iodphenylamino)-N-((R)-1-hydroxypropan-2-yl)furo[3,2-c]pyridin-2-carboxamid;
(3-(2-Fluor-4-iodphenylamino)furo[3,2-c]pyridin-2-yl)-(3-(hydroxylmethyl)piperidin-1-yl)methanon;
(3-(2-Fluor-4-iodphenylamino)furo[3,2-c]pyridin-2-yl)((R)-2-(hydroxylmethyl)pyrrolidin-1-yl)methanon;
(3-(2-Fluor-4-iodphenylamino)furo[3,2-c]pyridin-2-yl)-(4-hydroxypiperidin-1-yl)methanon;
(3-(2-Fluor-4-iodphenylamino)furo[3,2-c]pyridin-2-yl)((R)-3-hydroxypiperidin-1-yl)methanon;
(3-(2-Fluor-4-iodphenylamino)furo[3,2-c]pyridin-2-yl)(morpholino)methanon;
3-(2-Fluor-4-iodphenylamino)-N-methylfuro[3,2-c]pyridin-2-carboxamid;
3-(2-Fluor-4-iodphenylamino)-N-(2-(2,2-dimethyl-1,3-dioxolan-4-yl)ethyl)furo[3,2-c]pyridin-2-carboxamid;
3-(2-Fluor-4-iodphenylamino)-N-(3,4-dihydroxybutyl)furo[3,2-c]pyridin-2-carboxamid;
3-(2-Fluor-4-iodphenylamino)-N-(2-((S)-2,2-dimethyl-1,3-dioxolan-4-yl)ethyl)furo[3,2-c]pyridin-2-carboxamid;
3-(2-Fluor-4-iodphenylamino)-N-((S)-3,4-dihydroxybutyl)furo[3,2-c]pyridin-2-carboxamid;
3-(2-Fluor-4-iodphenylamino)-N-isopentylfuro[3,2-c]pyridin-2-carboxamid;
3-(2-Fluor-4-iodphenylamino)-N-phenyloxyfuro[3,2-c]pyridin-2-carboxamid;
3-(2-Fluor-4-iodphenylamino)-N-(2-hydroxy-2-methylpropoxy)furo[3,2-c]pyridin-2-carboxamid;
N-(1-Hydroxy-2-methylpropan-2-yloxy)-3-(2-fluor-4-iodphenylamino)furo[3,2-c]pyridin-2-carboxamid;
3-(2-Fluor-4-iodphenylamino)-N-(4-hydroxy-2-methylbutan-2-yloxy)furo[3,2-c]pyridin-2-carboxamid;
N-((Pyridin-2-yl)methoxy)-3-(2-fluor-4-iodphenylamino)furo[3,2-c]pyridin-2-carboxamid;
N-(1-Phenylethoxy)-3-(2-fluor-4-iodphenylamino)furo[3,2-c]pyridin-2-carboxamid;
3-(2-Fluor-4-iodphenylamino)furo[3,2-c]pyridin-2-carbonsäure-((R)-2-hydroxypropoxy)amid;
3-(2-Fluor-4-iodphenylamino)furo[3,2-c]pyridin-2-carbonsäure-((S)-2-hydroxypropoxy)amid;
(3-(2-Fluor-4-iodphenylamino)furo[3,2-c]pyridin-2-yl)-(3-hydroxy-3-methylpyrrolidin-1-yl)methanon;
(3-(2-Fluor-4-iodphenylamino)furo[3,2-c]pyridin-2-yl)((3R,4R)-3-amino-4-hydroxypyrrolidin-1-yl)methanon;
(3-(2-Fluor-4-iodphenylamino)furo[3,2-c]pyridin-2-yl)-(3-hydroxyazetidin-1-yl)methanon;
(3-(2-Fluor-4-iodphenylamino)furo[3,2-c]pyridin-2-yl)((2R,3R)-3-hydroxy-2-(hydroxymethyl)pyrrolidin-1-yl)methanon;
(3-(2-Fluor-4-iodphenylamino)furo[3,2-c]pyridin-2-yl)((2R,3R,4R)-3,4-dihydroxy-2-(hydroxymethyl)pyrrolidin-1-yl)methanon;
(3-(2-Fluor-4-iodphenylamino)furo[3,2-c]pyridin-2-yl)(pyrrolidin-1-yl)methanon;
(3-(2-Fluor-4-iodphenylamino)furo[3,2-c]pyridin-2-yl)((2R,4R)-4-hydroxy-2-(hydroxymethyl)pyrrolidin-1-yl)methanon;
(3-(2-Fluor-4-iodphenylamino)furo[3,2-c]pyridin-2-yl)((2S,4R)-4-hydroxy-2-(hydroxymethyl)pyrrolidin-1-yl)methanon;
(3-(2-Fluor-4-iodphenylamino)furo[3,2-c]pyridin-2-yl)((2R,4R)-4-amino-2-(hydroxymethyl)pyrrolidin-1-yl)methanon;
3-(2-Fluor-4-iodphenylamino)-N-(3-hydroxypropyl)-N-methylfuro[3,2-c]pyridin-2-carboxamid;
3-(2-Fluor-4-iodphenylamino)-N-(1H-pyrazol-3-yl)furo[3,2-c]pyridin-2-carboxamid;
(3-(2-Fluor-4-iodphenylamino)furo[3,2-c]pyridin-2-yl)((2R,3S)-3-hydroxy-2-(hydroxymethyl)pyrrolidin-1-yl)methanon;
(3-(2-Fluor-4-iodphenylamino)furo[3,2-c]pyridin-2-yl)((2R,3R,4S)-3,4-dihydroxy-2-(hydroxylmethyl)pyrrolidin-1-yl)methanon;
Azetidin-1-yl-(3-(2-fluor-4-iodphenylamino)furo[3,2-c]pyridin-2-yl)methanon;
(3-(2-Fluor-4-iodphenylamino)furo[3,2-c]pyridin-2-yl)-(3-(hydroxymethyl)azetidin-1-yl)-methanon;
(3-(2-Fluor-4-iodphenylamino)furo[3,2-c]pyridin-2-yl)((2R,3S,4S)-3,4-dihydroxy-2-(hydroxymethyl)pyrrolidin-1-yl)methanon; 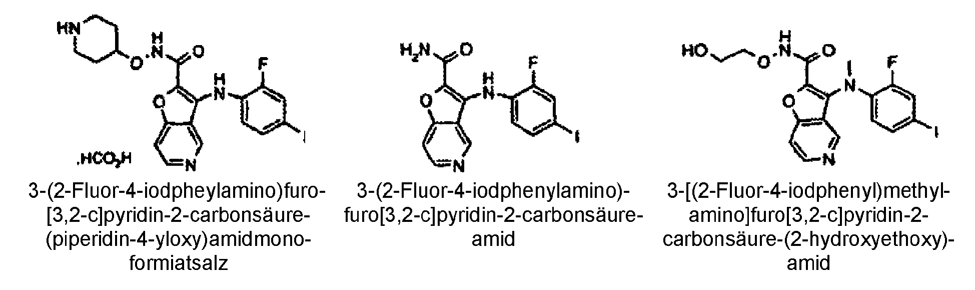 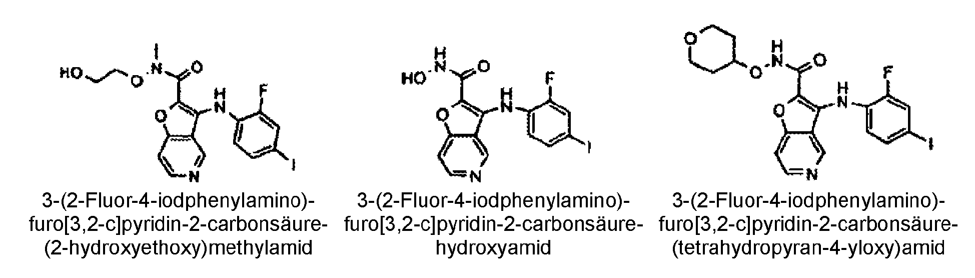 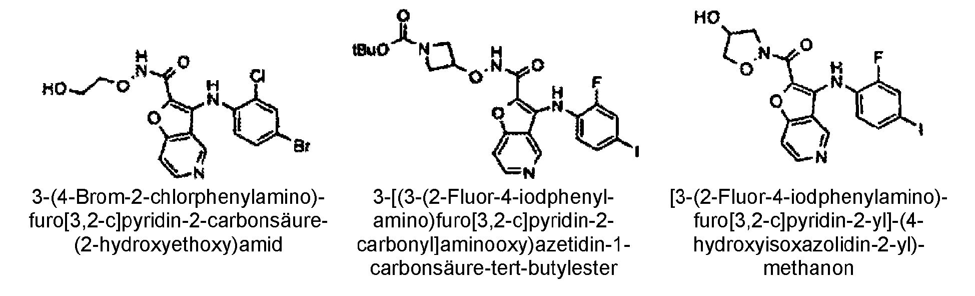 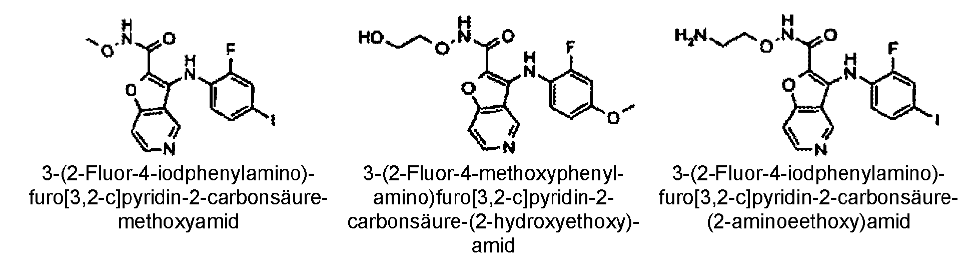 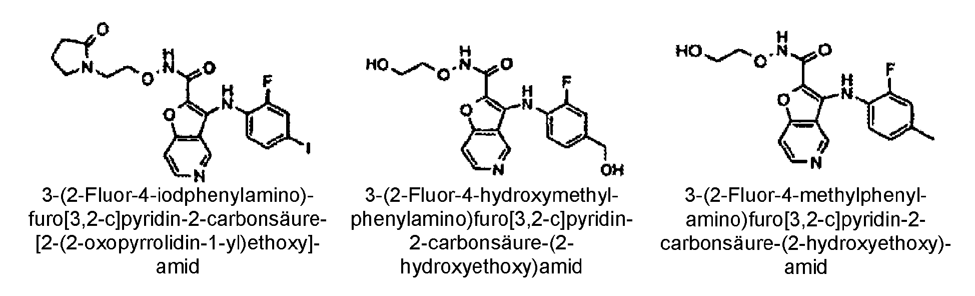 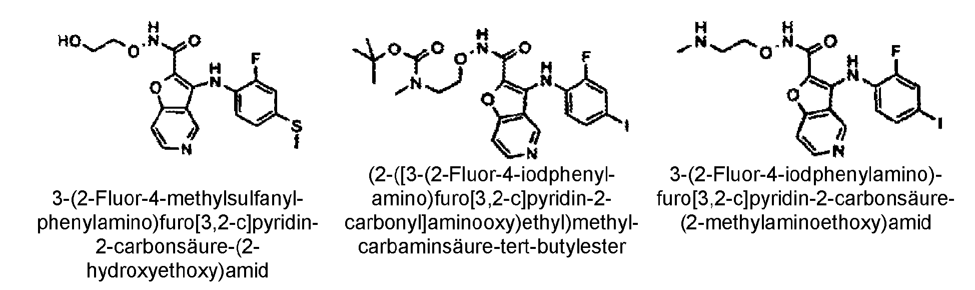 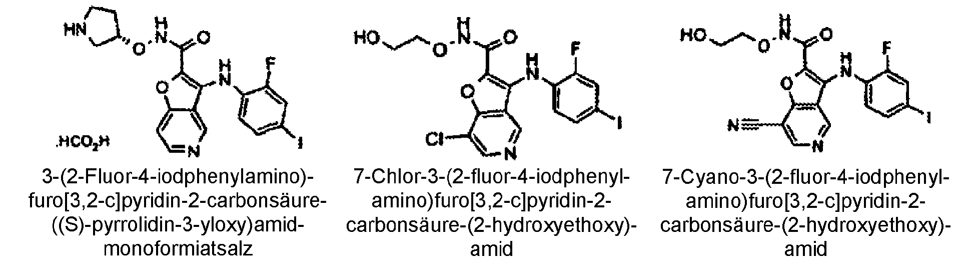 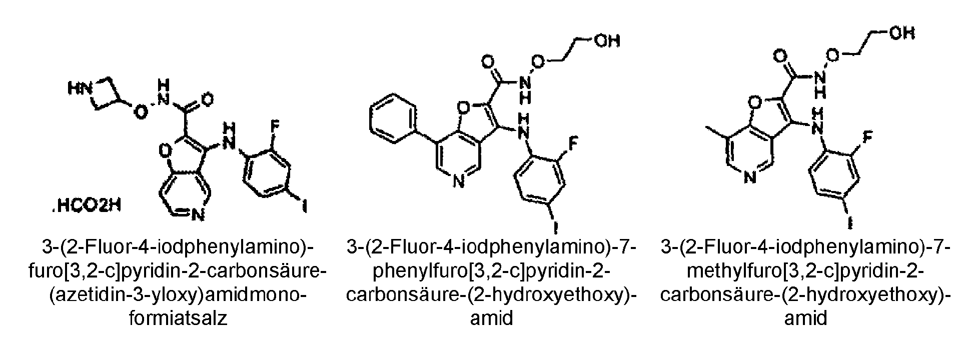 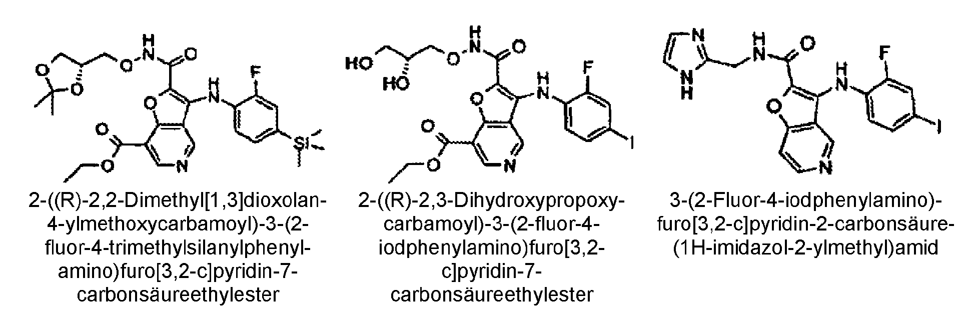 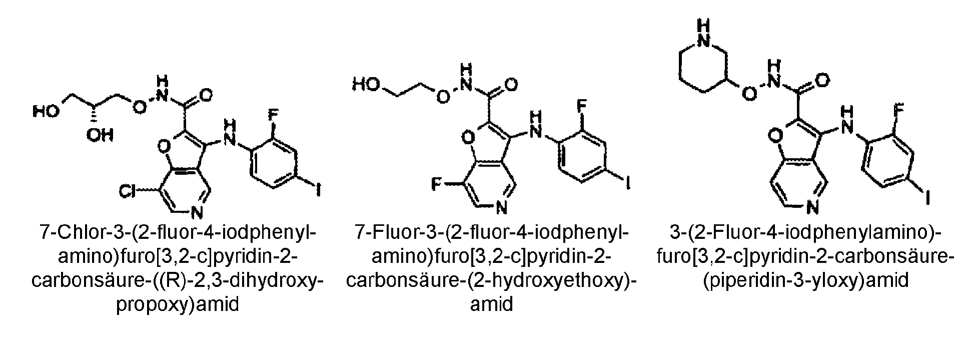 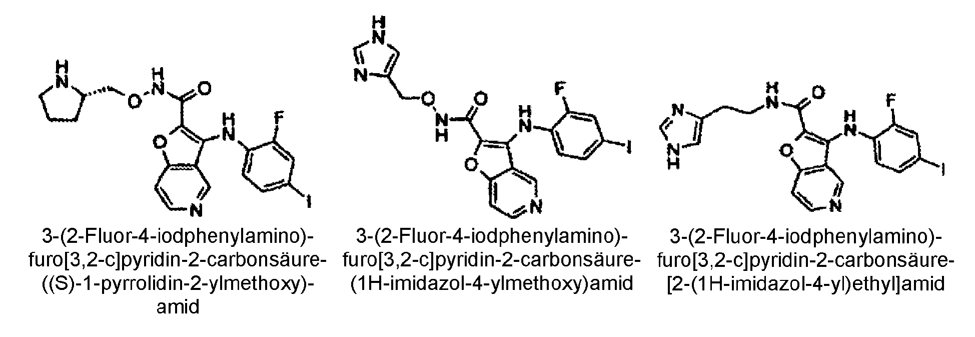 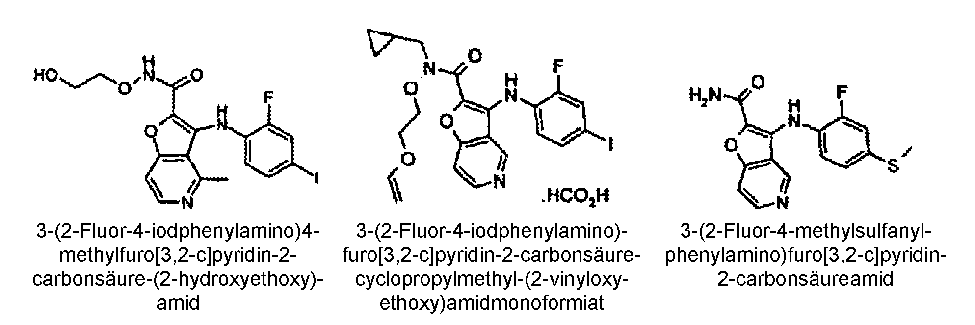 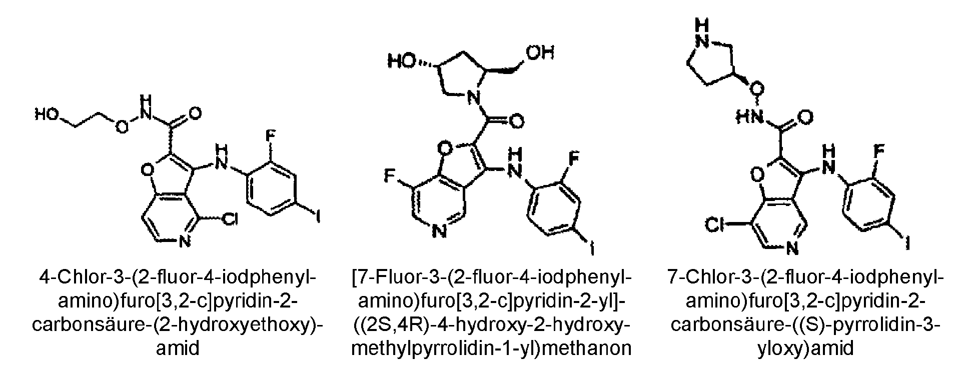 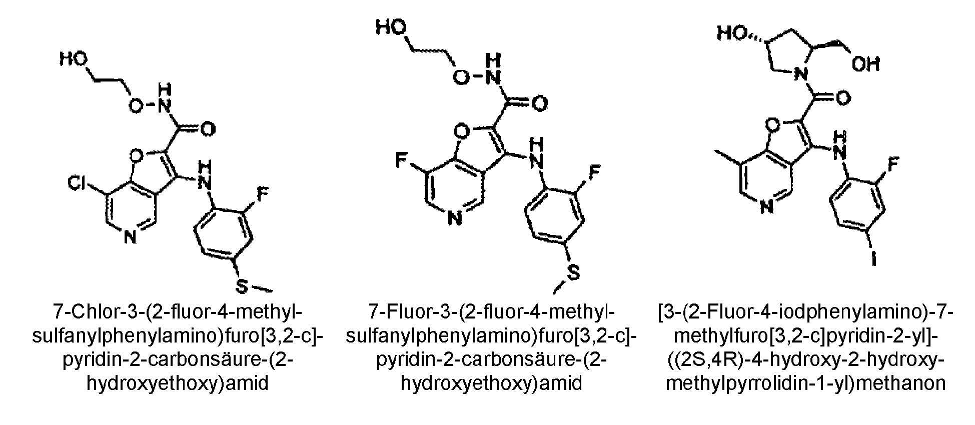 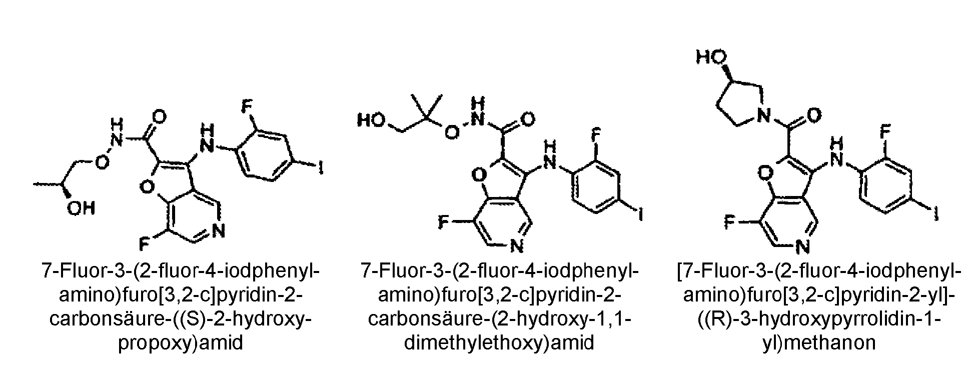 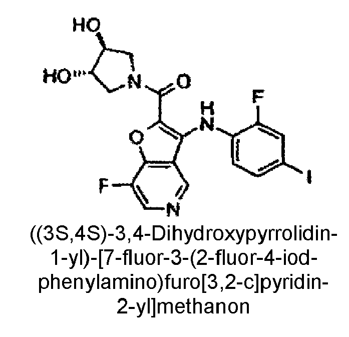 und

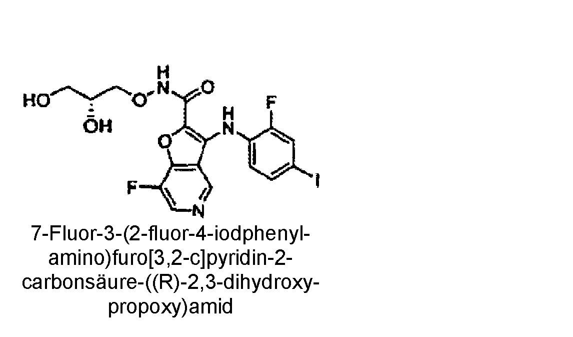

16. Pharmazeutische Zusammensetzung, die eine Verbindung nach einem der Ansprüche 1 bis 15 und einen pharmazeutisch annehmbaren Träger umfasst.

17. Pharmazeutische Zusammensetzung nach Anspruch 16, die weiters ein zweites Chemotherapeutikum oder ein zweites Antiphlogistikum umfasst.

18. Verbindung nach einem der Ansprüche 1 bis 15 zur Verwendung in einem Behandlungsverfahren, wobei das Verfahren zur Hemmung von anomalem Zellwachstum oder zur Behandlung einer hyperproliferativen Störung bei einem Säugetier dient.

19. Verwendung einer Verbindung nach einem der Ansprüche 1 bis 15 und gegebenenfalls eines zweiten Chemotherapeutikums zur Herstellung eines Medikaments zur Hemmung von anomalem Zellwachstum oder zur Behandlung einer hyperproliferativen Störung bei einem Säugetier.

20. Verbindung nach einem der Ansprüche 1 bis 15 zur Verwendung in einem Behandlungsverfahren, wobei das Verfahren zur Behandlung einer Entzündungserkrankung bei einem Säugetier dient.

21. Verwendung einer Verbindung nach einem der Ansprüche 1 bis 15 und gegebenenfalls eines zweiten Antiphlogistikums zur Herstellung eines Medikaments zur Behandlung einer Entzündungserkrankung bei einem Säugetier.

22. Verbindung nach einem der Ansprüche 1 bis 15 zur Verwendung in einem Behandlungsverfahren, wobei das Verfahren zur Behandlung einer Autoimmunerkrankung, destruktiven Knochenerkrankung, proliferativen Erkrankung, Infektionserkrankung, Viruserkrankung, fibrotischen Erkrankung, neurodegenerativen Erkrankung, Pankreatitis oder Nierenerkrankung bei einem Säugetier dient.

23. Verwendung einer Verbindung nach einem der Ansprüche 1 bis 15 und gegebenenfalls eines zweiten Therapeutikums zur Herstellung eines Medikaments zur Behandlung einer Autoimmunerkrankung, destruktiven Knochenerkrankung, proliferativen Erkrankung, Infektionserkrankung, Viruserkrankung, fibrotischen Erkrankung, neurodegenerativen Erkrankung, Pankreatitis oder Nierenerkrankung bei einem Säugetier.

24. Verwendung nach Anspruch 19, 21 oder 23, wobei das zweite Chemotherapeutikum, Antiphlogistikum oder Therapeutikum zur sequentiellen oder nachfolgenden Verabreichung an das Säugetier dient.

## Revendications

1. Composé choisi entre un composé de formule I : 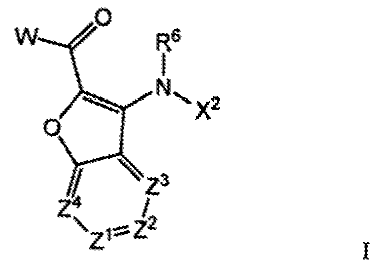 et ses produits de solvatation et ses sels, formule dans laquelle :
Z¹ représente un groupe CR¹
Z² représente un groupe CR² ou N ;
Z³ représente un groupe CR³ ou N ;
Z⁴ représente un groupe CR⁴ ou N ;
où un ou deux de Z², Z³ et Z⁴ représentent N ;
R¹, R², R³ et R⁴ sont choisis indépendamment entre H, des groupes halogéno, CN, CF₃, -OCF₃, -NO₂, -(CR¹⁴R¹⁵)ₙC(=Y)R¹¹, -(CR¹⁴R¹⁵)ₙC(=Y)OR¹¹, -(CR¹⁴R¹⁵)ₙC(=Y)NR¹¹R¹², -(CR¹⁴R¹⁵)ₙNR¹¹R¹², -(CR¹⁴R¹⁵)ₙOR¹¹, -(CR¹⁴R¹⁵)ₙNR¹²C(=Y)R¹¹, alkyle en C₁ à C_{12,} carbocyclyle, hétérocyclyle, aryle et hétéroaryle ;
W représente un groupe 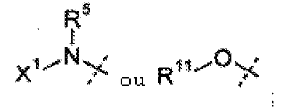
R⁵ et R⁶ sont choisis indépendamment entre H et un groupe alkyle en C₁ à C₁₂ ;
X¹ est choisi entre des groupes R¹¹, -OR¹¹, -S(O)R¹¹ et -S(O)₂R¹¹ ; lorsque X¹ représente un groupe R¹¹ ou -OR¹¹, R¹¹ ou -OR¹¹ de X¹ et -R⁵ sont pris facultativement conjointement avec l'atome d'azote auquel ils sont fixés pour former un noyau tétra- à heptagonal saturé ou insaturé ayant 0 à 2 hétéroatomes supplémentaires choisis entre O, S et N, ledit noyau étant facultativement substitué avec un ou plusieurs groupes choisis entre des groupes halogéno, CN, CF₃, -OC F₃, -NO₂, oxo, -(CR¹⁹R²⁰)ₙNR¹⁶R¹⁷, -(CR¹⁹R²⁰)ₙOR¹⁶, -(CR¹⁹R²⁰)ₙS(O)R¹⁶, -(CR¹⁹R²⁰)ₙS(O)₂R¹⁶, et R²¹ ;
X² représente un groupe aryle ;
R¹¹, R¹² et R¹³ représentent indépendamment H, un groupe alkyle en C₁ à C₁₂, alcényle en C₂ à C₈, alcynyle en C₂ à C₈, carbocyclyle, hétérocyclyle, aryle ou hétéroaryle ;
R¹⁴ et R¹⁵ sont choisis indépendamment entre H et un groupe alkyle en C₁ à C₁₂ ;
m et n sont choisis indépendamment entre 0, 1, 2, 3, 4, 5 et 6 ;
Y représente indépendamment O, un groupe NR¹¹ ou S ;
où chacun desdits groupes alkyle, alcényle, alcynyle, carbocyclyle, hétérocyclyle, aryle et hétéroaryle de R¹, R², R³, R⁴, R⁵, R⁶, X¹, X², R¹¹, R¹², R¹³, R¹⁴ et R¹⁵ est facultativement substitué indépendamment avec un ou plusieurs groupes choisis indépendamment entre des groupes halogéno, CN, CF₃, -OCF₃, -NO₂, oxo, -Si (alkyle en C₁ à C₆), -(CR¹⁹R²⁰)ₙ-C(=Y')R¹⁶, -(CR¹⁹R²⁰)ₙC(=Y)OR¹⁶, -(CR¹⁹R²⁰)ₙC(=Y')NR¹⁶R¹⁷, -(CR¹⁹R²⁰)ₙNR¹⁶R¹⁷, -(CR¹⁹R²⁰)ₙ-OR¹⁶, -(CR¹⁹R^{2o})ₙ-SR¹⁶, -(CR¹⁹R²⁰ )ₙ-NR¹⁶C(=Y')R¹⁷, -(CR¹⁹R²⁰)ₙNR¹⁶C(=Y')OR¹⁷, -(CR¹⁹R²⁰)ₙNR¹⁸C(=Y')-NR¹⁶R¹⁷, -(CR¹⁹R²⁰)ₙ-NR¹⁷SO²R¹⁶, -(CR¹⁹R²⁰)ₙOC(=Y')R¹⁶, -(CR¹⁹R²⁰)ₙ-OC(=Y')OR¹⁶, -(CR¹⁹R²⁰)ₙOC(=Y')NR¹⁶R¹⁷ et R²¹ ;
chacun des groupes R¹⁶, R¹⁷ et R¹⁸ représente indépendamment H, un groupe alkyle en C₁ à C₁₂, alcényle en C₂ à C₈, alcynyle en C₂ à C₈, carbocyclyle, hétérocyclyle, aryle ou hétéroaryle, où ledit groupe alkyle, alcényle, alcynyle, carbocyclyle, hétérocyclyle, aryle ou hétéroaryle est facultativement substitué avec un ou plusieurs groupes choisis entre des groupe halogéno, CN, -OCF₃, CF₃, -NO₂, alkyle en C₁ à C₆, -OH, -O(alkyle en C₁ à C₆), -NH₂, -NH-(alkyle en C₁ à C₆), -N(alkyle en C₁ à C₆)₂, -CO₂H et -CO₂-(alkyle en C₁ à C₆) ;
R¹⁹ et R²⁰ sont choisis indépendamment entre H et un groupe alkyle en C₁ à C₁₂ ;
R²¹ représente un groupe alkyle en C₁ à C₁₂, alcényle en C₂ à C₈, alcynyle en C₂ à C₈, carbocyclyle, hétérocyclyle, aryle ou hétéroaryle, où chaque membre de R²¹ est facultativement substitué avec un ou plusieurs groupes choisis entre des groupes halogéno, oxo, CN, -OCF₃, CF₃, -NO₂, alkyle en C₁ à C₆, -OH, -O(alkyle en C₁ à C₆), -NH₂, -NH(alkyle en C₁ à C₆) , -N(alkyle en C₁ à C₆)₂, -CO₂H et -CO₂(alkyle en C₁ à C₆) ;
chaque groupe Y' représente indépendamment H, un groupe NR²² ou S ; et
R²² représente H ou un groupe alkyle en C₁ à C₁₂.

2. Composé suivant la revendication 1, choisi parmi les formules I-b, I-f et I-g :

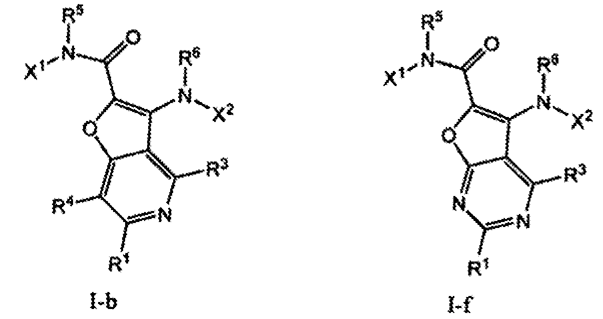

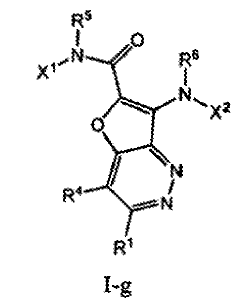

3. Composé suivant la revendication 2, dans lequel X¹ est choisi entre des groupes :

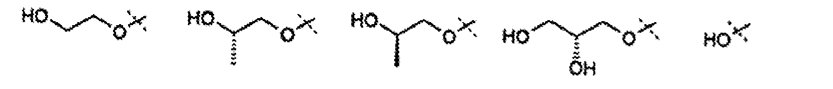

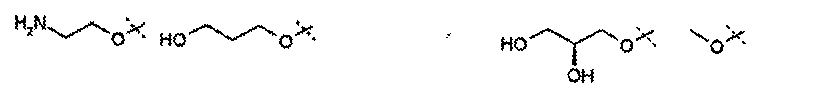

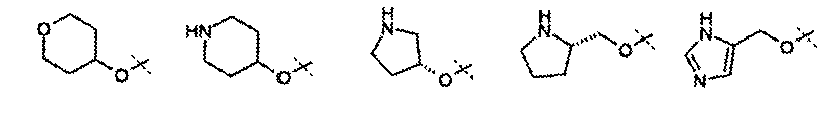

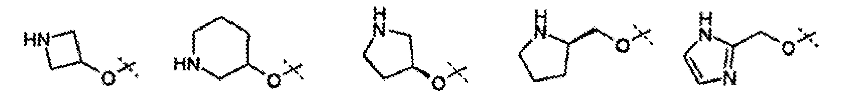

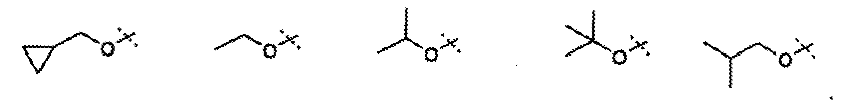

4. Composé suivant la revendication 2, dans lequel X¹ est choisi entre des groupes :

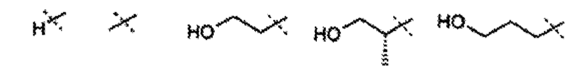

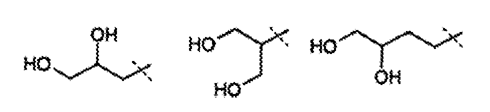

5. Composé suivant la revendication 2, dans lequel X¹ représente R¹¹, et R¹¹ et R⁵ sont pris conjointement avec l'atome d'azote auquel ils sont fixés pour former un groupe :

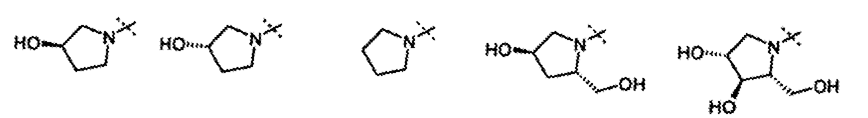

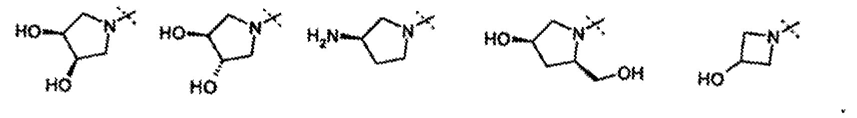

6. Composé suivant la revendication 2, dans lequel X² représente un groupe :

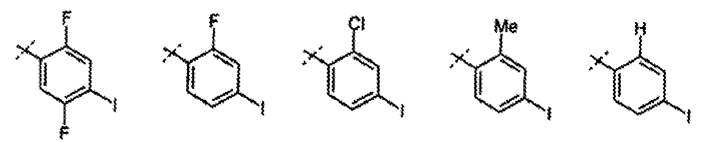

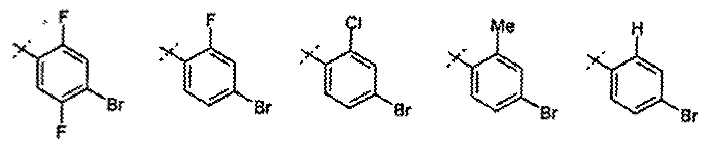

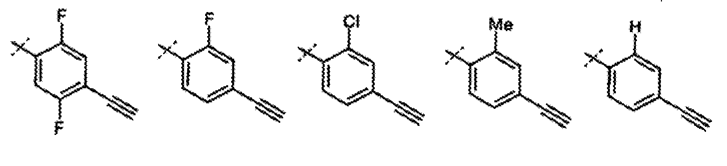

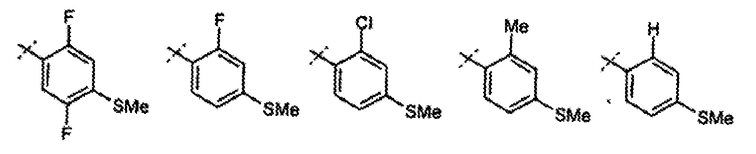

7. Composé suivant la revendication 2, dans lequel R¹ est choisi entre H, CH₃, CF₃, CN, -NR¹¹R¹², -OR¹¹ et -C(=O=)NR¹¹R¹².

8. Composé suivant la revendication 2, dans lequel R³ est choisi entre H, CH₃, F ou CF₃.

9. Composé suivant la revendication 2, dans lequel R⁴ est choisi entre CF₃, Br, Cl, CN, -NR¹¹R¹², -OR¹¹ et -C(=O)NR¹¹R¹².

10. Composé suivant la revendication 9, dans lequel R⁴ est choisi entre Cl, Br, Me, Et, F, CHF₂, CF₃ et -OH.

11. Composé suivant la revendication 2, dans lequel R⁵ représente H ou un groupe méthyle.

12. Composé suivant la revendication 2, dans lequel R⁶ représente H ou un groupe méthyle.

13. Composé suivant la revendication 1, dans lequel W représente un groupe OR¹¹.

14. Composé suivant la revendication 1, dans lequel W représente un groupe OH.

15. Composé choisi entre :
le ((R)-2,3-dihydroxy-propoxy)-amide d'acide 3-(2-fluoro-4-iodo-phénylamino)-furo[3,2-c]pyridine-2-carboxylique ;
le ((R)-2,3-dihydroxy-propoxy)-amide d'acide 3-(2-fluoro-4-bromo-phénylamino)-furo[3,2-c]pyridine-2-carboxylique ;
le ((R)-2,3-dihydroxy-propoxy)-amide d'acide 3-(4-éthynyl-2-fluoro-phénylamino)-furo[3,2-c]pyridine-2-carboxylique ;
le cyclopropylméthoxy-amide d'acide 3-(2-fluoro-4-iodophénylamino)-furo[3,2-c]pyridine-2-carboxylique ;
le ((R)-2,3-dihydroxy-propoxy)-amide d'acide 3-(2-fluoro-4-iodo-phénylamino)-furo[2,3-c]pyridine-2-carboxylique ;
le (2-hydroxy-éthoxy)-amide d'acide 3-(2-fluoro-4-iodophénylamino)-furo[3,2-c]pyridine-2-carboxylique ;
le (2-vinyloxy-éthoxy)-amide d'acide 3-(2-fluoro-4-iodophénylamino)-furo[3,2-c]pyridine-2-carboxylique ;
le ((R)-2,3-dihydroxy-propoxy)-amide d'acide 3-(4-iodophénylamino)-furo[3,2-c]pyridine-2-carboxylique ;
le ((R)-2,3-dihydroxy-propoxy)-amide d'acide 3-(2-chloro-4-iodo-phénylamino)-furo[3,2-c]pyridine-2-carboxylique ;
le ((R)-2,3-dihydroxy-propoxy)-amide d'acide 3-(2,6-difluoro-4-iodo-phénylamino)-furo[3,2-c]pyridine-2-carboxylique ;
le ((R)-2,3-dihydroxy-propoxy)-amide d'acide 3-(2,5-difluoro-4-iodo-phénylamino)-furo[3,2-c]pyridine-2-carboxylique ;
l'ester tertiobutylique d'acide 4-{[3-(2-fluoro-4-iodophénylamino)-furo[3,2-c]pyridine-2-carbonyl]-aminooxy}-pipéridine-1-carboxylique ;
le (2-morpholine-4-yl-éthoxy)-amide d'acide 3-(2-fluoro-4-iodo-phénylamino)-furo[3,2-c]pyridine-2-carboxylique ;
le ((R)-2,3-dihydroxy-propoxy)-amide d'acide 7-bromo-3-(2-fluoro-4-iodo-phénylamino)-furo[3,2-c]pyridine-2-carboxylique ;
le ((R)-2,3-dihydroxy-propoxy)-amide d'acide 5-(2-fluoro-4-iodo-phénylamino)-furo[2,3-d]pyrimidine-6-carboxylique ;
le (1-méthylpipéridine-4-yloxy)-amide d'acide 3-(2-fluoro-4-iodophénylamino)-furo[3,2-c]pyridine-2-carboxylique ;
le (2-diméthylaminoéthoxy)-amide d'acide 3-(2-fluoro-4-iodo-phénylamino)-furo[2,3-d]pyridine-2-carboxylique ;
le (2-fluoro-4-iodo-phénylamino)-N-tertiobutoxyfuro-[3,2-c]pyridine-2-carboxamide ;
la (3-(2-fluoro-4-iodophénylamino)furo[3,2-c]pyridine-2-yl)(1-oxothiazolidine-3-yl)méthanone ;
la (3-(2-fluoro-4-iodophénylamino)furo[3,2-c]pyridine-2-yl)(l,l-dioxothiomorpholine-4-yl)méthanone ;
la (3-(2,5-difluoro-4-(4-pyrazolyl)phénylamino)furo-[3,2-c]pyridine-2-yl)((R)-3-hydroxypyrrolidine-1-yl)méthanone ;
l'ester éthylique d'acide 2-diméthylcarbamoyl-3-(2-fluoro-4-iodo-phénylamino)-furo[3,2-c]pyridine-7-carboxylique ;
le diméthylamide d'acide 3-(2-fluoro-4-iodo-phénylamino)-7-hydroxyméthyl-furo[3,2-c]pyridine-2-carboxylique ;
le diméthylamide d'acide 3-(2-fluoro-4-iodo-phénylamino)-7-phénoxyméthyl-furo[3,2-c]pyridine-2-carboxylique ;
le sel de sodium d'acide 7-chloro-3-(2-fluoro-4-iodophénylamino)-furo[3,2-c]pyridine-2-carboxylique ;
l'amide d'acide 7-chloro-3-(2-fluoro-4-iodo-phénylamino)-furo[3,2-c]pyridine-2-carboxylique ;
le (2-méthanesulfonylamino-éthoxy)-amide d'acide 3-(2-fluoro-4-iodo-phénylamino)-furo[3,2-c]pyridine-2-carboxylique ;
le ((R)-2,3-dihydroxy-propoxy)-amide d'acide 7-chloro-3-(2-fluoro-4-iodo-phénylamino)-furo[3,2-c]pyridine-2-carboxylique ;
le (2-hydroxyéthoxy)-amide d'acide 7-fluoro-3-(2-fluoro-4-iodophénylamino)-furo[3,2-c]pyridine-2-carboxylique ;
le (2-fluoro-4-iodo-phénylamino)-N-isobutoxyfuro[3,2-c]-pyridine-2-carboxamide ;
le (2-fluoro-4-iodo-phénylamino)-N-isopropoxyfuro[3,2-c]-pyridine-2-carboxamide ;
le (2-fluoro-4-iodo-phénylamino)-N-benzyloxyfuro[3,2-c]-pyridine-2-carboxamide ;
le (2-fluoro-4-iodo-phénylamino)-N-(3-hydroxy-propoxy)-furo[3,2-c]pyridine-2-carboxamide ;
le (2-fluoro-4-iodo-phénylamino)-N-[3-(pyridine-2-carboxamide)-propoxy]-furo[3,2-c]pyridine-2-carboxamide ;
le (2-fluoro-4-iodo-phénylamino)-N-[3-(nicotinamide)-propoxy]-furo[3,2-c]pyridine-2-carboxamide ;
le (2-fluoro-4-iodo-phénylamino)-N-[3-(isonicotinamide)-propoxy]-furo[3,2-c]pyridine-2-carboxamide ;
le (2-fluoro-4-iodo-phénylamino)-N-propoxyfuro[3,2-c]-pyridine-2-carboxamide ;
le (2-fluoro-4-iodo-phénylamino)-N-éthoxyfuro[3,2-c]-pyridine-2-carboxamide ;
le N-(2-(benzyloxy)-2-méthylpropoxy)-3-(2-fluoro-4-iodo-phénylamino)-furo[3,2-c]pyridine-2-carboxamide ;
le 2-hydroxy-2-méthylpropyl-3-(2-fluoro-4-iodo-phénylamino)-furo[3,2-c]pyridine-2-carboxamide ;
le N-(2-hydroxyéthoxy)-3-(4-bromo-2-fluorophényl-amino)-furo[3,2-c]pyridine-2-carboxamide ;
le (2-hydroxy-1-méthyl-éthoxy)-amide d'acide 3-(2-fluoro-4-iodo-phénylamino)-furo[3,2-c]pyridine-2-carboxylique ;
le (2-hydroxy-propoxy)-amide d'acide 3-(2-fluoro-4-iodophénylamino)-furo[3,2-c]pyridine-2-carboxylique ;
le (2-hydroxy-1-hydroxyméthyl-éthoxy)-amide d'acide 3-(2-fluoro-4-iodo-phénylamino)-furo[3,2-c]pyridine-2-carboxylique ;
le N-[3-(2-fluoro-4-iodo-phénylamino)-furo[3,2-c]pyridine-2-carbonyl]-méthanesulfonamide ;
le (2-pipéridine-1-yl-éthoxy)-amide d'acide 3-(2-fluoro-4-iodo-phénylamino)-furo[3,2-c]pyridine-2-carboxylique ;
la (3-(2-fluoro-4-iodophénylamino)-furo[3,2-c]pyridine-2-yl)((R)-3-hydroxypyrrolidine-1-yl)-méthanone ;
la (3-(2-fluoro-4-iodophénylamino)-furo[3,2-c]pyridine-2-yl)((3S,4S)-3,4-dihydroxypyrrolidine-1-yl)-méthanone ;
le 3-(2-fluoro-4-iodophénylamino)-N-(3-hydroxypropyl)-furo[3,2-c]pyridine-2-carboxamide ;
le 3-(2-fluoro-4-iodophénylamino)-N-(2-méthoxyéthyl)-furo[3,2-c]pyridine-2-carboxamide ;
le 3-(2-fluoro-4-iodophénylamino)-N-isopropylfuro[3,2-c]-pyridine-2-carboxamide ;
le 3-(2-fluoro-4-iodophénylamino)-N-(2-cyclopropyléthyl)-furo[3,2-c]pyridine-2-carboxamide ;
le 3-(2-fluoro-4-iodophénylamino)-N-(3-(diméthylamino)-propyl)-furo[3,2-c]pyridine-2-carboxamide ;
le 3-(2-fluoro-4-iodophénylamino)-N-(2-(2-hydroxyéthoxy)-éthyl)-furo[3,2-c]pyridine-2-carboxamide ;
le N-(3-(1H-imidazole-1-yl)-propyl)-3-(2-fluoro-4-iodophénylamino)-furo[3,2-c]pyridine-2-carboxamide ;
le 3-(2-fluoro-4-iodophénylamino)-N-(1,3-dihydroxypropane-2-yl)-furo[3,2-c]pyridine-2-carboxamide ;
la (3-(2-fluoro-4-iodophénylamino)furo[3,2-c]pyridine-2-yl)(thiazolidine-3-yl)méthanone ;
la (3-(2-fluoro-4-iodophénylamino)furo[3,2-c]pyridine-2-yl)((S)-3-hydroxy-pyrrolidine-1-yl)méthanone ;
la (3-(4-bromo-2,5-difluorophénylamino)furo[3,2-c]-pyridine-2-yl)((R)-3-hydroxypyrrolidine-1-yl)méthanone ;
la (3-(4-bromo-2-fluorophénylamino)furo[3,2-c]pyridine-2-yl)((R)-3-hydroxypyrrolidine-1-yl)méthanone ;
la (3-(2-fluoro-4-iodophénylamino)furo[3,2-c]pyridine-2-yl)((3S,4R)-3,4-dihydroxypyrrolidine-1-yl)méthanone ;
la (3-(2-fluoro-4-iodophénylamino)furo[3,2-c]pyridine-2-yl)((R)-3-aminopyrrolidine-1-yl)méthanone ;
la (3-(2-fluoro-4-iodophénylamino)furo[3,2-c]pyridine-2-yl)((S)-3-hydroxypipéridine-1-yl)méthanone ;
le 3-(2-fluoro-4-iodophénylamino)-N-((S)-1-hydroxypropane-2-yl)furo[3,2-c]pyridine-2-carboxamide ;
la (3-(2-fluoro-4-iodophénylamino)furo[3,2-c]pyridine-2-yl)(2-(thiazole-2-yl)pyrrolidine-1-yl)méthanone ;
la (3-(2-fluoro-4-iodophénylamino)furo[3,2-c]pyridine-2-yl)(3-(méthylsulfonyl)pyrrolidine-1-yl)méthanone ;
la (3-(2-fluoro-4-iodophénylamino)furo[3,2-c]pyridine-2-yl)((S)-2-(hydroxyméthyl)pyrrolidine-1-yl)méthanone ;
la (3-(2-fluoro-4-iodophénylamino)furo[3,2-c]pyridine-2-yl)(2-(hydroxylméthyl)pipéridine-1-yl)méthanone ;
le 3-(2-fluoro-4-iodophénylamino)-N-(2-hydroxyéthyl)-N-méthylfuro[3,2-c]pyridine-2-carboxamide ;
le 3-(2-fluoro-4-iodophénylamino)-N-(2-hydroxyéthyl)-furo[3,2-c]pyridine-2-carboxamide ;
le 3-(2-fluoro-4-iodophénylamino)-N,N-diméthylfuro[3,2-c]-pyridine-2-carboxamide ;
le 3-(2-fluoro-4-iodophénylamino)-N-(2,3-dihydroxypropyl)-furo[3,2-c]pyridine-2-carboxamide ;
le 3-(2-fluoro-4-iodophénylamino)-N-((R)-1-hydroxypropane-2-yl)furo[3,2-c]pyridine-2-carboxamide ;
la (3-(2-fluoro-4-iodophénylamino)furo[3,2-c]pyridine-2-yl)(3-(hydroxylméthyl)pipéridine-1-yl)méthanone ;
la (3-(2-fluoro-4-iodophénylamino)furo[3,2-c]pyridine-2-yl)((R)-2-(hydroxylméthyl)pyrrolidine-1-yl)méthanone ;
la (3-(2-fluoro-4-iodophénylamino)furo[3,2-c]pyridine-2-yl)(4-hydroxypipéridine-1-yl)méthanone ;
la (3-(2-fluoro-4-iodophénylamino)furo[3,2-c]pyridine-2-yl)((R)-3-hydroxypipéridine-1-yl)méthanone ;
la (3-(2-fluoro-4-iodophénylamino)furo[3,2-c]pyridine-2-yl)(morpholino)méthanone ;
le 3-(2-fluoro-4-iodophénylamino)-N-méthylfuro[3,2-c]-pyridine-2-carboxamide ;
le 3-(2-fluoro-4-iodophénylamino)-N-(2-(2,2-diméthyl-1,3-dioxolanne-4-yl)éthyl)furo[3,2-c]pyridine-2-carboxamide ;
le 3-(2-fluoro-4-iodophénylamino)-N-(3,4-dihydroxybutyl)furo[3,2-c]pyridine-2-carboxamide ;
le 3-(2-fluoro-4-iodophénylamino)-N-(2-((S)-2,2-diméthyl-1,3-dioxolanne-4-yl)éthyl)furo[3,2-c]pyridine-2-carboxamide ;
le 3-(2-fluoro-4-iodophénylamino)-N-((S)-3,4-dihydroxybutyl)furo[3,2-c]pyridine-2-carboxamide ;
le 3-(2-fluoro-4-iodo-phénylamino)-N-isopentylfuro-[3,2-c]pyridine-2-carboxamide ;
le 3-(2-fluoro-4-iodo-phénylamino)-N-phényloxyfuro-[3,2-c]pyridine-2-carboxamide ;
le 3-(2-fluoro-4-iodophénylamino)-N-(2-hydroxy-2-méthylpropoxy)furo[3,2-c]pyridine-2-carboxamide ;
le N-(1-hydroxy-2-méthylpropane-2-yloxy)-3-(2-fluoro-4-iodo-phénylamino)-furo[3,2-c]pyridine-2-carboxamide ;
le 3-(2-fluoro-4-iodo-phénylamino)-N-(4-hydroxy-2-méthylbutane-2-yloxy)furo[3,2-c]pyridine-2-carboxamide ;
le N-((pyridine-2-yl)méthoxy)-3-(2-fluoro-4-iodo-phénylamino)furo[3,2-c]pyridine-2-carboxamide ;
le N-(1-phényléthoxy)-3-(2-fluoro-4-iodo-phénylamino)-furo[3,2-c]pyridine-2-carboxamide ;
le ((R)-2-hydroxy-propoxy)-amide d'acide 3-(2-fluoro-4-iodo-phénylamino)-furo[3,2-c]pyridine-2-carboxylique ;
le ((S)-2-hydroxy-propoxy)-amide d'acide 3-(2-fluoro-4-iodo-phénylamino)-furo[3,2-c]pyridine-2-carboxylique ;
la (3-(2-fluoro-4-iodophénylamino)furo[3,2-c]pyridine-2-yl)(3-hydroxy-3-méthylpyrrolidine-1-yl)méthanone ;
la (3-(2-fluoro-4-iodophénylamino)furo[3,2-c]pyridine-2-yl)((3R,4R)-3-amino-4-hydroxypyrrolidine-1-yl)méthanone ;
la (3-(2-fluoro-4-iodophénylamino)furo[3,2-c]pyridine-2-yl)(3-hydroxyazétidine-1-yl)méthanone ;
la (3-(2-fluoro-4-iodophénylamino)furo[3,2-c]pyridine-2-yl)((2R,3R)-3-hydroxy-2-(hydroxyméthyl)pyrrolidine-1-yl)-méthanone ;
la (3-(2-fluoro-4-iodophénylamino)furo[3,2-c]pyridine-2-yl)((2R,3R,4R)-3,4-dihydroxy-2-(hydroxyméthyl)pyrrolidine-1-yl)méthanone ;
la (3-(2-fluoro-4-iodophénylamino)furo[3,2-c]pyridine-2-yl)(pyrrolidine-1-yl)méthanone ;
la (3-(2-fluoro-4-iodophénylamino)furo[3,2-c]pyridine-2-yl)((2R,4R)-4-hydroxy-2-(hydroxyméthyl)pyrrolidine-1-yl)-méthanone ;
la (3-(2-fluoro-4-iodophénylamino)furo[3,2-c]pyridine-2-yl)((2S,4R)-4-hydroxy-2-(hydroxyméthyl)pyrrolidine-1-yl)-méthanone ;
la (3-(2-fluoro-4-iodophénylamino)furo[3,2-c]pyridine-2-yl)((2R,4R)-4-amino-2-(hydroxyméthyl)pyrrolidine-1-yl)-méthanone ;
le 3-(2-fluoro-4-iodophénylamino)-N-(3-hydroxypropyl)-N-méthylfuro[3,2-c]pyridine-2-carboxamide ;
le 3-(2-fluoro-4-iodophénylamino)-N-(1H-pyrazole-3-yl)-furo[3,2-c]pyridine-2-carboxamide ;
la 3-(2-fluoro-4-iodophénylamino)furo[3,2-c]pyridine-2-yl)((2R,3S)-3-hydroxy-2-(hydroxyméthyl)pyrrolidine-1-yl)-méthanone ;
la 3-(2-fluoro-4-iodophénylamino)furo[3,2-c]pyridine-2-yl)((2R,3R,4S)-3,4-dihydroxy-2-(hydroxylméthyl)pyrrolidine-1-yl)méthanone ;
l'azéridine-1-yl-(3-(2-fluoro-4-iodophénylamino)furo-[3,2-c]pyridine-2-yl)méthanone ;
la 3-(2-fluoro-4-iodophénylamino)furo[3,2-c]pyridine-2-yl)(3-(hydroxyméthyl)azétidine-1-yl)méthanone ;
la 3-(2-fluoro-4-iodophénylamino)furo[3,2-c]pyridine-2-yl)((2R,3S,4S)-3,4-dihydroxy-2-(hydroxyméthyl)pyrrolidine-1-yl)méthanone ; 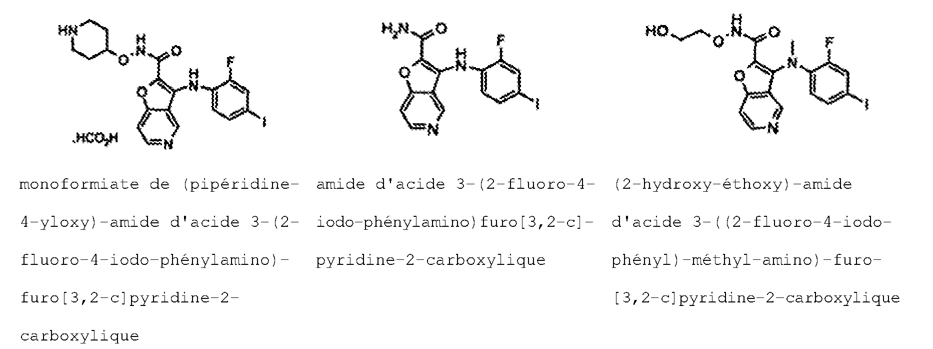 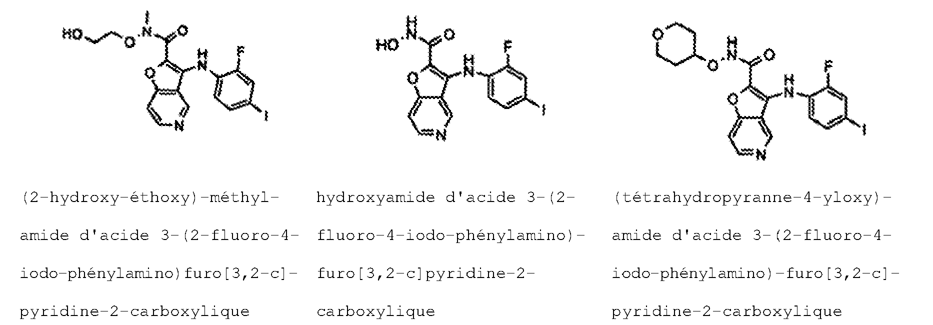 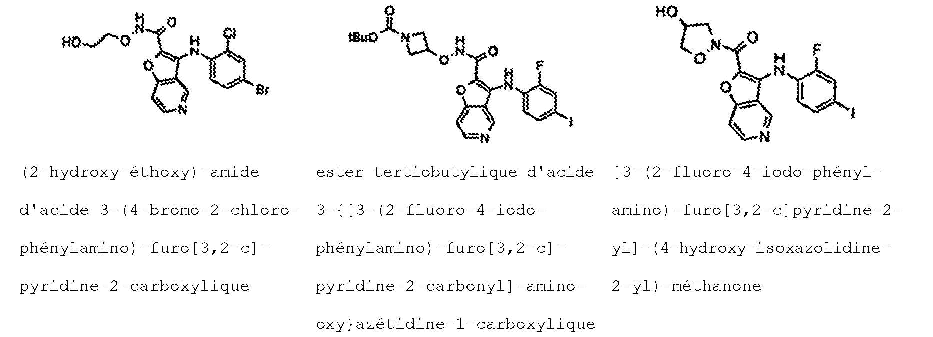 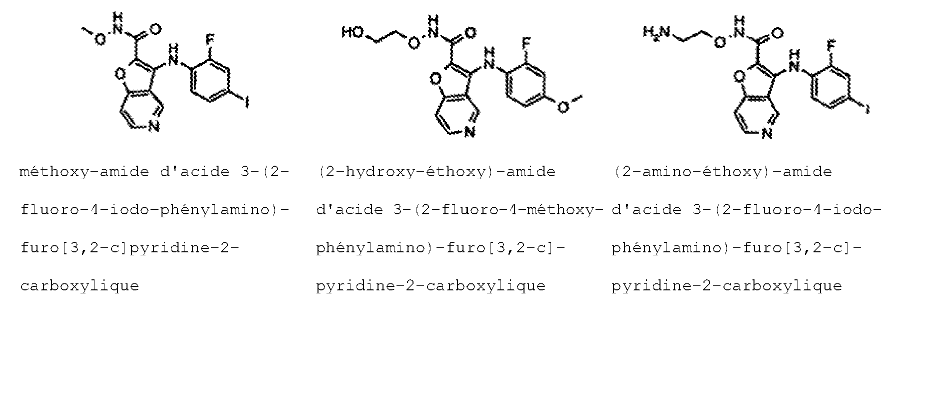 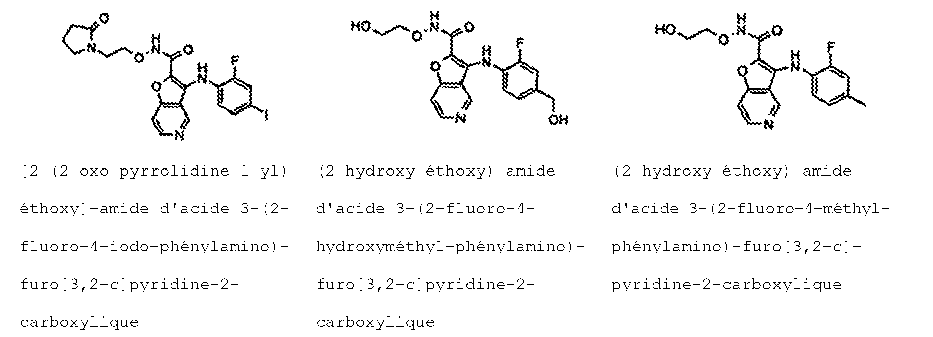 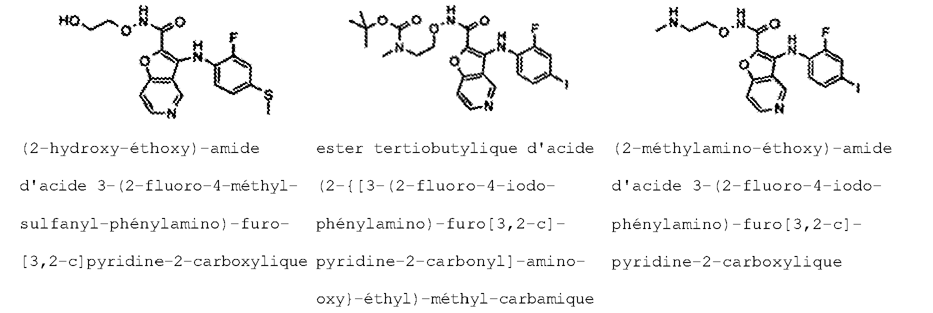 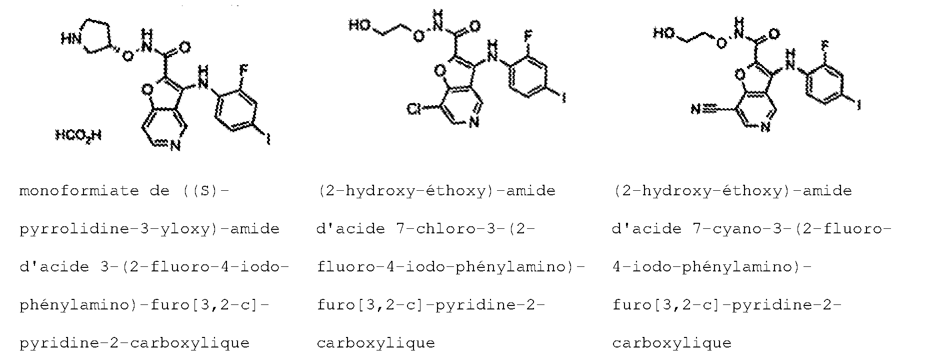 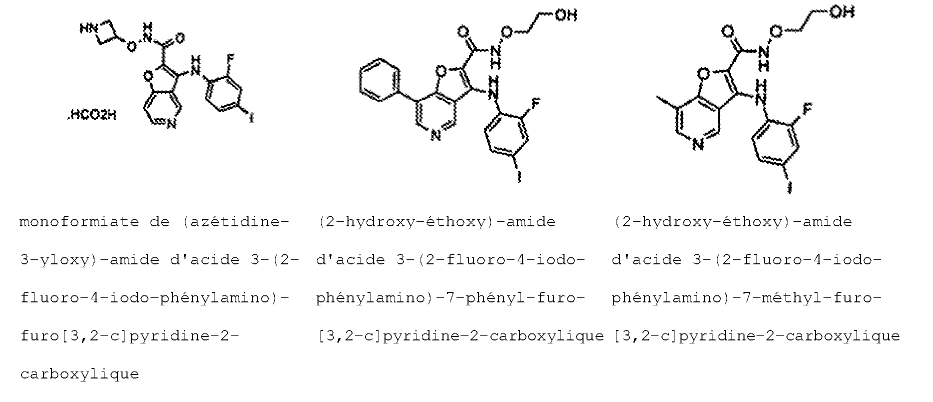 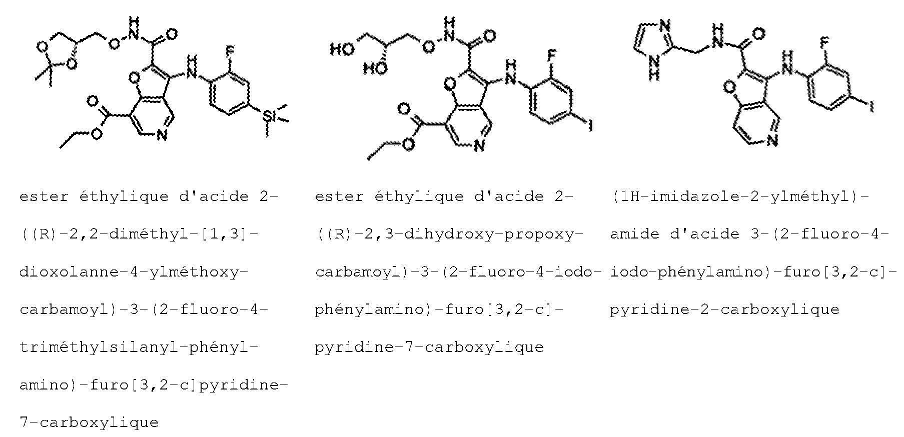 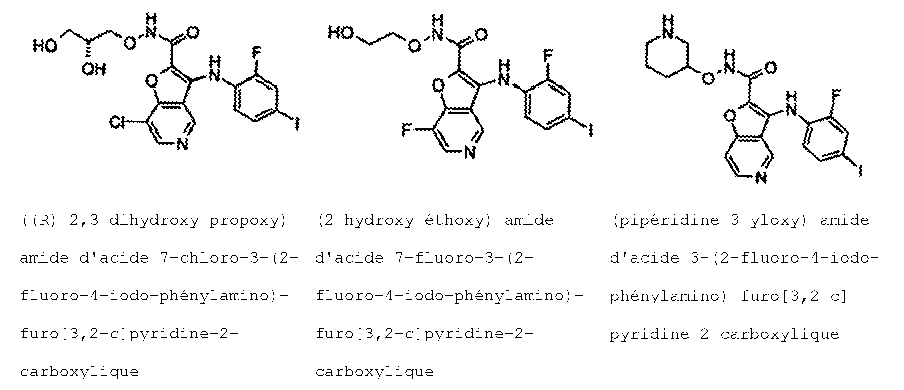 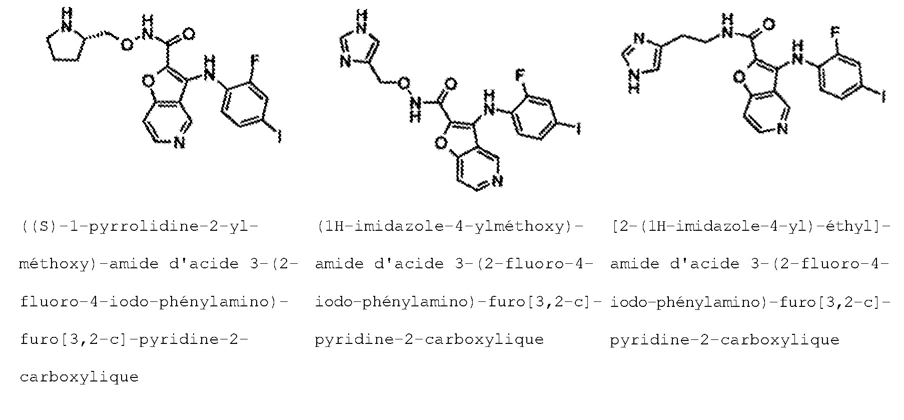 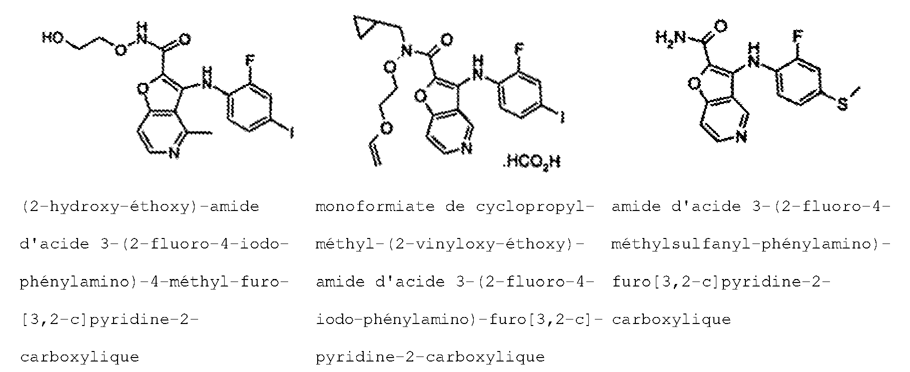 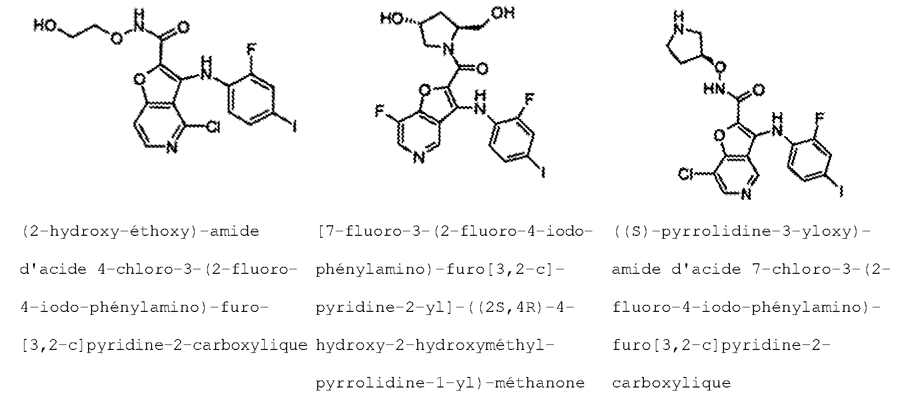 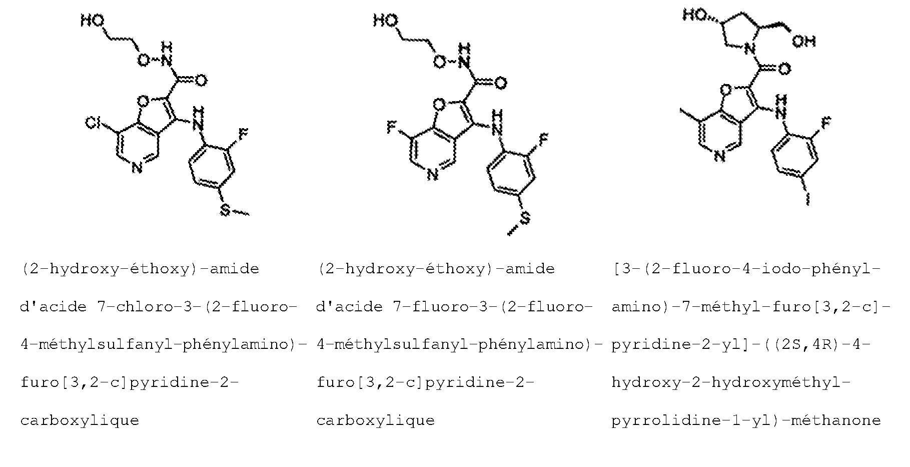 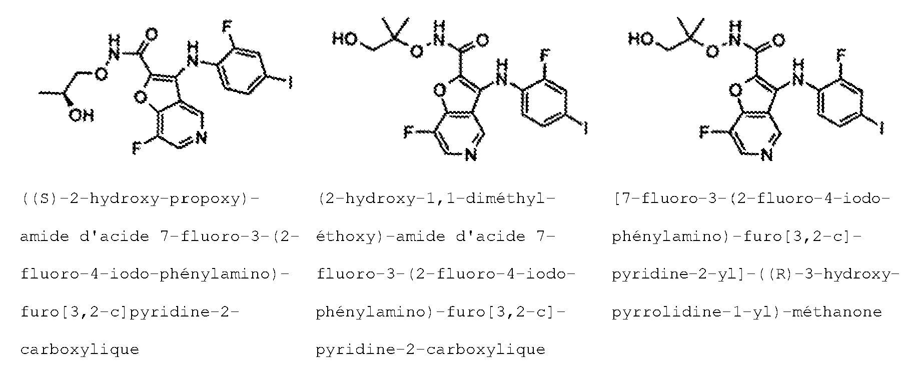 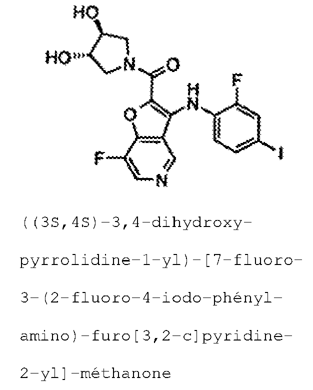 et

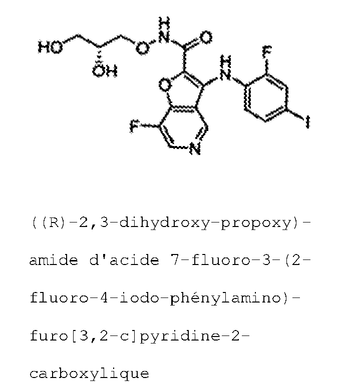

16. Composition pharmaceutique comprenant un composé de l'une quelconque des revendications 1 à 15, et un support pharmaceutiquement acceptable.

17. Composition pharmaceutique suivant la revendication 16, comprenant en outre un second agent chimiothérapeutique ou un second agent anti-inflammatoire.

18. Composé suivant l'une quelconque des revendications 1 à 15, pour son utilisation dans une méthode de traitement, ladite méthode étant pour l'inhibition d'une croissance cellulaire anormale ou le traitement d'un trouble hyperprolifératif chez un mammifère.

19. Utilisation d'un composé de l'une quelconque des revendications 1 à 15, et facultativement d'un second agent chimiothérapeutique, dans la production d'un médicament pour l'inhibition d'une croissance cellulaire anormale ou le traitement d'un trouble hyperprolifératif chez un mammifère.

20. Composé suivant l'une quelconque des revendications 1 à 15, pour son utilisation dans une méthode de traitement, ladite méthode étant pour le traitement d'une maladie inflammatoire chez un mammifère.

21. Utilisation d'un composé de l'une quelconque des revendications 1 à 15, et facultativement d'un second agent anti-inflammatoire, dans la production d'un médicament pour le traitement d'une maladie inflammatoire chez un mammifère.

22. Composé de l'une quelconque des revendications 1 à 15, pour une utilisation dans une méthode de traitement, ladite méthode étant pour le traitement d'une maladie auto-immune, d'un trouble de destruction du tissu osseux, de troubles prolifératifs, d'une maladie infectieuse, d'une maladie virale, d'une maladie fibrotique, d'une maladie neurodégénérative, de la pancréatique ou d'une maladie rénale chez un mammifère.

23. Utilisation d'un composé de l'une quelconque des revendications 1 à 15, et facultativement d'un second agent thérapeutique, dans la production d'un médicament pour le traitement d'une maladie auto-immune, d'un trouble de destruction du tissu osseux, de troubles prolifératifs, d'une maladie infectieuse, d'une maladie virale, d'une maladie fibrotique, d'une maladie neurodégénérative, de la pancréatique ou d'une maladie rénale chez un mammifère.

24. Utilisation suivant la revendication 19, 21 ou 23, dans laquelle ledit second agent chimiothérapeutique, anti-inflammatoire ou thérapeutique est destiné à l'administration séquentielle ou consécutive audit mammifère.
